# EUROPEAN PATENT APPLICATION

(11) **EP 3 450 435 A1**
(43) Date of publication of application: **06.03.2019**
(21) Application number: 17789570.3
(22) Date of filing: 26.04.2017
(51) Int. Cl.: C07D 519/00, A61K 31/437, A61P 3/10, A61P 43/00, C07D 471/04

(54) **5-SUBSTITUTED AZABENZIMIDAZOLE DERIVATIVE HAVING AMPK ACTIVATION ACTION**

(30) Priority: 26.04.2016 JP 2016088041
(71) Applicant: Shionogi & Co., Ltd, Osaka-shi, Osaka 541-0045 (JP)
(72) Inventor: TAMURA, Yuusuke, Toyonaka-shi Osaka 561-0825 (JP); SASAKI, Yoshikazu, Toyonaka-shi Osaka 561-0825 (JP)
(74) Representative: Vossius & Partner Patentanwälte Rechtsanwälte mbB
(86) International application number: PCT/JP2017/016452
(87) International publication number: WO 2017/188288

(57) **Abstract**

Provided is a compound which is useful as an AMPK activator. A compound represented by formula: 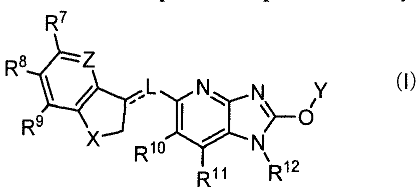 wherein
L is -NR¹-, =N-, -O-, -S-, -SO₂-, -CR²R³-, or =CR²-; dashed line indicates the presence or absence of a bond;
X is -CR⁴R⁵-, or -O-;
Y is substituted or unsubstituted heteroaryl, or substituted or unsubstituted heterocyclyl;
Z is =CR⁶-, or =N-;
R¹ is hydrogen, halogen, hydroxy, cyano, nitro, carboxy, substituted or unsubstituted alkyl, substituted or unsubstituted alkenyl, substituted or unsubstituted alkynyl or the like;
R² and R³ are each independently hydrogen, halogen, hydroxy, cyano, nitro, carboxy, substituted or unsubstituted alkyl, substituted or unsubstituted alkenyl, substituted or unsubstituted alkynyl or the like;
R⁴ and R⁵ are each independently hydrogen, halogen, hydroxy, cyano, nitro, carboxy, substituted or unsubstituted alkyl, substituted or unsubstituted alkenyl, substituted or unsubstituted alkynyl or the like;
R⁶ is hydrogen, halogen, hydroxy, cyano, nitro, carboxy, substituted or unsubstituted alkyl, substituted or unsubstituted alkenyl, substituted or unsubstituted alkynyl or the like;
R⁷, R⁸ and R⁹ are each independently hydrogen, halogen, hydroxy, cyano, nitro, carboxy, substituted or unsubstituted alkyl, substituted or unsubstituted alkenyl, substituted or unsubstituted alkynyl or the like;
R¹⁰ and R¹¹ are each independently hydrogen, halogen, hydroxy, cyano, nitro, carboxy, substituted or unsubstituted alkyl, substituted or unsubstituted alkenyl, substituted or unsubstituted alkynyl or the like; and
R¹² is hydrogen or substituted or unsubstituted alkyl, or its pharmaceutically acceptable salt.

## Description

### [Field of the Invention]

The present invention relates to a compound which has an activating effect on adenosine monophosphate-activated protein kinase (hereinafter referred to as AMPK) and is useful as a medicine.

### [Background Art]

AMPK is a serine-threonine kinase, which is activated by AMP, and has three subunits, α, β and γ. In each subunit, there exist multiple isoforms (α1, α2, 61, 62, γ1, γ2 and y3).

AMPK is involved in various physiological functions, such as suppression of gluconeogenesis and inhibition of fatty acid synthesis in hepatic and incorporation of sugars and an increase in fatty acid oxidation in skeletal muscles, as an energy sensor in living organisms, and has attracted attention as a target molecule of a therapeutic agent for diabetes. Therefore, an AMPK activator is expected to be effective in the treatment of diabetes as an insulin resistance improving drug, which has an insulin independent hypoglycemic effect and a lipid improving effect (Non-Patent Document 1).

Patent Documents 1 to 26 and 29 to 32 disclose a variety of compounds having an AMPK activating effect. However, a heterocyclic derivative like the compound of the present invention is not disclosed in any of the documents.

Patent Document 27 discloses the compounds shown below as a compound having an AMPK activating effect.

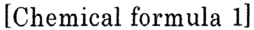

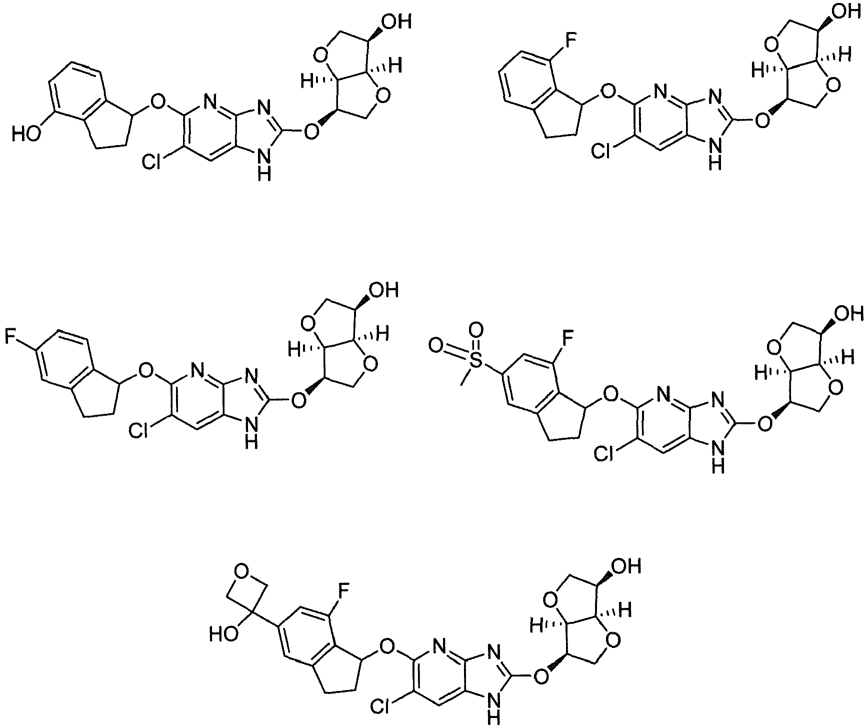

Patent Document 28 discloses the compounds shown below as a compound having an AMPK activating effect.

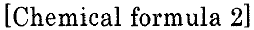

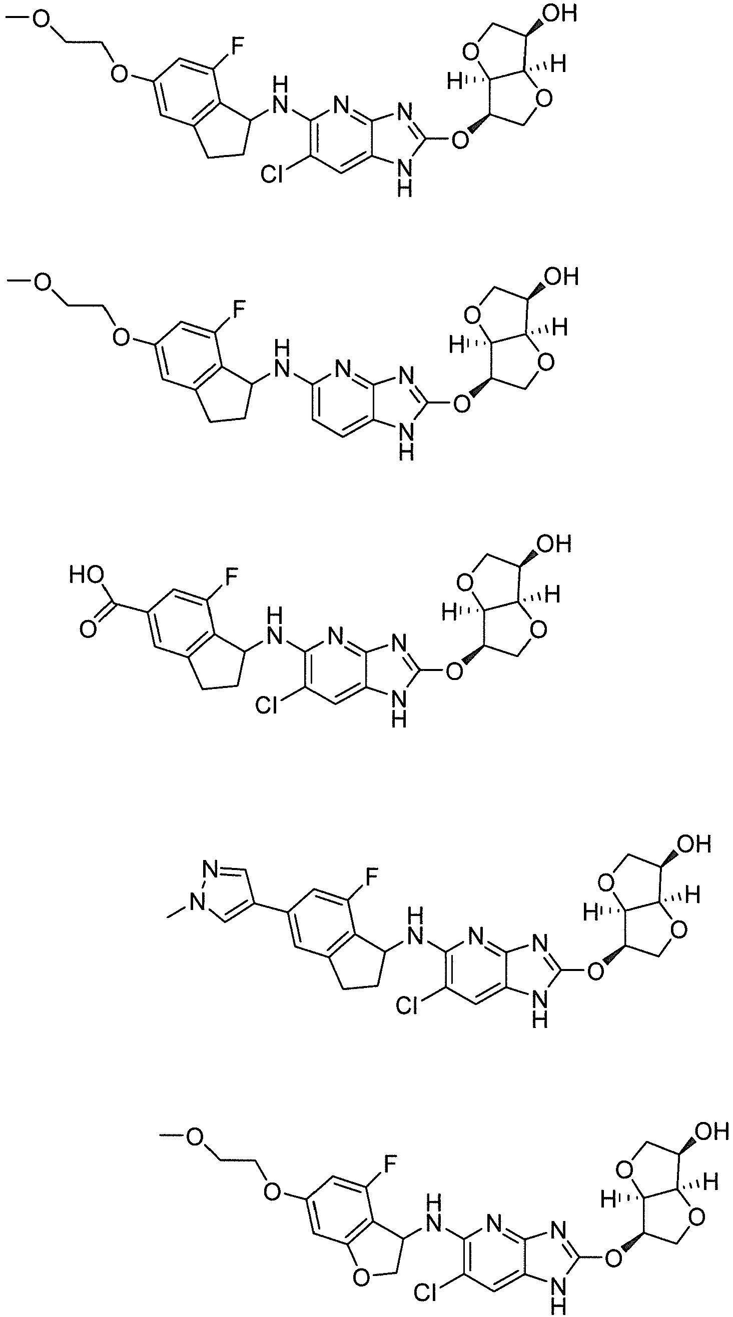

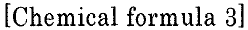

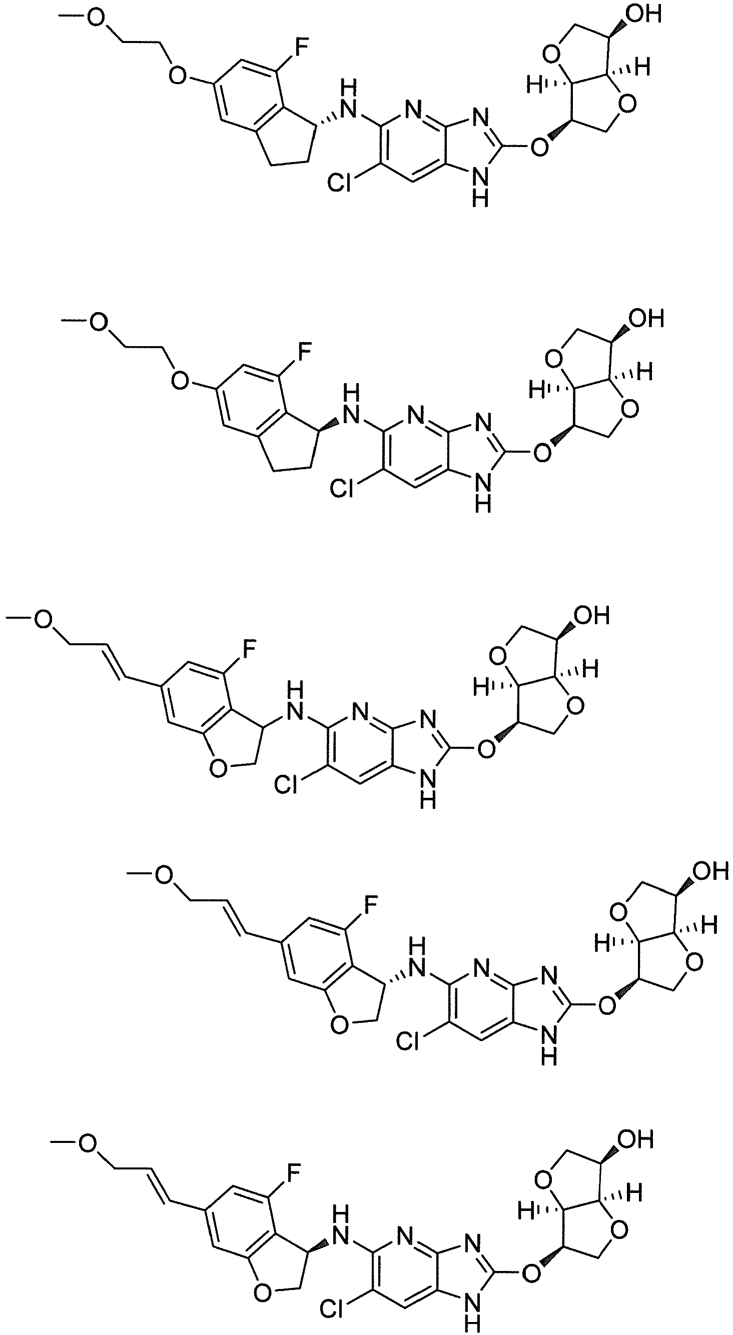

Patent Document 28 discloses the diastereomer mixture of the compounds shown below as a compound having an AMPK activating effect.

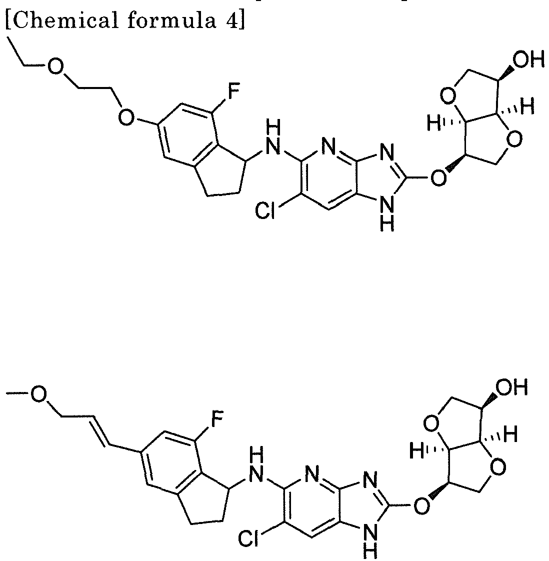

### [Prior Art Document]

### [Patent Document]

Patent Document 1: WO 2010/036613
Patent Document 2: WO 2010/047982
Patent Document 3: WO 2010/051176
Patent Document 4: WO 2010/051206
Patent Document 5: WO 2011/106273
Patent Document 6: WO 2012/116145
Patent Document 7: WO 2012/033149
Patent Document 8: WO 2013/011932
Patent Document 9: WO 2014/031441
Patent Document 10: WO 2014/031445
Patent Document 11: WO 2014/031468
Patent Document 12: WO 2014/031517
Patent Document 13: WO 2014/031465
Patent Document 14: WO 2014/031515
Patent Document 15: WO 2014/069426
Patent Document 16: WO 2008/096829
Patent Document 17: WO 2005/082905
Patent Document 18: JP 2012-167027A
Patent Document 19: WO 2012/147765
Patent Document 20: WO 2006/017214
Patent Document 21: JP 05-339224A
Patent Document 22: WO 2015/007669
Patent Document 23: WO 2015/063011
Patent Document 24: WO 2016/023789
Patent Document 25: WO 2014/139388
Patent Document 26: WO 2014/133008
Patent Document 27: WO 2014/175330
Patent Document 28: WO 2016/068099
Patent Document 29: WO 2016/001224
Patent Document 30: WO 2013/153479
Patent Document 31: WO 2014/140704
Patent Document 32: WO 2014/128549

### [Non-patent Document]

Non-Patent Document 1: Cell Metabolism Vol.9, Issue 5, 407-416, 2009

### [Disclosure of Invention]

### [Problems to be solved by the Invention]

An object of the present invention is to provide an excellent AMPK activator.

### [Means for Solving the Problem]

As a result of intensive research, the present inventors succeeded in synthesizing an excellent compound having an AMPK activating effect.

The present invention relates to the following.
(1) A compound represented by the formula (I): 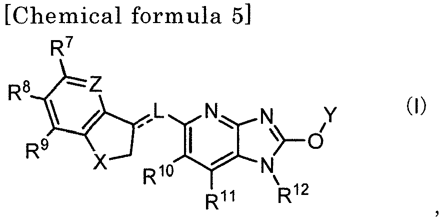 or its pharmaceutically acceptable salt, wherein
   L is -NR¹-, =N-, -O-, -S-, -SO₂-, -CR²R³ -, or =CR²-; dashed line indicates the presence or absence of a bond;
   X is -CR⁴R⁵ -, or -O-;
   Y is substituted or unsubstituted heteroaryl, or substituted or unsubstituted heterocyclyl;
   Z is =CR⁶-, or =N-;
   R¹ is hydrogen, halogen, hydroxy, cyano, nitro, carboxy, substituted or unsubstituted alkyl, substituted or unsubstituted alkenyl, substituted or unsubstituted alkynyl, substituted or unsubstituted aryl, substituted or unsubstituted heteroaryl, substituted or unsubstituted cycloalkyl, substituted or unsubstituted cycloalkenyl, substituted or unsubstituted heterocyclyl, substituted or unsubstituted acyl, substituted or unsubstituted carbamoyl, substituted or unsubstituted alkylsulfonyl, substituted or unsubstituted alkenylsulfonyl, substituted or unsubstituted alkynylsulfonyl, substituted or unsubstituted arylsulfonyl, substituted or unsubstituted heteroarylsulfonyl, substituted or unsubstituted cycloalkylsulfonyl, substituted or unsubstituted cycloalkenylsulfonyl, substituted or unsubstituted heterocyclylsulfonyl, or substituted or unsubstituted sulfamoyl;
   R² and R³ are each independently hydrogen, halogen, hydroxy, cyano, nitro, carboxy, substituted or unsubstituted alkyl, substituted or unsubstituted alkenyl, substituted or unsubstituted alkynyl, substituted or unsubstituted aryl, substituted or unsubstituted heteroaryl, substituted or unsubstituted cycloalkyl, substituted or unsubstituted cycloalkenyl, substituted or unsubstituted heterocyclyl, substituted or unsubstituted alkyloxy, substituted or unsubstituted aryloxy, substituted or unsubstituted heteroaryloxy, substituted or unsubstituted cycloalkyloxy, substituted or unsubstituted cycloalkenyloxy, substituted or unsubstituted heterocyclyloxy, substituted or unsubstituted alkylthio, substituted or unsubstituted arylthio, substituted or unsubstituted heteroarylthio, substituted or unsubstituted cycloalkylthio, substituted or unsubstituted cycloalkenylthio, substituted or unsubstituted heterocyclylthio, substituted or unsubstituted alkylsulfonyl, substituted or unsubstituted arylsulfonyl, substituted or unsubstituted heteroarylsulfonyl, substituted or unsubstituted cycloalkylsulfonyl, substituted or unsubstituted cycloalkenylsulfonyl, substituted or unsubstituted heterocyclylsulfonyl, substituted or unsubstituted acyl, substituted or unsubstituted carbamoyl, substituted or unsubstituted sulfamoyl, or substituted or unsubstituted amino;
   R⁴ and R⁵ are each independently hydrogen, halogen, hydroxy, cyano, nitro, carboxy, substituted or unsubstituted alkyl, substituted or unsubstituted alkenyl, substituted or unsubstituted alkynyl, substituted or unsubstituted aryl, substituted or unsubstituted heteroaryl, substituted or unsubstituted cycloalkyl, substituted or unsubstituted cycloalkenyl, substituted or unsubstituted heterocyclyl, substituted or unsubstituted alkyloxy, substituted or unsubstituted aryloxy, substituted or unsubstituted heteroaryloxy, substituted or unsubstituted cycloalkyloxy, substituted or unsubstituted cycloalkenyloxy, substituted or unsubstituted heterocyclyloxy, substituted or unsubstituted alkylthio, substituted or unsubstituted arylthio, substituted or unsubstituted heteroarylthio, substituted or unsubstituted cycloalkylthio, substituted or unsubstituted cycloalkenylthio, substituted or unsubstituted heterocyclylthio, substituted or unsubstituted alkylsulfonyl, substituted or unsubstituted arylsulfonyl, substituted or unsubstituted heteroarylsulfonyl, substituted or unsubstituted cycloalkylsulfonyl, substituted or unsubstituted cycloalkenylsulfonyl, substituted or unsubstituted heterocyclylsulfonyl, substituted or unsubstituted acyl, substituted or unsubstituted carbamoyl, substituted or unsubstituted sulfamoyl, or substituted or unsubstituted amino;
   R⁶ is hydrogen, halogen, hydroxy, cyano, nitro, carboxy, substituted or unsubstituted alkyl, substituted or unsubstituted alkenyl, substituted or unsubstituted alkynyl, substituted or unsubstituted aryl, substituted or unsubstituted heteroaryl, substituted or unsubstituted cycloalkyl, substituted or unsubstituted cycloalkenyl, substituted or unsubstituted heterocyclyl, substituted or unsubstituted alkyloxy, substituted or unsubstituted aryloxy, substituted or unsubstituted heteroaryloxy, substituted or unsubstituted cycloalkyloxy, substituted or unsubstituted cycloalkenyloxy, substituted or unsubstituted heterocyclyloxy, substituted or unsubstituted alkylthio, substituted or unsubstituted arylthio, substituted or unsubstituted heteroarylthio, substituted or unsubstituted cycloalkylthio, substituted or unsubstituted cycloalkenylthio, substituted or unsubstituted heterocyclylthio, substituted or unsubstituted alkylsulfonyl, substituted or unsubstituted arylsulfonyl, substituted or unsubstituted heteroarylsulfonyl, substituted or unsubstituted cycloalkylsulfonyl, substituted or unsubstituted cycloalkenylsulfonyl, substituted or unsubstituted heterocyclylsulfonyl, substituted or unsubstituted acyl, substituted or unsubstituted carbamoyl, substituted or unsubstituted sulfamoyl, or substituted or unsubstituted amino;
   R⁷ , R⁸ and R⁹ are each independently hydrogen, halogen, hydroxy, cyano, nitro, carboxy, substituted or unsubstituted alkyl, substituted or unsubstituted alkenyl, substituted or unsubstituted alkynyl, substituted or unsubstituted aryl, substituted or unsubstituted heteroaryl, substituted or unsubstituted cycloalkyl, substituted or unsubstituted cycloalkenyl, substituted or unsubstituted heterocyclyl, substituted or unsubstituted alkyloxy, substituted or unsubstituted aryloxy, substituted or unsubstituted heteroaryloxy, substituted or unsubstituted cycloalkyloxy, substituted or unsubstituted cycloalkenyloxy, substituted or unsubstituted heterocyclyloxy, substituted or unsubstituted alkylthio, substituted or unsubstituted arylthio, substituted or unsubstituted heteroarylthio, substituted or unsubstituted cycloalkylthio, substituted or unsubstituted cycloalkenylthio, substituted or unsubstituted heterocyclylthio, substituted or unsubstituted alkylsulfonyl, substituted or unsubstituted arylsulfonyl , substituted or unsubstituted heteroarylsulfonyl, substituted or unsubstituted cycloalkylsulfonyl, substituted or unsubstituted cycloalkenylsulfonyl, substituted or unsubstituted heterocyclylsulfonyl, substituted or unsubstituted carbamoyl, substituted or unsubstituted sulfamoyl, substituted or unsubstituted amino,
   R^{S}R^{S'} (O=)S=N-, R^{S}R^{S'} (O=)S=N-R^{2f}-, R^{S}R^{S'}(O=)S=N-C(=O)-, (R^{N})N=S(=O)(R^{S})-, (R^{N})N=S(=O)(R^{S})-R^{2f}-, R^{S}R^{S'}(R^{N'}-N=)S=N-, ((R^{N})N=)₂S(R^{S})-, (R^{N}R^{N'})N-C(=O)-O-, R^{O}O-C(=O)-N(R^{N})-, R^{O}O-C(=O)-O-, R^{S}(R^{N}R^{N'}N)(O=)S=N-, R^{S}(R^{N}R^{N'}N)(O=)S=N-R^{2f}-, (R^{N"})N=S(=O)(NR^{N}R^{N'})-, (R^{N"})N=S(=O)(NR^{N}R^{N'})-R^{2f}-, 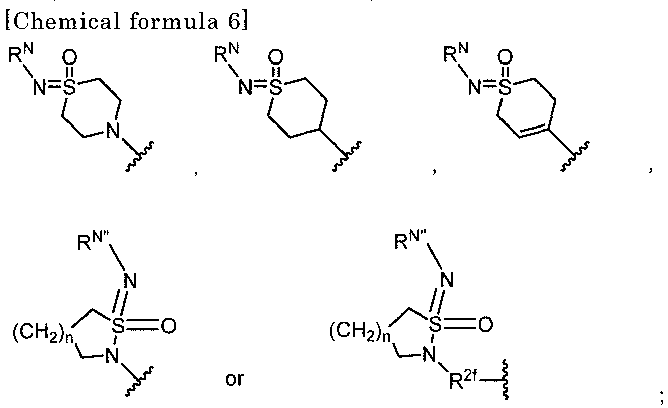 wherein n is an integer 1 or 2;
   R^{S} and R^{S'} are each independently substituted or unsubstituted alkyl, substituted or unsubstituted cycloalkyl, substituted or unsubstituted heterocyclyl, substituted or unsubstituted aryl, or substituted or unsubstituted heteroaryl;
   R^{S} and R^{S'} bound to the same sulfur atom may form a substituted or unsubstituted ring together with the sulfur atom;
   R^{2f} is substituted or unsubstituted alkylene;
   R^{N} is each independently hydrogen, cyano, substituted or unsubstituted alkyl, substituted or unsubstituted alkylcarbonyl, substituted or unsubstituted alkyloxycarbonyl, substituted or unsubstituted cycloalkyl, substituted or unsubstituted cycloalkylcarbonyl, substituted or unsubstituted heterocyclyl, substituted or unsubstituted heterocyclylcarbonyl, substituted or unsubstituted aryl, substituted or unsubstituted arylcarbonyl, substituted or unsubstituted heteroaryl, substituted or unsubstituted heteroarylcarbonyl, or substituted or unsubstituted carbamoyl;
   when R⁷ , R⁸ or R⁹ is ((R^{N})N=)₂ S(R^{S})-, two R^{N} together with the adjacent nitrogen atom may form a substituted or unsubstituted ring;
   R^{N'} is hydrogen, cyano, substituted or unsubstituted alkyl, substituted or unsubstituted alkylcarbonyl, substituted or unsubstituted alkyloxycarbonyl, substituted or unsubstituted cycloalkyl, substituted or unsubstituted cycloalkylcarbonyl, substituted or unsubstituted heterocyclyl, substituted or unsubstituted heterocyclylcarbonyl, substituted or unsubstituted aryl, substituted or unsubstituted arylcarbonyl, substituted or unsubstituted heteroaryl, substituted or unsubstituted heteroarylcarbonyl, or substituted or unsubstituted carbamoyl; R^{N} and R^{N'} bound to the same nitrogen atom may form a substituted or unsubstituted ring together with the nitrogen atom;
   R^{N'} is hydrogen, cyano, substituted or unsubstituted alkyl, substituted or unsubstituted alkenyl, substituted or unsubstituted alkynyl, substituted or unsubstituted alkylcarbonyl, substituted or unsubstituted alkyloxycarbonyl, substituted or unsubstituted alkylcarbamoyl, substituted or unsubstituted cycloalkyl, substituted or unsubstituted cycloalkenyl, substituted or unsubstituted cycloalkynyl, substituted or unsubstituted aryl, substituted or unsubstituted heteroaryl, substituted or unsubstituted heterocyclyl, substituted or unsubstituted acyl, substituted or unsubstituted aryloxycarbonyl, substituted or unsubstituted arylsulfonyl, substituted or unsubstituted alkylsulfonyl, or substituted or unsubstituted carbamoyl;
   R^{O} is hydrogen, substituted or unsubstituted alkyl, substituted or unsubstituted alkenyl, substituted or unsubstituted alkynyl, substituted or unsubstituted aryl, substituted or unsubstituted heteroaryl, substituted or unsubstituted cycloalkyl, substituted or unsubstituted cycloalkenyl, or substituted or unsubstituted heterocyclyl;
   provided that R⁷, R⁸ and R⁹ are not hydrogen at the same time;
   R¹⁰ and R¹¹ are each independently hydrogen, halogen, hydroxy, cyano, nitro, carboxy, substituted or unsubstituted alkyl, substituted or unsubstituted alkenyl, substituted or unsubstituted alkynyl, substituted or unsubstituted aryl, substituted or unsubstituted heteroaryl, substituted or unsubstituted cycloalkyl, substituted or unsubstituted cycloalkenyl, substituted or unsubstituted heterocyclyl, substituted or unsubstituted alkyloxy, substituted or unsubstituted aryloxy, substituted or unsubstituted heteroaryloxy, substituted or unsubstituted cycloalkyloxy, substituted or unsubstituted cycloalkenyloxy, substituted or unsubstituted heterocyclyloxy, substituted or unsubstituted alkylthio, substituted or unsubstituted arylthio, substituted or unsubstituted heteroarylthio, substituted or unsubstituted cycloalkylthio, substituted or unsubstituted cycloalkenylthio, substituted or unsubstituted heterocyclylthio, substituted or unsubstituted alkylsulfonyl, substituted or unsubstituted arylsulfonyl, substituted or unsubstituted heteroarylsulfonyl, substituted or unsubstituted cycloalkylsulfonyl, substituted or unsubstituted cycloalkenylsulfonyl, substituted or unsubstituted heterocyclylsulfonyl, substituted or unsubstituted acyl, substituted or unsubstituted carbamoyl, substituted or unsubstituted sulfamoyl, or substituted or unsubstituted amino;
   R¹² is hydrogen, or substituted or unsubstituted alkyl; with the proviso that
   compounds wherein Z is =CR⁶-, and R⁶ and R⁸ are fuluoro at the same time; and compounds shown below 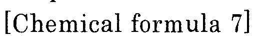 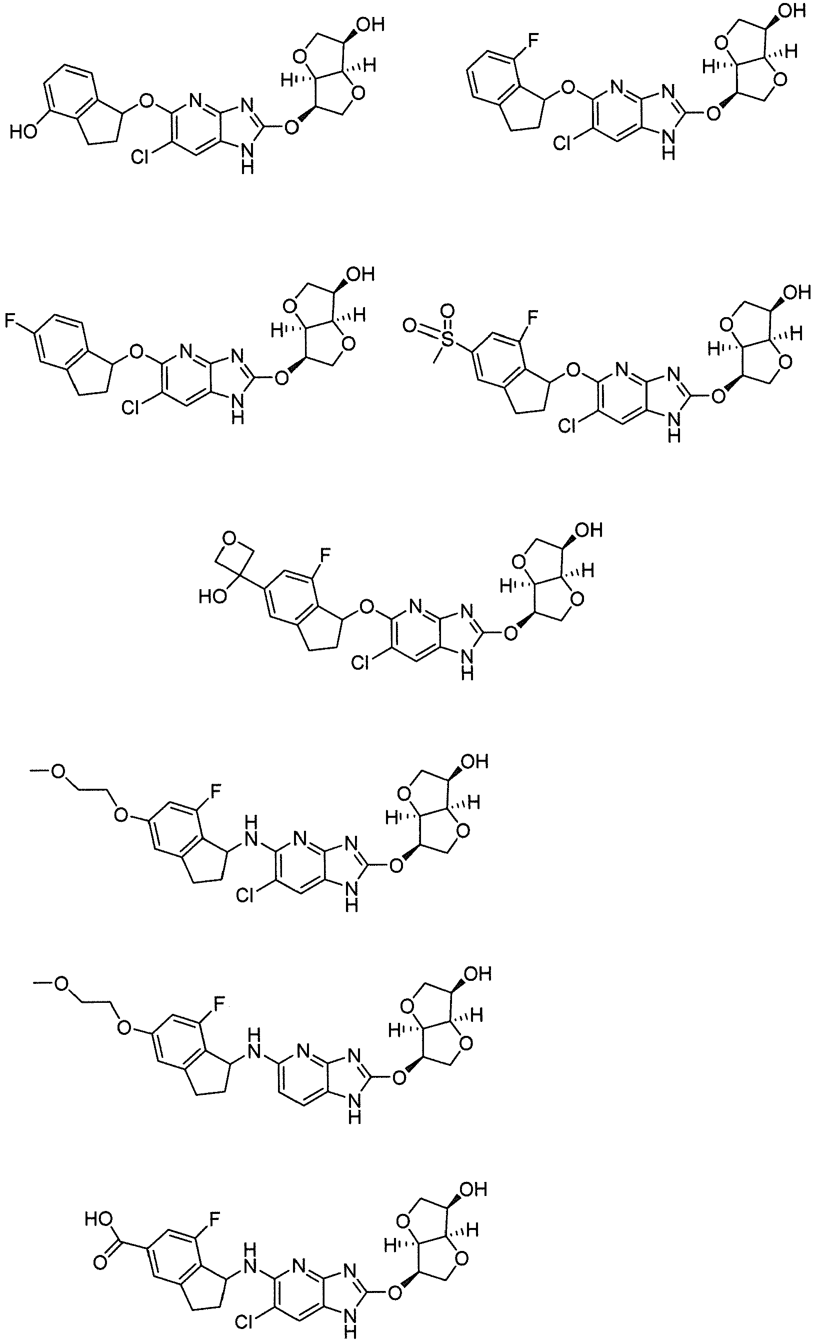 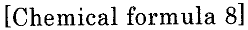 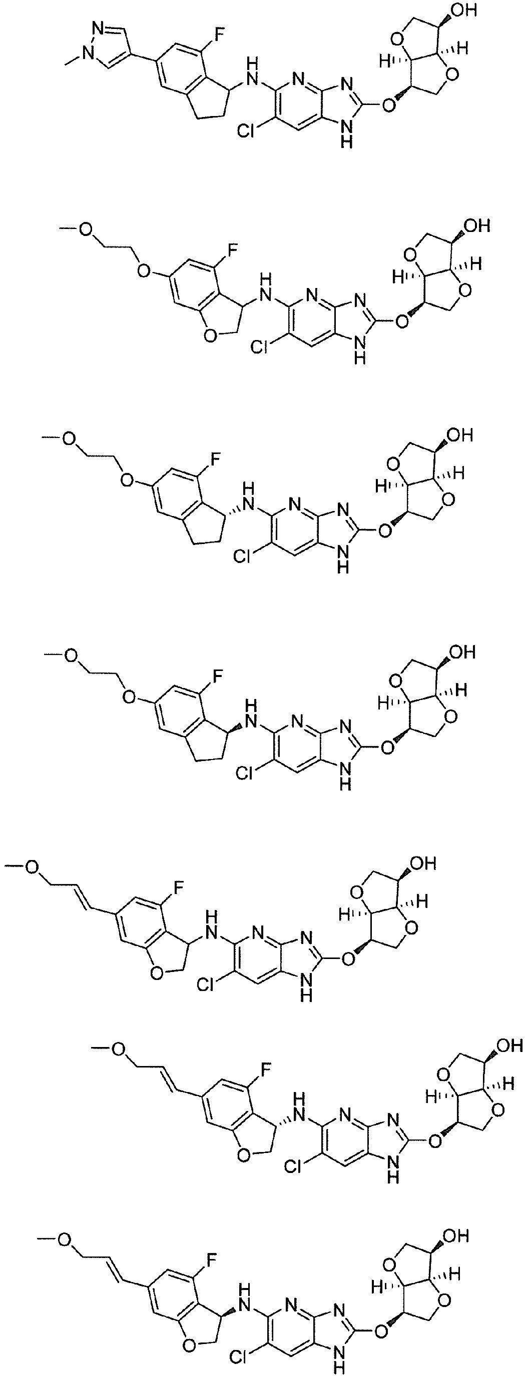
   and mixture of diastereomers of compounds shown below are excluded 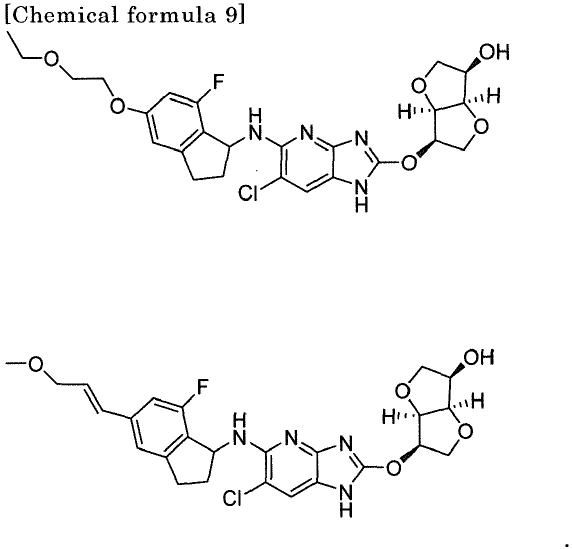
(2) The compound according to the above (1) or its pharmaceutically acceptable salt, wherein L is -NR¹-, or -O-.
(3) The compound according to the above (1) or its pharmaceutically acceptable salt, wherein L is -NR¹-.
(4) The compound according to the above (1) or its pharmaceutically acceptable salt, wherein the compound represented by the formula (I) is a compound represented by the formula (II): 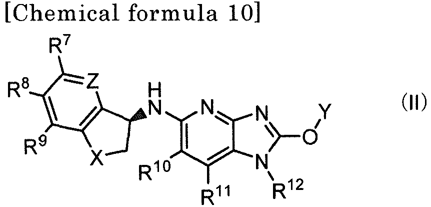 wherein each substituent in the formula (II) is defined in the above (1).
(5) The compound according to any one of the above (1) to (3), or its pharmaceutically acceptable salt, wherein R¹ is hydrogen.
(6) The compound according to any one of the above (1) to (5) or its pharmaceutically acceptable salt, wherein Y is substituted or unsubstituted heterocyclyl.
(7) The compound according to the above (6) or its pharmaceutically acceptable salt,
   wherein Y is 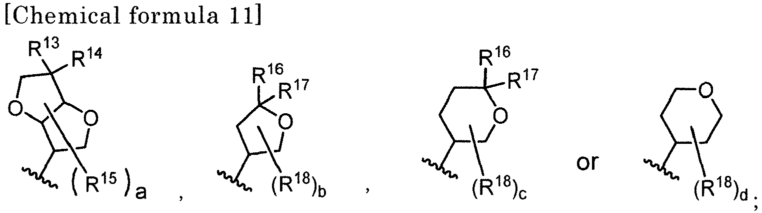
   wherein R¹³ and R¹⁴ are each independently hydrogen, halogen, hydroxy, cyano, nitro, carboxy, substituted or unsubstituted alkyl, substituted or unsubstituted alkenyl, substituted or unsubstituted alkynyl, substituted or unsubstituted alkyloxy, substituted or unsubstituted alkylthio, substituted or unsubstituted alkylsulfonyl, substituted or unsubstituted acyl, substituted or unsubstituted carbamoyl, substituted or unsubstituted sulfamoyl, or substituted or unsubstituted amino;
   R¹⁵ is each independently halogen, hydroxy, cyano, nitro, carboxy, substituted or unsubstituted alkyl, substituted or unsubstituted alkenyl, substituted or unsubstituted alkynyl, substituted or unsubstituted alkyloxy, substituted or unsubstituted alkylthio, substituted or unsubstituted alkylsulfonyl, substituted or unsubstituted acyl, substituted or unsubstituted carbamoyl, substituted or unsubstituted sulfamoyl, or substituted or unsubstituted amino;
   a is an integer from 0 to 6;
   R¹⁶ and R¹⁷ are each independently hydrogen, halogen, hydroxy, cyano, nitro, carboxy, substituted or unsubstituted alkyl, substituted or unsubstituted alkenyl, substituted or unsubstituted alkynyl, substituted or unsubstituted alkyloxy, substituted or unsubstituted alkylthio, substituted or unsubstituted alkylsulfonyl, substituted or unsubstituted acyl, substituted or unsubstituted carbamoyl, substituted or unsubstituted sulfamoyl, or substituted or unsubstituted amino; R¹⁸ is each independently halogen, hydroxy, cyano, nitro, carboxy, substituted or unsubstituted alkyl, substituted or unsubstituted alkenyl, substituted or unsubstituted alkynyl, substituted or unsubstituted alkyloxy, substituted or unsubstituted alkylthio, substituted or unsubstituted alkylsulfonyl, substituted or unsubstituted acyl, substituted or unsubstituted carbamoyl, substituted or unsubstituted sulfamoyl, or substituted or unsubstituted amino;
   b is an integer from 0 to 5;
   c is an integer from 0 to 7;
   d is an integer from 0 to 9.
(8) The compound according to any one of the above (1) to (7) or its pharmaceutically acceptable salt, wherein X is -CR⁴R⁵-.
(9) The compound according to any one of the above (1) to (8) or its pharmaceutically acceptable salt, wherein R⁴ and R⁵ are hydrogen.
(10) The compound according to any one of the above (1) to (9) or its pharmaceutically acceptable salt, wherein Z is =CR⁶-, and R⁶ is halogen.
(11) The compound according to any one of the above (1) to (9) or its pharmaceutically acceptable salt, wherein Z is =N-.
(12) The compound according to any one of the above (1) to (11) or its pharmaceutically acceptable salt, wherein R⁷, R⁸ and R⁹ are each independently hydrogen, halogen, hydroxy, cyano, nitro, carboxy, substituted or unsubstituted alkyl, substituted or unsubstituted alkenyl, substituted or unsubstituted alkynyl, substituted or unsubstituted aryl, substituted or unsubstituted heteroaryl, substituted or unsubstituted cycloalkyl, substituted or unsubstituted cycloalkenyl, substituted or unsubstituted heterocyclyl, substituted or unsubstituted alkyloxy, substituted or unsubstituted aryloxy, substituted or unsubstituted heteroaryloxy, substituted or unsubstituted cycloalkyloxy, substituted or unsubstituted cycloalkenyloxy, substituted or unsubstituted heterocyclyloxy, substituted or unsubstituted alkylsulfonyl, or substituted or unsubstituted amino.
(13) The compound according to any one of the above (1) to (12) or its pharmaceutically acceptable salt, wherein R¹⁰ is hydrogen, halogen, cyano, carboxy, or substituted or unsubstituted alkyl.
(14) The compound according to the above (13) or its pharmaceutically acceptable salt, wherein R¹⁰ is hydrogen, fluoro, chloro, cyano, or substituted or unsubstituted alkyl, wherein the substituent of the substituted alkyl is halogen.
(15) The compound according to the above (13) or its pharmaceutically acceptable salt, wherein R¹⁰ is fluoro, or chloro.
(16) The compound according to any one of the above (1) to (15) or its pharmaceutically acceptable salt, wherein R¹¹ is hydrogen.
(17) The compound according to any one of the above (1) to (16) or its pharmaceutically acceptable salt, wherein R¹² is hydrogen.
(18) The compound according to the above (1) or its pharmaceutically acceptable salt, wherein the compound is selected from compound (1-1-4), (1-1-6), (1-1-7), (1-1-10), (1-1-11), (1-1-13), (I-1-14), (I-1-15), (I-1-19), (1-1-20), (I-1-23), (1-1-24), (1-1-29), (I-1-32), (I-1-35), (I-1-38), (I-1-39), (1-1-42), (I-1-43), (I-1-44), (1-1-45), (I-1-46), (1-1-47), (I-1-48), (I-1-70), (1-1-72), (1-1-74), (I-1-76), (I-1-77), (I-1-79), (I-1-81), (I-1-83), (I-1-86), (I-1-88), (I-1-90), (I-1-91), (1-1-93), (1-1-94), (1-1-95), (1-1-96), (1-1-97), (1-1-98), (I-1-99), (1-1-100), (1-1-104), (I-1-105), (I-1-106), or (I-1-111).
(19) A compound represented by the formula (III) or (IV) or its pharmaceutically acceptable salt, wherein the compound represented by the formula (III) is 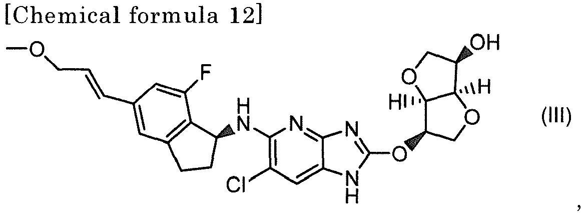 and the compound represented by the formula (IV) is 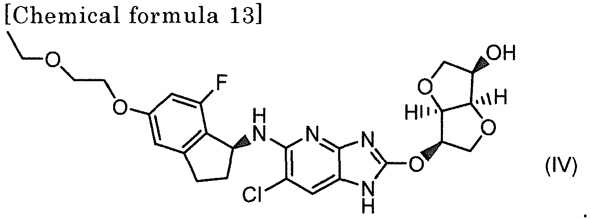
(20) The compound according to the above (1) or its pharmaceutically acceptable salt, wherein the compound is selected from compound (1-2-01), (1-2-02), (1-2-03), (I-2-04), (1-2-05), (1-2-06), (1-2-07), (1-2-08), (1-2-09), (1-2-10), (1-2-12), (1-2-13), (1-2-14), (1-2-17), (1-2-18), (1-2-21), (1-2-23), (1-2-26), (1-2-32), (1-2-33), (1-2-34), (1-2-35), (1-2-36), (1-2-37), (1-2-38), (1-2-39), (1-2-40), (I-2-41), (1-2-42), (1-2-43), (1-2-44), (1-2-45), (1-2-46), (I-2-47), (I-2-48), (1-2-49), (1-2-50), (1-2-51), (I-2-52), (1-2-53), (I-2-54), (I-2-55), (1-2-56), (1-2-57), (1-2-59), (1-2-60), (I-2-61), (1-2-62), (1-2-63), (1-2-64), (1-2-66), (1-2-67), (1-2-68), (1-2-69), (1-2-70), (I-2-71), (1-2-72), (1-2-73), (1-2-74), (1-2-75), (I-2-77) or (I-2-78).
(21) A pharmaceutical composition comprising the compound according to any one of the above (1) to (20) or its pharmaceutically acceptable salt.
(22) The pharmaceutical composition according to the above (21), which has an activating effect on adenosine monophosphate-activated protein kinase.
(23) The pharmaceutical composition according to the above (21) or (22), for the treatment and/or prevention of diabetes.
(24) A method for preventing or treating diabetes, comprising administering the compound according to any one of the above (1) to (20), or its pharmaceutically acceptable salt.
(25) The compound according to any one of the above (1) to (20), or its pharmaceutically acceptable salt, for the treatment and/or prevention of diabetes.
   (1A)
      A compound represented by the formula (I): 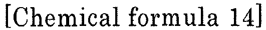 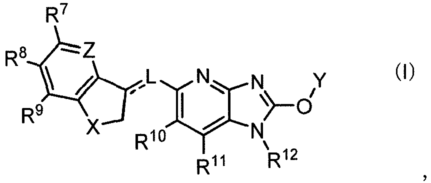 or its pharmaceutically acceptable salt,
      wherein
      L is -NR¹-, =N-, -O-, -S-, -SO₂ -, -CR²R³-, or =CR²-; dashed line indicates the presence or absence of a bond;
      X is -CR⁴R⁵-, or -O-;
      Y is substituted or unsubstituted heteroaryl, or substituted or unsubstituted heterocyclyl;
      Z is =CR⁶-, or =N-;
      R¹ is hydrogen, halogen, hydroxy, cyano, nitro, carboxy, substituted or unsubstituted alkyl, substituted or unsubstituted alkenyl, substituted or unsubstituted alkynyl, substituted or unsubstituted aryl, substituted or unsubstituted heteroaryl, substituted or unsubstituted cycloalkyl, substituted or unsubstituted cycloalkenyl, substituted or unsubstituted heterocyclyl, substituted or unsubstituted acyl, substituted or unsubstituted carbamoyl, substituted or unsubstituted alkylsulfonyl, substituted or unsubstituted alkenylsulfonyl, substituted or unsubstituted alkynylsulfonyl, substituted or unsubstituted arylsulfonyl, substituted or unsubstituted heteroarylsulfonyl, substituted or unsubstituted cycloalkylsulfonyl, substituted or unsubstituted cycloalkenylsulfonyl, substituted or unsubstituted heterocyclylsulfonyl, or substituted or unsubstituted sulfamoyl;
      R² and R³ are each independently hydrogen, halogen, hydroxy, cyano, nitro, carboxy, substituted or unsubstituted alkyl, substituted or unsubstituted alkenyl, substituted or unsubstituted alkynyl, substituted or unsubstituted aryl, substituted or unsubstituted heteroaryl, substituted or unsubstituted cycloalkyl, substituted or unsubstituted cycloalkenyl, substituted or unsubstituted heterocyclyl, substituted or unsubstituted alkyloxy, substituted or unsubstituted aryloxy, substituted or unsubstituted heteroaryloxy, substituted or unsubstituted cycloalkyloxy, substituted or unsubstituted cycloalkenyloxy, substituted or unsubstituted heterocyclyloxy, substituted or unsubstituted alkylthio, substituted or unsubstituted arylthio, substituted or unsubstituted heteroarylthio, substituted or unsubstituted cycloalkylthio, substituted or unsubstituted cycloalkenylthio, substituted or unsubstituted heterocyclylthio, substituted or unsubstituted alkylsulfonyl, substituted or unsubstituted arylsulfonyl, substituted or unsubstituted heteroarylsulfonyl, substituted or unsubstituted cycloalkylsulfonyl, substituted or unsubstituted cycloalkenylsulfonyl, substituted or unsubstituted heterocyclylsulfonyl, substituted or unsubstituted acyl, substituted or unsubstituted carbamoyl, substituted or unsubstituted sulfamoyl, or substituted or unsubstituted amino;
      R⁴ and R⁵ are each independently hydrogen, halogen, hydroxy, cyano, nitro, carboxy, substituted or unsubstituted alkyl, substituted or unsubstituted alkenyl, substituted or unsubstituted alkynyl, substituted or unsubstituted aryl, substituted or unsubstituted heteroaryl, substituted or unsubstituted cycloalkyl, substituted or unsubstituted cycloalkenyl, substituted or unsubstituted heterocyclyl, substituted or unsubstituted alkyloxy, substituted or unsubstituted aryloxy, substituted or unsubstituted heteroaryloxy, substituted or unsubstituted cycloalkyloxy, substituted or unsubstituted cycloalkenyloxy, substituted or unsubstituted heterocyclyloxy, substituted or unsubstituted alkylthio, substituted or unsubstituted arylthio, substituted or unsubstituted heteroarylthio, substituted or unsubstituted cycloalkylthio, substituted or unsubstituted cycloalkenylthio, substituted or unsubstituted heterocyclylthio, substituted or unsubstituted alkylsulfonyl, substituted or unsubstituted arylsulfonyl, substituted or unsubstituted heteroarylsulfonyl, substituted or unsubstituted cycloalkylsulfonyl, substituted or unsubstituted cycloalkenylsulfonyl, substituted or unsubstituted heterocyclylsulfonyl, substituted or unsubstituted acyl, substituted or unsubstituted carbamoyl, substituted or unsubstituted sulfamoyl, or substituted or unsubstituted amino;
      R⁶ is hydrogen, halogen, hydroxy, cyano, nitro, carboxy, substituted or unsubstituted alkyl, substituted or unsubstituted alkenyl, substituted or unsubstituted alkynyl, substituted or unsubstituted aryl, substituted or unsubstituted heteroaryl, substituted or unsubstituted cycloalkyl, substituted or unsubstituted cycloalkenyl, substituted or unsubstituted heterocyclyl, substituted or unsubstituted alkyloxy, substituted or unsubstituted aryloxy, substituted or unsubstituted heteroaryloxy, substituted or unsubstituted cycloalkyloxy, substituted or unsubstituted cycloalkenyloxy, substituted or unsubstituted heterocyclyloxy, substituted or unsubstituted alkylthio, substituted or unsubstituted arylthio, substituted or unsubstituted heteroarylthio, substituted or unsubstituted cycloalkylthio, substituted or unsubstituted cycloalkenylthio, substituted or unsubstituted heterocyclylthio, substituted or unsubstituted alkylsulfonyl, substituted or unsubstituted arylsulfonyl, substituted or unsubstituted heteroarylsulfonyl, substituted or unsubstituted cycloalkylsulfonyl, substituted or unsubstituted cycloalkenylsulfonyl, substituted or unsubstituted heterocyclylsulfonyl, substituted or unsubstituted acyl, substituted or unsubstituted carbamoyl, substituted or unsubstituted sulfamoyl, or substituted or unsubstituted amino;
      R⁷ , R⁸ and R⁹ are each independently hydrogen, halogen, hydroxy, cyano, nitro, carboxy, substituted or unsubstituted alkyl, substituted or unsubstituted alkenyl, substituted or unsubstituted alkynyl, substituted or unsubstituted aryl, substituted or unsubstituted heteroaryl, substituted or unsubstituted cycloalkyl, substituted or unsubstituted cycloalkenyl, substituted or unsubstituted heterocyclyl, substituted or unsubstituted alkyloxy, substituted or unsubstituted aryloxy, substituted or unsubstituted heteroaryloxy, substituted or unsubstituted cycloalkyloxy, substituted or unsubstituted cycloalkenyloxy, substituted or unsubstituted heterocyclyloxy, substituted or unsubstituted alkylthio, substituted or unsubstituted arylthio, substituted or unsubstituted heteroarylthio, substituted or unsubstituted cycloalkylthio, substituted or unsubstituted cycloalkenylthio, substituted or unsubstituted heterocyclylthio, substituted or unsubstituted alkylsulfonyl, substituted or unsubstituted arylsulfonyl , substituted or unsubstituted heteroarylsulfonyl, substituted or unsubstituted cycloalkylsulfonyl, substituted or unsubstituted cycloalkenylsulfonyl, substituted or unsubstituted heterocyclylsulfonyl, substituted or unsubstituted carbamoyl, substituted or unsubstituted sulfamoyl, substituted or unsubstituted amino,
      R^{S}R^{S'}(O=)S=N-, R^{S}R^{S'}(O=)S=N-R^{2f}-, R^{S}R^{S'}(O=)S=N-C(=O)-, (R^{N})N=S(=O)(R^{S})-, (R^{N})N=S(=O)(R^{S})-R^{2f}-, R^{S}R^{S'}(R^{N'}-N=)S=N-, ((R^{N})N=)₂S(R^{S})-, (R^{N}R^{N'})N-C(=O)-O-, R^{O}O-C(=O)-N(R^{N})-, R^{O}O-C(=O)-O-, R^{S}(R^{N}R^{N'}N)(O=)S=N-, R^{S}(R^{N}R^{N'}N)(O=)S=N-R^{2f}-, (R^{N"})N=S(=O)(NR^{N}R^{N'})-, (R^{N"})N=S(=O)(NR^{N}R^{N'})-R^{2f}-, 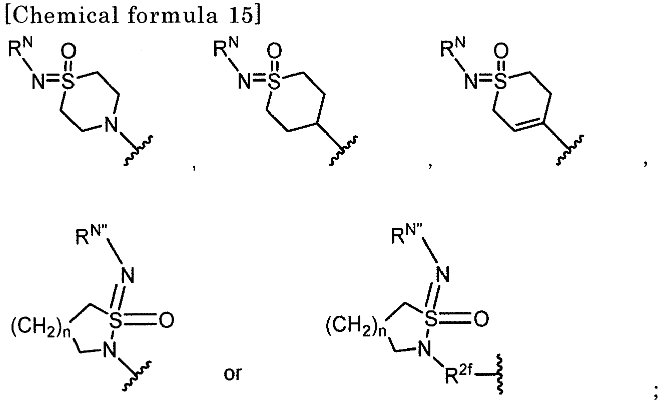
      wherein n is an integer 1 or 2;
      R^{S} and R^{S'} are each independently substituted or unsubstituted alkyl, substituted or unsubstituted cycloalkyl, substituted or unsubstituted heterocyclyl, substituted or unsubstituted aryl, or substituted or unsubstituted heteroaryl;
      R^{S} and R^{S'} bound to the same sulfur atom may form a substituted or unsubstituted ring together with the sulfur atom;
      R^{2f} is substituted or unsubstituted alkylene;
      R^{N} is each independently hydrogen, cyano, substituted or unsubstituted alkyl, substituted or unsubstituted alkylcarbonyl, substituted or unsubstituted alkyloxycarbonyl, substituted or unsubstituted cycloalkyl, substituted or unsubstituted cycloalkylcarbonyl, substituted or unsubstituted heterocyclyl, substituted or unsubstituted heterocyclylcarbonyl, substituted or unsubstituted aryl, substituted or unsubstituted arylcarbonyl, substituted or unsubstituted heteroaryl, substituted or unsubstituted heteroarylcarbonyl, or substituted or unsubstituted carbamoyl;
      when R⁷ , R⁸ or R⁹ is ((R^{N})N=)₂S(R^{S})-, two R^{N} together with the adjacent nitrogen atom may form a substituted or unsubstituted ring;
      R^{N'} is hydrogen, cyano, substituted or unsubstituted alkyl, substituted or unsubstituted alkylcarbonyl, substituted or unsubstituted alkyloxycarbonyl, substituted or unsubstituted cycloalkyl, substituted or unsubstituted cycloalkylcarbonyl, substituted or unsubstituted heterocyclyl, substituted or unsubstituted heterocyclylcarbonyl, substituted or unsubstituted aryl, substituted or unsubstituted arylcarbonyl, substituted or unsubstituted heteroaryl, substituted or unsubstituted heteroarylcarbonyl, or substituted or unsubstituted carbamoyl; R^{N} and R^{N'} bound to the same nitrogen atom may form a substituted or unsubstituted ring together with the nitrogen atom
      R^{N"} is hydrogen, cyano, substituted or unsubstituted alkyl, substituted or unsubstituted alkenyl, substituted or unsubstituted alkynyl, substituted or unsubstituted alkylcarbonyl, substituted or unsubstituted alkyloxycarbonyl, substituted or unsubstituted alkylcarbamoyl, substituted or unsubstituted cycloalkyl, substituted or unsubstituted cycloalkenyl, substituted or unsubstituted cycloalkynyl, substituted or unsubstituted aryl, substituted or unsubstituted heteroaryl, substituted or unsubstituted heterocyclyl, substituted or unsubstituted acyl, substituted or unsubstituted aryloxycarbonyl, substituted or unsubstituted arylsulfonyl, substituted or unsubstituted alkylsulfonyl, or substituted or unsubstituted carbamoyl;
      R^{O} is hydrogen, substituted or unsubstituted alkyl, substituted or unsubstituted alkenyl, substituted or unsubstituted alkynyl, substituted or unsubstituted aryl, substituted or unsubstituted heteroaryl, substituted or unsubstituted cycloalkyl, substituted or unsubstituted cycloalkenyl, or substituted or unsubstituted heterocyclyl;
      provided that R⁷, R⁸ and R⁹ are not hydrogen at the same time;
      R¹⁰ and R¹¹ are each independently hydrogen, halogen, hydroxy, cyano, nitro, carboxy, substituted or unsubstituted alkyl, substituted or unsubstituted alkenyl, substituted or unsubstituted alkynyl, substituted or unsubstituted aryl, substituted or unsubstituted heteroaryl, substituted or unsubstituted cycloalkyl, substituted or unsubstituted cycloalkenyl, substituted or unsubstituted heterocyclyl, substituted or unsubstituted alkyloxy, substituted or unsubstituted aryloxy, substituted or unsubstituted heteroaryloxy, substituted or unsubstituted cycloalkyloxy, substituted or unsubstituted cycloalkenyloxy, substituted or unsubstituted heterocyclyloxy, substituted or unsubstituted alkylthio, substituted or unsubstituted arylthio, substituted or unsubstituted heteroarylthio, substituted or unsubstituted cycloalkylthio, substituted or unsubstituted cycloalkenylthio, substituted or unsubstituted heterocyclylthio, substituted or unsubstituted alkylsulfonyl, substituted or unsubstituted arylsulfonyl, substituted or unsubstituted heteroarylsulfonyl, substituted or unsubstituted cycloalkylsulfonyl, substituted or unsubstituted cycloalkenylsulfonyl, substituted or unsubstituted heterocyclylsulfonyl, substituted or unsubstituted acyl, substituted or unsubstituted carbamoyl, substituted or unsubstituted sulfamoyl, or substituted or unsubstituted amino;
      R¹² is hydrogen, or substituted or unsubstituted alkyl;
      with the proviso that
      compounds wherein Z is =CR⁶-, and R⁶ and R⁸ are fuluoro at the same time; and compounds shown below are excluded 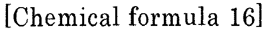 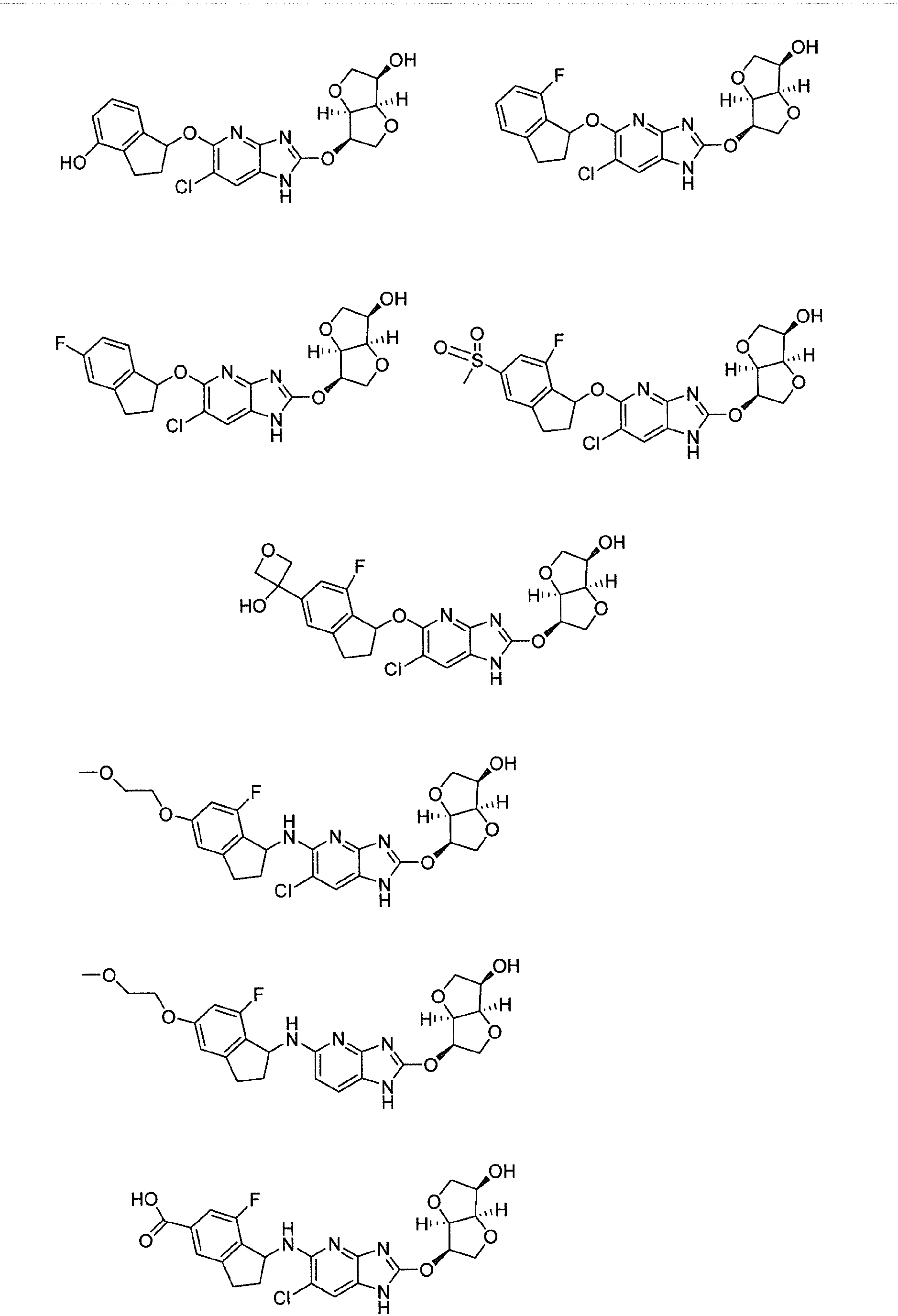 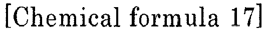 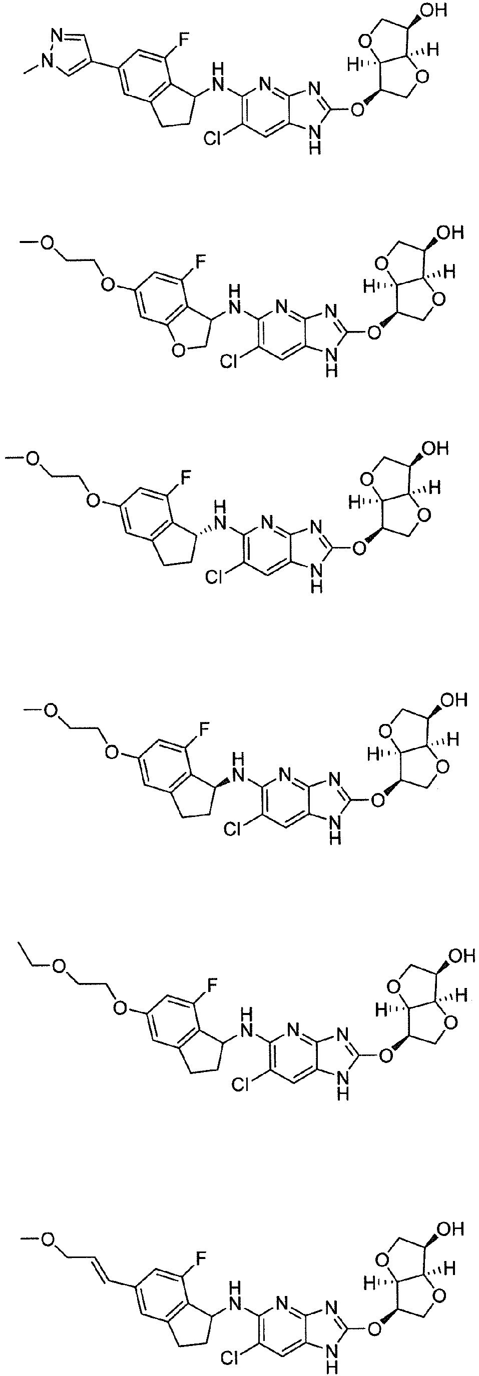 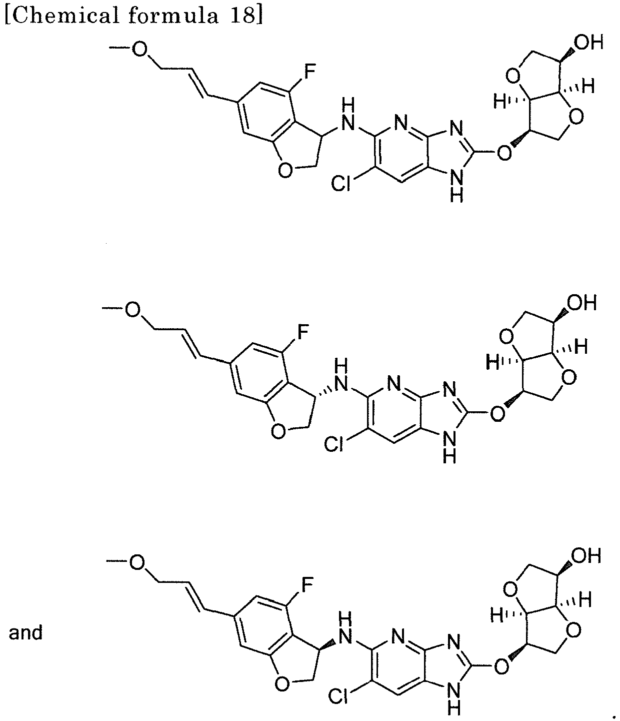
   (2A) The compound according to the above (1A), or its pharmaceutically acceptable salt, wherein L is -NR¹-, or -O-.
   (3A) The compound according to the above (1A) or (2A), or its pharmaceutically acceptable salt, wherein R¹ is hydrogen.
   (4A) The compound according to any one of the above (1A) to (3A), or its pharmaceutically acceptable salt, wherein Y is substituted or unsubstituted heterocyclyl.
   (5A) The compound according to the above (4A), or its pharmaceutically acceptable salt,
      wherein Y is 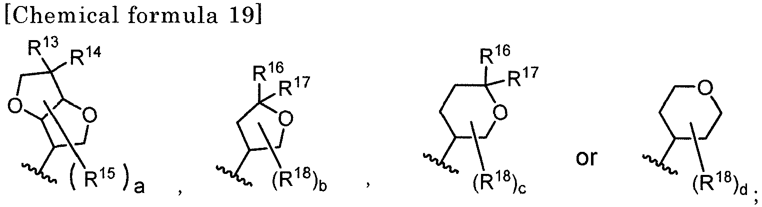
      wherein R¹³ and R¹⁴ are each independently hydrogen, halogen, hydroxy, cyano, nitro, carboxy, substituted or unsubstituted alkyl, substituted or unsubstituted alkenyl, substituted or unsubstituted alkynyl, substituted or unsubstituted alkyloxy, substituted or unsubstituted alkylthio, substituted or unsubstituted alkylsulfonyl, substituted or unsubstituted acyl, substituted or unsubstituted carbamoyl, substituted or unsubstituted sulfamoyl, or substituted or unsubstituted amino;
      R¹⁵ is each independently halogen, hydroxy, cyano, nitro, carboxy, substituted or unsubstituted alkyl, substituted or unsubstituted alkenyl, substituted or unsubstituted alkynyl, substituted or unsubstituted alkyloxy, substituted or unsubstituted alkylthio, substituted or unsubstituted alkylsulfonyl, substituted or unsubstituted acyl, substituted or unsubstituted carbamoyl, substituted or unsubstituted sulfamoyl, or substituted or unsubstituted amino;
      a is an integer from 0 to 6;
      R¹⁶ and R¹⁷ are each independently halogen, hydroxy, cyano, nitro, carboxy, substituted or unsubstituted alkyl, substituted or unsubstituted alkenyl, substituted or unsubstituted alkynyl, substituted or unsubstituted alkyloxy, substituted or unsubstituted alkylthio, substituted or unsubstituted alkylsulfonyl, substituted or unsubstituted acyl, substituted or unsubstituted carbamoyl, substituted or unsubstituted sulfamoyl, or substituted or unsubstituted amino; R¹⁸ is each independently halogen, hydroxy, cyano, nitro, carboxy, substituted or unsubstituted alkyl, substituted or unsubstituted alkenyl, substituted or unsubstituted alkynyl, substituted or unsubstituted alkyloxy, substituted or unsubstituted alkylthio, substituted or unsubstituted alkylsulfonyl, substituted or unsubstituted acyl, substituted or unsubstituted carbamoyl, substituted or unsubstituted sulfamoyl, or substituted or unsubstituted amino;
      b is an integer from 0 to 5;
      c is an integer from 0 to 7;
      d is an integer from 0 to 9.
   (6A) The compound according to any one of the above (1A) to (5A), or its pharmaceutically acceptable salt, wherein X is -CR⁴R⁵-.
   (7A) The compound according to any one of the above (1A) to (6 A), or its pharmaceutically acceptable salt, wherein R⁴ and R⁵ are hydrogen.
   (8A) The compound according to any one of the above (1A) to (7A), or its pharmaceutically acceptable salt, wherein Z is =CR⁶-, and R⁶ is halogen.
   (9A) The compound according to any one of the above (1A) to (7A), or its pharmaceutically acceptable salt, wherein Z is =N-.
   (10A) The compound according to any one of the above (1A) to (9A), or its pharmaceutically acceptable salt, wherein R⁷, R⁸ and R⁹ are each independently halogen, hydroxy, cyano, nitro, carboxy, substituted or unsubstituted alkyl, substituted or unsubstituted alkenyl, substituted or unsubstituted alkynyl, substituted or unsubstituted aryl, substituted or unsubstituted heteroaryl, substituted or unsubstituted cycloalkyl, substituted or unsubstituted cycloalkenyl, substituted or unsubstituted heterocyclyl, substituted or unsubstituted alkyloxy, substituted or unsubstituted aryloxy, substituted or unsubstituted heteroaryloxy, substituted or unsubstituted cycloalkyloxy, substituted or unsubstituted cycloalkenyloxy, substituted or unsubstituted heterocyclyloxy, substituted or unsubstituted alkylsulfonyl, or substituted or unsubstituted amino.
   (11A) The compound according to any one of the above (1A) to (10A), or its pharmaceutically acceptable salt, wherein R¹⁰ is hydrogen, halogen, cyano, carboxy, or substituted or unsubstituted alkyl.
   (12A) The compound according to the above (11A), or its pharmaceutically acceptable salt, wherein R¹⁰ is hydrogen, fluoro, chloro, cyano, or substituted or unsubstituted alkyl, wherein the substituent of the substituted alkyl is halogen.
   (13A) The compound according to the above (11A), or its pharmaceutically acceptable salt, wherein R¹⁰ is fluoro, or chloro.
   (14A) The compound according to any one of the above (1A) to (13A), or its pharmaceutically acceptable salt, wherein R¹¹ is hydrogen.
   (15A) The compound according to any one of the above (1A) to (14A), or its pharmaceutically acceptable salt, wherein R¹² is hydrogen.
   (16A) The compound according to the above (1A), or its pharmaceutically acceptable salt, wherein the compound is selected from compound (I-1-4), (I-1-6), (I-1-7), (1-1-10), (1-1-11), (I-1-13), (I-1-14), (I-1-15), (I-1-19), (I-1-20), (I-1-23), (I-1-24), (I-1-29), (I-1-32), (1-1-35), (I-1-38), (1-1-39), (1-1-42), (I-1-43), (1-1-44), (I-1-45), (1-1-46), (I-1-47), (I-1-48), (I-1-70), (I-1-72), (I-1-74), (I-1-76), (I-1-77), (1-1-79), (I-1-81), (1-1-83), (1-1-86), (I-1-88), (I-1-90), (I-1-91), (I-1-93), (I-1-94), (1-1-95), (I-1-96), (1-1-97), (I-1-98), (I-1-99), (1-1-100), (1-1-104), (I-1-105), (1-1-106), or (I-1-111).
   (17A) A pharmaceutical composition comprising the compound according to any one of the above (1A) to (16A), or its pharmaceutically acceptable salt.
   (18A) The pharmaceutical composition according to the above (17A), which has an activating effect on adenosine monophosphate-activated protein kinase.
   (19A) A method for preventing or treating diabetes, comprising administering the compound according to any one of the above (1A) to (16A), or its pharmaceutically acceptable salt.
   (20A) The compound according to any one of the above (1A) to (16A), or its pharmaceutically acceptable salt, for the treatment and/or prevention of diabetes.
   (21A) The pharmaceutical composition according to the above (17A) or (18A), for the treatment and/or prevention of diabetes.
   (25A) A pharmaceutical composition for oral administration, comprising a compound represented by formula (I) or its pharmaceutically acceptable salt,
   (26A) The pharmaceutical composition according to the above (25A), which is a tablet, powder, granule, capsule, pill, film, suspension, emulsion, elixir, syrup, lemonade, spirit, aromatic water, extract, decoction or tincture.
   (27A) The pharmaceutical composition according to the above (26A), which is a sugar-coated tablet, film-coated tablet, enteric-coated tablet, sustained-release tablet, troche tablet, sublingual tablet, buccal tablet, chewable tablet, orally disintegrating tablet, dry syrup, soft capsule, micro capsule or sustained-release capsule.
   (28A) A pharmaceutical composition for parenteral administration, comprising a compound represented by formula (I), or its pharmaceutically acceptable salt.
   (29A) The pharmaceutical composition according to the above (28A), for dermal, subcutaneous, intravenous, intraarterial, intramuscular, intraperitoneal, transmucosal, inhalation, transnasal, ophthalmic, inner ear or vaginal administration.
   (30A) The pharmaceutical composition according to the above (28A) or (29A), which is injection, infusion, eye drop, nose drop, ear drop, aerosol, inhalation, lotion, impregnation, liniment, mouthwash, enema, ointment, plaster, jelly, cream, patch, cataplasm, external powder or suppository.
   (31A) A pharmaceutical composition for a pediatric or geriatric patient, comprising a compound represented by formula (I), or its pharmaceutically acceptable salt.
   (32A) A pharmaceutical composition consisting of a combination of a compound represented by formula (I) or its pharmaceutically acceptable salt, and an insulin secretagogue, a fast-acting insulin secretagogue, a glucose uptake inhibitor, an insulin resistance improving drug, a thiazolidine derivative, an insulin formulation, a peptidyl peptidase IV inhibitor, a GLP-1 receptor agonist, a sodium-dependent glucose transporter 1 inhibitor, or a sodium-dependent glucose transporter 2 inhibitor.
   (33A) A pharmaceutical composition comprising a compound represented by formula (I) or its pharmaceutically acceptable salt, for a combination therapy with an insulin secretagogue, a fast-acting insulin secretagogue, a glucose uptake inhibitor, an insulin resistance improving drug, a thiazolidine derivative, an insulin formulation, a peptidyl peptidase IV inhibitor, a GLP-1 receptor agonist, a sodium-dependent glucose transporter 1 inhibitor, or a sodium-dependent glucose transporter 2 inhibitor.

### [Effect of the Invention]

The compound of the present invention has an AMPK activating effect, and thus a pharmaceutical composition comprising a compound of the present invention is very useful as a medicinal product, particularly, a medicine for treating and/or preventing type II diabetes, hyperglycemia, metabolic syndrome, obesity, hypercholesterolemia and/or hypertension. Further, the compound of the present invention is a compound which has usefulness as a medicine. The usefulness as a medicine herein comprises good metabolic stability, slight induction of a drug-metabolizing enzyme, slight inhibition of drug-metabolizing enzymes which metabolize other drugs, high oral absorption, low clearance, a sufficiently long half-life period to express the efficacy of a medicine, a high enzyme activity, a high maximal activation rate, a low protein binding rate, high penetration into target tissue, high solubility, high safety, an insulin resistance improving effect based on an energy consumption increase, the effect of decreasing hemoglobin Aic(HbAlc), the effect of improving fatty hepatic or the like.

### [Mode for Carrying Out the Invention]

Each term used in this description will be described below. In this description, even when each term is used individually or used with other terms, the term has the same meaning.

"Halogen" includes fluorine, chlorine, bromine and iodine.

"Alkyl" means a C1 to C10 straight or branched alkyl group, and examples thereof include methyl, ethyl, n-propyl, isopropyl, n-butyl, isobutyl, sec-butyl, tert-butyl, n-pentyl, isopentyl, neopentyl, n-hexyl, isohexyl, n-heptyl, n-octyl, n-nonyl, n-decyl and the like. Preferred examples include C1 to C6 or C1 to C4 alkyl, and examples thereof include methyl, ethyl, n-propyl, isopropyl, n-butyl, isobutyl, sec-butyl, tert-butyl, n-pentyl, isopentyl, neopentyl, n-hexyl and isohexyl.

"Alkenyl" means C2 to C8 straight or branched alkenyl having one or more double bond(s) in the above "alkyl", and examples thereof include vinyl, 1-propenyl, 2-propenyl, 1-butenyl, 2-butenyl, 3-butenyl, 1,3-butadienyl, 3-methyl-2-butenyl and the like.

"Alkynyl" means C2 to C8 straight or branched alkynyl having one or more triple bond(s) in the above "alkyl", and examples thereof include ethynyl, propynyl, butynyl and the like. Furthermore, an "alkynyl" may have a double bond.

"Cycloalkyl" means a C3 to C15 cyclic saturated hydrocarbon group, and examples thereof include cyclopropyl, cyclobutyl, cyclopentyl, cyclohexyl, cycloheptyl, cyclooctyl, bridged cyclic hydrocarbon group, spiro hydrocarbon group and the like. Preferred examples include cyclopropyl, cyclobutyl, cyclopentyl, cyclohexyl and bridged cyclic hydrocarbon group.

A "bridged cyclic hydrocarbon group" includes a group which is derived by removing one hydrogen from a C5 to C8 aliphatic cycle which consists of two or more rings that share two or more atoms. Specific examples thereof include bicyclo[2.1.0]pentyl, bicyclo[2.2.1]heptyl, bicyclo[2.2.2]octyl, bicyclo[3.2.1]octyl, tricyclo[2.2.1.0]heptyl and the like.

A "spiro hydrocarbon group" includes a group which is derived by removing one hydrogen from a cycle which consists of two hydrocarbon rings that share one carbon atom. Specific examples thereof include spiro[3.4]octyl and the like.

"Cycloalkenyl" means C3 to C10 cyclic unsaturated aliphatic hydrocarbon group, and examples thereof include cyclopropenyl (e.g., 1-cyclopropenyl), cyclobutenyl (e.g., 1-cyclobutenyl), cyclopentenyl (e.g., 1-cyclopenten-1-yl, 2-cyclopenten-1-yl and 3-cyclopenten-1-yl), cyclohexenyl (e.g., 1-cyclohexen-1-yl, 2-cyclohexen-1-yl and 3-cyclohexen-1-yl), cycloheptenyl (e.g., 1-cycloheptenyl), cyclooctenyl (e.g., 1-cyclooctenyl) and the like. Preferable is cyclopropenyl, cyclobutenyl, cyclopentenyl or cyclohexenyl. Cycloalkenyls also include bridged cyclic hydrocarbon group and spiro hydrocarbon group which both have an unsaturated bond in the ring. Cycloalkenyls also include cyclic groups in which cycloalkene or a benzene ring is condensed with the cycloalkyl. For examples, cycloalkenyls also include the groups shown below.

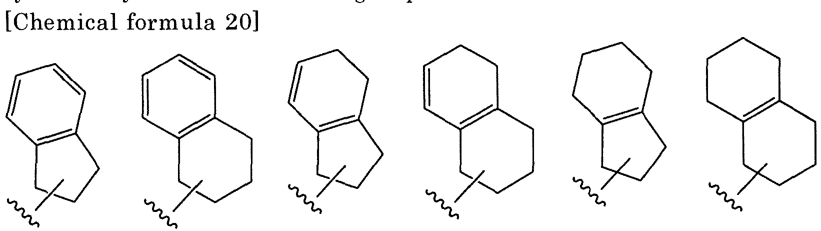

"Aryl" means a monocyclic aromatic hydrocarbon group (e.g., phenyl) and a polycyclic aromatic hydrocarbon group (e.g., 1-naphthyl, 2-naphthyl, 1-anthryl, 2-anthryl, 9-anthryl, 1-phenanthryl, 2-phenanthryl, 3-phenanthryl, 4-phenanthryl, 9-phenanthryl, etc.). Preferred examples include phenyl or naphthyl (1-naphthyl or 2-naphthyl).

"Heteroaryl" means a monocyclic aromatic heterocyclic group and a fused aromatic heterocyclic group.

A "monocyclic aromatic heterocyclic group" means a group which is derived from a 5 to 8-membered aromatic ring which has one or more same or different heteroatoms optionally selected from oxygen, sulfur and nitrogen atoms in the ring, which group may have a bond at any substitutable position.

A "fused aromatic heterocyclic group" means a group in which a 5 to 8-membered aromatic ring which has one or more same or different heteroatoms optionally selected from oxygen, sulfur and nitrogen atoms in the ring is fused with one to four 5 to 8-membered aromatic carbocyclic rings or another 5 to 8-membered aromatic hetero ring, which group may have a bond at any substitutable position.

Examples of a "heteroaryl" include furyl (e.g., 2-furyl, 3-furyl), thienyl (e.g., 2-thienyl, 3-thienyl), pyrrolyl (e.g., 1-pyrrolyl, 2-pyrrolyl, 3-pyrrolyl), imidazolyl (e.g., 1-imidazolyl, 2-imidazolyl, 4-imidazolyl), pyrazolyl (e.g., 1-pyrazolyl, 3-pyrazolyl, 4-pyrazolyl), triazolyl (e.g., 1,2,4-triazol-1-yl, 1,2,4-triazol-3-yl, 1,2,4-triazol-4-yl), tetrazolyl (e.g., 1-tetrazolyl, 2-tetrazolyl, 5-tetrazolyl), oxazolyl (e.g., 2-oxazolyl, 4-oxazolyl, 5-oxazolyl), isoxazolyl (e.g., 3-isoxazolyl, 4-isoxazolyl, 5-isoxazolyl), thiazolyl (e.g., 2-thiazolyl, 4-thiazolyl, 5-thiazolyl), thiadiazolyl, isothiazolyl (e.g., 3-isothiazolyl, 4-isothiazolyl, 5-isothiazolyl), pyridyl (e.g., 2-pyridyl, 3-pyridyl, 4-pyridyl), pyridazinyl (e.g., 3-pyridazinyl, 4-pyridazinyl), pyrimidinyl (e.g., 2-pyrimidinyl, 4-pyrimidinyl, 5-pyrimidinyl), furazanyl (e.g., 3-furazanyl), pyrazinyl (e.g., 2-pyrazinyl), oxadiazolyl (e.g., 1,3,4-oxadiazol-2-yl), benzofuryl (e.g., 2-benzo[b]furyl, 3-benzo[b]furyl, 4-benzo[b]furyl, 5-benzo[b]furyl, 6-benzo[b]furyl, 7-benzo[b]furyl), benzothienyl (e.g., 2-benzo[b]thienyl, 3-benzo[b]thienyl, 4-benzo[b]thienyl, 5-benzo[b]thienyl, 6-benzo[b]thienyl, 7-benzo[b]thienyl), benzimidazolyl (e.g., 1-benzimidazolyl, 2-benzimidazolyl, 4-benzimidazolyl, 5-benzimidazolyl), benzopyrazolyl, dibenzofuryl, benzoxazolyl, benzothiazolyl, quinoxalinyl (e.g., 2-quinoxalinyl, 5-quinoxalinyl, 6-quinoxalinyl), cinnolinyl (e.g., 3-cinnolinyl, 4-cinnolinyl, 5-cinnolinyl, 6-cinnolinyl, 7-cinnolinyl, 8-cinnolinyl), quinazolinyl (e.g., 2-quinazolinyl, 4-quinazolinyl, 5-quinazolinyl, 6-quinazolinyl, 7-quinazolinyl, 8-quinazolinyl), quinolyl (e.g., 2-quinolyl, 3-quinolyl, 4-quinolyl, 5-quinolyl, 6-quinolyl, 7-quinolyl, 8-quinolyl), phthalazinyl (e.g., 1-phthalazinyl, 5-phthalazinyl, 6-phthalazinyl), isoquinolyl (e.g., 1-isoquinolyl, 3-isoquinolyl, 4-isoquinolyl, 5-isoquinolyl, 6-isoquinolyl, 7-isoquinolyl, 8-isoquinolyl), puryl, pteridinyl (e.g., 2-pteridinyl, 4-pteridinyl, 6-pteridinyl, 7-pteridinyl), carbazolyl, phenanthridinyl, acridinyl (e.g., 1-acridinyl, 2-acridinyl, 3-acridinyl, 4-acridinyl, 9-acridinyl), indolyl (e.g., 1-indolyl, 2-indolyl, 3-indolyl, 4-indolyl, 5-indolyl, 6-indolyl, 7-indolyl), isoindolyl, phenazinyl (e.g., 1-phenazinyl, 2-phenazinyl), phenothiazinyl (e.g., 1-phenothiazinyl, 2-phenothiazinyl, 3-phenothiazinyl, 4-phenothiazinyl) and the like.

"Heterocyclyl" means a non-aromatic heterocyclic group, which may have a bond at any substitutable position of a ring which has at least one or more nitrogen, oxygen or sulfur atoms in the ring, or a ring in which such ring is fused with a cycloalkane (preferably 5 to 6-membered), a benzene ring and/or a ring which has at least one or more nitrogen, oxygen or sulfur atoms in the ring. A "non-aromatic heterocyclic group" can be saturated or unsaturated as long as it is non-aromatic. Preferable is a 5- to 10-membered ring. Examples thereof include 1-pyrrolinyl, 2-pyrrolinyl, 3-pyrrolinyl, 1-pyrrolidinyl, 2-pyrrolidinyl, 3-pyrrolidinyl, 1-imidazolinyl, 2-imidazolinyl, 4-imidazolinyl, 1-imidazolidinyl, 2-imidazolidinyl, 4-imidazolidinyl, 1-pyrazolinyl, 3-pyrazolinyl, 4-pyrazolinyl, 1-pyrazolidinyl, 3-pyrazolidinyl, 4-pyrazolidinyl, piperidino, 2-piperidinyl, 3-piperidinyl, 4-piperidinyl, 1-piperazinyl, 2-piperazinyl, 2-morpholinyl, 3-morpholinyl, morpholino, tetrahydropyranyl, tetrahydrofuranyl, 1,2,3,4-tetrahydroisoquinolinyl, 1,2,3,4-tetrahydroquinolinyl, 1,3-dihydro-2H-isoindol-5-yl, the following group and the like.

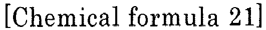

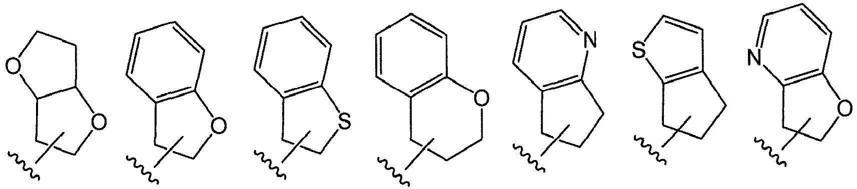

Further, examples of a "heterocyclyl" group also include a bridged group or a spiro ring forming group shown below.

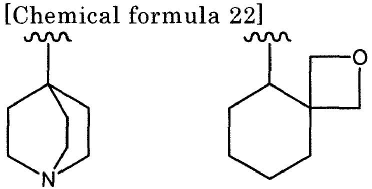

"Acyl" means formyl, substituted or unsubstituted alkylcarbonyl, substituted or unsubstituted alkenylcarbonyl, substituted or unsubstituted cycloalkylcarbonyl, substituted or unsubstituted cycloalkenylcarbonyl, substituted or unsubstituted arylcarbonyl, substituted or unsubstituted heteroarylcarbonyl or substituted or unsubstituted heterocyclylcarbonyl. The "alkyl" part of "alkylcarbonyl", the "alkenyl" part of "alkenylcarbonyl", the "cycloalkyl" part of "cycloalkylcarbonyl", the "cycloalkenyl" part of "cycloalkenylcarbonyl", the "aryl" part of "arylcarbonyl", the "heteroaryl" part of "heteroarylcarbonyl" and the "heterocyclyl" part of "heterocyclylcarbonyl" respectively mean the above "alkyl", the above "alkenyl", the above "cycloalkyl", the above "cycloalkenyl", the above "aryl", the above "heteroaryl" and the above "heterocyclyl".

The alkyl parts of "alkyloxy", "alkylthio" and "alkylsulfonyl" mean the above "alkyl".

The "alkenyl" part of "alkenylsulfonyl" means the above "alkenyl".

The "alkynyl" part of "alkynylsulfonyl" means the above "alkynyl".

The aryl parts of "aryloxy", "arylthio" and "arylsulfonyl" mean the above "aryl".

The heteroaryl parts of "heteroaryloxy", "heteroarylthio" and "heteroarylsulfonyl" mean the above "heteroaryl".

The cycloalkyl parts of "cycloalkyloxy", "cycloalkylthio" and "cycloalkylsulfonyl" mean the above "cycloalkyl".

The cycloalkenyl parts of "cycloalkenyloxy", "cycloalkenylthio" and "cycloalkenylsulfonyl" mean the above "cycloalkenyl".

The heterocyclyl parts of "heterocyclyloxy", "heterocyclylthio" and "heterocyclylsulfonyl" mean the above "heterocyclyl".

Examples of substituents of "substituted alkyl", "substituted alkenyl", "substituted alkynyl", "substituted aryl", "substituted heteroaryl", "substituted cycloalkyl", "substituted cycloalkenyl", "substituted heterocyclyl", "substituted acyl", "substituted alkylsulfonyl", "substituted alkenylsulfonyl", "substituted alkynylsulfonyl", "substituted arylsulfonyl", "substituted heteroarylsulfonyl", "substituted cycloalkylsulfonyl", "substituted cycloalkenylsulfonyl", "substituted heterocyclylsulfonyl", "substituted alkyloxy", "substituted aryloxy", "substituted heteroaryloxy", "substituted cycloalkyloxy", "substituted cycloalkenyloxy", "substituted heterocyclyloxy", "substituted alkylthio", "substituted arylthio", "substituted heteroarylthio", "substituted cycloalkylthio", "substituted cycloalkenylthio" and "substituted heterocyclylthio" include groups selected from the group consisting of halogen; hydroxy; carboxy; nitro; cyano; substituted or unsubstituted alkyl (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, substituted or unsubstituted amino (when substituted, substituents include alkylsulfonyl, alkyl, alkylcarbonyl.), aryloxy, alkylsulfonyl, acylamino, alkyloxycarbonyl. e.g., methyl, ethyl, isopropyl, tert-butyl, or CF₃); substituted or unsubstituted alkenyl (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino, alkyloxy. e.g., vinyl); substituted or unsubstituted alkynyl (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino, alkyloxy. e.g., ethynyl); substituted or unsubstituted aryl (when substituted, substituents include halogen, hydroxy, carboxy, nitro, cyano, substituted or unsubstituted alkyl (when substituted, substituents include halogen, hydroxy, heterocyclyl, heterocyclylcarbonyl, substituted or unsubstituted amino (when substituted, substituents include alkyl.).), substituted or unsubstituted alkenyl (when substituted, substituents include hydroxy, alkyloxy, amino, carboxy.), substituted or unsubstituted alkynyl (when substituted, substituents include hydroxy, substituted or unsubstituted amino (when substituted, substituents include alkyl.), alkyloxy, carboxy.), aryl, cycloalkyl, substituted or unsubstituted cycloalkyloxy (when substituted, substituents include halogen.), substituted or unsubstituted cycloalkenyl (when substituted, substituents include carboxy.), substituted or unsubstituted heteroaryl (when substituted, substituents include alkyl, oxo, heterocyclylalkyl.), substituted or unsubstituted heterocyclyl (when substituted, substituents include alkyl, alkylsulfonyl, cycloalkylcarbonyl, oxo.), substituted or unsubstituted heterocyclyloxy (when substituted, substituents include oxo, alkyl, alkylcarbonyl, alkyloxycarbonyl.), substituted or unsubstituted carbamoyl (when substituted, substituents include alkyl), sulfamoyl, amino, alkylcarbonylamino, substituted or unsubstituted alkyloxy (when substituted, substituents include halogen, hydroxy, cycloalkyl, substituted or unsubstituted alkyloxy (when substituted, substituents include alkyloxy, halogen, CF₃.), substituted or unsubstituted heterocyclyl (when substituted, substituents include halogen, alkyl, alkylsulfonyl, acyl, hydroxyalkyl, alkyloxycarbonyl, oxo.), substituted or unsubstituted heteroaryl (when substituted, substituents include alkyl.), alkylamino, alkylsulfonyl, alkylsulfonylamino, oxo, alkylcarbonyl, alkylcarbonylamino, carbamoyl, carbamoylamino, carboxy, cyano.), alkylsulfonyl, alkyloxycarbonyl, SF₅, R^{S}R^{S'}(O=)S=N-, R^{S}R^{S'}(O=)S=N-R^{2f}-, R^{S}R^{S'}(O=)S=N-C(=O)-, (R^{N})N=S(=O)(R^{S})-, (R^{N})N=S(=O)(R^{S})-R^{2f}-, R^{S}R^{S'}(R^{N'}-N=)S=N-, ((R^{N})N=)₂S(R^{S})-, (R^{N}R^{N'})N-C(=O)-O-, R^{O}O-C(=O)-N(R^{N})-, R^{O}O-C(=O)-O-, R^{S}(R^{N}R^{N'}N)(O=)S=N-, R^{S}(R^{N}R^{N'}N)(O=)S=N-R^{2f}-, (R^{N"})N=S(=O)(NR^{N}R^{N'})- (R^{N"})N=S(=O)(NR^{N}R^{N'})-R^{2f}-, 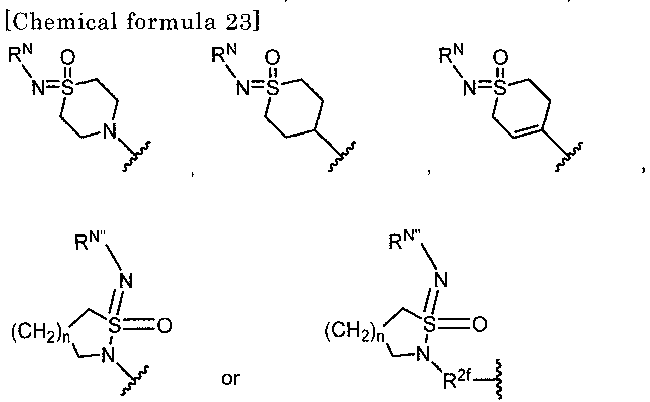
- e.g., phenyl, or naphthyl);
   substituted or unsubstituted cycloalkyl (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino, SF₅, R^{S}R^{S'}(O=)S=N-, R^{S}R^{S'}(O=)S=N-R^{2f}-, R^{S}R^{S'}(O=)S=N-C(=O)-, (R^{N})N=S(=O)(R^{S})-, (R^{N})N=S(=O)(R^{S})-R^{2f}-, R^{S}R^{S'}(R^{N'}-N=)S=N-, ((R^{N})N=)₂S(R^{S})-, (R^{N}R^{N'})N-C(=O)-O-, R^{O}O-C(=O)-N(R^{N})-, R^{O}O-C(=O)-O-, R^{S}(R^{N}R^{N'}N)(O=)S=N-, R^{S}(R^{N}R^{N'}N)(O=)S=N-R^{2f}-, (R^{N"})N=S(=O)(NR^{N}R^{N'})-, (R^{N"})N=S(=O)(NR^{N}R^{N'})-R^{2f}-, 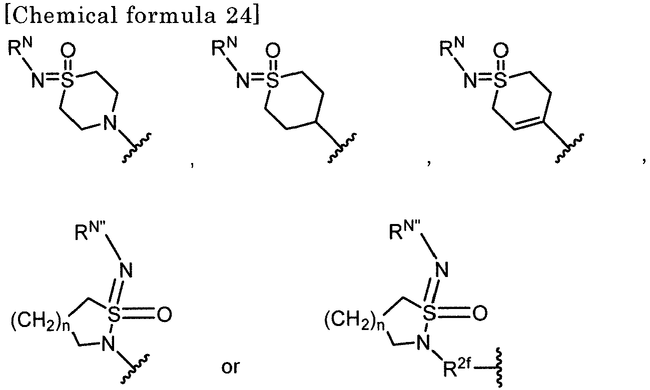
- e.g., cyclopropyl, or cyclobutyl);
   substituted or unsubstituted cycloalkenyl (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino, SF₅, R^{S}R^{S'}(O=)S=N-, R^{S}R^{S'}(O=)S=N-R^{2f}-, R^{S}R^{S'}(O=)S=N-C(=O)-, (R^{N})N=S(=O)(R^{S})-, (R^{N})N=S(=O)(R^{S})-R^{2f}-, R^{S}R^{S'}(R^{N'}-N=)S=N-, ((R^{N})N=)₂S(R^{S})-, (R^{N}R^{N'})N-C(=O)-O-, R^{O}O-C(=O)-N(R^{N})-, R^{O}O-C(=O)-O-, R^{S}(R^{N}R^{N'}N)(O=)S=N-, R^{S}(R^{N}R^{N'}N)(O=)S=N-R^{2f}-, (R^{N"})N=S(=O)(NR^{N}R^{N'})-, (R^{N"})N=S(=O)(NR^{N}R^{N'})-R^{2f}-, 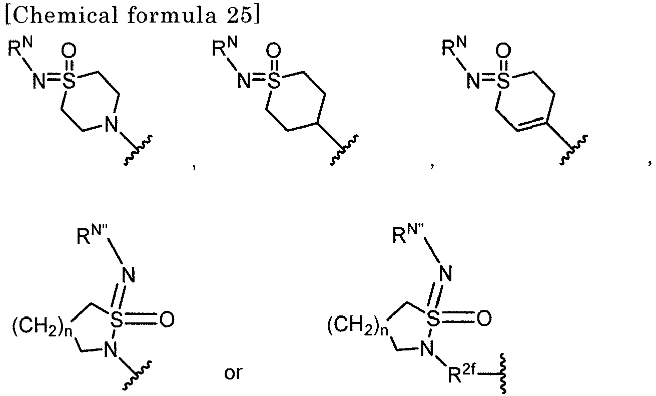
- e.g., cyclopropenyl);
   substituted or unsubstituted heteroaryl (when substituted, substituents include halogen, hydroxy, carboxy, nitro, cyano, substituted or unsubstituted alkyl (when substituted, substituents include hydroxy, alkyl, substituted or unsubstituted alkyloxy (when substituted, substituents include alkyloxy.), aryl, carbamoyl, alkylsulfonylamino.), alkenyl, alkynyl, aryl, cycloalkyl, cycloalkylalkyl, cycloalkenyl, heteroaryl, heteroarylalkyl, heterocyclyl, heterocyclyloxy, heterocyclylalkyl, heterocyclylalkylcarbamoyl, sulfamoyl, amino, alkylamino, alkyloxy, alkyloxycarbonyl, hydroxyalkyl.);
   substituted or unsubstituted heterocyclyl (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino, SF₅, R^{S}R^{S'}(O=)S=N-, R^{S}R^{S'}(O=)S=N-R^{2f-}, R^{S}R^{S'}(O=)S=N-C(=O)-, (R^{N})N=S(=O)(R^{S})-, (R^{N})N=S(=O)(R^{S})-R^{2f-}, R^{S}R^{S'}(R^{N'}-N=)S=N-, ((R^{N})N=)₂S(R^{S})-, (R^{N}R^{N'})N-C(=O)-O-, R^{O}O-C(=O)-N(R^{N})-, R^{O}O-C(=O)-O-, R^{S}(R^{N}R^{N'}N)(O=)S=N-, R^{S}(R^{N}R^{N'}N)(O=)S=N-R^{2f}-, (R^{N"})N=S(=O)(NR^{N}R^{N'})-, (R^{N"})N=S(=O)(NR^{N}R^{N'})-R^{2f}-, 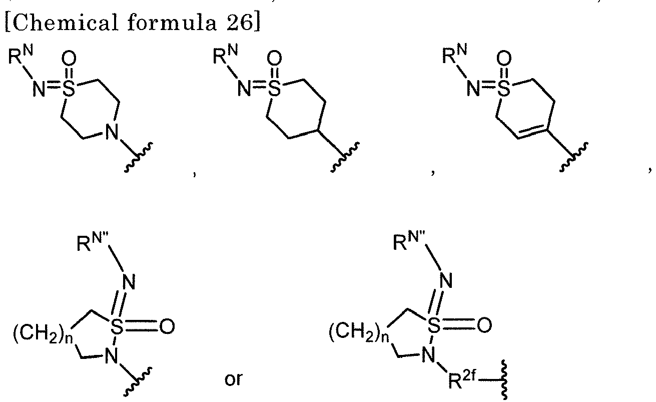
- e.g., morpholinyl, piperidyl, or pyrrolidinyl);
   substituted or unsubstituted alkyloxy (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, substituted or unsubstituted amino (when substituted, substituents include alkyl.), alkyloxy, alkylsulfonylamino, alkylsulfonyl, R^{S}R^{S'}(O=)S=N-, R^{S}R^{S'}(O=)S=N-R^{2f}-, R^{S}R^{S'}(O=)S=N-C(=O)-, (R^{N})N=S(=O)(R^{S})-, (R^{N})N=S(=O)(R^{S})-R^{2f-}, R^{S}R^{S'}(R^{N'}-N=)S=N-, ((R^{N})N=)₂S(R^{S})-, (R^{N}R^{N'})N-C(=O)-O-, R^{O}O-C(=O)-N(R^{N})-, R^{O}O-C(=O)-O-, R^{S}(R^{N}R^{N'}N)(O=)S=N-, R^{S}(R^{N}R^{N'}N)(O=)S=N-R^{2f}-, (R^{N"})N=S(=O)(NR^{N}R^{N'})-, (R^{N"})N=S(=O)(NR^{N}R^{N'})-R^{2f}-, 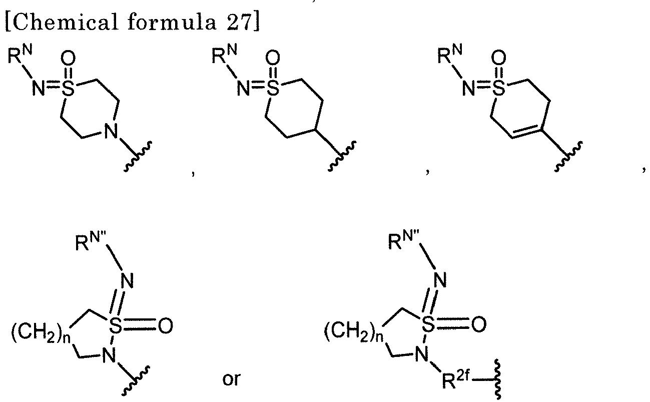
- e.g., methoxy, or ethoxy);
   substituted or unsubstituted alkenyloxy (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino. e.g., vinyloxy, or aryloxy);
   substituted or unsubstituted aryloxy (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino. e.g., phenyloxy);
   substituted or unsubstituted cycloalkyloxy (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino.);
   substituted or unsubstituted cycloalkenyloxy (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino.); substituted or unsubstituted heteroaryloxy (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino.);
   substituted or unsubstituted heterocyclyloxy (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino, alkylcarbonyl.);
   substituted or unsubstituted arylalkyl (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino. e.g., benzyl);
   substituted or unsubstituted amino (e.g., alkylamino (e.g., methylamino, ethylamino, or dimethylamino), arylamino, cycloalkylamino, cycloalkenylamino, heteroarylamino, heterocyclylamino, substituted or unsubstituted acylamino (when substituted, substituents include alkyloxy, e.g., acetylamino, or benzoylamino), arylalkylamino (e.g., benzylamino, or tritylamino), hydroxyamino, alkyloxycarbonylamino, carbamoylamino, substituted or unsubstituted alkylsulfonylamino (when substituted, substituents include halogen), arylsulfonylamino, cycloalkylsulfonylamino, cycloalkenylsulfonylamino, heteroarylsulfonylamino, heterocyclylsulfonylamino.);
   substituted or unsubstituted carbamoyl (when substituted, substituents include halogen, hydroxy, carboxy, nitro, cyano, substituted or unsubstituted alkyl (when substituted, substituents include heterocyclyl), alkenyl, alkynyl, aryl, cycloalkyl, cycloalkenyl, heteroaryl, heterocyclyl. e.g., alkylcarbamoyl (e.g., methylcarbamoyl, ethylcarbamoyl, dimethylcarbamoyl, phenylethylcarbamoyl, dimethylaminoethylcarbamoyl, isopropylcarbamoyl, or hydroxyethylcarbamoyl), alkylsulfonylcarbamoyl, heteroarylalkylcarbamoyl, alkyloxycarbamoyl.); substituted or unsubstituted carbamoyloxy (when substituted, substituents include halogen, hydroxy, carboxy, nitro, cyano, alkyl, alkenyl, alkynyl, aryl, cycloalkyl, cycloalkenyl, heteroaryl, heterocyclyl.);
   substituted or unsubstituted acyl (when substituted, substituents include halogen, hydroxy, hydroxyalkyl, carboxy, nitro, cyano, alkyl, alkenyl, alkynyl, aryl, cycloalkyl, cycloalkenyl, heteroaryl, heterocyclyl, carbamoyl, sulfamoyl, amino. e.g., alkylcarbonyl, arylcarbonyl, cycloalkylcarbonyl, cycloalkenylcarbonyl, heteroarylcarbonyl, heterocyclylcarbonyl, formyl, or acetyl.);
   substituted or unsubstituted alkylsulfonyl (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino. e.g., methanesulfonyl, or ethanesulfonyl);
   substituted or unsubstituted arylsulfonyl (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino.);
   substituted or unsubstituted cycloalkylsulfonyl (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino.); substituted or unsubstituted cycloalkenylsulfonyl (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino.); substituted or unsubstituted heteroarylsulfonyl (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino.); substituted or unsubstituted heterocyclylsulfonyl (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino.); substituted or unsubstituted alkylthio (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino.);
   substituted or unsubstituted arylthio (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino.);
   substituted or unsubstituted cycloalkylthio (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino.);
   substituted or unsubstituted cycloalkenylthio (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino.); substituted or unsubstituted heteroarylthio (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino.); substituted or unsubstituted heterocyclylthio (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino.); substituted or unsubstituted sulfamoyl (when substituted, substituents include halogen, hydroxy, carboxy, nitro, cyano, alkyl, alkenyl, alkynyl, aryl, cycloalkyl, cycloalkenyl, heteroaryl, heterocyclyl.);
   substituted or unsubstituted alkyloxycarbonyl (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino. e.g., methoxycarbonyl, ethoxycarbonyl, or tert-butoxycarbonyl);
   substituted or unsubstituted aryloxycarbonyl (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino.); substituted or unsubstituted cycloalkyloxycarbonyl (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino.); substituted or unsubstituted cycloalkenyloxycarbonyl (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino.);
   substituted or unsubstituted heteroaryloxycarbonyl (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino.);
   substituted or unsubstituted heterocyclyloxycarbonyl (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino.);
   substituted or unsubstituted alkylsulfinyl (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino.);
   substituted or unsubstituted arylsulfinyl (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino.);
   substituted or unsubstituted cycloalkylsulfinyl (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino.); substituted or unsubstituted cycloalkenylsulfinyl (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino.); substituted or unsubstituted heteroarylsulfinyl (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino.); substituted or unsubstituted heterocyclylsulfinyl (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino.); nitroso;
   azido;
   isocyano; isocyanato; thiocyanato; isothiocyanato; mercapto; formyloxy; haloformyl; oxalo; thioformyl; thiocarboxy; dithiocarboxy; thiocarbamoyl; sulfino; sulfo; sulfoamino; hydrazino; ureido; amidino; guanidino; phthalimido; oxo; SF₅; R^{S}R^{S'}(O=)S=N-; R^{S}R^{S'}(O=)S=N-R^{2f}-; R^{S}R^{S'}(O=)S=N-C(=O)-; (R^{N})N=S(=O)(R^{S})-; (R^{N})N=S(=O)(R^{S})-R^{2f}-; R^{S}R^{S'}(R^{N'}-N=)S=N-; ((R^{N})N=)₂S(R^{S})-; (R^{N}R^{N'})N-C(=O)-O-; R^{O}O-C(=O)-N(R^{N})-; R^{O}O-C(=O)-O-; R^{S}(R^{N}R^{N'}N)(O=)S=N-; R^{S}(R^{N}R^{N'}N)(O=)S=N-R^{2f}-; (R^{N"})N=S(=O)(NR^{N}R^{N'})-; (R^{N"})N=S(=O)(NR^{N}R^{N'})-R^{2f}-;

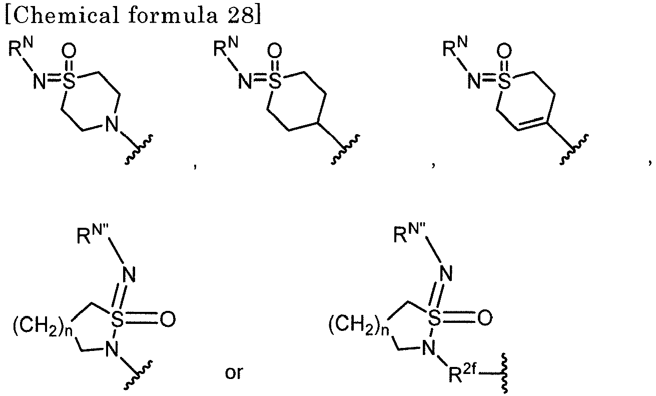

The above-described substituted groups may have one to four of these substituents.

Preferred examples of substituents of "substituted carbamoyl", "substituted sulfamoyl" or "substituted amino" include
substituted or unsubstituted alkyl (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino. e.g., methyl, ethyl, isopropyl, tert-butyl, or CF₃);
substituted or unsubstituted alkenyl (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino. e.g., vinyl); substituted or unsubstituted aryl (when substituted, substituents include halogen, hydroxy, carboxy, nitro, cyano, alkyl, alkenyl, alkynyl, aryl, cycloalkyl,
cycloalkenyl, heteroaryl, heterocyclyl, carbamoyl, sulfamoyl, amino. e.g., phenyl, or naphthyl);
substituted or unsubstituted cycloalkyl (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino. e.g., cyclopropyl, or cyclobutyl);
substituted or unsubstituted cycloalkenyl (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino. e.g., cyclopropenyl);
substituted or unsubstituted heteroaryl (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino.);
substituted or unsubstituted heterocyclyl (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino.);
substituted or unsubstituted arylalkyl (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino.);
substituted or unsubstituted alkyloxy (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino. e.g., methoxy, or ethoxy);
substituted or unsubstituted aryloxy (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino. e.g., phenyloxy);
substituted or unsubstituted cycloalkyloxy (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino.);
substituted or unsubstituted cycloalkenyloxy (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino.); substituted or unsubstituted heteroaryloxy (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino.);
substituted or unsubstituted heterocyclyloxy (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino.); substituted or unsubstituted acyl (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino.);
substituted or unsubstituted alkyloxycarbonyl (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino. e.g., methoxycarbonyl, ethoxycarbonyl, or tert-butoxycarbonyl);
substituted or unsubstituted aryloxycarbonyl (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino.); substituted or unsubstituted cycloalkyloxycarbonyl (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino.); substituted or unsubstituted cycloalkenyloxycarbonyl (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino.);
substituted or unsubstituted heteroaryloxycarbonyl (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino.);
substituted or unsubstituted heterocyclyloxycarbonyl (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino.);
substituted or unsubstituted sulfamoyl (when substituted, substituents include halogen, hydroxy, carboxy, nitro, cyano, alkyl, alkenyl, alkynyl, aryl, cycloalkyl, cycloalkenyl, heteroaryl, heterocyclyl.);
substituted or unsubstituted alkylsulfonyl (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino. e.g., methanesulfonyl, or ethanesulfonyl);
substituted or unsubstituted arylsulfonyl (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino.);
substituted or unsubstituted heteroarylsulfonyl (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino.); substituted or unsubstituted cycloalkylsulfonyl (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino.); substituted or unsubstituted cycloalkenylsulfonyl (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino.); substituted or unsubstituted heterocyclylsulfonyl (when substituted, substituents include halogen, hydroxy, carboxy, nitro, cyano, alkyl, alkenyl, alkynyl, aryl, cycloalkyl, cycloalkenyl, heteroaryl, heterocyclyl, carbamoyl, sulfamoyl, amino.); substituted or unsubstituted carbamoyl (when substituted, substituents include halogen, hydroxy, carboxy, nitro, cyano, alkyl, alkenyl, alkynyl, aryl, cycloalkyl, cycloalkenyl, heteroaryl, heterocyclyl.);
halogen; hydroxy; carboxy; nitro; cyano; alkylsulfinyl; cycloalkylsulfinyl; cycloalkenylsulfinyl; arylsulfinyl; heteroarylsulfinyl; heterocyclylsulfinyl; amino; SF₅; R^{S}R^{S'}(O=)S=N-; R^{S}R^{S'}(O=)S=N-R^{2f}-; R^{S}R^{S'}(O=)S=N-C(=O)-; (R^{N})N=S(=O)(R^{S})- ; (R^{N})N=S(=O)(R^{S})-R^{2f}-; R^{S}R^{S'}(R^{N'}-N=)S=N-; ((R^{N})N=)₂S(R^{S})-; (R^{N}R^{N'})N-C(=O)-O-; R^{O}O-C(=O)-N(R^{N})-; R^{O}O-C(=O)-O-; R^{S}(R^{N}R^{N'}N)(O=)S=N-; R^{S}(R^{N}R^{N'}N)(O=)S=N-R^{2f}-;
(R^{N"})N=S(=O)(NR^{N}R^{N'})-; (R^{N"})N=S(=O)(NR^{N}R^{N'})-R^{2f}-;

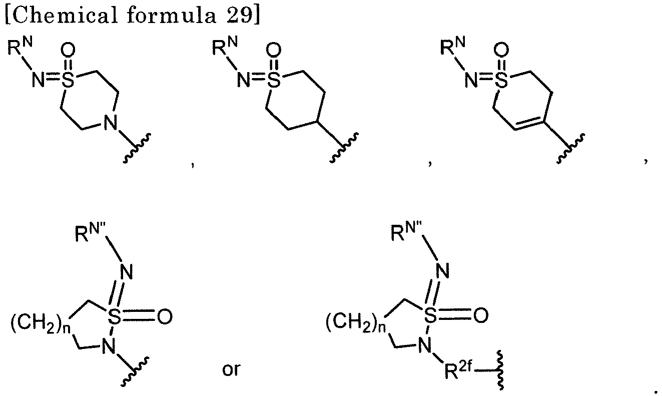

The alkyl parts of "alkylamino", "arylalkylamino", "alkyloxycarbonylamino", "alkylsulfonylamino", "alkylcarbamoyl", "alkylsulfonylcarbamoyl", "heteroarylalkylcarbamoyl", "alkyloxycarbamoyl", "alkyloxycarbonyl" and "arylalkyl" mean the above "alkyl".

The alkenyl part of "alkenyloxy" means the above "alkenyl".

The aryl parts of "arylalkyl", "arylamino", "arylalkylamino", "arylsulfonylamino", "aryloxycarbonyl" and "arylsulfinyl" mean the above "aryl".

The heteroaryl parts of "heteroarylamino", "heteroarylsulfonylamino", "heteroarylalkylcarbamoyl", "heteroaryloxycarbonyl" and "heteroarylsulfinyl" mean the above "heteroaryl".

The cycloalkyl parts of "cycloalkylamino", "cycloalkylsulfonylamino", "cycloalkyloxycarbonyl" and "cycloalkylsulfinyl" mean the above "cycloalkyl".

The cycloalkenyl parts of "cycloalkenylamino", "cycloalkenylsulfonylamino", "cycloalkenyloxycarbonyl" and "cycloalkenylsulfinyl" mean the above "cycloalkenyl".

The heterocyclyl parts of "heterocyclylamino", "heterocyclylsulfonylamino", "heterocyclyloxycarbonyl" and "heterocyclylsulfinyl" mean the above "heterocyclyl".

Among the compounds of the present invention, the compounds in the following embodiments are preferred.

L is -NR¹-, =N-, -O-, -S-, -SO₂-, -CR²R³-, or =CR²-.

Preferably, L is -NR¹-, =N-, -O-, or =CR²-.

Further preferably, L is -NR¹- or -O-.

R¹ is hydrogen, halogen, hydroxy, cyano, nitro, carboxy, substituted or unsubstituted alkyl, substituted or unsubstituted alkenyl, substituted or unsubstituted alkynyl, substituted or unsubstituted aryl, substituted or unsubstituted heteroaryl, substituted or unsubstituted cycloalkyl, substituted or unsubstituted cycloalkenyl, substituted or unsubstituted heterocyclyl, substituted or unsubstituted acyl, substituted or unsubstituted carbamoyl, substituted or unsubstituted alkylsulfonyl, substituted or unsubstituted alkenylsulfonyl, substituted or unsubstituted alkynylsulfonyl, substituted or unsubstituted arylsulfonyl, substituted or unsubstituted heteroarylsulfonyl, substituted or unsubstituted cycloalkylsulfonyl, substituted or unsubstituted cycloalkenylsulfonyl, substituted or unsubstituted heterocyclylsulfonyl, or substituted or unsubstituted sulfamoyl;
R² and R³ are each independently hydrogen, halogen, hydroxy, cyano, nitro, carboxy, substituted or unsubstituted alkyl, substituted or unsubstituted alkenyl, substituted or unsubstituted alkynyl, substituted or unsubstituted aryl, substituted or unsubstituted heteroaryl, substituted or unsubstituted cycloalkyl, substituted or unsubstituted cycloalkenyl, substituted or unsubstituted heterocyclyl, substituted or unsubstituted alkyloxy, substituted or unsubstituted aryloxy, substituted or unsubstituted heteroaryloxy, substituted or unsubstituted cycloalkyloxy, substituted or unsubstituted cycloalkenyloxy, substituted or unsubstituted heterocyclyloxy, substituted or unsubstituted alkylthio, substituted or unsubstituted arylthio, substituted or unsubstituted heteroarylthio, substituted or unsubstituted cycloalkylthio, substituted or unsubstituted cycloalkenylthio, substituted or unsubstituted heterocyclylthio, substituted or unsubstituted alkylsulfonyl, substituted or unsubstituted arylsulfonyl, substituted or unsubstituted heteroarylsulfonyl, substituted or unsubstituted cycloalkylsulfonyl, substituted or unsubstituted cycloalkenylsulfonyl, substituted or unsubstituted heterocyclylsulfonyl, substituted or unsubstituted acyl, substituted or unsubstituted carbamoyl, substituted or unsubstituted sulfamoyl, or substituted or unsubstituted amino.

Preferably, R² and R³ are hydrogen, or substituted or unsubstituted alkyl.

Further preferably, R² and R³ are hydrogen.

Y is substituted or unsubstituted heteroaryl, or substituted or unsubstituted heterocyclyl.

Preferably, Y is substituted or unsubstituted heterocyclyl.

Further preferably, Y is

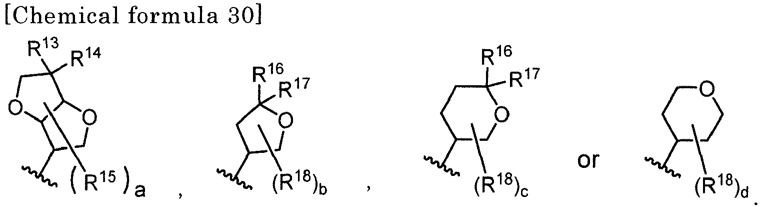

R¹³ and R¹⁴ are each independently hydrogen, halogen, hydroxy, cyano, nitro, carboxy, substituted or unsubstituted alkyl, substituted or unsubstituted alkenyl, substituted or unsubstituted alkynyl, substituted or unsubstituted alkyloxy, substituted or unsubstituted alkylthio, substituted or unsubstituted alkylsulfonyl, substituted or unsubstituted acyl, substituted or unsubstituted carbamoyl, substituted or unsubstituted sulfamoyl, or substituted or unsubstituted amino.

Preferably, one of R¹³ and R¹⁴ is hydroxy, and the other of R¹³ and R¹⁴ is hydrogen, halogen, hydroxy, cyano, nitro, carboxy, substituted or unsubstituted alkyl, substituted or unsubstituted alkenyl, substituted or unsubstituted alkynyl, substituted or unsubstituted alkyloxy, substituted or unsubstituted alkylthio, substituted or unsubstituted alkylsulfonyl, substituted or unsubstituted acyl, substituted or unsubstituted carbamoyl, substituted or unsubstituted sulfamoyl, or substituted or unsubstituted amino.

Further preferably, one of R¹³ and R¹⁴ is hydroxy, and the other of R¹³ and R¹⁴ is hydrogen, or substituted or unsubstituted alkyl.

Particularly preferably, one of R¹³ and R¹⁴ is hydroxy, and the other of R¹³ and R¹⁴ is hydrogen.

R¹⁵ is each independently halogen, hydroxy, cyano, nitro, carboxy, substituted or unsubstituted alkyl, substituted or unsubstituted alkenyl, substituted or unsubstituted alkynyl, substituted or unsubstituted alkyloxy, substituted or unsubstituted alkylthio, substituted or unsubstituted alkylsulfonyl, substituted or unsubstituted acyl, substituted or unsubstituted carbamoyl, substituted or unsubstituted sulfamoyl, or substituted or unsubstituted amino.
a is an integer from 0 to 7. Preferably, a is 0 or 1. Further preferably, a is 0.

R¹⁶ and R¹⁷ are each independently hydrogen, halogen, hydroxy, cyano, nitro, carboxy, substituted or unsubstituted alkyl, substituted or unsubstituted alkenyl, substituted or unsubstituted alkynyl, substituted or unsubstituted alkyloxy, substituted or unsubstituted alkylthio, substituted or unsubstituted alkylsulfonyl, substituted or unsubstituted acyl, substituted or unsubstituted carbamoyl, substituted or unsubstituted sulfamoyl, or substituted or unsubstituted amino.

Preferably, one of R¹⁶ and R¹⁷ is hydrogen, and the other of R¹⁶ and R¹⁷ is hydrogen, or substituted or unsubstituted alkyl.

Further preferably, one of R¹⁶ and R¹⁷ is hydrogen, and the other of R¹⁶ and R¹⁷ is substituted or unsubstituted alkyl.

R¹⁸ is each independently halogen, hydroxy, cyano, nitro, carboxy, substituted or unsubstituted alkyl, substituted or unsubstituted alkenyl, substituted or unsubstituted alkynyl, substituted or unsubstituted alkyloxy, substituted or unsubstituted alkylthio, substituted or unsubstituted alkylsulfonyl, substituted or unsubstituted acyl, substituted or unsubstituted carbamoyl, substituted or unsubstituted sulfamoyl, or substituted or unsubstituted amino.

Preferably, R¹⁸ is each independently halogen, hydroxy, or substituted or unsubstituted alkyloxy.

b is an integer from 0 to 5. Preferably, b is an integer from 0 to 2.

c is an integer from 0 to 7. Preferably, c is an integer from 0 to 2.

d is an integer from 0 to 9. Preferably, d is an integer from 0 to 3.

X is -CR⁴R⁵- or -O-.

R⁴ and R⁵ are each independently hydrogen, halogen, hydroxy, cyano, nitro, carboxy, substituted or unsubstituted alkyl, substituted or unsubstituted alkenyl, substituted or unsubstituted alkynyl, substituted or unsubstituted aryl, substituted or unsubstituted heteroaryl, substituted or unsubstituted cycloalkyl, substituted or unsubstituted cycloalkenyl, substituted or unsubstituted heterocyclyl, substituted or unsubstituted alkyloxy, substituted or unsubstituted aryloxy, substituted or unsubstituted heteroaryloxy, substituted or unsubstituted cycloalkyloxy, substituted or unsubstituted cycloalkenyloxy, substituted or unsubstituted heterocyclyloxy, substituted or unsubstituted alkylthio, substituted or unsubstituted arylthio, substituted or unsubstituted heteroarylthio, substituted or unsubstituted cycloalkylthio, substituted or unsubstituted cycloalkenylthio, substituted or unsubstituted heterocyclylthio, substituted or unsubstituted alkylsulfonyl, substituted or unsubstituted arylsulfonyl, substituted or unsubstituted heteroarylsulfonyl, substituted or unsubstituted cycloalkylsulfonyl, substituted or unsubstituted cycloalkenylsulfonyl, substituted or unsubstituted heterocyclylsulfonyl, substituted or unsubstituted acyl, substituted or unsubstituted carbamoyl, substituted or unsubstituted sulfamoyl, or substituted or unsubstituted amino.

Preferably, R⁴ and R⁵ are each independently hydrogen, or halogen.

Z is =CR⁶- or =N-.

R⁷, R⁸ and R⁹ are each independently hydrogen, halogen, hydroxy, cyano, nitro, carboxy, substituted or unsubstituted alkyl, substituted or unsubstituted alkenyl, substituted or unsubstituted alkynyl, substituted or unsubstituted aryl, substituted or unsubstituted heteroaryl, substituted or unsubstituted cycloalkyl, substituted or unsubstituted cycloalkenyl, substituted or unsubstituted heterocyclyl, substituted or unsubstituted alkyloxy, substituted or unsubstituted aryloxy, substituted or unsubstituted heteroaryloxy, substituted or unsubstituted cycloalkyloxy, substituted or unsubstituted cycloalkenyloxy, substituted or unsubstituted heterocyclyloxy, substituted or unsubstituted alkylthio, substituted or unsubstituted arylthio, substituted or unsubstituted heteroarylthio, substituted or unsubstituted cycloalkylthio, substituted or unsubstituted cycloalkenylthio, substituted or unsubstituted heterocyclylthio, substituted or unsubstituted alkylsulfonyl, substituted or unsubstituted arylsulfonyl, substituted or unsubstituted heteroarylsulfonyl, substituted or unsubstituted cycloalkylsulfonyl, substituted or unsubstituted cycloalkenylsulfonyl, substituted or unsubstituted heterocyclylsulfonyl, substituted or unsubstituted carbamoyl, substituted or unsubstituted sulfamoyl, substituted or unsubstituted amino, R^{S}R^{S'}(O=)S=N-, R^{S}R^{S'}(O=)S=N-R^{2f}-, R^{S}R^{S'}(O=)S=N-C(=O)-, (R^{N})N=S(=O)(R^{S})-, (R^{N})N=S(=O)(R^{S})-R^{2f}-, R^{S}R^{S'}(R^{N'}-N=)S=N-, ((R^{N})N=)₂S(R^{S})-, (R^{N}R^{N'})N-C(=O)-O-, R^{O}O-C(=O)-N(R^{N})-, R^{O}O-C(=O)-O-, R^{S}(R^{N}R^{N'}N)(O=)S=N-, R^{S}(R^{N}R^{N'}N)(O=)S=N-R^{2f}-, (R^{N"})N=S(=O)(NR^{N}R^{N'})-, (R^{N"})N=S(=O)(NR^{N}R^{N'})-R^{2f}-,

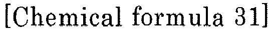

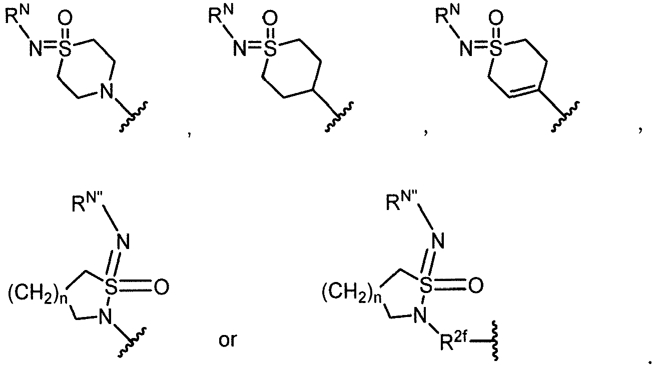

Preferably, R⁷, R⁸ and R⁹ are each independently hydrogen, halogen, hydroxy, cyano, nitro, carboxy, substituted or unsubstituted alkyl, substituted or unsubstituted alkenyl, substituted or unsubstituted alkynyl, substituted or unsubstituted aryl, substituted or unsubstituted heteroaryl, substituted or unsubstituted cycloalkyl, substituted or unsubstituted cycloalkenyl, substituted or unsubstituted heterocyclyl, substituted or unsubstituted alkyloxy, substituted or unsubstituted aryloxy, substituted or unsubstituted heteroaryloxy, substituted or unsubstituted cycloalkyloxy, substituted or unsubstituted cycloalkenyloxy, substituted or unsubstituted heterocyclyloxy, substituted or unsubstituted alkylsulfonyl, or substituted or unsubstituted amino.

Further preferably, R⁷, R⁸ and R⁹ are each independently hydrogen, halogen, substituted or unsubstituted alkyl, substituted or unsubstituted alkenyl, substituted or unsubstituted aryl, substituted or unsubstituted heteroaryl, substituted or unsubstituted heterocyclyl, substituted or unsubstituted alkyloxy, substituted or unsubstituted aryloxy, substituted or unsubstituted heteroaryloxy, substituted or unsubstituted cycloalkyloxy, substituted or unsubstituted cycloalkenyloxy, substituted or unsubstituted heterocyclyloxy, substituted or unsubstituted alkylsulfonyl, or substituted or unsubstituted amino.

More preferably, R⁷, R⁸ and R⁹ are each independently hydrogen, halogen, substituted or unsubstituted alkyl, substituted or unsubstituted alkenyl, substituted or unsubstituted aryl, substituted or unsubstituted heteroaryl, substituted or unsubstituted heterocyclyl, substituted or unsubstituted alkyloxy, substituted or unsubstituted heterocyclyloxy, substituted or unsubstituted alkylsulfonyl, or substituted or unsubstituted amino.

R^{S} and R^{S'} are each independently substituted or unsubstituted alkyl, substituted or unsubstituted cycloalkyl, substituted or unsubstituted heterocyclyl, substituted or unsubstituted aryl, or substituted or unsubstituted heteroaryl. R^{S} and R^{S'} bound to the same sulfur atom may form a substituted or unsubstituted ring together with the sulfur atom.

Preferably, R^{S} and R^{S'} are each independently substituted or unsubstituted alkyl.

The ring, which is formed by R^{S} and R^{S'} which are bound to the same sulfur atom, together with the sulfur atom, means a 3 to 15-membered saturated or unsaturated hetero ring that may contain one to four oxygen, nitrogen and/or sulfur atom(s) in the ring, other than the sulfur atom. Preferred is a non-aromatic ring, and such non-aromatic ring may be further cross-linked by a C1 to C4 alkyl chain, and may be fused with cycloalkane (preferably 5 to 6-membered) and a benzene ring. Examples thereof include as follows.

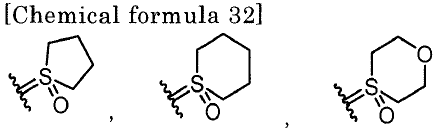

R^{2f} is substituted or unsubstituted alkylene.

R^{N} is each independently hydrogen, cyano, substituted or unsubstituted alkyl, substituted or unsubstituted alkylcarbonyl, substituted or unsubstituted alkyloxycarbonyl, substituted or unsubstituted cycloalkyl, substituted or unsubstituted cycloalkylcarbonyl, substituted or unsubstituted heterocyclyl, substituted or unsubstituted heterocyclylcarbonyl, substituted or unsubstituted aryl, substituted or unsubstituted arylcarbonyl, substituted or unsubstituted heteroaryl, substituted or unsubstituted heteroarylcarbonyl, or substituted or unsubstituted carbamoyl.

In the case of -S(R^{S})(=N-R^{N})₂, R^{N} together with the adjacent nitrogen atom may form a substituted or unsubstituted ring.

Preferably, R^{N} is hydrogen, substituted or unsubstituted alkyl, or substituted or unsubstituted carbamoyl.

In the case of -S(R^{S})(=N-R^{N})₂, examples of the ring formed by R^{N} together with the adjacent nitrogen atom include as follows.

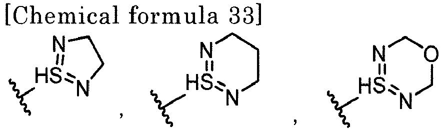

R^{N'} is hydrogen, cyano, substituted or unsubstituted alkyl, substituted or unsubstituted alkylcarbonyl, substituted or unsubstituted alkyloxycarbonyl, substituted or unsubstituted cycloalkyl, substituted or unsubstituted cycloalkylcarbonyl, substituted or unsubstituted heterocyclyl, substituted or unsubstituted heterocyclylcarbonyl, substituted or unsubstituted aryl, substituted or unsubstituted arylcarbonyl, substituted or unsubstituted heteroaryl, substituted or unsubstituted heteroarylcarbonyl, or substituted or unsubstituted carbamoyl.

Preferably, R^{N'} is hydrogen, cyano, substituted or unsubstituted alkyl, substituted or unsubstituted cycloalkyl, substituted or unsubstituted aryl, or substituted or unsubstituted heteroaryl.

R^{O} is hydrogen, substituted or unsubstituted alkyl, substituted or unsubstituted alkenyl, substituted or unsubstituted alkynyl, substituted or unsubstituted aryl, substituted or unsubstituted heteroaryl, substituted or unsubstituted cycloalkyl, substituted or unsubstituted cycloalkenyl, or substituted or unsubstituted heterocyclyl.

R¹⁰ and R¹¹ are each independently hydrogen, halogen, hydroxy, cyano, nitro, carboxy, substituted or unsubstituted alkyl, substituted or unsubstituted alkenyl, substituted or unsubstituted alkynyl, substituted or unsubstituted aryl, substituted or unsubstituted heteroaryl, substituted or unsubstituted cycloalkyl, substituted or unsubstituted cycloalkenyl, substituted or unsubstituted heterocyclyl, substituted or unsubstituted alkyloxy, substituted or unsubstituted aryloxy, substituted or unsubstituted heteroaryloxy, substituted or unsubstituted cycloalkyloxy, substituted or unsubstituted cycloalkenyloxy, substituted or unsubstituted heterocyclyloxy, substituted or unsubstituted alkylthio, substituted or unsubstituted arylthio, substituted or unsubstituted heteroarylthio, substituted or unsubstituted cycloalkylthio, substituted or unsubstituted cycloalkenylthio, substituted or unsubstituted heterocyclylthio, substituted or unsubstituted alkylsulfonyl, substituted or unsubstituted arylsulfonyl, substituted or unsubstituted heteroarylsulfonyl, substituted or unsubstituted cycloalkylsulfonyl, substituted or unsubstituted cycloalkenylsulfonyl, substituted or unsubstituted heterocyclylsulfonyl, substituted or unsubstituted acyl, substituted or unsubstituted carbamoyl, substituted or unsubstituted sulfamoyl, or substituted or unsubstituted amino.

Preferably, R¹⁰ is hydrogen, halogen, cyano, carboxy, or substituted or unsubstituted alkyl.

Further preferably, R¹⁰ is hydrogen, fluoro, chloro, cyano or substituted alkyl, and the substituent of the substituted alkyl is halogen.

More preferably, R¹⁰ is fluoro, or chloro.

Preferably, R¹¹ is hydrogen.

R¹² is hydrogen, or substituted or unsubstituted alkyl.
More preferably, R¹² is hydrogen.

Preferred combinations of substituents of a compound represented by formula (I) include the following 1) to 4):
1) A compound wherein L is NR¹, R¹ is hydrogen, X is -CR⁴R⁵-, R⁴ and R⁵ are each independently hydrogen or halogen, Y is substituted or unsubstituted heterocyclyl, Z is =CR⁶-, R⁶ is hydrogen or halogen, R⁷ is hydrogen, R⁸ is halogen, substituted or unsubstituted alkyl, substituted or unsubstituted alkenyl, substituted or unsubstituted aryl, substituted or unsubstituted heteroaryl, substituted or unsubstituted heterocyclyl, substituted or unsubstituted alkyloxy, substituted or unsubstituted heterocyclyloxy, substituted or unsubstituted alkylsulfonyl or substituted or unsubstituted amino, R⁹ is hydrogen, R¹⁰ is hydrogen, halogen or substituted or unsubstituted alkyl, R¹¹ is hydrogen, and R¹² is hydrogen.
2) A compound wherein L is NR¹, R¹ is hydrogen, X is -CR⁴R⁵-, R⁴ and R⁵ are each independently hydrogen or halogen, Y is substituted or unsubstituted heterocyclyl, Z is =N-, R⁷ is hydrogen, R⁸ is halogen, substituted or unsubstituted alkyl, substituted or unsubstituted alkenyl, substituted or unsubstituted aryl, substituted or unsubstituted heteroaryl, substituted or unsubstituted heterocyclyl, substituted or unsubstituted alkyloxy, substituted or unsubstituted heterocyclyloxy, substituted or unsubstituted alkylsulfonyl or substituted or unsubstituted amino, R⁹ is hydrogen, R¹⁰ is hydrogen, halogen or substituted or unsubstituted alkyl, R¹¹ is hydrogen, and R¹² is hydrogen.
3) A compound wherein L is -O-, X is -CR⁴R⁵-, R⁴ and R⁵ are each independently hydrogen or halogen, Y is substituted or unsubstituted heterocyclyl, Z is =CR⁶-, R⁶ is hydrogen or halogen, R⁷ is hydrogen, R⁸ is halogen, substituted or unsubstituted alkyl, substituted or unsubstituted alkenyl, substituted or unsubstituted aryl, substituted or unsubstituted heteroaryl, substituted or unsubstituted heterocyclyl, substituted or unsubstituted alkyloxy, substituted or unsubstituted heterocyclyloxy, substituted or unsubstituted alkylsulfonyl or substituted or unsubstituted amino, R⁹ is hydrogen, R¹⁰ is hydrogen, halogen or substituted or unsubstituted alkyl, R¹¹ is hydrogen, and R¹² is hydrogen.
4) A compound wherein L is -O-, X is -CR⁴R⁵-, R⁴ and R⁵ are each independently hydrogen or halogen, Y is substituted or unsubstituted heterocyclyl, Z is =N-, R⁷ is hydrogen, R⁸ is halogen, substituted or unsubstituted alkyl, substituted or unsubstituted alkenyl, substituted or unsubstituted aryl, substituted or unsubstituted heteroaryl, substituted or unsubstituted heterocyclyl, substituted or unsubstituted alkyloxy, substituted or unsubstituted heterocyclyloxy, substituted or unsubstituted alkylsulfonyl or substituted or unsubstituted amino, R⁹ is hydrogen, R¹⁰ is hydrogen, halogen or substituted or unsubstituted alkyl, R¹¹ is hydrogen, and R¹² is hydrogen.

One or more hydrogen, carbon or other atoms of the compounds of formula (I) of the present invention can be replaced by an isotope of the hydrogen, carbon or other atoms.

For example, the compounds of formula (I) include all radiolabeled forms of compounds of formula (I). Such "radioactive labeling," "radiolabeled form" and the like of the compounds of formula (I) are encompassed by the present invention and useful as a research and/or diagnostic tool in metabolism pharmacokinetic studies and in binding assays. Examples of isotopes that can be incorporated into a compound of formula (I) of the present invention include isotopes of hydrogen, carbon, nitrogen, oxygen, phosphorus, sulfur, fluorine and chlorine, such as ²H, ³H, ¹³C, ¹⁴C, ¹⁵N, ¹⁸O, ¹⁷O, ³¹P, ³²P, ³⁵S, ¹⁸F and ³⁶Cl, respectively. Radiolabeled compounds of the present invention can be prepared by methods well-known in the art. For example, tritium-labeled compounds of formula (I) can be prepared by introducing tritium into a particular compound of formula (I), for example, by catalytic dehalogenation with tritium. This method may include reacting a suitably halogen-substituted precursor of a compound of formula (I) with tritium gas in the presence of a suitable catalyst such as Pd/C, in the presence or absence of a base. Other suitable methods for preparing tritiated compounds can be found in Isotopes in the Physical and Biomedical Sciences, Vol. 1, Labeled Compounds (Part A), Chapter 6 (1987). ¹⁴C-labeled compounds can be prepared by employing starting materials having a ¹⁴C carbon.

As a pharmaceutically acceptable salt of the compound of the present invention, the following salts can be included.

As a basic salt, examples include alkali metal salts such as sodium salts and potassium salts; alkaline earth metal salts such as calcium salts and strontium salts; beryllium salts, magnesium salts; transition metal salts such as zinc salts; ammonium salts; aliphatic amine salts such as trimethylamine salts, triethylamine salts, dicyclohexylamine salts, ethanolamine salts, diethanolamine salts, triethanolamine salts, procaine salts, meglumine salts, diethanolamine salts and ethylenediamine salts; aralkylamine salts such as N,N-dibenzylethylenediamine and benethamine salts; heterocyclic aromatic amine salts such as pyridine salts, picoline salts, quinoline salts, and isoquinoline salts; quaternary ammonium salts such as tetramethylammonium salts, tetraethylammonium salts, benzyltrimethylammonium salts, benzyltriethylammonium salts, benzyltributylammonium salts, methyltrioctylammonium salts, and tetrabutylammonium salts; basic amino acid salts such as arginine salts and lysine salts; and the like.

As an acidic salt, examples include inorganic acid salts such as hydrochlorides, sulfates, nitrates, phosphates, carbonates, hydrogencarbonates, and perchlorates; organic acid salts such as acetates, propionates, lactates, maleates, fumarates, tartrates, malates, citrates and ascorbates; sulfonate salts such as methanesulfonates, isethionates, benzenesulfonates and p-toluenesulfonates; acidic amino acid salts such as aspartates and glutamates; and the like.

A compound represented by formula (I) of the present invention or its pharmaceutically acceptable salt may form a solvate (e.g., hydrate, etc.), cocrystal and/or a crystal polymorph, and the present invention also contains such various types of solvates, cocrystal and crystal polymorphs. In a "solvate", any number of solvent molecules (e.g., water molecule, etc.) may be coordinated with a compound represented by formula (I). When left in the atmosphere, a compound represented by formula (I) or its pharmaceutically acceptable salt may absorb water, and a case where adsorbed water is attached thereto or a case where hydrate is formed may arise. In addition, by recrystallization of a compound represented by formula (I) or its pharmaceutically acceptable salt, a crystal polymorph thereof can be formed. The "cocrystal" means that a compound represented by formula (I) or its salt and a counter molecule are present in the same crystal lattice, and may be formed with any number of counter molecule.

A compound represented by formula (I) of the present invention or its pharmaceutically acceptable salt may form a prodrug, and the present invention also contains such various types of prodrugs. The prodrugs are a derivative of the compound of the present invention, which has a chemically or metabolically decomposable group, and a compound which is changed into the compound of the present invention, which is pharmaceutically active, by solvolysis or in vivo under physiological conditions. The prodrugs contain a compound which is converted into a compound represented by formula (I) by enzymatic oxidation, reduction, hydrolysis and the like in living organisms under physiological conditions; a compound which is converted into a compound represented by formula (I) by hydrolysis by e.g., gastric acid; and the like. A method for selecting and a method for producing a proper prodrug derivative are described in e.g., Design of Prodrugs, Elsevier, Amsterdam 1985. Prodrugs can have activity in themselves.

When a compound represented by formula (I) or its pharmaceutically acceptable salt has a hydroxyl group, prodrugs such as acyloxy derivatives and sulfonyloxy derivatives are exemplified, which derivatives are produced, for example, by a reaction of a compound having a hydroxy group and a proper acyl halide, a proper acid anhydride, a proper sulfonyl chloride, a proper sulfonyl anhydride and a mixed anhydride, or a reaction using a condensing agent. Examples thereof include CH₃COO-, C₂H₅COO-, tert-BuCOO-, C₁₅H₃₁COO-, PhCOO-, (m-NaOOCPh)COO-, NaOOCCH₂CH₂COO-, CH₃CH(NH₂)COO-, CH₂N(CH₃)₂COO-, CH₃SO₃-, CH₃CH₂SO₃-, CF₃SO₃-, CH₂FSO₃-, CF₃CH₂SO₃-, p-CH₃O-PhSO₃-, PhSO₃- and p-CH₃PhSO₃-.

The term "activating" means that the compound of the present invention activates the function of AMPK.

The term "pharmaceutically acceptable" means preventively or therapeutically harmless.

A general method for producing the compound of the present invention will be illustrated below. For extraction, purification and the like, treatment which is carried out in common experiments in organic chemistry may be carried out.

A compound represented by formula (I-1) can be synthesized as follows. 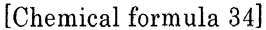 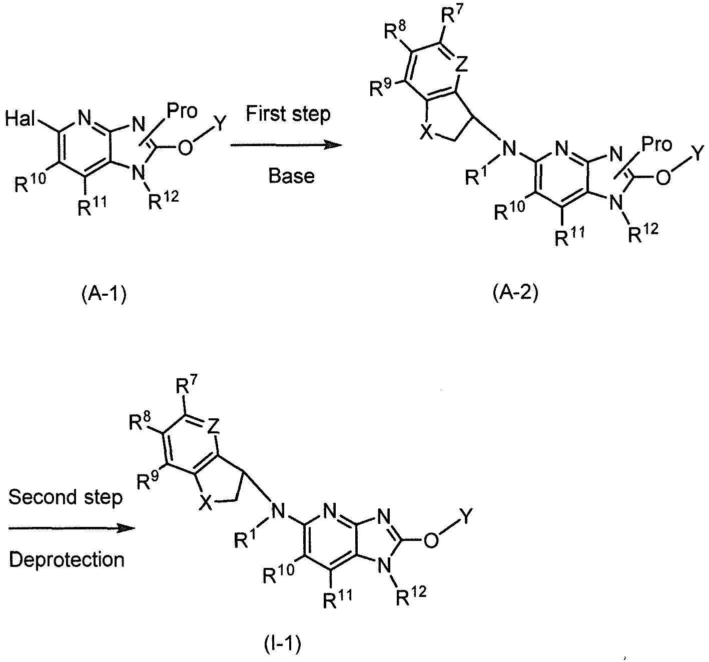 wherein each symbol has the same meaning as above, and as a compound represented by formula (A-1), a known compound can be used, or a compound which is derived from a known compound by a conventional method may be used. "Hal" means a halogen, and Pro means a protecting group. Pro includes a benzyl group, a benzoyl group, a p-methoxybenzyl group, an acetyl group, a benzoyl group, SEM (trimethylsilylethoxymethyl), THP (tetrahydropyran), TBS (tertbutyldimethylsilyl), TBDPS (tert-butyldiphenylsilyl) and the like.

### First step

The first step is a step in which a compound represented by formula (A-2) is produced from a compound represented by formula (A-1).

As a reaction solvent, examples include N,N-dimethylformamide, dimethyl sulfoxide, aromatic hydrocarbons (e.g., toluene, benzene, xylene, etc.), saturated hydrocarbons (e.g., cyclohexane, hexane, etc.), halogenated hydrocarbons (e.g., dichloromethane, chloroform, 1,2-dichloroethane, etc.), ethers (e.g., tetrahydrofuran, diethyl ether, dioxane, 1,2-dimethoxyethane, etc.), esters (e.g., methyl acetate, ethyl acetate, etc.), ketones (e.g., acetone, methyl ethyl ketone, etc.), nitriles (e.g., acetonitrile, etc.), alcohols (e.g., methanol, ethanol, t-butanol, etc.), water, a mixed solvent thereof and the like.

Preferably, aromatic hydrocarbons (e.g., toluene, benzene, xylene, etc.), N,N-dimethylformamide or ethers (e.g., tetrahydrofuran, diethyl ether, dioxane, 1,2-dimethoxyethane, etc.) and the like can be used.

As a base, examples include metal hydrides (e.g., sodium hydride, etc.), metal hydroxides (e.g., sodium hydroxide, potassium hydroxide, lithium hydroxide, barium hydroxide, etc.), metal carbonates (e.g., sodium carbonate, calcium carbonate, cesium carbonate, etc.), metal alkoxides (e.g., sodium methoxide, sodium ethoxide, potassium t-butoxide, etc.), sodium hydrogencarbonate, metal sodium, metal amides, organic amines (e.g., triethylamine, diisopropylethylamine, DBU, 2,6-lutidine, etc.), pyridine, alkyllithiums (n-BuLi, sec-BuLi, tert-BuLi) and the like.

Preferred examples of the base include metal carbonates (e.g., sodium carbonate, calcium carbonate, cesium carbonate, etc.), metal alkoxides (e.g., sodium methoxide, sodium ethoxide, potassium t-butoxide, etc.), and the like.

The reaction may be carried out in the presence of a palladium catalyst (e.g., Pd(PPh₃)₄, PdCl₂, Pd(OAc)₂, Pd(dba)₂, Pd₂(dba)₃, etc.) and a phosphine ligand (e.g., PPh₃, BINAP, Ruphos, etc.).

The reaction can be carried out at 50 to 120°C for 0.5 to 24 hours.

When using microwave, the reaction can be carried out at 80 to 200°C for 5 minutes to 3 hours.

### Second step

The second step is a step in which a compound represented by formula (I-1) is produced by deprotection of a compound represented by formula (A-2).

As a reaction solvent, solvents described for the first step can be used. Preferred examples include halogenated hydrocarbons (e.g., dichloromethane, chloroform, 1,2-dichloroethane, etc.), ethers (e.g., tetrahydrofuran, diethyl ether, dioxane, 1,2-dimethoxyethane, etc.), alcohols (e.g., methanol, ethanol, t-butanol, etc.), water, and a mixed solvent thereof and the like.

The reaction can be carried out in the presence of hydrochloric acid, PPTs (pyridinium p-toluenesulfonate), TFA (trifluoroacetic acid), TBAF (tetrabutylammoniumfluoride) or the like at 0 to 100°C for 0.5 to 24 hours.

Among compounds represented by formula (I), a compound, wherein R¹ is substituted or unsubstituted alkyl, can be synthesized, for example, from a compound represented by formula (I-1) by an alkylation reaction using sodium hydride and an alkyl halide.

A compound represented by formula (1-2) can be synthesized as follows. 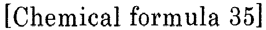 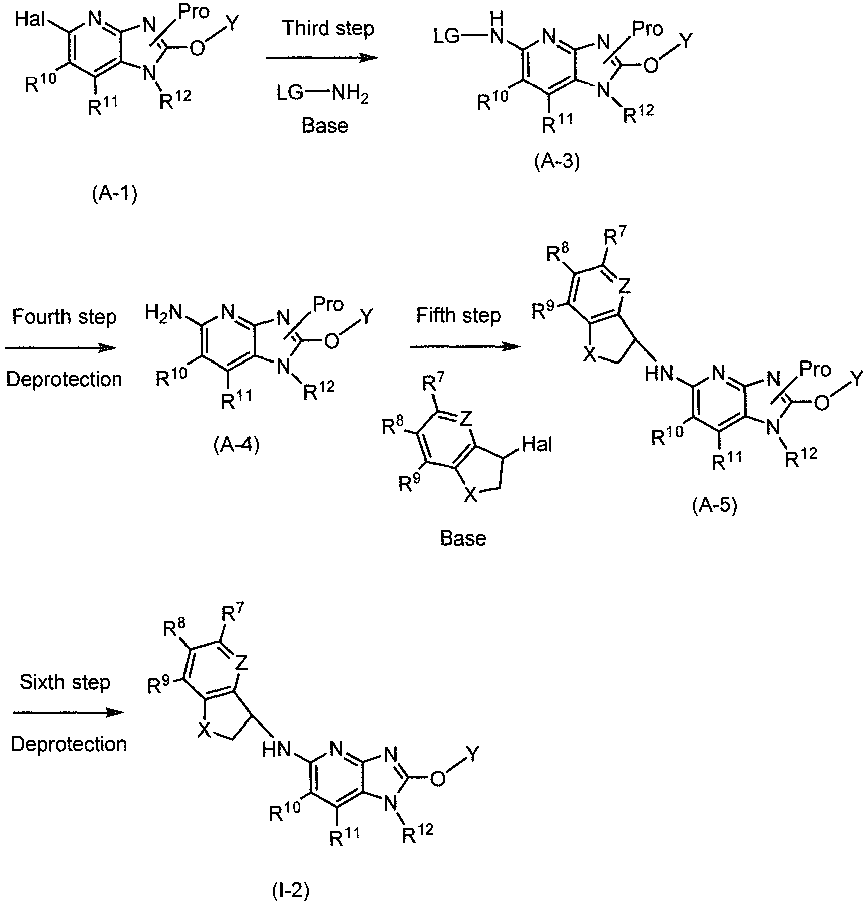 wherein each symbol has the same meaning as above, and as a compound represented by formula (A-1), a known compound can be used, or a compound which is derived from a known compound by a conventional method may be used. "Hal" means a halogen, Pro means a protecting group, and LG means a leaving group. Pro includes a benzyl group, a p-methoxybenzyl group, an acetyl group, a benzoyl group, SEM (trimethylsilylethoxymethyl), THP (tetrahydropyran), TBS (tertbutyldimethylsilyl), TBDPS (tert-butyldiphenylsilyl) and the like. LG includes p-methoxybenzylamine and the like.

### Third step

The third step is a step in which a compound represented by formula (A-3) is produced from a compound represented by formula (A-1).

As a reaction solvent, solvents described for the first step can be used. Preferably, aromatic hydrocarbons (e.g., toluene, benzene, xylene, etc.), N,N-dimethylformamide or ethers (e.g., tetrahydrofuran, diethyl ether, dioxane, 1,2-dimethoxyethane, etc.) and the like can be used.

As a base, examples include metal hydrides (e.g., sodium hydride, etc.), metal hydroxides (e.g., sodium hydroxide, potassium hydroxide, lithium hydroxide, barium hydroxide, etc.), metal carbonates (e.g., sodium carbonate, calcium carbonate, cesium carbonate, etc.), metal alkoxides (e.g., sodium methoxide, sodium ethoxide, potassium t-butoxide, etc.), sodium hydrogencarbonate, metal sodium, metal amides, organic amines (e.g., triethylamine, diisopropylethylamine, DBU, 2,6-lutidine, etc.), pyridine, alkyllithiums (n-BuLi, sec-BuLi, tert-BuLi) and the like.

Preferred examples of the base include metal carbonates (e.g., sodium carbonate, calcium carbonate, cesium carbonate, etc.), metal alkoxides (e.g., sodium methoxide, sodium ethoxide, potassium t-butoxide, etc.), and the like.

The reaction may be carried out in the presence of a palladium catalyst (e.g., Pd(PPh₃)₄, PdCl₂, Pd(OAc)₂, Pd(dba)₂, Pd₂(dba)₃, etc.) and a phosphine ligand (e.g., PPh₃, BINAP, Ruphos, etc.).

The reaction can be carried out at 50 to 120°C for 0.5 to 24 hours.

When using microwave, the reaction can be carried out at 80 to 200°C for 5 minutes to 3 hours.

Examples of a compound represented by formula: LG-NH₂ include p-methoxybenzylamine and the like.

### Fourth step

The fourth step is a step in which a compound represented by formula (A-4) is produced by deprotection of a compound represented by formula (A-3).

As a reaction solvent, solvents described for the first step can be used. Preferred examples include N,N-dimethylformamide, halogenated hydrocarbons (e.g., dichloromethane, chloroform, 1,2-dichloroethane, etc.), ethers (e.g., tetrahydrofuran, diethyl ether, dioxane, 1,2-dimethoxyethane, etc.), alcohols (e.g., methanol, ethanol, t-butanol, etc.), water, and a mixed solvent thereof and the like.

The reaction can be carried out in the presence of hydrochloric acid, PPTs (pyridinium p-toluenesulfonate), TFA (trifluoroacetic acid) or the like at 0 to 100°C for 0.5 to 24 hours.

### Fifth step

The fifth step is a step in which a compound represented by formula (A-5) is produced from a compound represented by formula (A-4).

As a reaction solvent, solvents described for the first step can be used. Preferably, N,N-dimethylformamide, halogenated hydrocarbons (e.g., dichloromethane, chloroform, 1,2-dichloroethane, etc.), ethers (e.g., tetrahydrofuran, diethyl ether, dioxane, 1,2-dimethoxyethane, etc.), esters (e.g., methyl acetate, ethyl acetate, etc.), nitriles (e.g., acetonitrile, etc.), water or the like may be used.

As a base, bases described for the first step can be used. Preferred examples include metal hydrides (e.g., sodium hydride, etc.), metal hydroxides (e.g., sodium hydroxide, potassium hydroxide, lithium hydroxide, barium hydroxide, etc.), metal carbonates (e.g., sodium carbonate, calcium carbonate, cesium carbonate, etc.), metal alkoxides (e.g., sodium methoxide, sodium ethoxide, potassium t-butoxide, etc.), sodium hydrogencarbonate, metal sodium, metal amides, organic amines (e.g., triethylamine, diisopropylethylamine, DBU, 2,6-lutidine, etc.), pyridine, alkyllithiums (n-BuLi, sec-BuLi, tert-BuLi) and the like.

The reaction can be carried out at 0 to 200°C for 0.5 to 24 hours.

### Sixth step

The sixth step is a step in which a compound represented by formula (1-2) is produced by deprotection of a compound represented by formula (A-5).

The reaction may be carried out in the same manner as the second step.

A compound represented by formula (1-3) can be synthesized as follows. 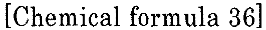 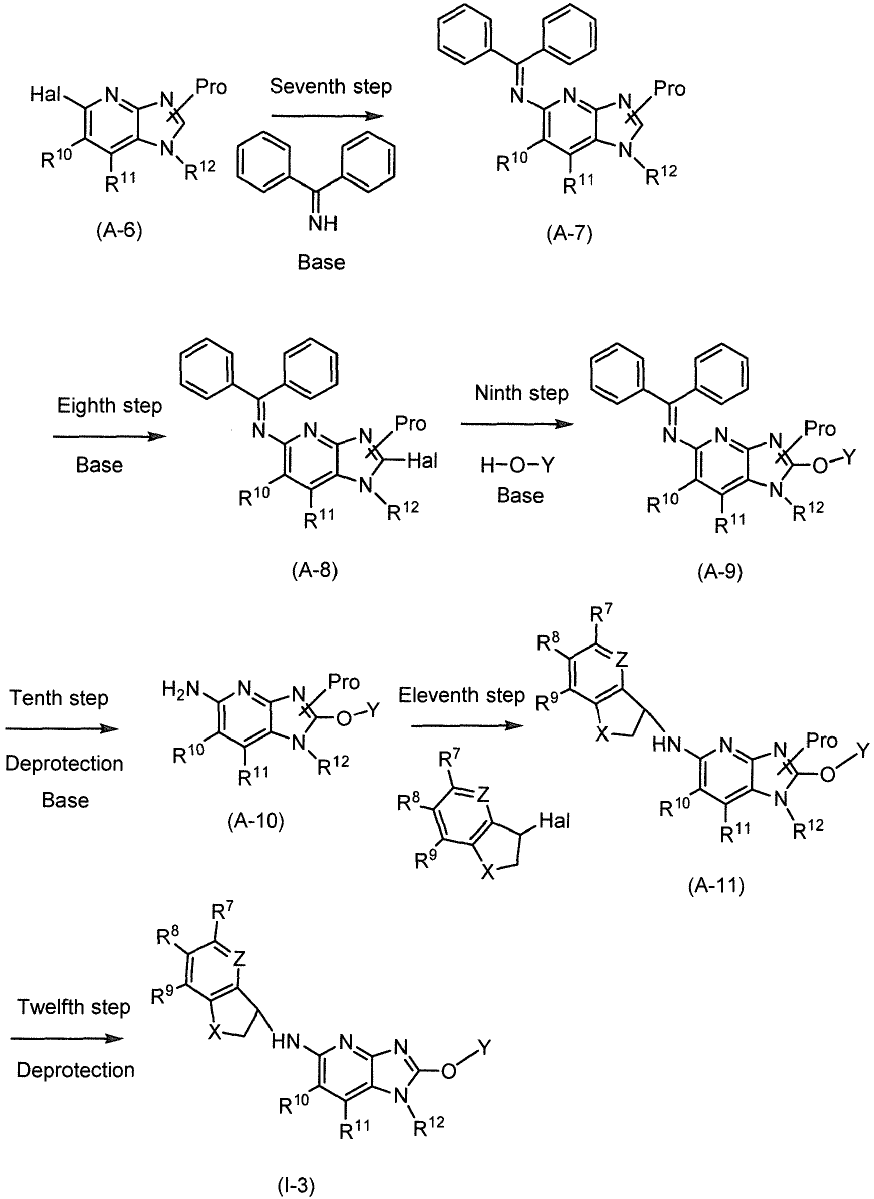 wherein each symbol has the same meaning as above, and as a compound represented by formula (A-6), a known compound can be used and a compound which is derived from a known compound by a conventional method can be used. "Hal" means a halogen, and Pro means a protecting group. Pro includes a benzyl group, a p-methoxybenzyl group, an acetyl group, a benzoyl group, SEM (trimethylsilylethoxymethyl), THP (tetrahydropyran), TBS (tertbutyldimethylsilyl), TBDPS (tert-butyldiphenylsilyl) and the like.

### Seventh step

The seventh step is a step in which a compound represented by formula (A-7) is produced from a compound represented by formula (A-6).

As a reaction solvent, solvents described for the first step can be used. Preferably, aromatic hydrocarbons (e.g., toluene, benzene, xylene, etc.), N,N-dimethylformamide or ethers (e.g., tetrahydrofuran, diethyl ether, dioxane, 1,2-dimethoxyethane, etc.) and the like can be used.

As a base, bases described for the first step can be used. Preferred examples of the base include metal carbonates (e.g., sodium carbonate, calcium carbonate, cesium carbonate, etc.), metal alkoxides (e.g., sodium methoxide, sodium ethoxide, potassium t-butoxide, etc.), and the like.

The reaction may be carried out in the presence of a palladium catalyst (e.g., Pd(PPh₃)₄, PdCl₂, Pd(OAc)₂, Pd(dba)₂, Pd₂(dba)₃, etc.) and a phosphine ligand (e.g., PPh₃, BINAP, Ruphos, dppf, etc.).

The reaction can be carried out at 50 to 120°C for 0.5 to 24 hours.

When using microwave, the reaction can be carried out at 80 to 200°C for 5 minutes to 3 hours.

### Eighth step

The eighth step is a step in which a compound represented by formula (A-8) is produced by halogenation of a compound represented by formula (A-7). As a reaction solvent, solvents described for the first step can be used. Preferred examples include N,N-dimethylformamide, halogenated hydrocarbons (e.g., dichloromethane, chloroform, 1,2-dichloroethane, etc.), ethers (e.g., tetrahydrofuran, diethyl ether, dioxane, 1,2-dimethoxyethane, etc.) or nitriles (e.g., acetonitrile, etc.) and the like. Further preferably, alcohols (e.g., methanol, ethanol, t-butanol, etc.) can be used.

As a base, bases described for the first step can be used. Preferred examples include metal hydrides (e.g., sodium hydride, etc.), metal amides (e.g., lithium hexamethyldisilazide, etc.), alkyllithiums (n-BuLi, sec-BuLi, tert-BuLi) and the like.

The reaction can be carried out at -78 to 50°C for 0.5 to 24 hours.

As a halogenating agent, I₂, Br₂, NIS (N-iodosuccinimide), NBS (N-bromosuccinimide), NCS (N-chlorosuccinimide), CCl₃-CCl₃ (hexachloroethane) and the like can be used.

### Ninth step

The ninth step is the step for producing a compound represented by formula (A-9) by reacting a compound represented by formula (A-8) and a compound represented by formula: H-O-Y.

As a compound represented by formula: H-O-Y, examples include phenol, methanol, ethanol and the like.

As a reaction solvent, solvents described for the first step can be used. Preferred examples include N,N-dimethylformamide, dimethyl sulfoxide, ethers (e.g., tetrahydrofuran, diethyl ether, dioxane, 1,2-dimethoxyethane, etc.), nitriles (e.g., acetonitrile, etc.) and the like.

As a base, bases described for the first step can be used. Preferred examples include metal hydrides (e.g., sodium hydride, etc.), metal carbonates (e.g., sodium carbonate, calcium carbonate, cesium carbonate, etc.), metal amides, organic amines (e.g., triethylamine, diisopropylethylamine, DBU, 2,6-lutidine, etc.), pyridine, alkyllithiums (n-BuLi, sec-BuLi, tert-BuLi) and the like.

Further preferably, metal hydrides (e.g., sodium hydride, etc.) or metal carbonates (e.g., sodium carbonate, calcium carbonate, cesium carbonate, etc.) can be used.

The reaction can be carried out at 0 to 100°C for 0.5 to 12 hours.

### (When Hal is bromine or iodine)

The reaction can be carried out using conditions for a reaction which is known as the Ullmann reaction.

As a reaction solvent, solvents described for the first step can be used. Preferred examples include N,N-dimethylformamide, dimethyl sulfoxide, ethers (e.g., tetrahydrofuran, diethyl ether, dioxane, 1,2-dimethoxyethane, etc.), nitriles (e.g., acetonitrile, etc.) and the like.

As a base, bases described for the first step can be used. Preferred examples include metal hydrides (e.g., sodium hydride, etc.), metal carbonates (e.g., sodium carbonate, calcium carbonate, cesium carbonate, etc.), metal amides, organic amines (e.g., triethylamine, diisopropylethylamine, DBU, 2,6-lutidine, etc.), pyridine, alkyllithiums (n-BuLi, sec-BuLi, tert-BuLi) and the like.

Further preferably, metal carbonates (e.g., sodium carbonate, calcium carbonate, cesium carbonate, etc.) can be used.

As a catalyst, copper iodide can be used.

The reaction can be carried out at room temperature to 100°C for 0.5 to 12 hours.

### Tenth step

The tenth step is a step in which a compound represented by formula (A-10) is produced by deprotection of a compound represented by formula (A-9).

As a reaction solvent, solvents described for the first step can be used. Preferably, N,N-dimethylformamide, halogenated hydrocarbons (e.g., dichloromethane, chloroform, 1,2-dichloroethane, etc.), ethers (e.g., tetrahydrofuran, diethyl ether, dioxane, 1,2-dimethoxyethane, etc.), esters (e.g., methyl acetate, ethyl acetate, etc.), nitriles (e.g., acetonitrile, etc.), alcohols (e.g., methanol, ethanol, t-butanol, etc.), water, a mixed solvent thereof or the like may be used.

As a base, bases described for the first step can be used. Preferred examples include metal hydrides (e.g., sodium hydride, etc.), metal hydroxides (e.g., sodium hydroxide, potassium hydroxide, lithium hydroxide, barium hydroxide, etc.), metal carbonates (e.g., sodium carbonate, calcium carbonate, cesium carbonate, etc.), metal alkoxides (e.g., sodium methoxide, sodium ethoxide, potassium t-butoxide, etc.), sodium hydrogencarbonate, metal sodium, metal amides, organic amines (e.g., triethylamine, diisopropylethylamine, DBU, 2,6-lutidine, etc.), pyridine, alkyllithiums (n-BuLi, sec-BuLi, tert-BuLi), hydroxylamine and the like.
The reaction can be carried out at 0 to 200°C for 0.5 to 24 hours.

### Eleventh step

The eleventh step is a step in which a compound represented by formula (A-11) is produced from a compound represented by formula (A-10).

The reaction may be carried out in the same manner as the fifth step.

### Twelfth step

The twelfth step is a step in which a compound represented by formula (1-3) is produced by deprotection of a compound represented by formula (A-11).

The reaction may be carried out in the same manner as the second step.

A compound represented by formula (1-4) can be synthesized as follows. 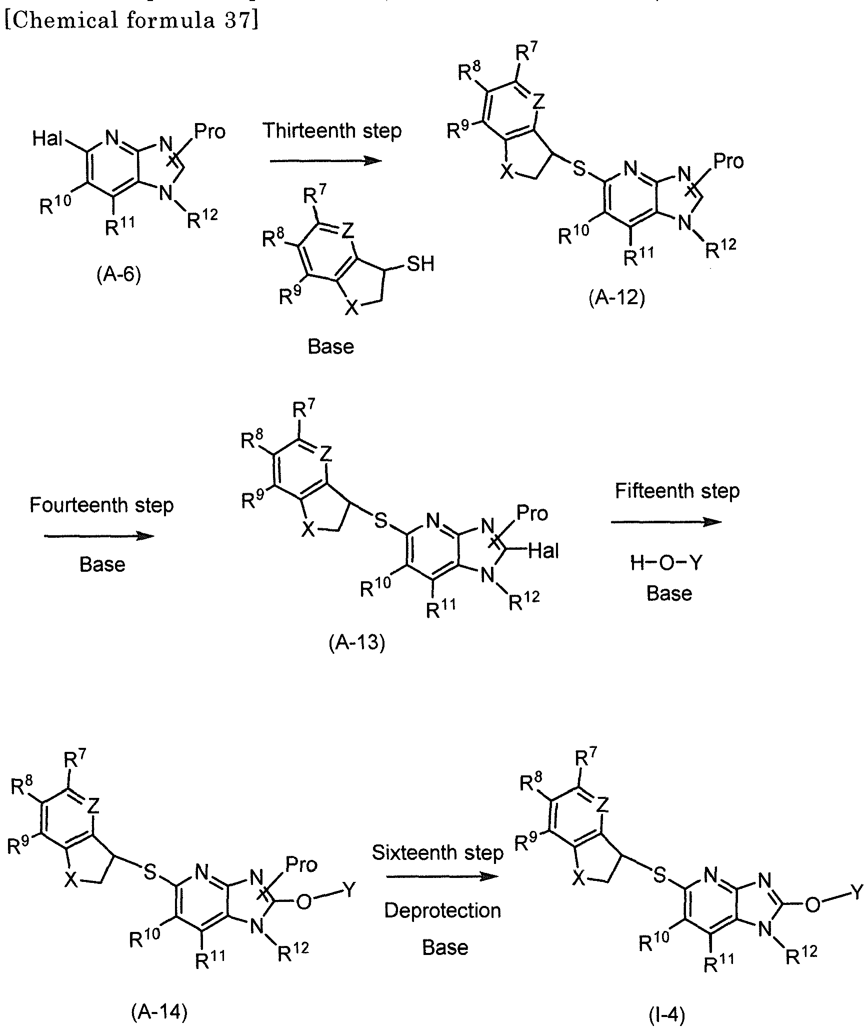 wherein each symbol has the same meaning as above, and as a compound represented by formula (A-6), a known compound can be used and a compound which is derived from a known compound by a conventional method can be used. "Hal" means a halogen, and Pro means a protecting group. Pro includes a benzyl group, a p-methoxybenzyl group, an acetyl group, a benzoyl group, SEM (trimethylsilylethoxymethyl), THP (tetrahydropyran), TBS (tert-butyldimethylsilyl), TBDPS (tert-butyldiphenylsilyl) and the like.

### Thirteenth step

The thirteenth step is a step in which a compound represented by formula (A-12) is produced from a compound represented by formula (A-6).

As a reaction solvent, solvents described for the first step can be used. Preferably, N,N-dimethylformamide, halogenated hydrocarbons (e.g., dichloromethane, chloroform, 1,2-dichloroethane, etc.), ethers (e.g., tetrahydrofuran, diethyl ether, dioxane, 1,2-dimethoxyethane, etc.), esters (e.g., methyl acetate, ethyl acetate, etc.), nitriles (e.g., acetonitrile, etc.) or the like may be used.

As a base, bases described for the first step can be used. Preferred examples include metal hydrides (e.g., sodium hydride, etc.), metal hydroxides (e.g., sodium hydroxide, potassium hydroxide, lithium hydroxide, barium hydroxide, etc.), metal carbonates (e.g., sodium carbonate, calcium carbonate, cesium carbonate, etc.), metal alkoxides (e.g., sodium methoxide, sodium ethoxide, potassium t-butoxide, etc.), sodium hydrogencarbonate, metal sodium, metal amides, organic amines (e.g., triethylamine, diisopropylethylamine, DBU, 2,6-lutidine, etc.), pyridine, and the like.

The reaction can be carried out at 0 to 200°C for 0.5 to 24 hours.

### Fourteenth step

The fourteenth step is a step in which a compound represented by formula (A-13) is produced by halogenation of a compound represented by formula (A-12).

The reaction may be carried out in the same manner as the eighth step.

### Fifteenth step

The fifteenth step is the step for producing a compound represented by formula (A-14) by reacting a compound represented by formula (A-13) and a compound represented by formula: H-O-Y.

The reaction may be carried out in the same manner as the ninth step.

### Sixteenth step

The sixteenth step is a step in which a compound represented by formula (I-4) is produced by deprotection of a compound represented by formula (A-14).

The reaction may be carried out in the same manner as the second step.

A compound represented by formula (I-5) can be synthesized as follows. 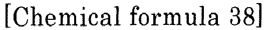 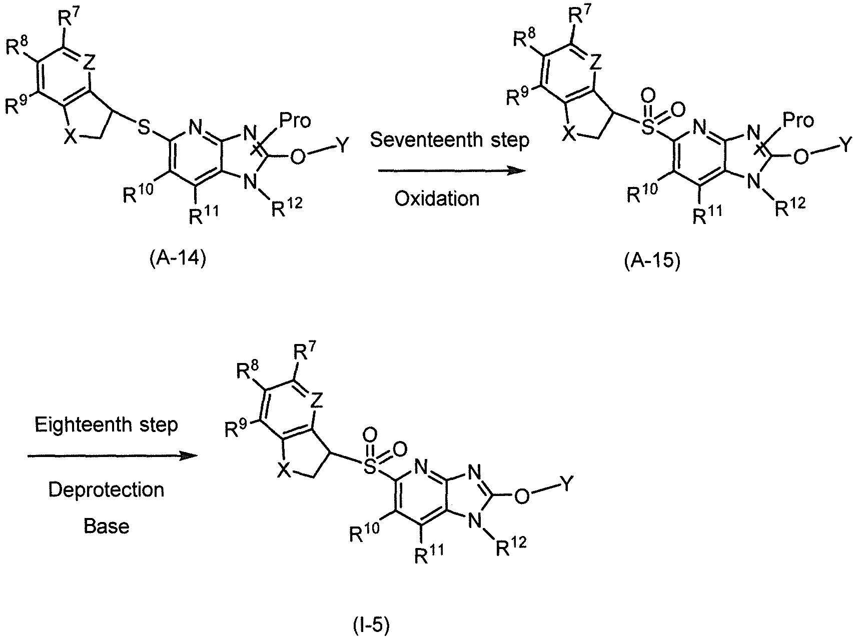 wherein each symbol has the same meaning as above, and "Hal" means a halogen, and Pro means a protecting group. Pro includes a benzyl group, a p-methoxybenzyl group, an acetyl group, a benzoyl group, SEM (trimethylsilylethoxymethyl), THP (tetrahydropyran), TBS (tert-butyldimethylsilyl), TBDPS (tert-butyldiphenylsilyl) and the like.

### Seventeenth step

The seventeenth step is a step in which a compound represented by formula (A-15) is produced by oxidation of a compound represented by formula (A-14).

As a reaction solvent, solvents described for the first step can be used. Preferably, N,N-dimethylformamide, halogenated hydrocarbons (e.g., dichloromethane, chloroform, 1,2-dichloroethane, etc.), ethers (e.g., tetrahydrofuran, diethyl ether, dioxane, 1,2-dimethoxyethane, etc.), esters (e.g., methyl acetate, ethyl acetate, etc.), nitriles (e.g., acetonitrile, etc.), alcohols (e.g., methanol, ethanol, t-butanol, etc.) or the like may be used.

As an oxidizing agent, examples include hydrogen peroxide, mCPBA (m-chloroperoxybenzoic acid) and the like.
The reaction can be carried out at 0 to 200°C for 0.5 to 24 hours.

### Eighteenth step

The eighteenth step is a step in which a compound represented by formula (1-5) is produced by deprotection of a compound represented by formula (A-15).

The reaction may be carried out in the same manner as the second step.

Among compounds represented by formula (I), a compound, wherein X is -O-, can be synthesized as follows. 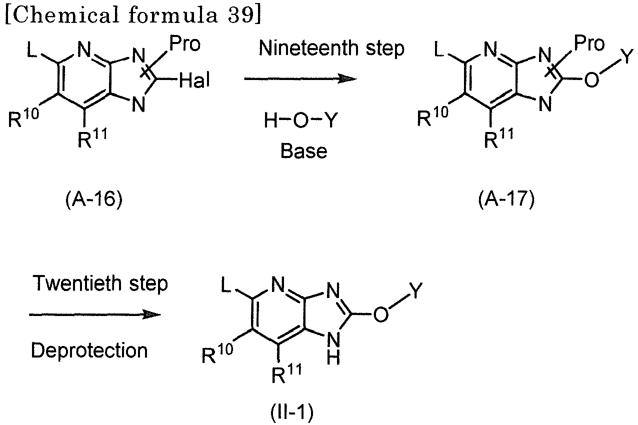 wherein, each symbol has the same meaning as above. "Hal" means a halogen, and Pro means a protecting group. Pro includes a benzyl group, a p-methoxybenzyl group, an acetyl group, a benzoyl group, SEM (trimethylsilylethoxymethyl), THP (tetrahydropyran), TBS (tert-butyldimethylsilyl), TBDPS (tert-butyldiphenylsilyl) and the like.

### Nineteenth step

The nineteenth step is the step for producing a compound represented by formula (A-17) by reacting a compound represented by formula (A-16) and a compound represented by formula: H-O-Y.

As a compound represented by formula: H-O-Y, examples include phenol, methanol, ethanol and the like.

The reaction may be carried out in the same manner as the ninth step.

### Twentieth step

The twentieth step is a step in which a compound represented by formula (II-1) is produced by deprotection of a compound represented by formula (A-17).

The reaction may be carried out in the same manner as the second step.

The substituents R¹⁰, R¹¹ and R¹² can be introduced in any step of the above-described first to twentieth steps.

Various types of substituents on compounds of the present invention can be introduced by reference to (1) Alan R. Katriszly et al., Comprehensive Heterocyclic Chemistry, (2) Alan R. Katriszly et al., Comprehensive Heterocyclic Chemistry II, (3) RODD'S CHEMISTRY OF CARBON COMPOUNDS VOLUME IV HETEROCYCLIC COMPOUNDS and the like.

A compound of the present invention has an excellent AMPK activating effect. Therefore, the compound can be used for the treatment or prevention of diseases associated with AMPK, particularly disease such as type I diabetes, type II diabetes, hyperglycemia, metabolic syndrome, obesity, hypercholesterolemia and/or hypertension. Particularly, the compound is useful in the treatment or prevention of type II diabetes, hyperglycemia, metabolic syndrome or obesity.

A pharmaceutical composition of the present invention can be administered orally or parenterally. Methods for parenteral administration include dermal, subcutaneous, intravenous, intraarterial, intramuscular, intraperitoneal, transmucosal, inhalation, transnasal, ophthalmic, inner ear or vaginal administration and the like.

In the case of oral administration, any forms, which are usually used, such as oral solid formulations (e.g., tablets, powders, granules, capsules, pills, films or the like), oral liquid formulations (e.g., suspension, emulsion, elixir, syrup, lemonade, spirit, aromatic water, extract, decoction, tincture or the like) and the like may be prepared according to the usual method and administered. The tablets can be sugar-coated tablets, film-coated tablets, enteric-coating tablets, sustained-release tablets, troche tablets, sublingual tablets, buccal tablets, chewable tablets or orally disintegrating tablets. Powders and granules can be dry syrups. Capsules can be soft capsules, micro capsules or sustained-release capsules.

In the case of parenteral administration, any forms, which are usually used, such as injections, drips, external preparations (e.g., ophthalmic drops, nasal drops, ear drops, aerosols, inhalations, lotion, infusion, liniment, mouthwash, enema, ointment, plaster, jelly, cream, patch, cataplasm, external powder, suppository or the like) and the like can be preferably administered. Injections can be emulsions whose type is O/W, W/O, O/W/O, W/O/W or the like.

The pharmaceutical composition may be manufactured by mixing an effective amount of the compound of the present invention with various pharmaceutical additives suitable for the formulation, such as excipients, binders, disintegrants, lubricants and the like. Furthermore, the pharmaceutical composition can be for pediatric patients, geriatric patients, serious cases or operations by appropriately changing the effective amount of the compound of the present invention, formulation and/or various pharmaceutical additives. The pediatric pharmaceutical compositions are preferably administered to patients under 12 or 15 years old. In addition, the pediatric pharmaceutical compositions can be administered to patients who are under 27 days old after the birth, 28 days to 23 months old after the birth, 2 to 11 years old, 12 to 16 years old, or 18 years old. The geriatric pharmaceutical compositions are preferably administered to patients who are 65 years old or over.

Although the dosage of a pharmaceutical composition of the present invention should be determined in consideration of the patient's age and body weight, the type and degree of diseases, the administration route and the like, a usual oral dosage is 0.05 to 100 and preferably 0.1 to 10 mg/kg/day. For parenteral administration, although the dosage highly varies with administration routes, a usual dosage is 0.005 to 10 and preferably 0.01 to 1 mg/kg/day. The dosage may be administered in one to several divisions per day.

A compound of the present invention can be used in combination with an insulin secretagogue (e.g., a sulfonylurea (SU) drug), a fast-acting insulin secretagogue (e.g., a phenylalanine derivative), a glucose uptake inhibitor (e.g., an α-glucosidase inhibitor (α-GI drug)), an insulin resistance improving drug (e.g., a biguanide drug (BG drug), a thiazolidine derivative (TZD drug)), an insulin formulation, a peptidyl peptidase IV (DPP-IV) inhibitor, a GLP-1 receptor agonist, a sodium-dependent glucose transporter 1 (SGLT1) inhibitor, a sodium-dependent glucose transporter 2 (SGLT2) inhibitor and the like (hereinafter, abbreviated as concomitant drugs) for the purpose of an increase in the effect of the compound, a decrease in a dose of the compound or the like. In this case, the time when a compound of the present invention and a concomitant drug are administered is not restricted, and they can be administered to a subject of administration simultaneously or at intervals. Further, a compound of the present invention and a concomitant drug can be administered as two kinds of formulations comprising each active ingredient and as a single formulation comprising both active ingredients.

The dose of a concomitant drug can be suitably selected on the basis of a dosage which is clinically used. In addition, the mixing ratio of a compound of the present invention and a concomitant drug can be suitably selected depending on a subject of administration, an administration route, a target disease, symptoms, combination and the like. When a subject of administration is a human, for example, 0.01 to 100 parts by weight of a concomitant drug can be used per part by weight of a compound of the present invention.

The present invention is described in more detail below with reference to Examples, which are not intended to limit the scope of the present invention.

NMR spectrum data of the compounds of the present invention and intermediates thereof are shown. NMR analysis obtained in each example was performed at 400 MHz using deuterated chloroform (CDCl₃) or dimethyl sulfoxide (d6-DMSO).

LC/MS was measured under the following conditions.

### (Method A)

Column: ACQUITY UPLC BEH C18 (1.7 µm, i.d. 2.1 x 50mm) (Waters)
Flow rate: 0.8 mL/min
UV detection wavelength: 254 nm
Mobile phase: [A] 0.1% formic acid-containing aqueous solution, [B] 0.1% formic acid-containing acetonitrile solution
Gradient: a linear gradient of the solvent [B] from 5 to 100% was carried out for 3.5 minutes, and the solvent [B] at 100% was maintained for 0.5 minutes.

### (Method B)

Column: Shim-pack XR-ODS (2.2 µm, i.d. 3.0 x 50 mm) (Shimadzu)
Flow rate: 1.6 mL/min
UV detection wavelength: 254 nm
Mobile phase: [A] 0.1% formic acid-containing aqueous solution, [B] 0.1% formic acid-containing acetonitrile solution
Gradient: a linear gradient of the solvent [B] from 10 to 100% was carried out for 3 minutes, and the solvent [B] at 100% was maintained for 0.5 minutes.

### (Method C)

Column: ACQUITY UPLC BEH C18 (1.7 µm. i.d. 2.1 x 50mm) (Waters)
Flow rate: 0.55 mL/min
UV detection wavelength: 254 nm
Mobile phase: [A] 0.1% formic acid-containing aqueous solution, [B] 0.1% formic acid-containing acetonitrile solution
Gradient: a linear gradient of the solvent [B] from 5 to 100% was carried out for 3 minutes, and the solvent [B] at 100% was maintained for 0.5 minutes.

The meaning of each term in Examples is as follows.
Ruphos: 2-Dicyclohexylphosphino-2',6'-diisopropoxybiphenyl
Pd₂(dba)₃: Tris(dibenzylideneacetone)dipalladium
NaOt-Bu: Sodium tert-butoxide
TBAF: Tetrabutylammonium fluoride
THF: Tetrahydrofuran
Pd(OAc)₂: Palladium(II) acetate
BINAP: 2,2'-Bis(diphenylphosphino)-1,1'-binaphthyl
TFA: Trifluoroacetic acid
DMF: N,N-Dimethylformamide
PPTS: Pyridinium p-toluenesulfonate
SEM: Trimethylsilylethoxymethyl
TBS: tert-Butyldimethylsilyl
THP: Tetrahydropyran
BrettPhos: 2-(Dicyclohexylphosphino)-3,6-dimethoxy-2',4',6'-triisopropyl-1,1'-biphenyl
CDI: 1,1'-Carbonyldiimidazole
Pd(PPh₃)₄: Tetrakis(triphenylphosphine)palladium(0)
POCl₃: Phosphorus oxychloride
PdCl₂(dtbpf): [1,1'-Bis(di-tert-butylphosphino)ferrocene]dichloropalladium(II)
NCS: N-Chlorosuccinimide
NBS: N-Bromosuccinimide
NIS: N-Iodosuccinimide
DIAD: Diisopropyl azodicarboxylate
UHP: Urea-hydrogen peroxide
mCPBA: m-Chloroperoxybenzoic acid
HMPA: Hexamethylphosphoric triamide
DMAP: N,N-Dimethyl-4-aminopyridine
MTBE: Methyl tert-butyl ether
TEMPO: 2,2,6,6-Tetramethylpiperidine 1-oxyl free radical
MS4A: Molecular sieve 4A
TMEDA: N,N,N',N'-Tetramethylethylenediamine
NFSI: N-Fluorobenzenesulfonimide
PdCl₂(dppf)CH₂Cl₂: [1,1'-Bis(diphenylphosphino)ferrocene]palladium(II) dichloride dichloromethane adduct
NMO: 4-Methylmorpholine N-oxide
DMSO: Dimethyl sulfoxide
DME: 1,2-Dimethoxyethane
HOBt: 1-Hydroxybenzotriazole
EDC·HCl: 1-(3-Dimethylaminopropyl)-3-ethylcarbodiimide hydrochloride
HATU: O-(7-Azabenzotriazol-1-yl)-N,N,N',N'-tetramethyluronium hexafluorophosphate
DMT-MM: 4-(4,6-Dimethoxy-1,3,5-triazin-2-yl)-4-methylmorpholinium chloride Xantphos: 4,5-Bis(diphenylphosphino)-9,9-dimethylxanthene
DMEAD: Bis(2-methoxyethyl) azodicarboxylate
X-phos: 2-Dicyclohexylphosphino-2',4',6'-triisopropylbiphenyl
NaBH₄: Sodium borohydride
nBuLi: n-Butyllithium
CMBP; Cyanomethylenetributylphosphorane

### [Example 1]

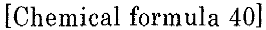

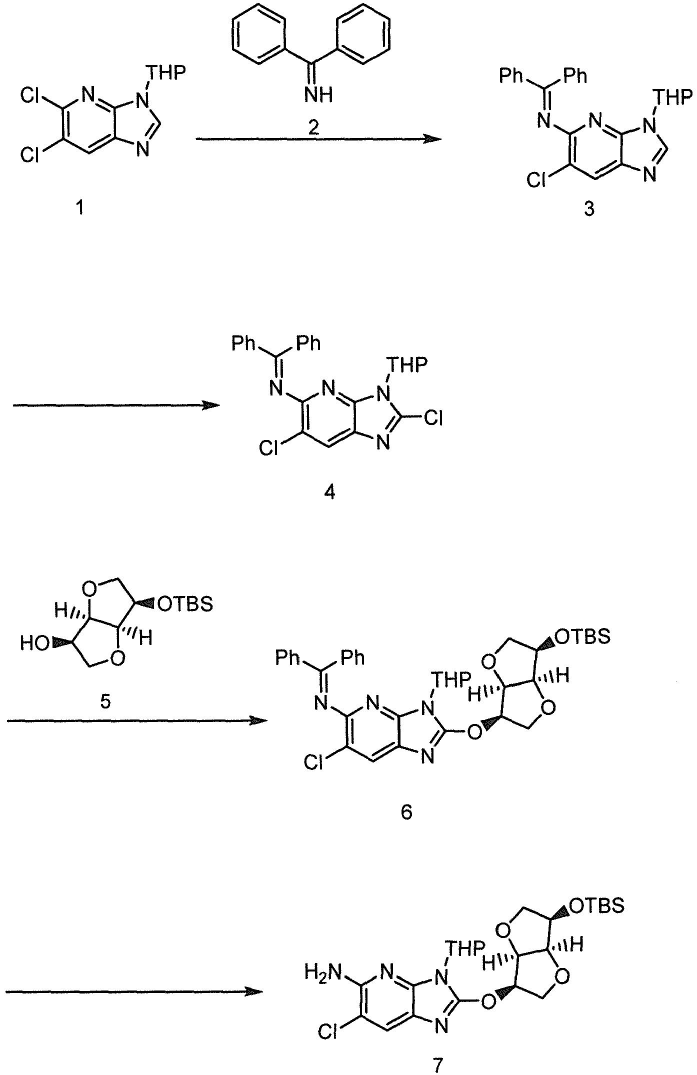

To degassed toluene (60 mL) were added tris(dibenzylideneacetone)dipalladium (168 mg, 0.184 mmol) and diphenylphosphinoferrocene (306 mg, 0.551 mmol) under nitrogen atmosphere, and the mixture was stirred at room temperature for 10 minutes. Subsequently, sodium t-butoxide (1.41 g, 14.7 mmol), Compound 1 (2.00 g, 7.35 mmol), and Compound 2 (1.48 mL, 8.82 mmol) were added thereto, and the mixture was stirred at 80°C for 2 hours. The reaction mixture was cooled to room temperature, and water (30 mL) was added thereto, then the mixture was extracted with ethyl acetate. The extract was washed with water and then concentrated, and the obtained residue was purified by silica gel column chromatography and crystallized with ethyl acetate (2 mL) and hexane (10 mL) to obtain Compound 3 (29.5 mg, 60.5%) as a yellow crystal.

Compound 3; ¹H-NMR (DMSO-d₆) δ: 1.61-1.66 (4H, m), 1.91 (1H, dd, J = 12.3, 0.8 Hz), 2.03-2.13 (1H, m), 3.59-3.66 (1H, m), 3.96-4.01 (1H, m), 5.50 (1H, d, J = 10.8 Hz), 7.19 (2H, d, J = 15.8 Hz), 7.29 (3H, s), 7.53 (2H, t, J = 6.7 Hz), 7.58-7.65 (1H, m), 7.71-7.80 (2H, m), 8.17 (1H, s), 8.45 (1H, s).

To a solution of Compound 3 (1.00 mg, 2.40 mmol) in THF (10 mL) was added hexachloroethane (596 mg, 2.52 mmol), and the mixture was cooled to -50°C. Then, a 1 mol/L solution of hexamethyldisilazide in toluene (2.64 mL, 2.64 mmol) was added dropwise thereto over 10 minutes, and the mixture was stirred at the same temperature for 10 minutes. The reaction mixture was quenched with water, and extracted with ethyl acetate. The extract was washed with water, then filtered and concentrated, and the obtained residue was suspended with ethyl acetate (15 mL) and hexane (30 mL) to obtain Compound 4 (937 mg, 82.5%) as a white powder.

Compound 4; ¹H-NMR (DMSO-d₆) δ: 1.46-1.67 (4H, m), 1.86-1.88 (1H, m), 2.52-2.55 (6H, m), 3.57-3.63 (1H, m), 3.98 (1H, d, J = 11.3 Hz), 5.50 (1H, d, J = 10.8 Hz), 7.16 (2H, s), 7.31 (3H, s), 7.54 (2H, t, J = 7.0 Hz), 7.63 (1H, dt, J = 10.0, 2.2 Hz), 7.77 (2H, d, J = 7.0 Hz), 8.17 (1H, d, J = 5.3 Hz).

To a solution of Compound 4 (200 mg, 0.443 mmol) and Compound 5 (150 mg, 0.576 mmol) in THF (4 mL) was added potassium t-butoxide (64.6 mg, 0.576 mmol), and the mixture was stirred at room temperature for 2 hours. Similarly, to a solution of Compound 4 (727 mg, 1.61 mmol) and Compound 5 (545 mg, 2.09 mmol) in THF (14.5 mL) was added potassium t-butoxide (235 mg, 2.09 mmol), and the mixture was stirred at room temperature for 40 minutes. Two reaction mixtures were combined and quenched with water, and extracted with ethyl acetate. The extract was washed with water, then filtered and concentrated, and the obtained residue was suspended with ethyl acetate (2 mL) and hexane (4 mL) to obtain Compound 6 (714 mg, 51.5%) as a white crystal. Also, the mother liquor was concentrated and purified by silica gel column chromatography to obtain Compound 6 (650.8 mg, 46.9%) as a white powder.
Compound 6; ¹H-NMR (DMSO-d₆) δ: 0.01 (3H, d, J = 5.8 Hz), 0.03 (3H, s), 0.81 (9H, d, J = 4.5 Hz), 0.99-1.85 (5H, m),2.26-2.49 (1H, m), 3.11 (2H, d, J = 5.0 Hz),3.68-3.73 (1H, m), 3.85-3.99 (3H, m), 4.17-4.31 (2H, m), 4.71 (1H, q, J = 5.5 Hz), 5.21-5.25 (1H, m), 5.31-5.34 (1H, m),7.08 (2H, dd, J = 2.5, 2.0 Hz), 7.25 (3H, d, J = 0.5 Hz), 7.46 (2H, t, J = 7.4 Hz), 7.55 (1H, dd, J = 9.2, 5.1 Hz), 7.69 (2H, d, J = 7.5 Hz), 7.80 (1H, s).

To a solution of Compound 6 (214 mg, 0.317 mmol) in methanol (8 mL) was added a 50% aqueous solution of hydroxylamine (41.9 mg, 0.635 mmol), and the mixture was stirred at room temperature for 1 hour. The mixture was diluted with THF (4 mL) and methanol (1 mL), and heated under reflux for 6 hours. In order to further raise the reaction temperature, the reaction mixture was concentrated, and ethanol (4 mL) was added thereto, then the mixture was heated under reflux for 1.5 hours. The reaction mixture was cooled to room temperature, and extracted with ethyl acetate. The extract was washed with water and concentrated, and the obtained residue was purified by silica gel column chromatography to obtain Compound 7 (127.2 mg, 78.4%) as a yellow powder.
Compound 7; Method C
LC/MS retention time =2.63 min.
MS (ESI) m/z =511.15 (M+H)+.

### [Example 2]

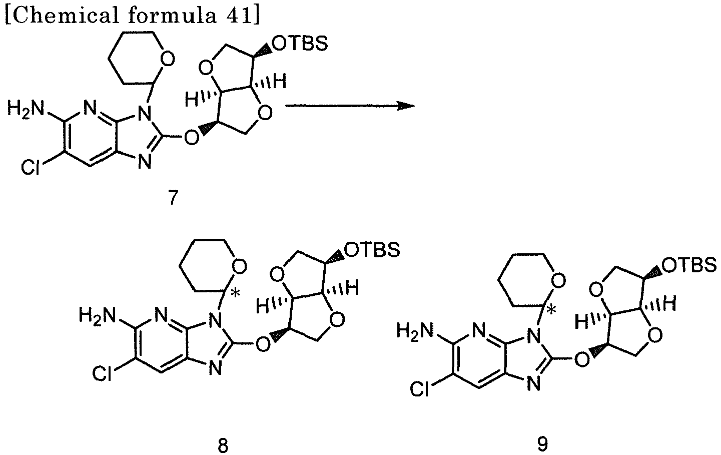

Optical resolution of Compound 7 was carried out in the following conditions, and Compound 8 and Compound 9 were obtained as 1^{st} peak and 2^{nd} peak, respectively (* shows that it is optically resolved (the same applies hereafter).).
- Instrument: SemiPrep SFC
- Column: CHIRALPAK IC/SFC manufactured by DAICEL Corporation (5 µm, i.d. 20 x 250 mm)
- Mobile phase: MeOH and carbon dioxide
- Flow rate: 30 mL/min

### [Example 3]

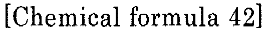

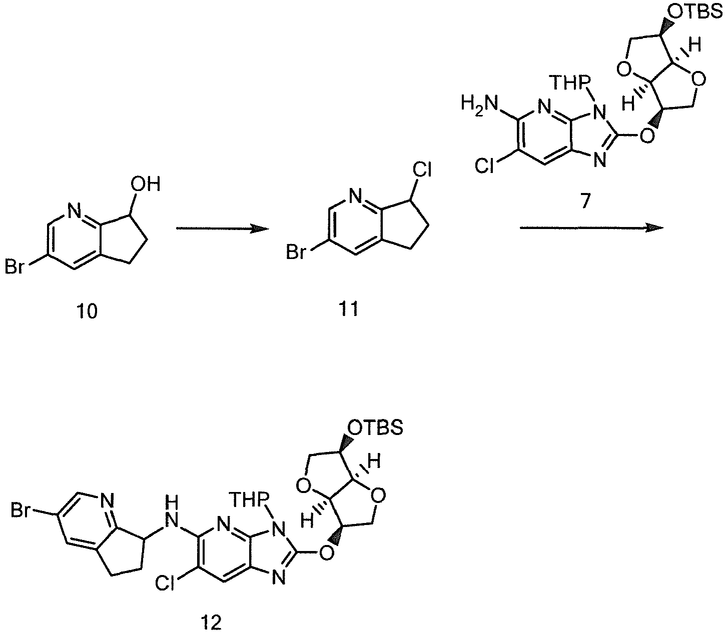

To Compound 10 (800 mg, 3.47 mmol) was added dichloromethane, and SOCl₂ (355 µL, 4.86 mmol) was added thereto under cooling, then the mixture was stirred at 0°C. After completion of the reaction, the reaction mixture was diluted with chloroform, and a saturated aqueous solution of sodium hydrogencarbonate was added thereto, then the mixture was subjected to a liquid-liquid separation. The obtained organic layer was dried over magnesium sulfate and filtered. The obtained filtrate was concentrated under reduced pressure, and purified by silica gel column chromatography to obtain Compound 11 (564 mg, 64.9%).

Compound 7 (150mg, 0.293 mmol) was dissolved in THF (1 mL) under nitrogen atmosphere, and the solution was cooled to -78°C, then a 2.66 mol/L n-butyllithium solution (110 µL, 0.293 mmol) was added thereto, and the mixture was stirred at -78°C for a while. Thereafter, Compound 11 (82 mg, 0.352 mmol) was added thereto. The temperature of the mixture was raised to room temperature, and Nal (132 mg, 0.880 mmol) was added thereto, then the mixture was stirred at 55°C. After completion of the reaction, ethyl acetate was added thereto, and the mixture was washed with water. The obtained organic layer was dried over magnesium sulfate and filtered. The obtained filtrate was concentrated under reduced pressure, and purified by silica gel column chromatography to obtain Compound 12 as a mixture with unreacted Compound 7

### [Example 4]

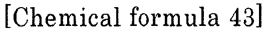

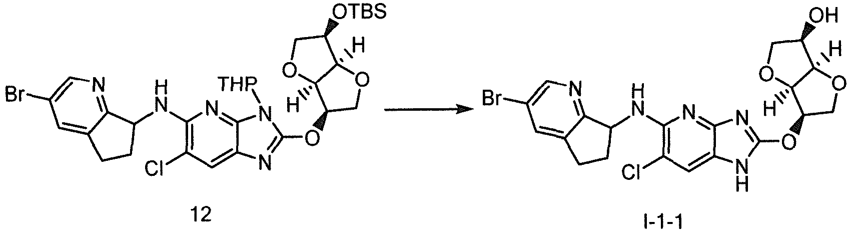

TFA (1 mL) was added to Compound 12 (25mg, 0.035 mmol), and the mixture was stirred at room temperature. After completion of the reaction, TFA was removed by concentration under reduced pressure, and chloroform was added to the residue, then the mixture was neutralized with saturated sodium hydrogencarbonate. The mixture was extracted with chloroform, and the obtained organic layer was dried over magnesium sulfate and filtered. The obtained filtrate was concentrated under reduced pressure, and purified by silica gel column chromatography to obtain Compound I-1-1 (11 mg, 61.2%).

### Compound 1-1-1; Method B

LC/MS retention time =1.68 min.
MS (ESI) m/z =510.2 (M+H)+.

### [Example 5]

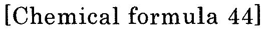

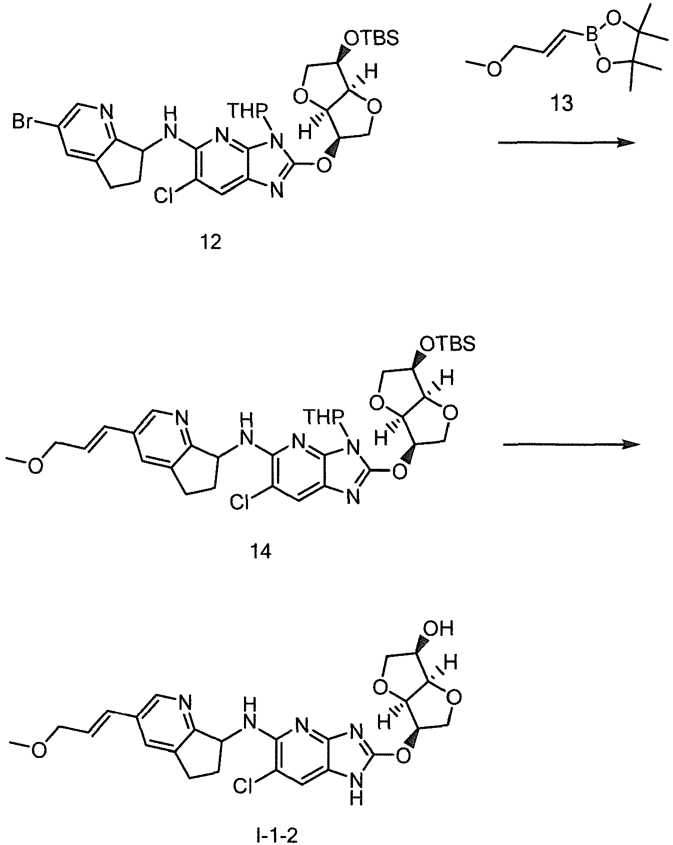

Compound 12 (equivalent to 37 µmol) and Compound 13 (9.34 mg, 45 µmol) were dissolved in 1,4-dioxane (0.8 mL), and PdCl₂(dtbpf) (4.87 mg, 7.47 µmol) and a 2 mol/L aqueous solution of potassium carbonate (28 µL, 56 µmol) were added thereto under nitrogen atmosphere, and the mixture was stirred at 55°C. After completion of the reaction, the resulting mixture was purified by silica gel column chromatography to obtain Compound 14 (25.3 mg, 97%).

Compound 14 (25 mg, 36 µmol) was dissolved in a mixed solvent of methanol and water (9:1, 1 ml), and PPTS (17.99 mg, 72 µmοl) was added thereto, and the mixture was stirred at 85°C. After completion of the reaction, a saturated aqueous solution of sodium hydrogencarbonate was added thereto, and the mixture was extracted with chloroform and a mixed solvent of chloroform and methanol. The obtained organic layer was dried over magnesium sulfate and filtered. The obtained filtrate was concentrated under reduced pressure, and purified by silica gel column chromatography to obtain Compound 1-1-2 (7.2 mg, 40.2%).

### Compound 1-1-2; Method B

LC/MS retention time =1.17 min.
MS (ESI) m/z =500.35 (M+H)+.

### [Example 6]

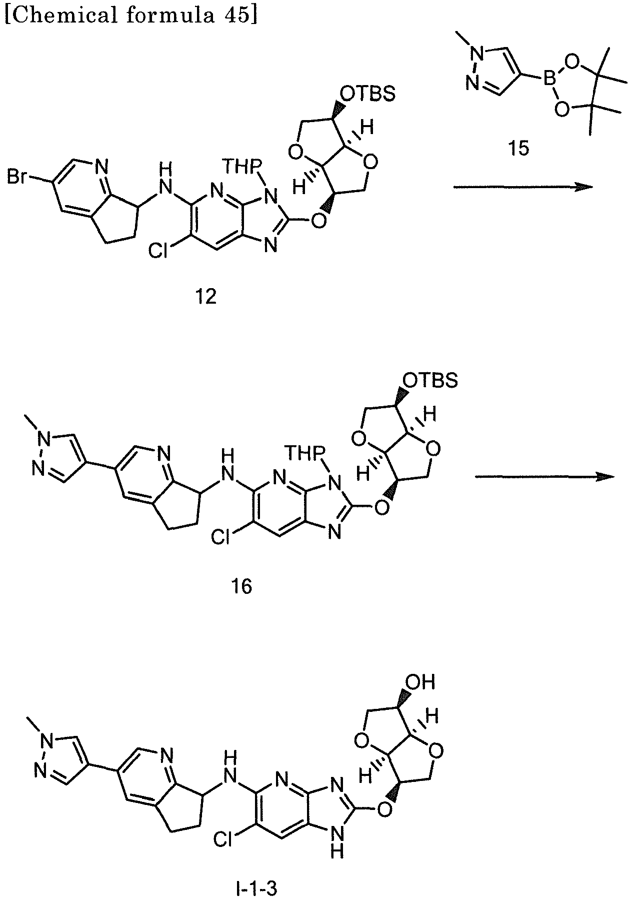

Compound 16 was synthesized from Compound 15, in a similar way that Compound 14 was synthesized from Compound 13. Compound 1-1-3 was synthesized from Compound 16, in a similar way that Compound I-1-1 was synthesized from Compound 12.

### Compound 1-1-3; Method B

LC/MS retention time =1.03 min.
MS (ESI) m/z =510.3 (M+H)+.

### [Example 7]

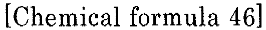

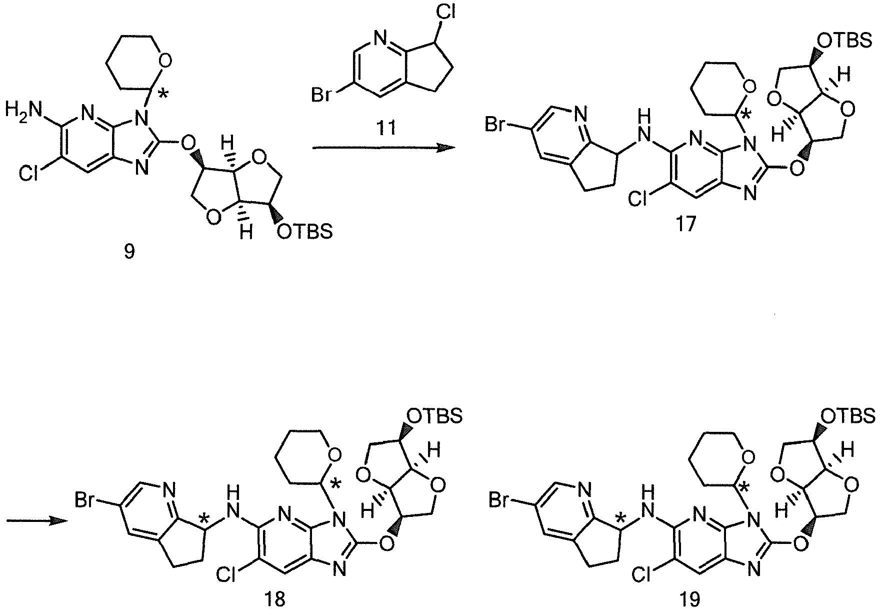

Compound 17 was synthesized from Compound 9, in a similar way that Compound 12 was synthesized from Compound 7. Optical resolution of Compound 17 was carried out in the following conditions, and Compound 18 and Compound 19 were obtained as 1^{st} peak and 2^{nd} peak, respectively.
- Instrument: SemiPrep SFC
- Column: CHIRALPAK IC/SFC manufactured by DAICEL Corporation (5 µm, i.d. 20 x 250 mm)
- Mobile phase: EtOH and carbon dioxide
- Flow rate: 20 mL/min

### [Example 8]

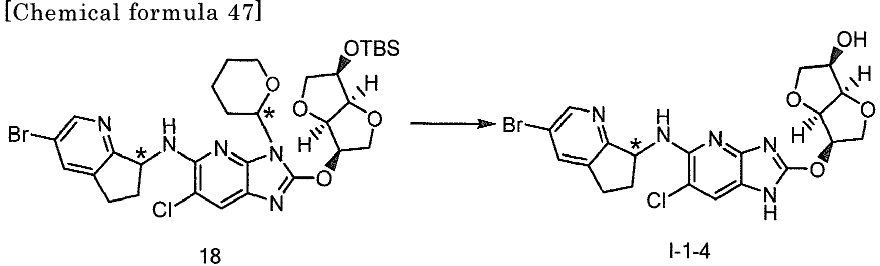

Compound I-1-4 was synthesized from Compound 18, in a similar way that Compound I-1-1 was synthesized from Compound 12.

### Compound I-1-4; Method B

LC/MS retention time =1.70 min.
MS (ESI) m/z =510.3 (M+H)+.

### [Example 9]

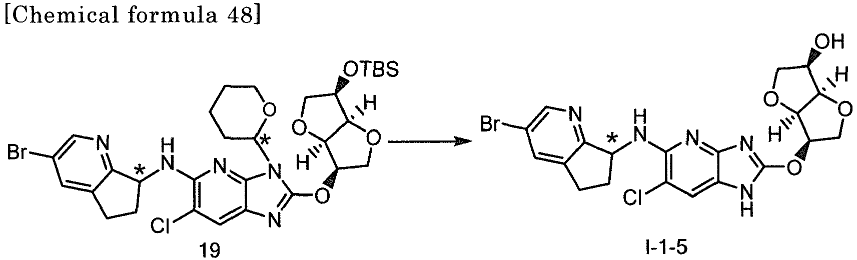

Compound 1-1-5 was synthesized from Compound 19, in a similar way that Compound I-1-1 was synthesized from Compound 12.

### Compound I-1-5; Method B

LC/MS retention time =1.70 min.
MS (ESI) m/z =510.2 (M+H)+.

### [Example 10]

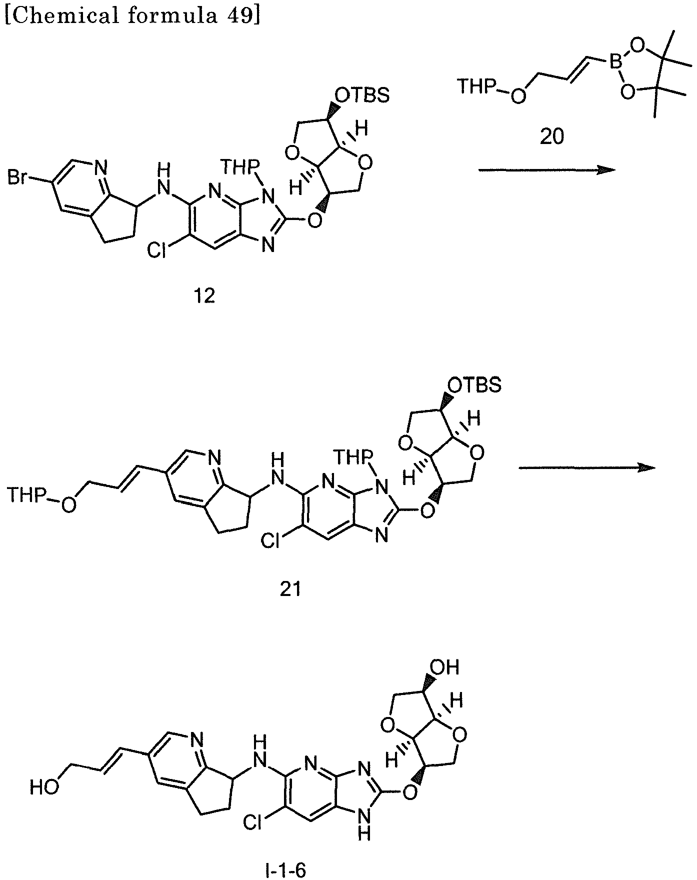

Compound 21 was synthesized from Compound 20, in a similar way that Compound 14 was synthesized from Compound 13. Compound 1-1-6 was synthesized from Compound 21, in a similar way that Compound 1-1-2 was synthesized from Compound 14.

### Compound I-1-6; Method B

LC/MS retention time =0.93 min.
MS (ESI) m/z =486.3 (M+H)+.

### [Example 11]

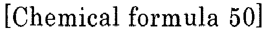

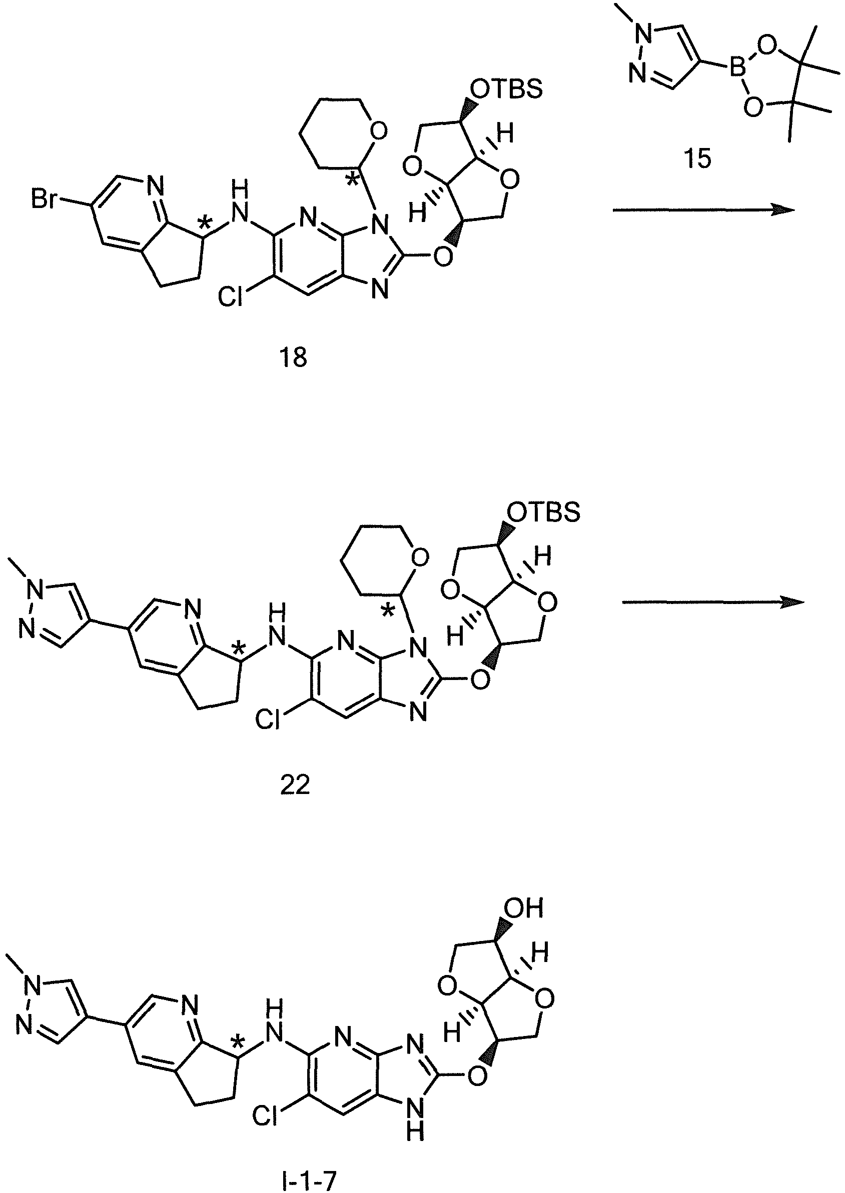

Compound 22 was synthesized from Compound 18, in a similar way that Compound 16 was synthesized from Compound 12. Compound 1-1-7 was synthesized from Compound 22, in a similar way that Compound 1-1-3 was synthesized from Compound 16.

### Compound 1-1-7; Method B

LC/MS retention time =1.04 min.
MS (ESI) m/z =510.3 (M+H)+.

### [Example 12]

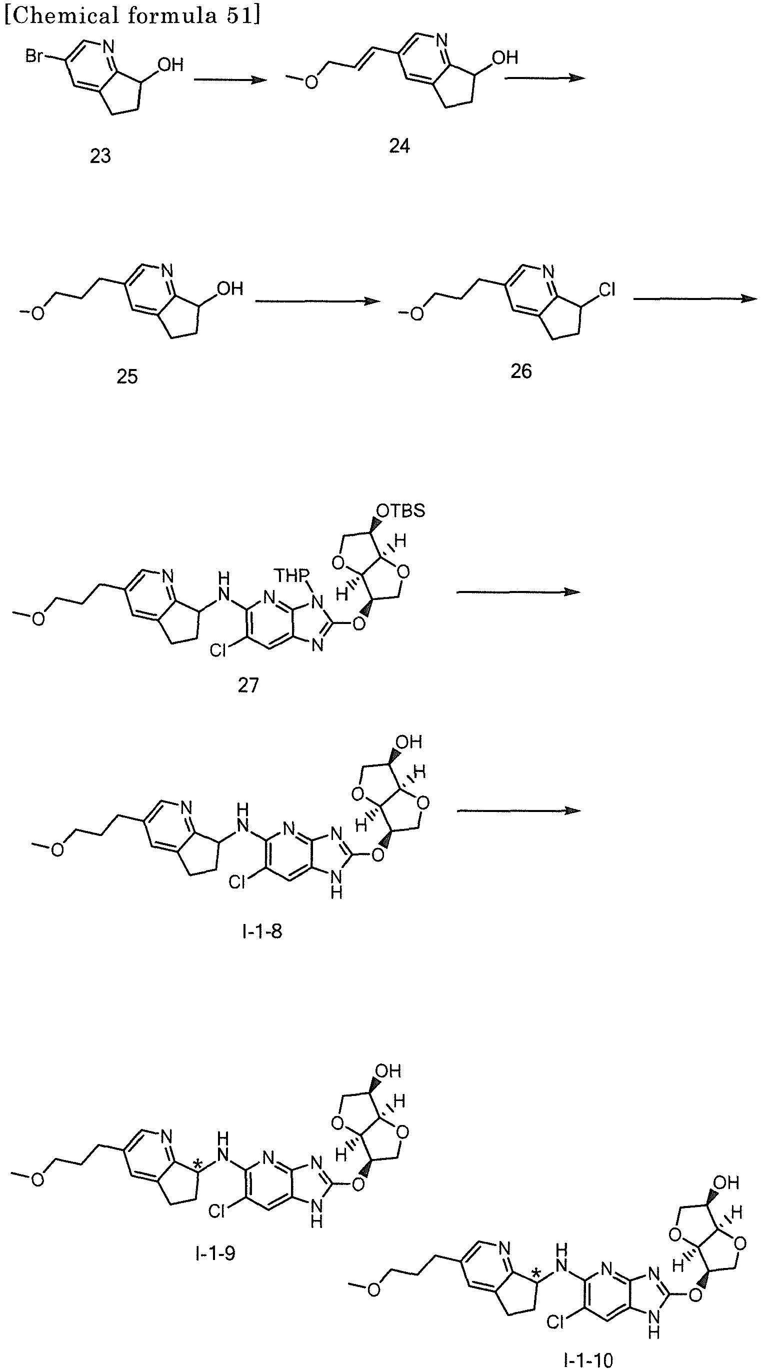

Compound 24 was synthesized from Compound 23, in a similar way that Compound 14 was synthesized from Compound 12. To Compound 24 (95 mg, 0.463 mmol) and Pd/carbone (10 wt % , 20 mg) was added methanol, and the mixture was stirred under a hydrogen atmosphere at room temperature. After completion of the reaction, the reaction mixture was filtered, and the obtained filtrate was concentrated under reduced pressure to obtain Compound 25 (78 mg, 81.3%). Compound 27 was synthesized from Compound 25, in a similar way that Compound 12 was synthesized from Compound 10. Compound 1-1-8 was synthesized from Compound 27, in a similar way that Compound I-1-1 was synthesized from Compound 12.

### Compound 1-1-8; Method B

LC/MS retention time =1.03 min.
MS (ESI) m/z =502.3 (M+H)+.

Optical resolution of Compound 1-1-8 was carried out in the following conditions, and Compound 1-1-9 and Compound I-1-10 were obtained as 1^{st} peak and 2^{nd} peak, respectively.
- Instrument: SemiPrep SFC
- Column: CHIRALPAK IC/SFC manufactured by DAICEL Corporation (5 µm, i.d. 20 x 250 mm)
- Mobile phase: MeOH and carbon dioxide
- Flow rate: 40 mL/min

### Compound 1-1-9; Method B

LC/MS retention time =0.98 min.
MS (ESI) m/z =502.3 (M+H)+.

### Compound I-1-10; Method B

LC/MS retention time =1.01 min.
MS (ESI) m/z =502.3 (M+H)+.

### [Example 13]

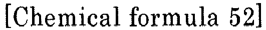

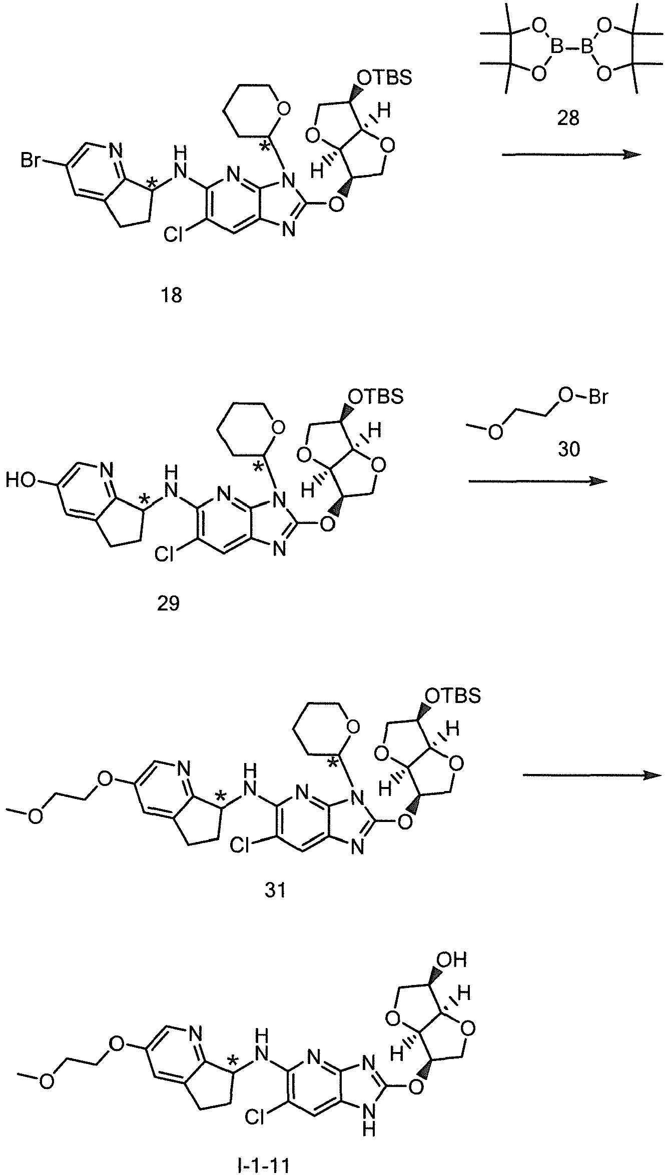

Compound 18 (60 mg, 85 µmol) and Compound 28 (32.3 mg, 127 µmol) were dissolved in 1,4-dioxane (1.0 mL), and PdCl₂(dtbpf) (11 mg, 17 µmol) and potassium acetate (16.7 mg, 170 µmol) were added thereto under nitrogen atmosphere, then the mixture was stirred at 70 to 75°C. After completion of the reaction, the reaction mixture was filtered with celite, and the filtrate was concentrated under reduced pressure. The obtained residue was dissolved in THF (1.0 mL), and water (0.5 mL) and a hydrate of NaBO₃ (25.4 mg, 25 5 µmol) were added thereto, then the mixture was stirred at room temperature. The reaction mixture was dried under magnesium sulfate, filtered, and washed with a mixture of chloroform and methanol. The obtained filtrate was concentrated under reduced pressure, and purified by silica gel column chromatography to obtain Compound 29. To Compound 29 was added Compound 30 (18 mg, 128 µmol) dissolved in DMF (1 mL), and cesium carbonate (42 mg, 128 µmol) was added thereto, and the reaction mixture was stirred at room temperature. To the reaction mixture was added ethyl acetate, and the mixture was washed with water. The obtained organic layer was concentrated under reduced pressure, and purified by silica gel column chromatography to obtain Compound 31. Compound I-1-11 was synthesized from Compound 31, in a similar way that Compound I-1-1 was synthesized from Compound 12.

### Compound I-1-11; Method B

LC/MS retention time =1.08 min.
MS (ESI) m/z =504.3 (M+H)+.

### [Example 14]

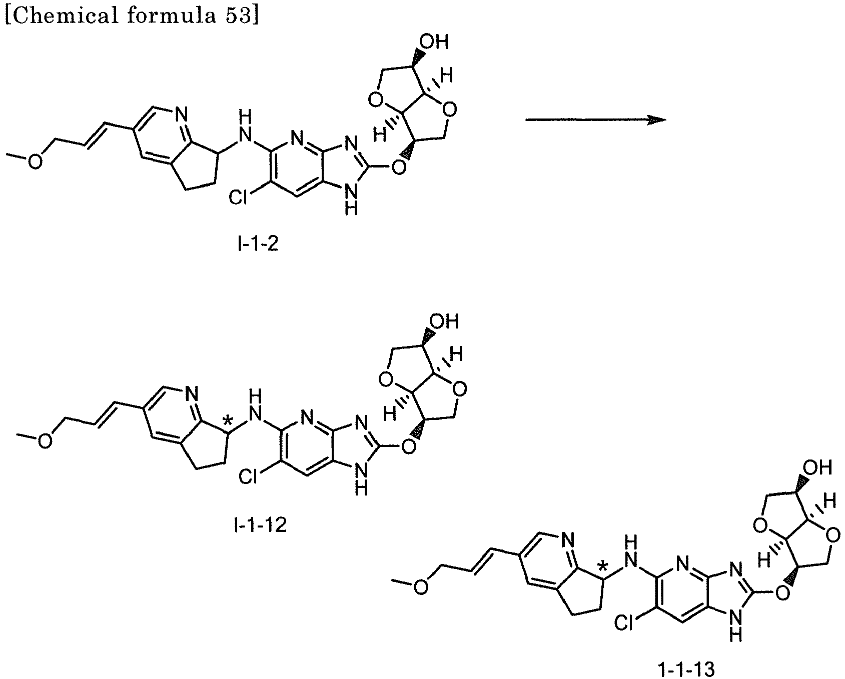

Optical resolution of Compound 1-1-2 was carried out in the following conditions, and Compound I-1-12 and Compound 1-1-13 were obtained as 1^{st} peak and 2^{nd} peak, respectively.
- Instrument: SemiPrep SFC
- Column: CHIRALPAK IA/SFC manufactured by DAICEL Corporation (5 µm, i.d. 20 x 250 mm)
- Mobile phase: EtOH and carbon dioxide
- Flow rate: 30 mL/min

### Compound 1-1-12; Method B

LC/MS retention time =1.15 min.
MS (ESI) m/z =500.35 (M+H)+.

### Compound I-1-13; Method B

LC/MS retention time =1.15 min.
MS (ESI) m/z =500.35 (M+H)+.

### [Example 15]

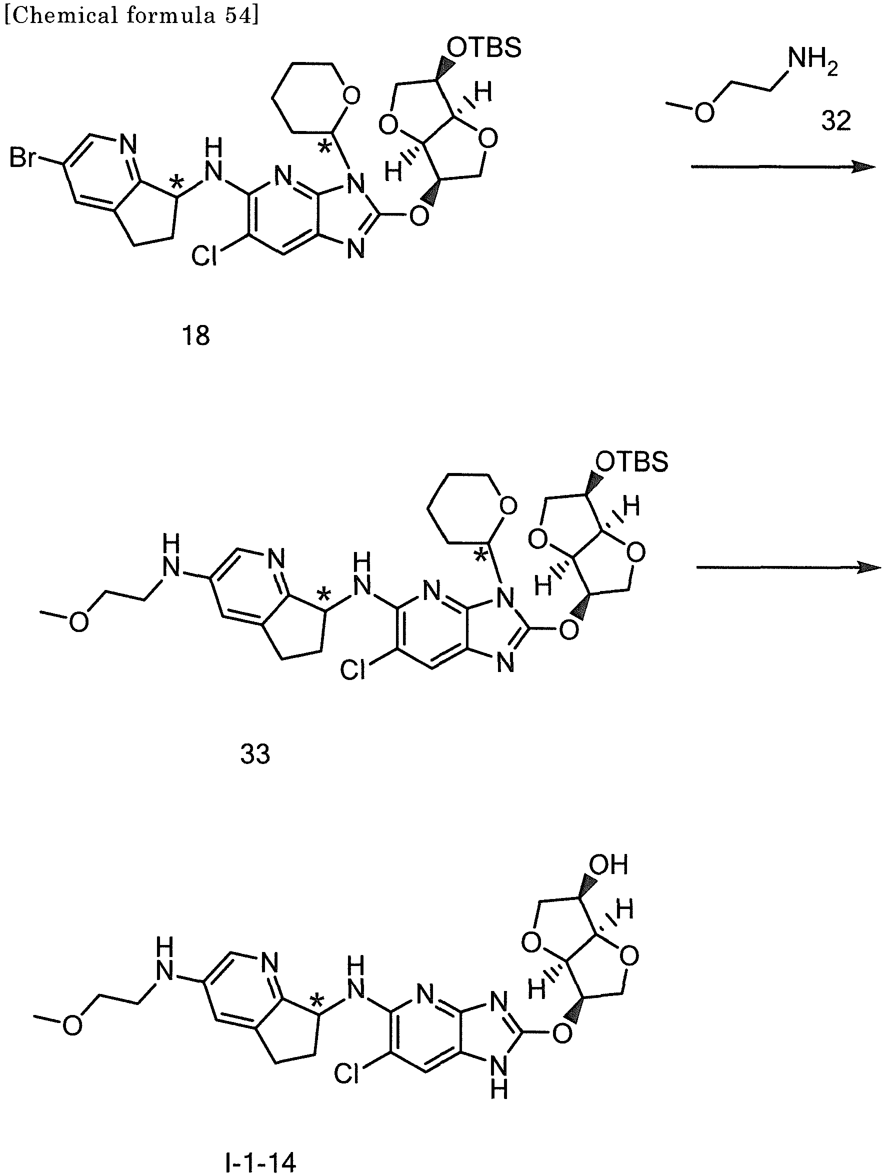

To Compound 18 (100 mg, 141 µmol) were added toluene (1 mL), Pd₂(dba)₃ (13 mg, 14 µmol), Xantphos (16 mg, 28 µmol), potassium tert-butoxide (27 mg, 283 µmol) and Compound 32 (18 µL, 212 µmol) under nitrogen atmosphere, and the mixture was stirred at 110°C. The reaction mixture was filtered with celite, and washed with ethyl acetate, and the obtained filtrate was concentrated under reduced pressure. The obtained residue was purified by silica gel column chromatography to obtain Compound 33. Compound I-1-14 was synthesized from Compound 33, in a similar way that Compound I-1-1 was synthesized from Compound 12.

### Compound I-1-14; Method B

LC/MS retention time =0.97 min.
MS (ESI) m/z =503.3 (M+H)+.

### [Example 16]

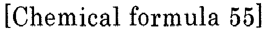

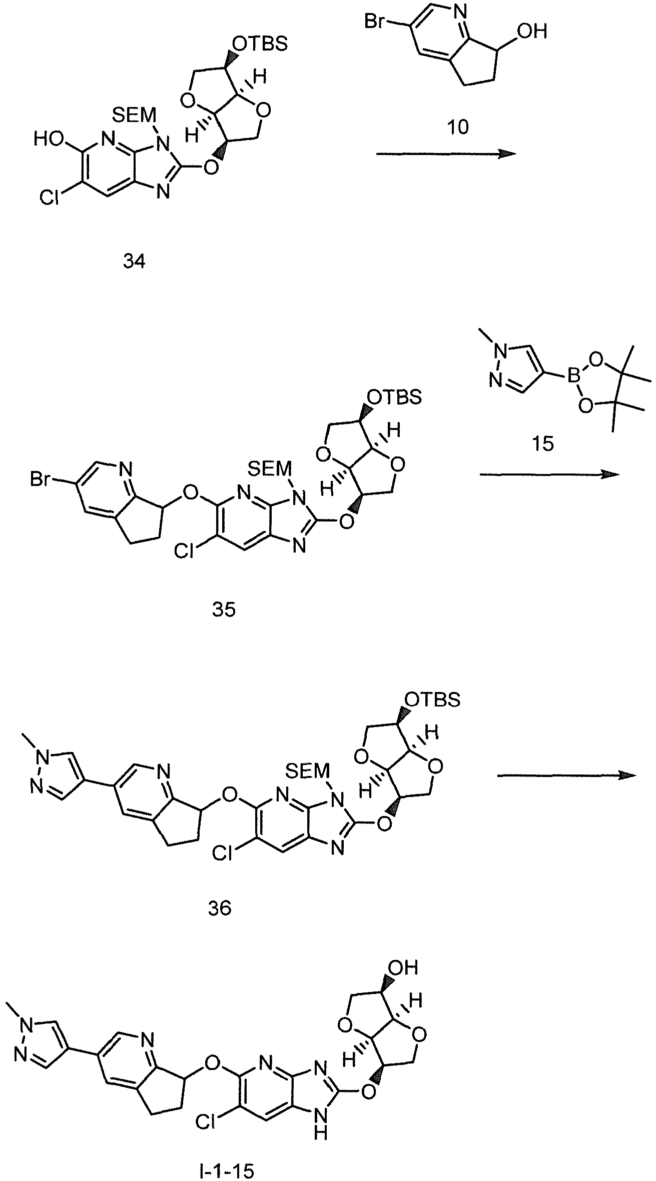

Compound 34 (100 mg, 179 µmol) and triphenylphosphine (70.5 mg, 269 µmοl) were dissolved in THF (1 mL), and Compound 10 (46 mg, 215 µmol) and DIAD (52.2 µL, 269 µmol) were added thereto, then the mixture was stirred at room temperature. After completion of the reaction, the resulting mixture was purified by silica gel column chromatography to obtain Compound 35. Compound 36 was synthesized from Compound 35, in a similar way that Compound 16 was synthesized from Compound 12. To Compound 36 (23.5 mg, 31 µmol) was added a 1 mol/L TBAF solution in THF (1 mL), and the mixture was stirred at 80°C. The reaction mixture was concentrated, and the residue was purified by silica gel column chromatography to obtain Compound I-1-15 (10.4 mg, 65.4%).

### Compound I-1-15; Method B

LC/MS retention time =1.24 min.
MS (ESI) m/z =511.3 (M+H)+.

### [Example 17]

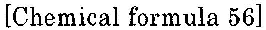

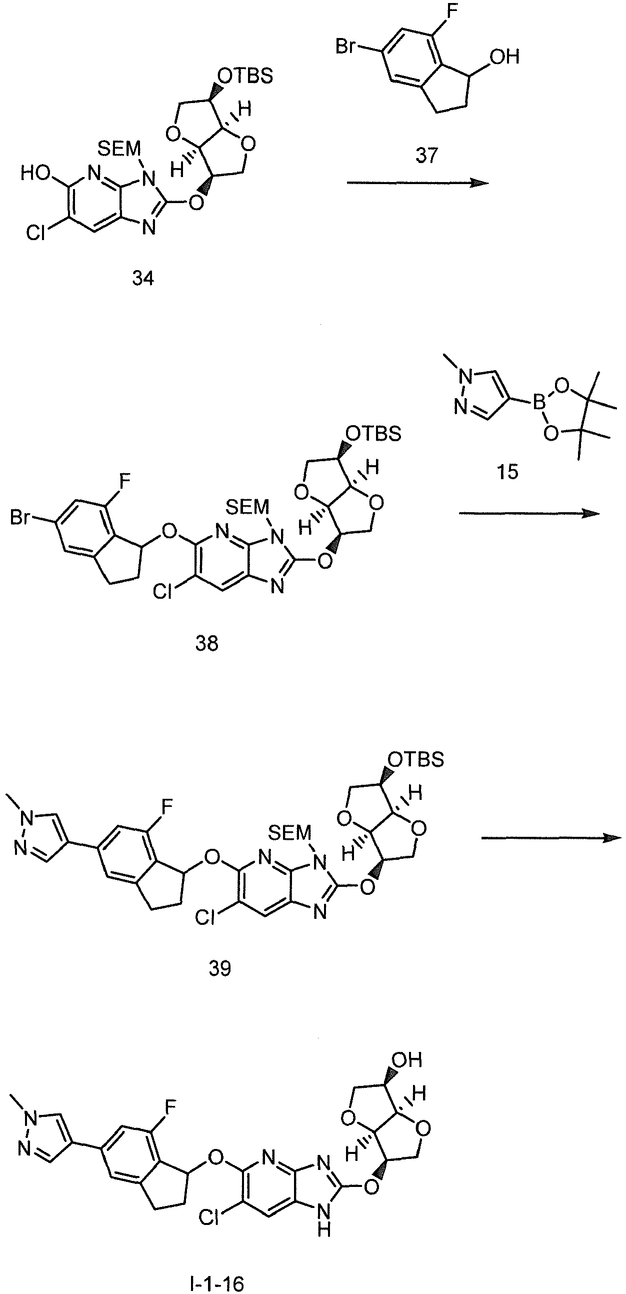

Compound I-1-16 was synthesized from Compound 37, in a similar way that Compound I-1-15 was synthesized from Compound 10.

### Compound 1-1-16; Method B

LC/MS retention time =1.65 min.
MS (ESI) m/z =528.25 (M+H)+.

### [Example 18]

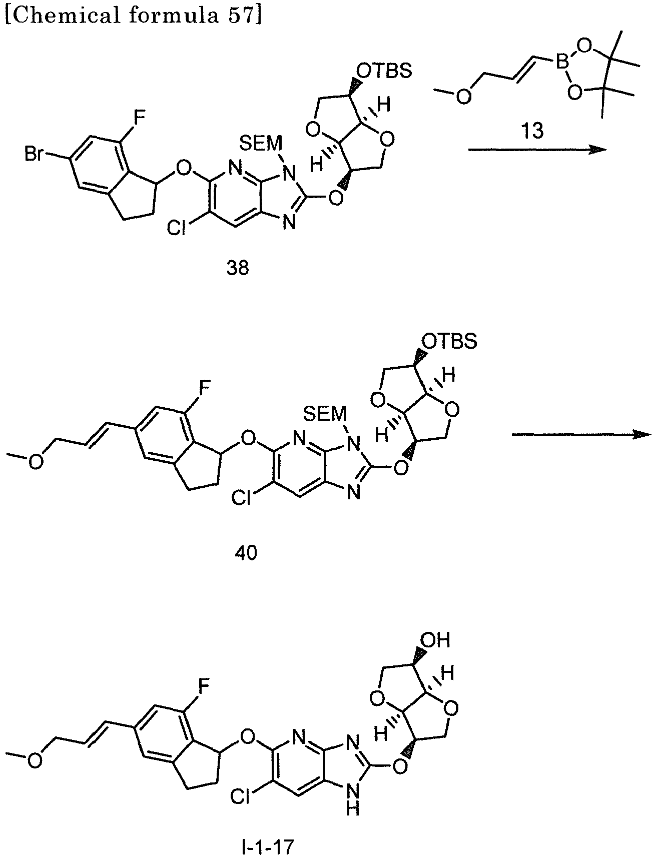

Compound 1-1-17 was synthesized from Compound 13, in a similar way that Compound I-1-16 was synthesized from Compound 15.

### Compound I-1-17; Method B

LC/MS retention time =1.92 min.
MS (ESI) m/z =518.35 (M+H)+.

### [Example 19]

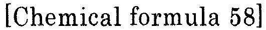

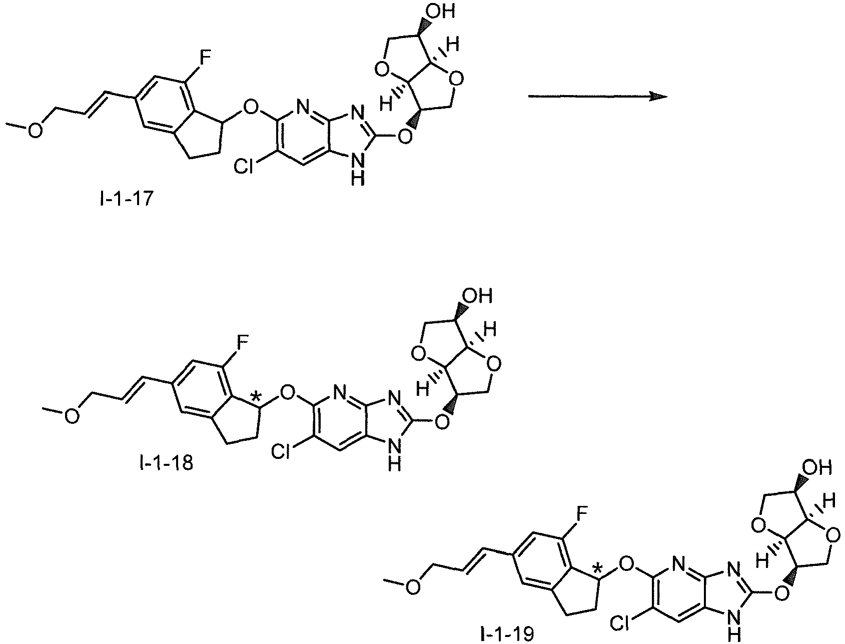

Optical resolution of Compound I-1-17 was carried out in the following conditions, and Compound 1-1-18 and Compound 1-1-19 were obtained as 1^{st} peak and 2^{nd} peak, respectively.
- Instrument: SemiPrep SFC
- Column: CHIRALPAK IA/SFC manufactured by DAICEL Corporation (5 µm, i.d. 20 x 250 mm)
- Mobile phase: MeOH containing 0.1% diethylamine and carbon dioxide
- Flow rate: 30 mL/min

### Compound 1-1-18; Method B

LC/MS retention time =1.93 min.
MS (ESI) m/z =518.25 (M+H)+.

### Compound 1-1-19; Method B

LC/MS retention time =1.93 min.
MS (ESI) m/z =518.25 (M+H)+.

### [Example 20]

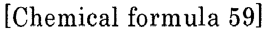

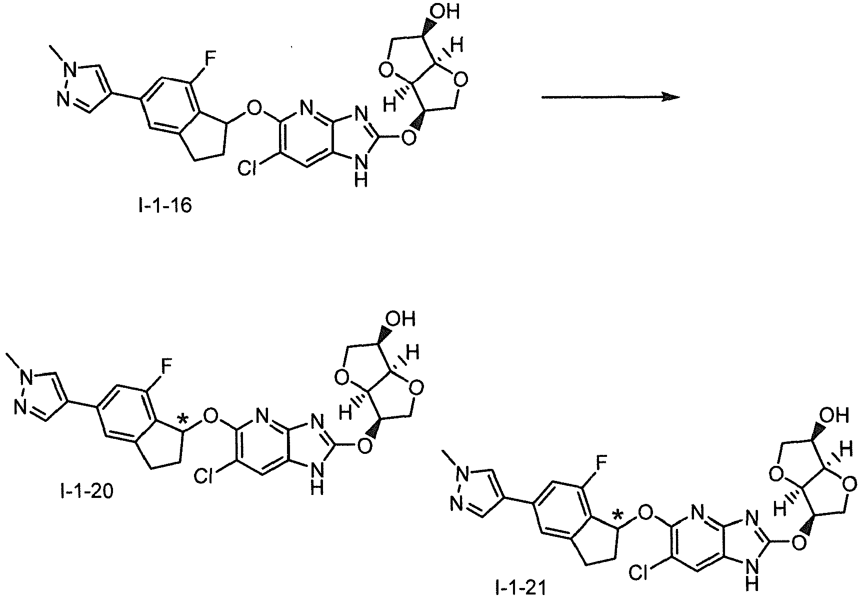

Optical resolution of Compound I-1-16 was carried out in the following conditions, and Compound 1-1-20 and Compound 1-1-21 were obtained as 1^{st} peak and 2^{nd} peak, respectively.
- Instrument: SemiPrep SFC
- Column: CHIRALPAK IC/SFC manufactured by DAICEL Corporation (5 µm, i.d. 20 x 250 mm)
- Mobile phase: EtOH containing 0.1% diethylamine and carbon dioxide
- Flow rate: 30 mL/min

### Compound I-1-20; Method B

LC/MS retention time =1.65 min.
MS (ESI) m/z =528.25 (M+H)+.

### Compound 1-1-21; Method B

LC/MS retention time =1.65 min.
MS (ESI) m/z =528.25 (M+H)+.

### [Example 21]

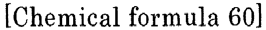

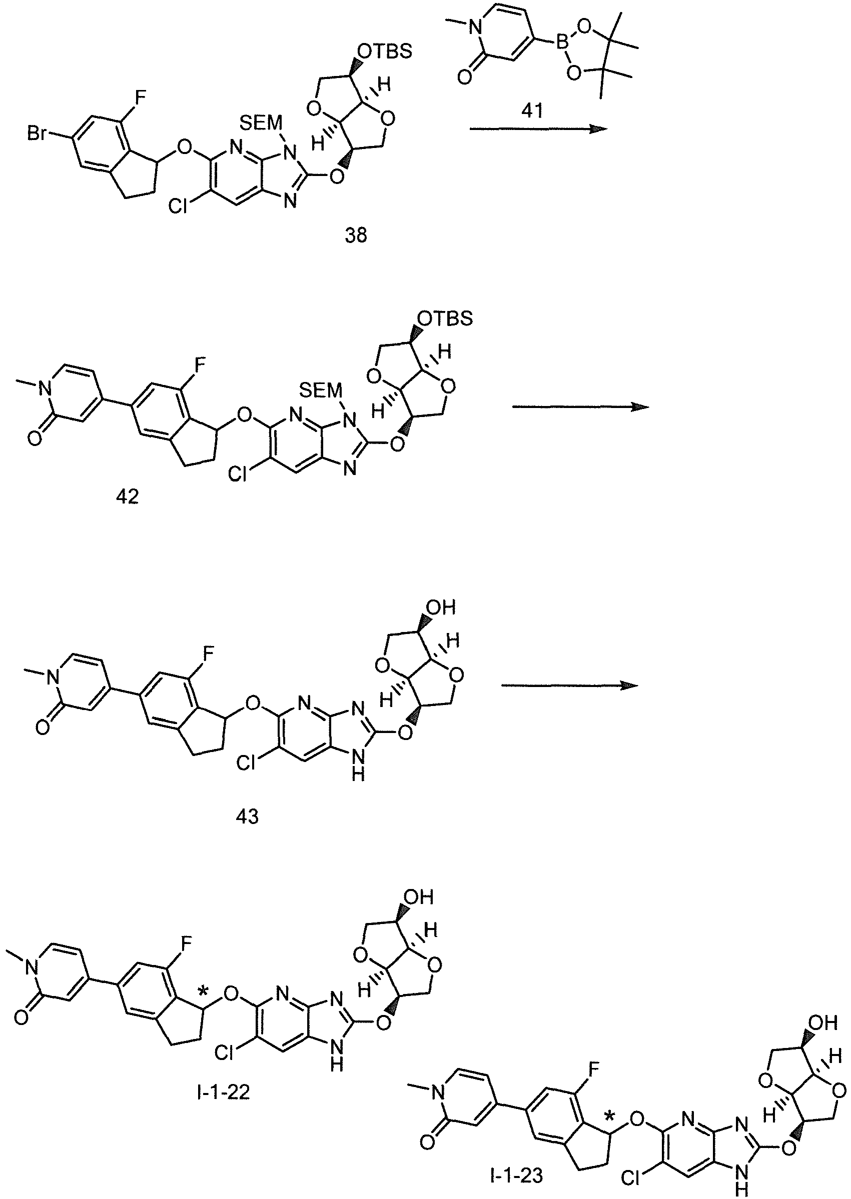

Compound 43 was synthesized from Compound 41, in a similar way that Compound I-1-16 was synthesized from Compound 15. Optical resolution of Compound 43 was carried out in the following conditions, and Compound I-1-22 and Compound 1-1-23 were obtained as 1^{st} peak and 2^{nd} peak, respectively.
- Instrument: SemiPrep SFC
- Column: CHIRALPAK ID/SFC manufactured by DAICEL Corporation (5 µm, i.d. 20 x 250 mm)
- Mobile phase: MeOH containing 0.1% diethylamine and carbon dioxide
- Flow rate: 20 mL/min

### Compound 1-1-22; Method B

LC/MS retention time =1.45 min.
MS (ESI) m/z =555.35 (M+H)+.

### Compound 1-1-23; Method B

LC/MS retention time =1.46 min.
MS (ESI) m/z =555.35 (M+H)+.

### [Example 22]

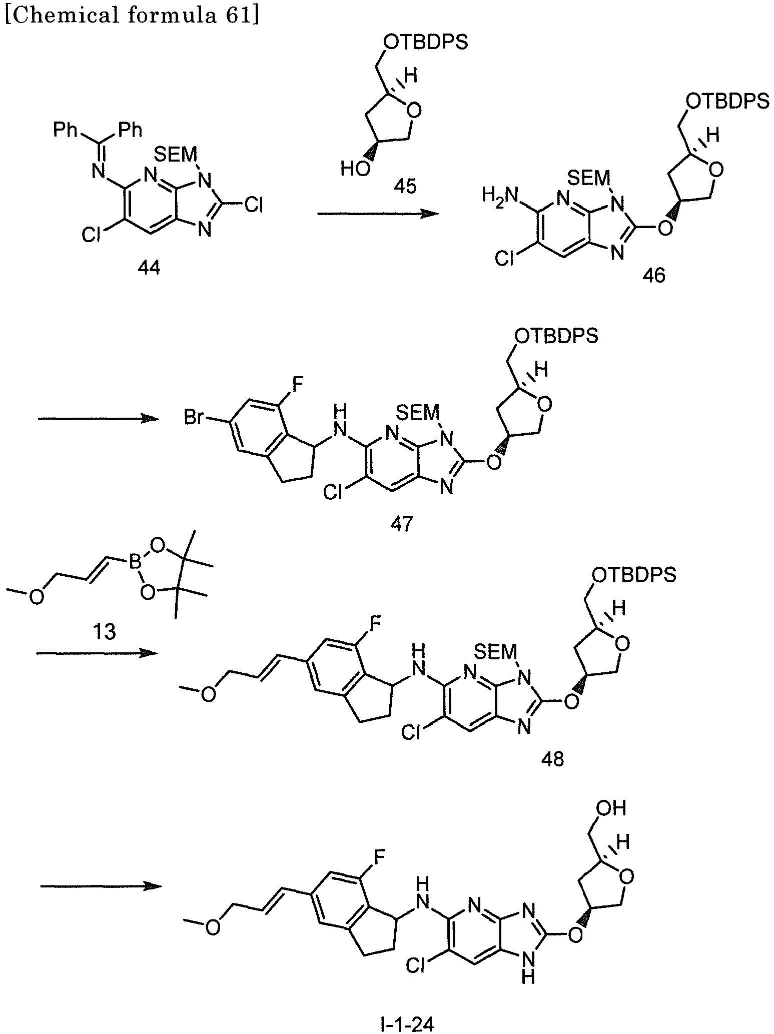

Compound 46 was synthesized from Compound 45, in a similar way that Compound 7 was synthesized from Compound 5. Compound 47 was synthesized from Compound 46, in a similar way that Compound 12 was synthesized from Compound 7. Compound 1-1-24 was synthesized from Compound 47, in a similar way that Compound 1-1-17 was synthesized from Compound 38. Compound 1-1-24; Method B

LC/MS retention time =1.91 min.
MS (ESI) m/z =489.3 (M+H)+.

### [Example 23]

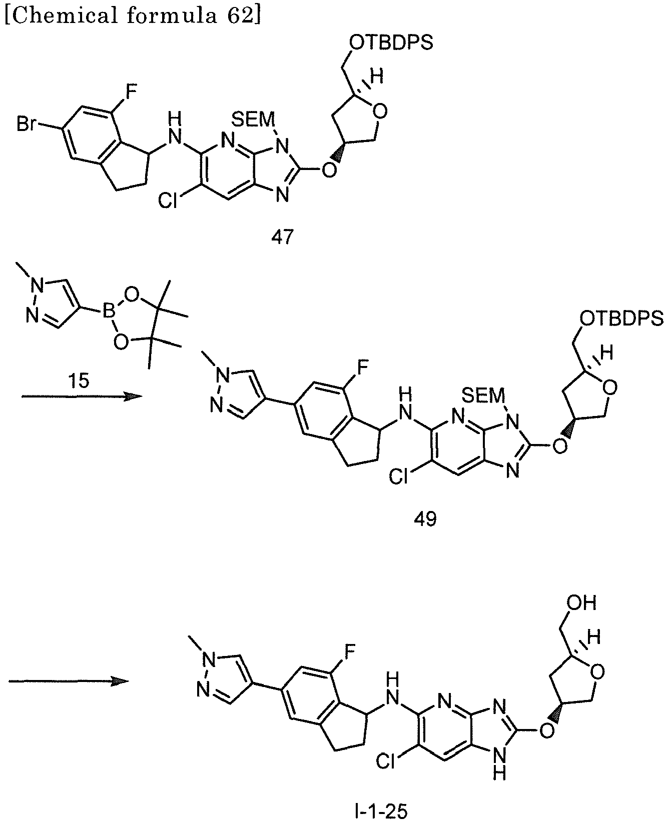

Compound I-1-25 was synthesized from Compound 15, in a similar way that Compound 1-1-24 was synthesized from Compound 13.

### Compound I-1-25; Method B

LC/MS retention time =1.63 min.
MS (ESI) m/z =499.25 (M+H)+.

### [Example 24]

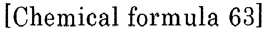

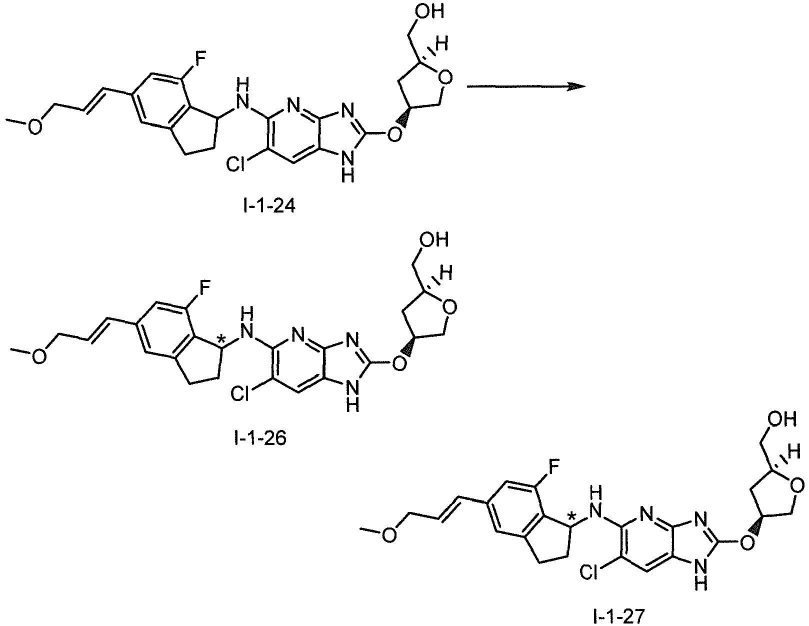

Optical resolution of Compound I-1-24 was carried out in the following conditions, and Compound 1-1-26 and Compound 1-1-27 were obtained as 1^{st} peak and 2^{nd} peak, respectively.
- Instrument: SemiPrep SFC
- Column: CHIRALPAK IA/SFC manufactured by DAICEL Corporation (5 µm, i.d. 20 x 250 mm)
- Mobile phase: EtOH containing 0.1% diethylamine and carbon dioxide
- Flow rate: 30 mL/min

### Compound 1-1-26; Method B

LC/MS retention time =1.913 min.
MS (ESI) m/z =489.30 (M+H)+.

### Compound 1-1-27; Method B

LC/MS retention time =1.913 min.
MS (ESI) m/z =489.30 (M+H)+.

### [Example 25]

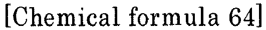

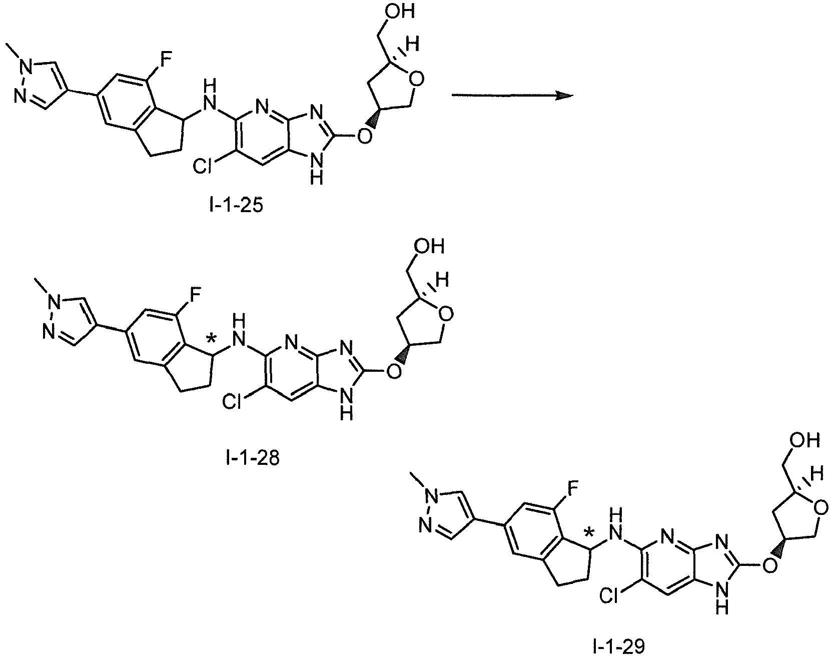

Optical resolution of Compound I-1-25 was carried out in the following conditions, and Compound I-1-28 and Compound I-1-29 were obtained as 1^{st} peak and 2^{nd} peak, respectively.
- Instrument: SemiPrep SFC
- Column: CHIRALPAK IA/SFC manufactured by DAICEL Corporation (5 µm, i.d. 20 x 250 mm)
- Mobile phase: MeOH containing 0.1% diethylamine and carbon dioxide
- Flow rate: 30 mL/min

### Compound 1-1-28; Method B

LC/MS retention time =1.64 min.
MS (ESI) m/z =499.25 (M+H)+.

### Compound 1-1-29; Method B

LC/MS retention time =1.64 min.
MS (ESI) m/z =499.5 (M+H)+.

### [Example 26]

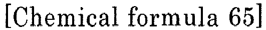

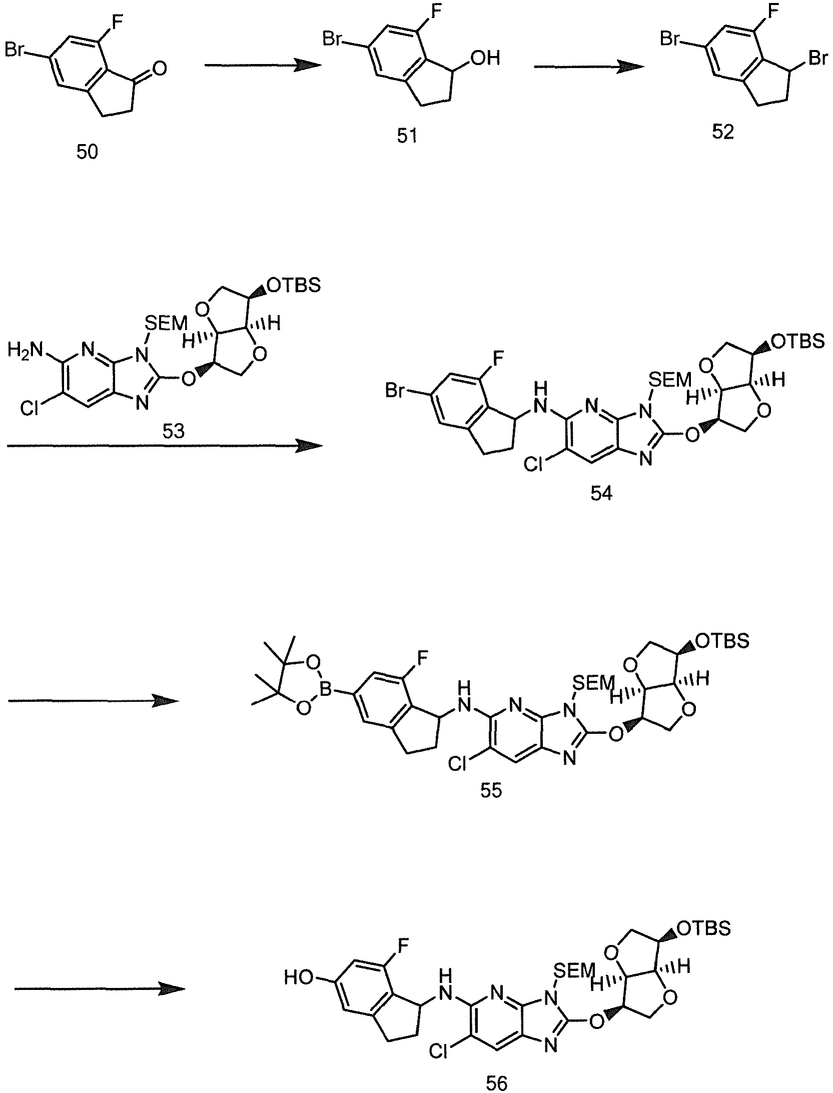

To 5-bromo-7-fluoro-2,3-dihydro-1H-inden-1-one (10 g, 54.7 mmol) were added 30 ml of methanol and 100 ml of THF, and the mixture was stirred at room temperature for 3 minutes. Subsequently, NaBH₄ (2.48 g, 65.5 mmol) was slowly added thereto while being kept at an inner temperature of 5°C or lower in an ice bath. Next, the ice bath was removed, and the mixture was stirred at room temperature for 30 minutes. Then, a saturated aqueous solution of ammonium chloride was added thereto in an ice bath, and the mixture was extracted with ethyl acetate. The extract was washed with water and then concentrated, and the obtained residue was purified by silica gel column chromatography to obtain Compound 51 (9.98 g, 98.9%) as a yellow oil.

Compound 51 (30 g, 130 mmol) was dissolved in 300 ml of dichloromethane, then pyridine (419 µl, 5.19 mmol) was added thereto, and the mixture was stirred in an ice bath for 5 minutes. Subsequently, phosphorus tribromide (6.12 ml, 64.9 mmol) was slowly added thereto, then the mixture was stirred in an ice bath for 30 minutes and concentrated under reduced pressure. The obtained residue was purified by diol silica gel column chromatography to obtain Compound 52 (36.9 g, 96.7%) as a yellow oil.

Compound 53 (155 mg, 0.278 mmol) was added to anhydrous THF (1 ml), and the mixture was stirred under nitrogen atmosphere at -78°C for 3 minutes. Subsequently, an nBuLi solution in hexane (2.66 M, 146 µl, 0.389 mmol) was added thereto, and the mixture was stirred for 5 minutes. Then, 500 µl of a solution of Compound 52 (114 mg, 0.389 mmol) in hexane was slowly added dropwise thereto at room temperature, and the mixture was stirred at 70°C for 2 hours. The reaction mixture was cooled to room temperature, and a saturated aqueous solution of ammonium chloride was added thereto, then the mixture was extracted with ethyl acetate. The extract was washed with water and then concentrated, and the obtained residue was purified by silica gel column chromatography to obtain Compound 54 (87.4 mg, 40.8%) as a yellow oil.

Compound 54 (20.6 g, 26.8 mmol), potassium acetate (7.89 g, 80 mmol), bis(pinacolato)diboron (10.2 g, 40.2 mol) and PdCl₂(dppf) were dissolved in degassed 1,4-dioxane (516 mL), then the mixture was stirred at 90°C for 2 hours under nitrogen atmosphere. Subsequently, the reaction mixture was added to a saturated aqueous solution of ammonium chloride in an ice bath, and the mixture was extracted with ethyl acetate. The extract was washed with saturated aqueous NaCl and then concentrated, and the obtained residue was purified by diol silica gel column chromatography to obtain Compound 55 (21.9 g, 99.9%) as a yellow oil.

Compound 55 (2.30 g, 2.81 mmol) was dissolved in acetone (40 ml), and oxone (2.24 g, 3.64 mmol) dissolved in 20 ml of water was added dropwise thereto under ice-cooling. The mixture was stirred for 30 minutes, then heated to room temperature and further stirred for 30 minutes. Subsequently, to the reaction mixture was added a saturated aqueous solution of sodium thiosulfate in an ice bath, and the mixture was extracted with ethyl acetate. The extract was washed with saturated aqueous NaCl and then concentrated, and the obtained residue was purified by silica gel column chromatography to obtain Compound 56 as a yellow powder.

### [Example 27]

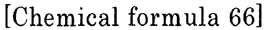

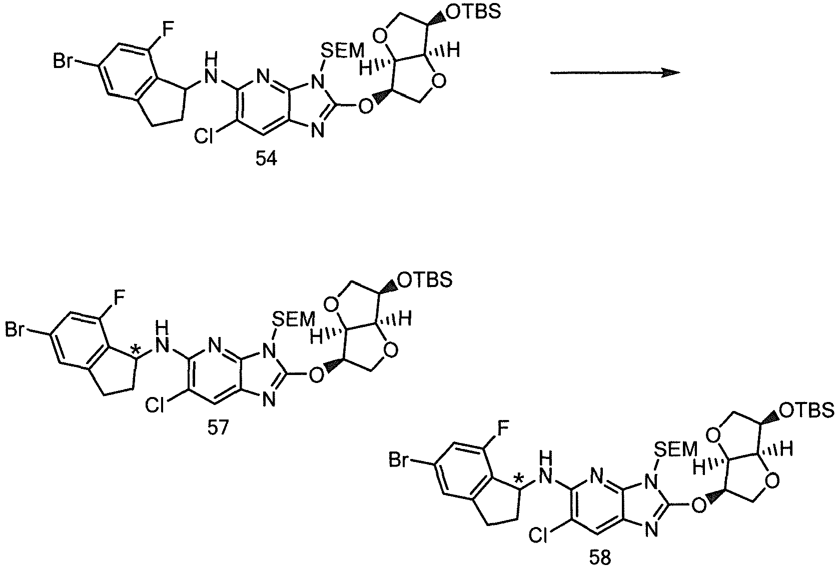

Optical resolution of Compound 54 was carried out in the following conditions, and Compound 57 and Compound 58 were obtained as 1^{st} peak and 2^{nd} peak, respectively.
- Instrument: 321 PUMP(GILSON), 215 LIQUID HANDLER(GILSON), UV/VIS-155(GILSON)
- Column: CHIRALPAK IC/SFC manufactured by DAICEL Corporation (5 µm, i.d. 20 x 250 mm)
- Mobile phase: only MeCN
- Flow rate: 11.4 mL/min

### [Example 28]

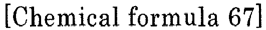

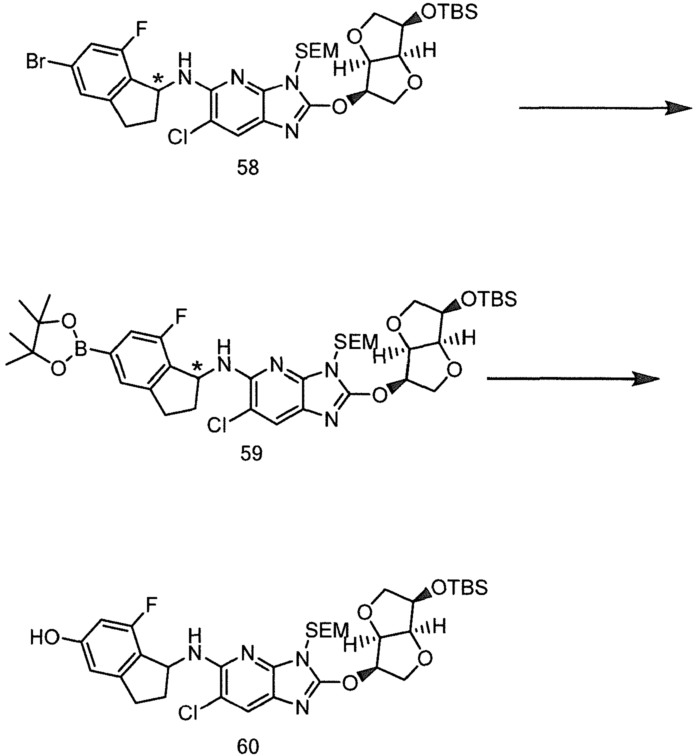

Compound 60 was obtained from Compound 58, in a similar way that Compound 56 was obtained from Compound 54.

### [Example 29]

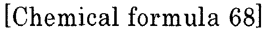

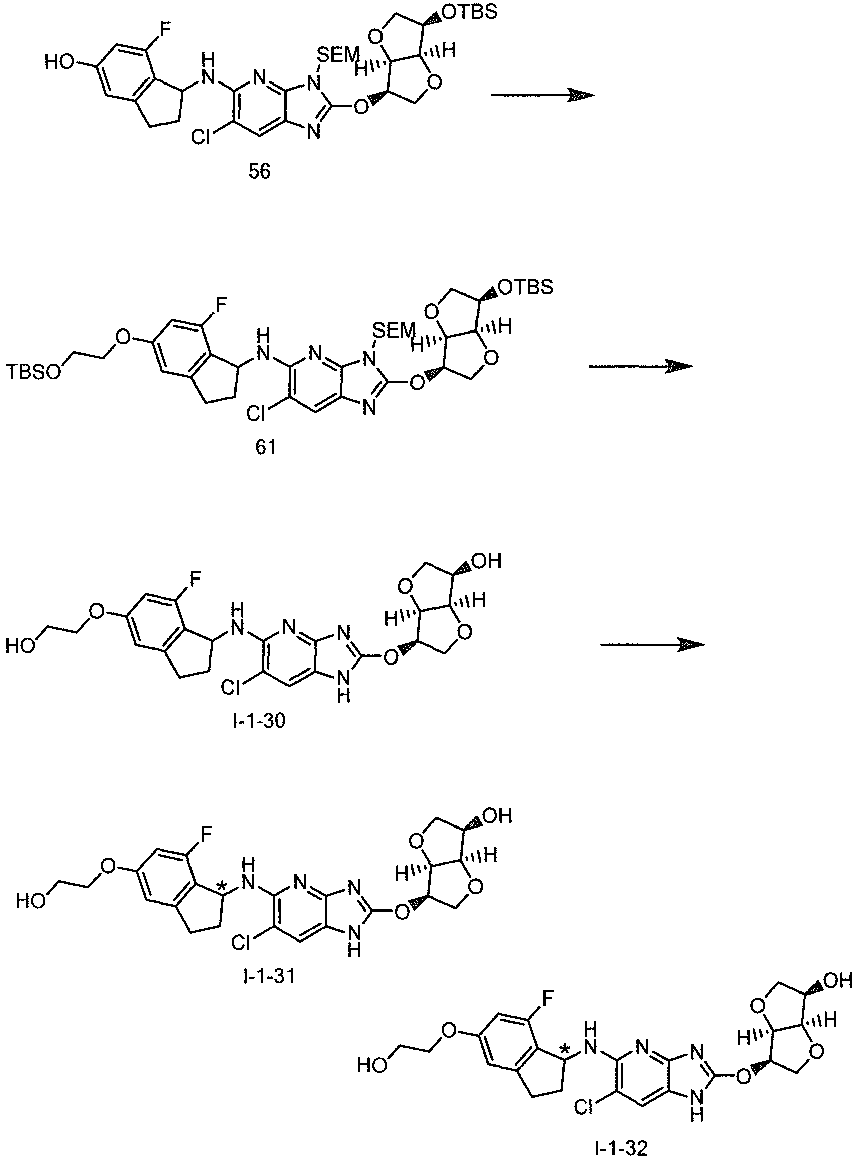

Compound 56 (70 mg, 0.099 mmol) was dissolved in DMF (700 µl), and (2-bromoethoxy)(tert-butyl)dimethylsilane (42 µl, 0.198 mmol) and cesium carbonate (64.5 mg, 0.198 mmol) were added thereto, then the mixture was stirred at 55°C. After completion of the reaction, a saturated aqueous solution of ammonium chloride was added thereto, and the mixture was extracted with ethyl acetate. The extract was washed with saturated aqueous NaCl and then concentrated, and the obtained residue was purified by silica gel column chromatography to obtain Compound 61 (71.4 mg) as a yellow powder.

To Compound 61 (71.4 mg, 0.082 mmol) was added 1.49 ml (1.48 mmol) of a 1 mol/L TBAF solution in tetrahydrofuran, and the mixture was sealed in a microwave reaction tube, and then stirred at 80°C for 20 hours. After completion of the reaction, a saturated aqueous solution of ammonium chloride was added thereto, and the mixture was extracted with ethyl acetate. The extract was washed with saturated aqueous NaCl and then concentrated, and the obtained residue was purified by silica gel column chromatography to obtain Compound I-1-30 (53.3 mg, 105 mmol) as a yellow powder.

### Compound I-1-30; Method B

LC/MS retention time =1.40 min.
MS (ESI) m/z =507.25 (M+H)+.

Optical resolution of Compound 1-1-30 was carried out in the following conditions, and Compound 1-1-31 and Compound 1-1-32 were obtained as 1^{st} peak and 2^{nd} peak, respectively.
- Instrument: SemiPrep SFC
- Column: CHIRALPAK ID/SFC manufactured by DAICEL Corporation (5 µm, i.d. 20 x 250 mm)
- Mobile phase: MeOH and carbon dioxide
- Flow rate: 30 mL/min

### Compound I-1-31; Method B

LC/MS retention time =1.47 min.
MS (ESI) m/z =507.00 (M+H)+.

### Compound I-1-32; Method B

LC/MS retention time =1.47 min.
MS (ESI) m/z =507.00 (M+H)+.

### [Example 30]

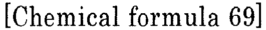

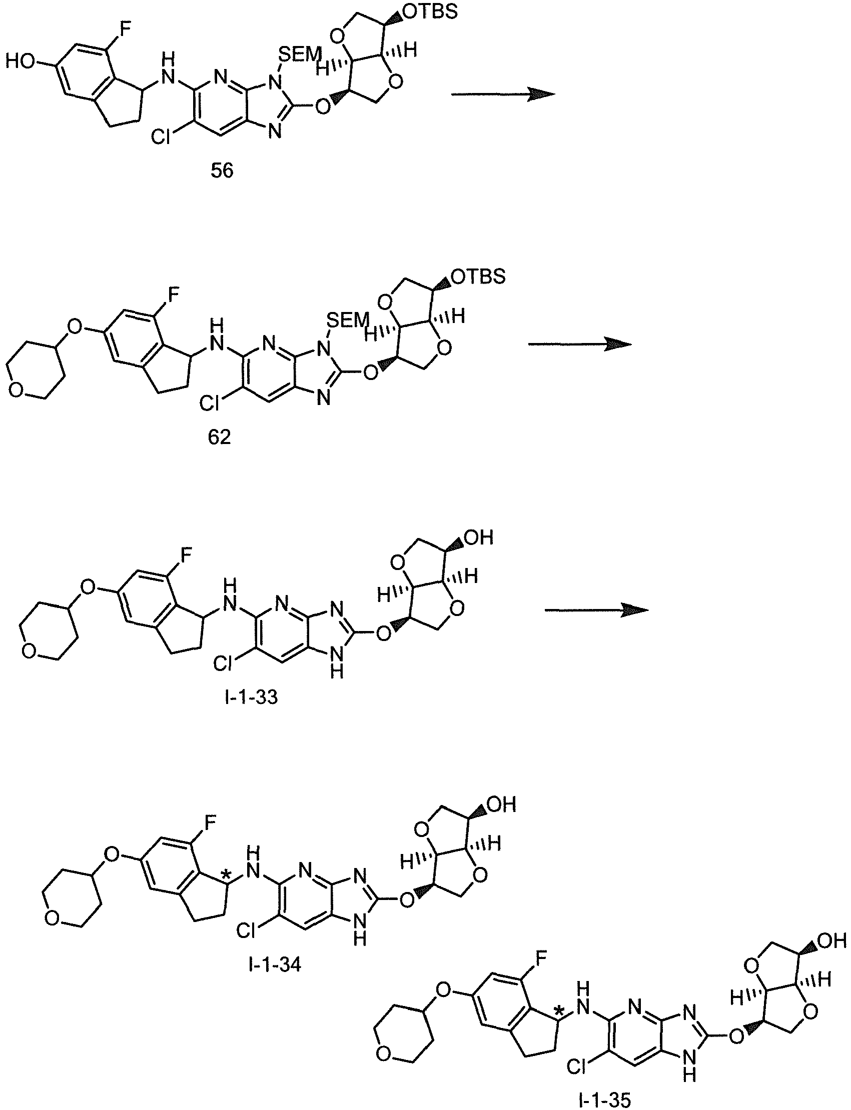

Compound 56 (50 mg, 0.071 mmol) was dissolved in THF (500 µl), and tetrahydropyran-4-ol (0.141 mmol) and DMEAD (33.1 mg, 0.141 mmol) were added thereto, then the mixture was stirred at room temperature. After completion of the reaction, the mixture was concentrated, and the obtained residue was purified by silica gel column chromatography to obtain Compound 62 (55.6 mg) as a yellow powder.

Compounds 1-1-34 and 1-1-35 were synthesized from Compound 62, in a similar way that Compounds 1-1-31 and 1-1-32 were synthesized from Compound 61.
- Instrument: SemiPrep SFC
- Column: CHIRALPAK ID/SFC manufactured by DAICEL Corporation (5 µm, i.d. 20 x 250 mm)
- Mobile phase: MeOH containing 0.1% diethylamine and carbon dioxide
- Flow rate: 30 mL/min

### Compound 1-1-34; Method B

LC/MS retention time =1.87 min.
MS (ESI) m/z =547.05 (M+H)+

### Compound I-1-35; Method B

LC/MS retention time =1.87 min.
MS (ESI) m/z =547.05 (M+H)+.

### [Example 31]

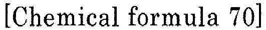

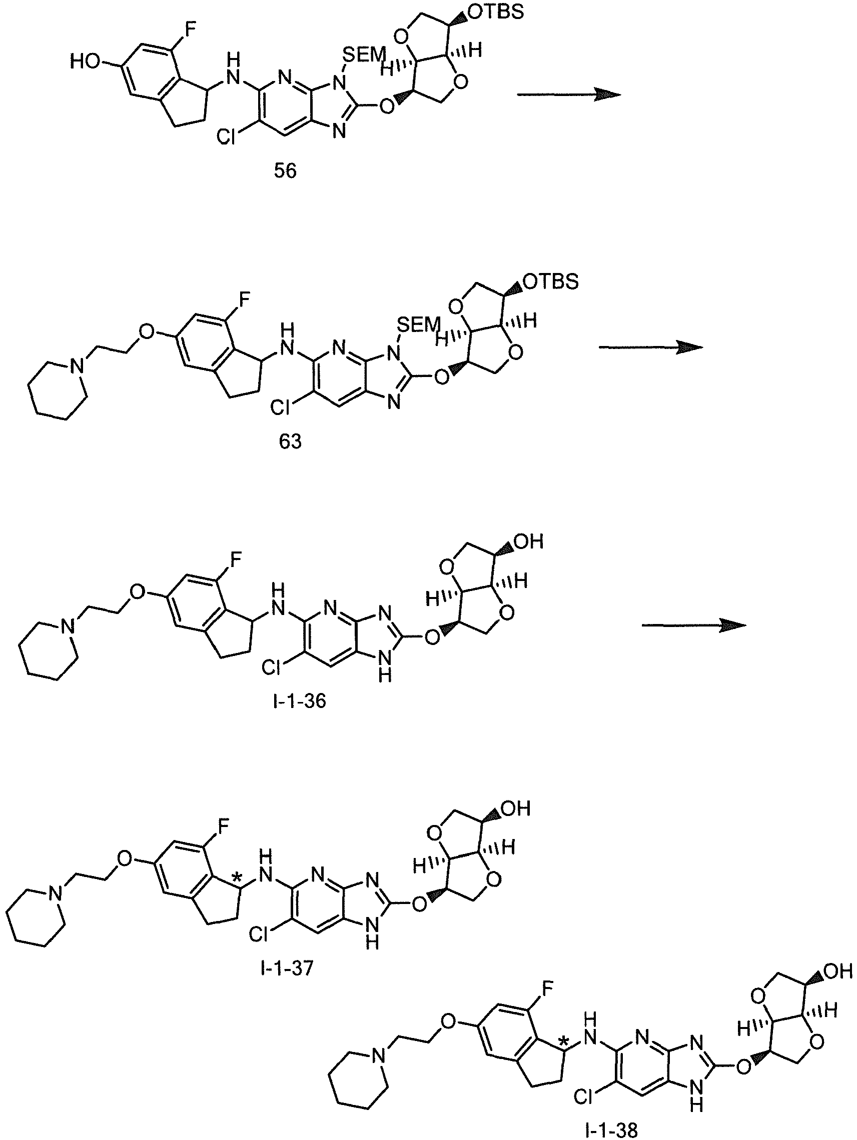

Compound 56 (50 mg, 0.071 mmol) was dissolved in THF (500 µl), and 2-(piperidin-1-yl)ethan-1-ol (0.141 mmol) and CMBP (0.141 mmol) were added thereto, then the mixture was stirred at 100°C. After completion of the reaction, and the obtained residue was purified by silica gel column chromatography to obtain Compound 63 as a yellow oil.

Compounds 1-1-37 and 1-1-38 were synthesized from Compound 63, in a similar way that Compounds 1-1-31 and 1-1-32 were synthesized from Compound 61.
- Instrument: SemiPrep SFC
- Column: CHIRALPAK ID/SFC manufactured by DAICEL Corporation (5 µm, i.d. 20 x 250 mm)
- Mobile phase: MeOH containing 0.1% diethylamine and carbon dioxide
- Flow rate: 30 mL/min

### Compound I-1-37; Method B

LC/MS retention time =1.08 min.
MS (ESI) m/z =574.55 (M+H)+.

### Compound I-1-38; Method B

LC/MS retention time =1.08 min.
MS (ESI) m/z =574.35 (M+H)+.

### [Example 32]

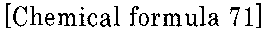

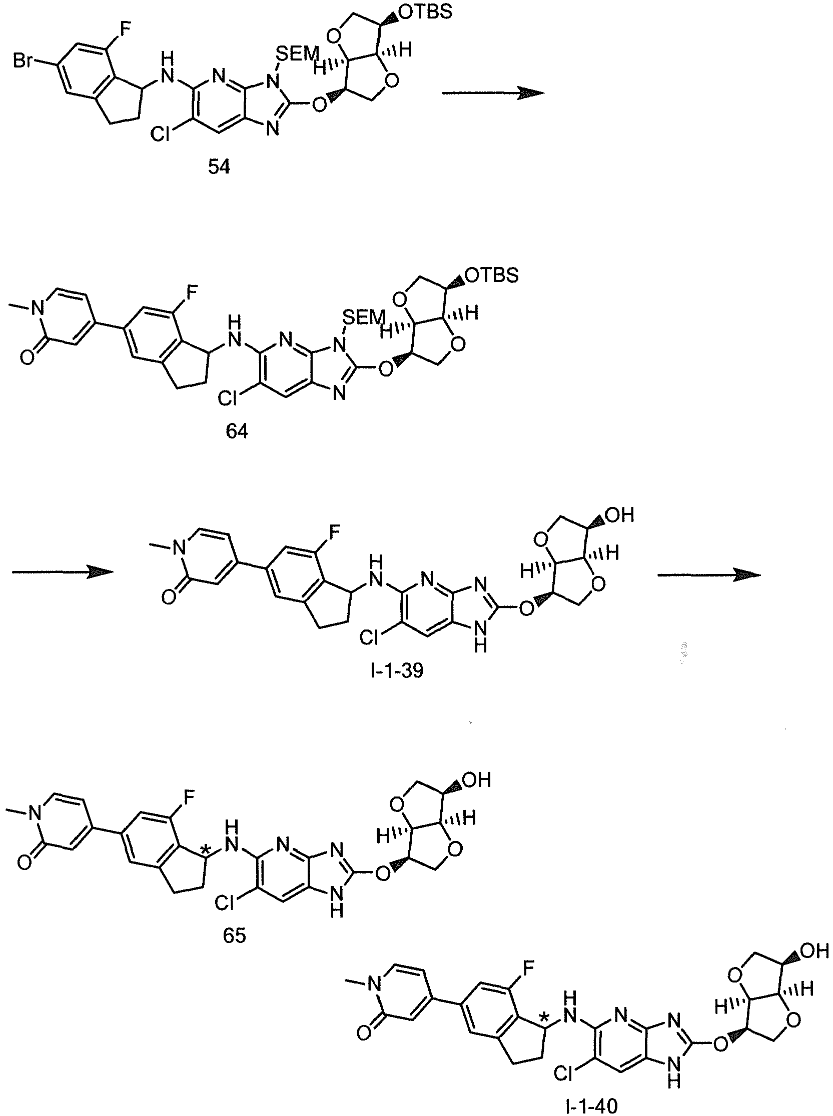

Compound 54 (50.0 mg, 0.065 mmol) and 1-methyl-4-(4,4,5,5-tetramethyl-1,3,2-dioxaboran-2-yl)pyridin-2(1H)-one (23 mg, 0.097 mmol) were dissolved in 1,4-dioxane (0.5 mL), and PdCl₂(dppf) (4.23 mg, 6.49 µmmol) and a 2 mol/L aqueous solution of potassium carbonate (97 µL, 0.195 mmol) were added thereto under nitrogen atmosphere, then the mixture was stirred at 80°C. After completion of the reaction, the resulting mixture was purified by silica gel column chromatography to obtain Compound 64.

Compounds 65 and 1-1-40 were synthesized from Compound 64, in a similar way that Compounds 1-1-31 and 1-1-32 were synthesized from Compound 61.
- Instrument: SemiPrep SFC
- Column: CHIRALPAK IA/SFC manufactured by DAICEL Corporation (5 µm, i.d. 20 x 250 mm)
- Mobile phase: MeOH containing 0.1% diethylamine and carbon dioxide
- Flow rate: 20 mL/min

### Compound I-1-40; Method B

LC/MS retention time =1.44 min.
MS (ESI) m/z =554.25 (M+H)+.

### [Example 33]

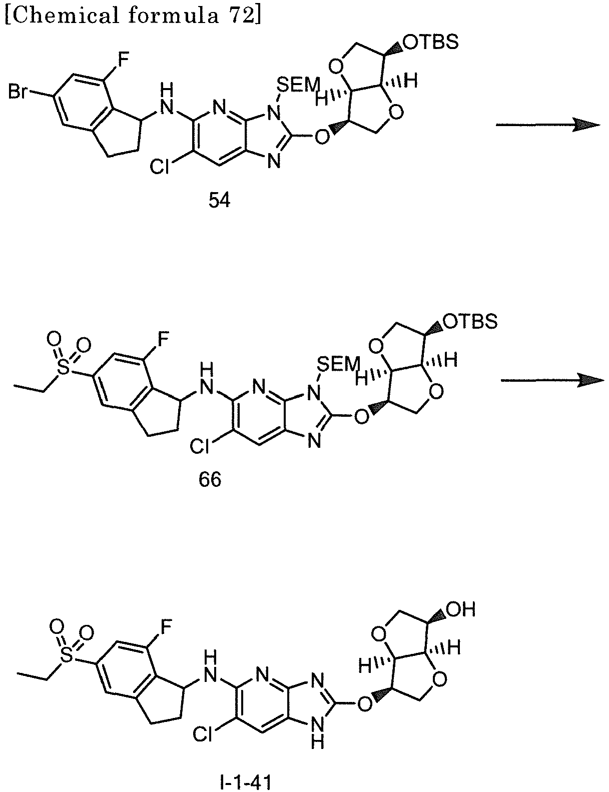

Compound 54 (50.0 mg, 0.065 mmol), sodium ethylsulfinate (14.9 mg, 0.130 mmol) and copper(II) trifluoromethanesulfonate (23.5 mg, 0.065 mmol) were dissolved in DMSO (0.3 mL), and 1,2-ethylenediamine (14.7 µl, 0.136 mmol) was added thereto, and the mixture was stirred at 120°C under nitrogen atmosphere. Subsequently, the reaction mixture was added to a saturated aqueous solution of ammonium chloride in an ice bath, and the mixture was extracted with ethyl acetate. The extract was washed with saturated aqueous NaCl and then concentrated, and the obtained residue was purified by diol silica gel column chromatography to obtain Compound 66 (12.8 mg, 25.2%).

Compound I-1-41 was synthesized from Compound 66, in a similar way that Compound 1-1-30 was synthesized from Compound 61.

### Compound I-1-41; Method B

LC/MS retention time =1.52 min.
MS (ESI) m/z =539.25 (M+H)+.

### [Example 34]

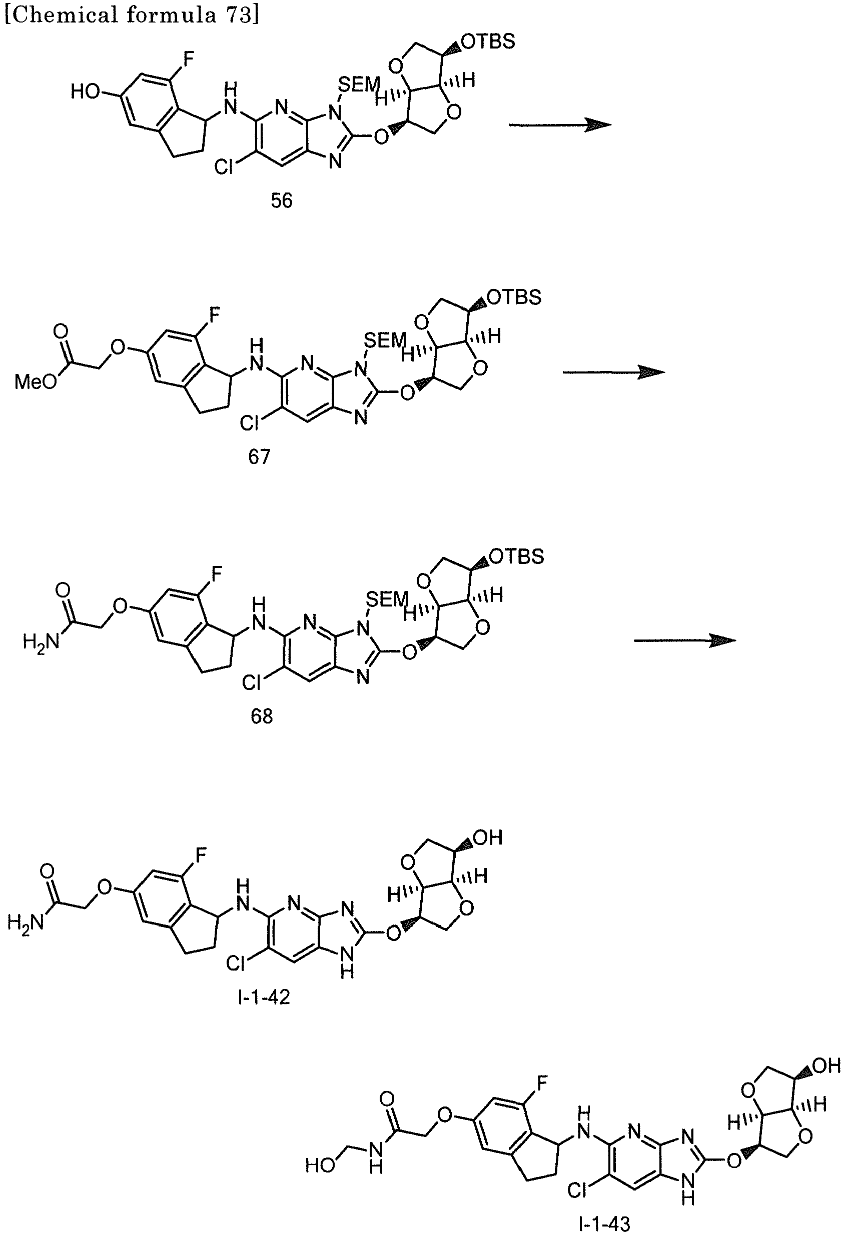

Compound 67 was synthesized, in a similar way that Compound 61 was synthesized from Compound 56.

Compound 67 (60 mg, 0.077 mmol) was dissolved in a mixed solvent of 150 µl of THF and 150 µl of methanol, then a 2 mol/L aqueous solution of sodium hydroxide (57.7 µl, 0.115 mmol) was added thereto, and the mixture was stirred at room temperature. After completion of the reaction, the mixture was concentrated and dried under reduced pressure, then dissolved in 600 µl of THF. Subsequently, isobutyl formate (22.2 µl, 0.154 mmol) and triethylamine (21.3 µl, 0.154 mmol) were added thereto in an ice bath, and the mixture was stirred for 30 minutes. Then, the ice bath was removed, a 28% aqueous solution of ammonia (59.3 µl, 0.770 mmol) was added thereto, and the mixture was further stirred for 1 hour. Subsequently, the reaction mixture was added to a saturated aqueous solution of ammonium chloride in an ice bath, and the mixture was extracted with ethyl acetate. The extract was washed with saturated aqueous NaCl and then concentrated, and the obtained residue was purified by diol silica gel column chromatography to obtain Compound 68 (30.6 mg, 52.0%).

To Compound 68 (30.6 mg) was added 722 µl of a 1 mol/L TBAF solution in tetrahydrofuran, and the mixture was sealed in a microwave reaction tube and then stirred at 80°C for 20 hours. After completion of the reaction, a saturated aqueous solution of ammonium chloride was added thereto, and the mixture was extracted with ethyl acetate. The extract was washed with saturated aqueous NaCl and then concentrated, and the obtained residue was purified by silica gel column chromatography to obtain Compound 1-1-42 and Compound I-1-43.

### Compound I-1-42; Method B

LC/MS retention time =1.25 min.
MS (ESI) m/z =520.30 (M+H)+.

### Compound 1-1-43; Method B

LC/MS retention time =1.23 min.
MS (ESI) m/z =550.30 (M+H)+.

### [Example 35]

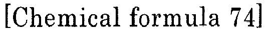

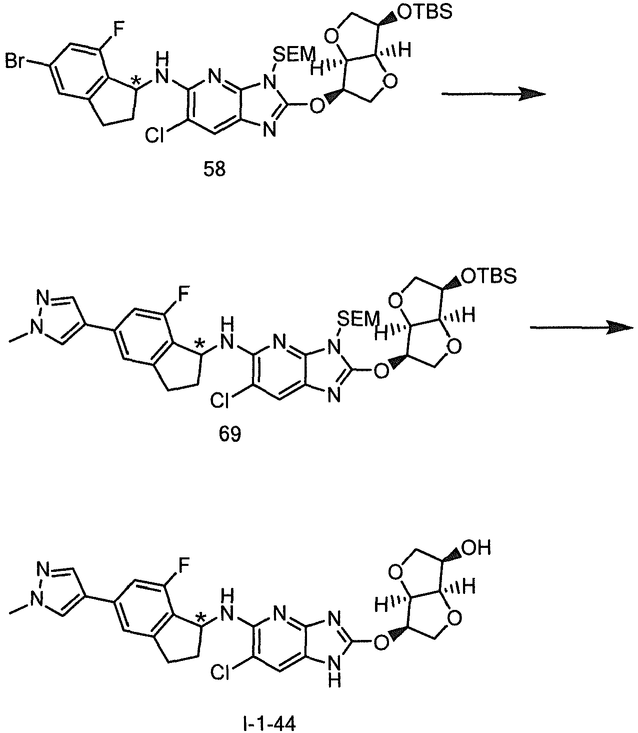

Compound I-1-44 was synthesized from Compound 58, in a similar way that Compound 1-1-39 was synthesized from Compound 54.

### Compound I-1-44; Method B

LC/MS retention time =1.62 min.
MS (ESI) m/z =527.25 (M+H)+.

### [Example 36]

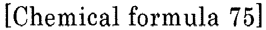

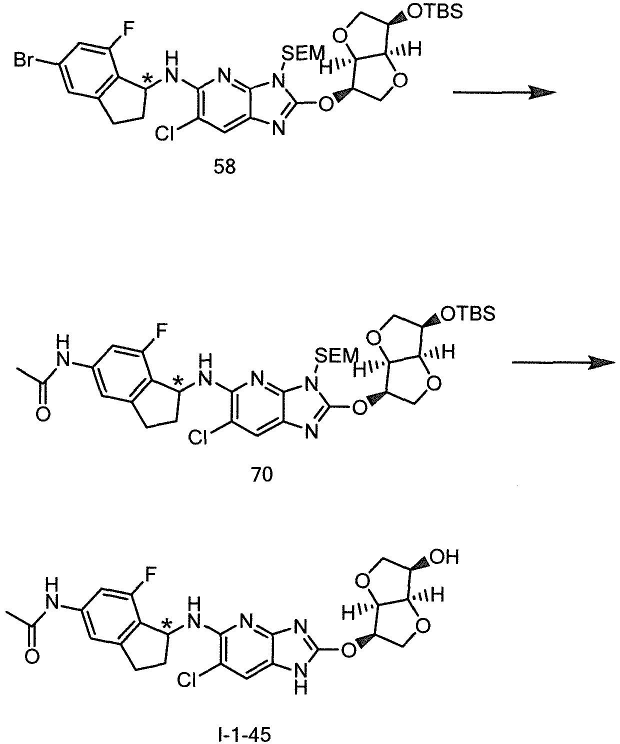

Compound 58 (50.0 mg, 0.065 mmol), acetamide (5.75 mg, 0.097 mmol), Xantphos (11.3 mg, 0.019 mmol) and Pd₂(dba)₃ (5.94 mg, 6.49 µmol) were dissolved in 1,4-dioxane (0.75 mL), and the mixture was stirred at 100°C under nitrogen atmosphere. After completion of the reaction, the reaction mixture was added to a saturated aqueous solution of ammonium chloride in an ice bath, and the mixture was extracted with ethyl acetate. The extract was washed with saturated aqueous NaCl and then concentrated, and the obtained residue was purified by diol silica gel column chromatography to obtain Compound 70 (48.4 mg, 99%).

Compound I-1-45 was synthesized from Compound 70, in a similar way that Compound 1-1-30 was synthesized from Compound 61.

### Compound 1-1-45; Method B

LC/MS retention time =1.37 min.
MS (ESI) m/z =504.2 (M+H)+.

### [Example 37]

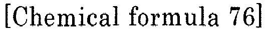

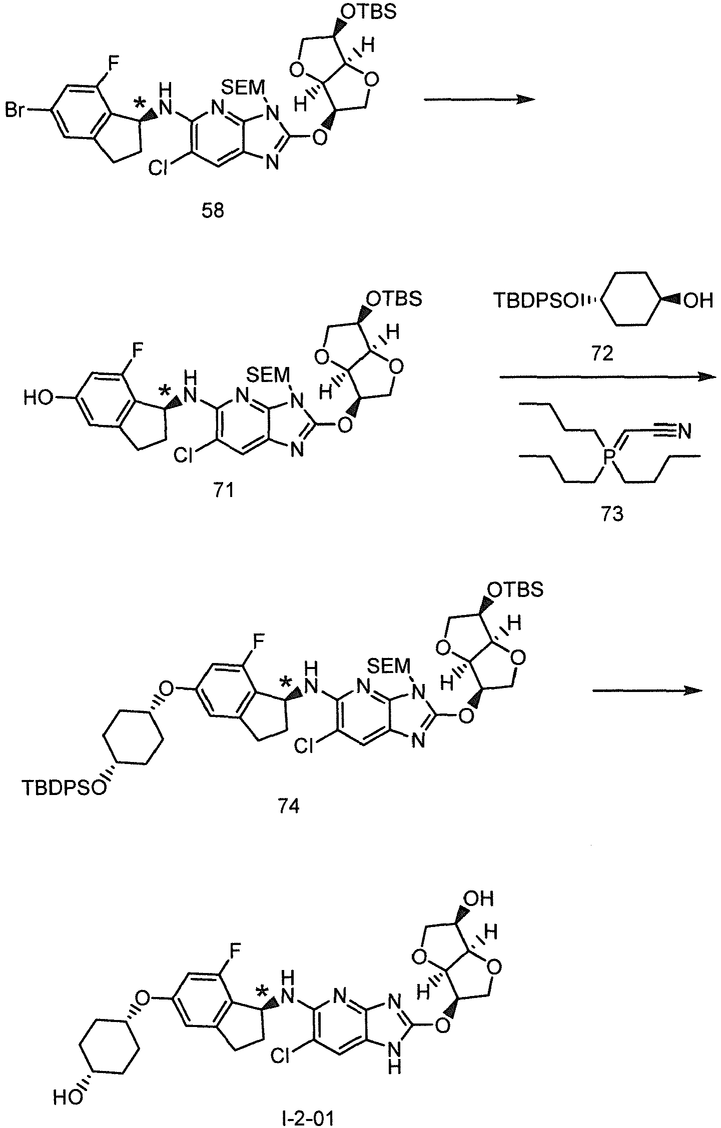

Compound 58 (266 mg, 0.345 mmol) was dissolved in 1,4-dioxane (2.66 mL) under nitrogen atmosphere, and bis (pinacolato) diboron (132 mg, 0.518 mmol), potassium acetate (102 mg, 1.036mmol) and PdCl₂(dppf) (25.3mg, 0.035 mmol) were added thereto, and the mixture was stirred at 90°C for 2 hours. After cooling, to the reaction mixture was added a saturated aqueous solution of ammonium chloride, and the mixture was extracted with ethyl acetate. The extract was washed with saturated aqueous sodium chloride and then concentrated under reduced pressure. To the obtained residue were added dichloromethane (5.32 mL) and methanol (2.66 mL). Hydrogen peroxide (30%, 0.353 mL, 3.45 mmol) was added thereto at 0°C, and the mixture was stirred at room temperature. To the reaction mixture was added a saturated aqueous solution of sodium thiosulfate, and the mixture was extracted with ethyl acetate. The extract was dried with sodium thiosulfate, filtered and concentrated under reduced pressure. The obtained residue was purified by silica gel column chromatography to obtain Compound 71. To Compound 71 (30.0 mg, 0.042 mmol) and Compound 72 (30.1 mg, 0.085 mmol) were added toluene (0.45 mL) and Compound 73 (20.5 mg, 0.085 mmol), and the mixture was stirred at 100°C. The reaction mixture was purified by silica gel column chromatography to obtain Compound 74 (36.3 mg, 82.0%). To Compound 74 (32 mg, 0.031 mmol) was added TBAF (1 mol/L in THF, 0.613 mL, 0.613 mmol), and the mixture was heated under reflux. The reaction mixture was cooled to room temperature, and poured into separating funnel. Ethyl acetate was added thereto and washed with a saturated aqueous solution of citric acid and a saturated aqueous solution of sodium chloride. The obtained organic layer was concentrated under reduced pressure. The obtained residue was purified by silica gel column chromatography to obtain Compound (I-2-01) (8.6mg, 50.0%).

### Compound (1-2-01); Method B

LC/MS retention time =1.65 min.
MS (ESI) m/z =561.2 (M+H)+.

### [Example 38]

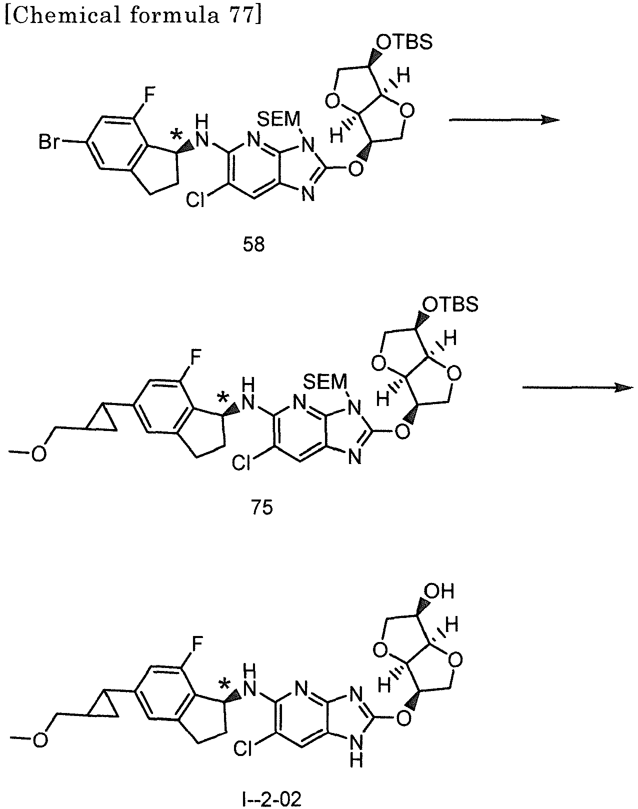

Compound 58 (50 mg, 0.0651 mmol) was dissolved in 1,4-dioxane (0.9 mL) under nitrogen atmosphere, and 2-(2-(methoxymethyl)cyclopropyl)-4,4,5,5-tetramethyl-1,3,2-dioxaborolane (20.7 mg, 0.97 mmol), a 2 mol/L aqueous solution of potassium acetate (97.3 ul, 0.195 mmol) and PdCl₂(dppf) (4.75 mg, 0.00648 mmol) were added thereto, and the mixture was stirred at 110°C for 3 hours. After cooling, the reaction mixture was added to a saturated aqueous solution of ammonium chloride, and the mixture was extracted with ethyl acetate. The extract was washed with saturated aqueous sodium chloride and then concentrated under reduced pressure. The obtained residue was purified by silica gel column chromatography to obtain Compound 75 (36mg, 71.5%). To Compound 75 (36 mg, 0.046 mmol) was added TBAF (1 mol/L in THF, 0.836 mL, 0.836 mmol), and the mixture was heated under reflux. The reaction mixture was cooled to room temperature, and poured into separating funnel. Ethyl acetate was added thereto, and the mixture was washed with a saturated aqueous solution of citric acid and a saturated aqueous solution of sodium chloride. The obtained organic layer was concentrated under reduced pressure, and the obtained residue was purified by silica gel column chromatography to obtain Compound (1-2-02) (23.9 mg, 97.0%).

### Compound (I-2-02); Method B

LC/MS retention time =1.92 min.
MS (ESI) m/z =531.3 (M+H)+.

### [Example 39]

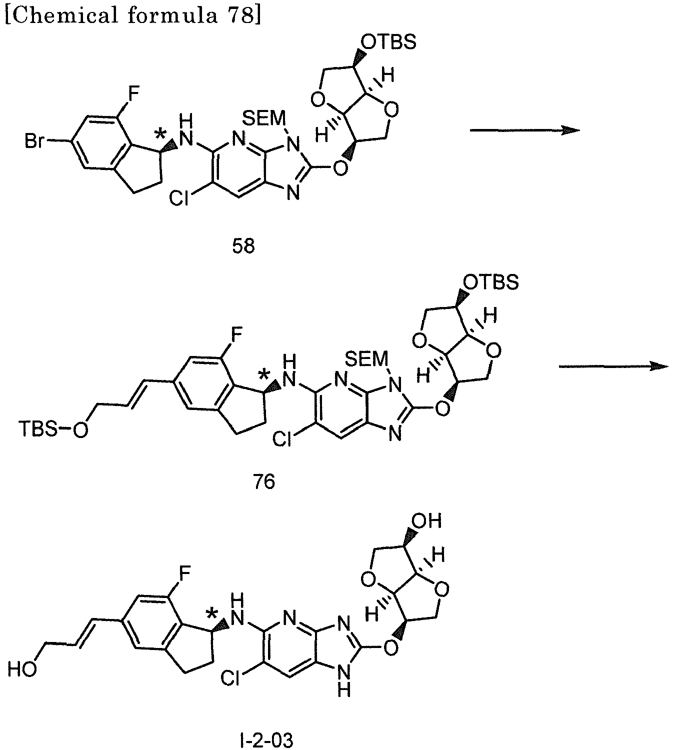

Compound 58 (35 mg, 0.035 mmol) was dissolved in 1,4-dioxane (0.35 mL) under nitrogen atmosphere, and (E)-tert-butyldimethyl ((3-(4,4,5,5-tetramethyl-1,3,2-dioxaborolane-2-yl) allyl)oxy) silane (20.1mg, 0.068mmol), a 2 mol/L aqueous solution of potassium carbonate (68.2 ul, 0.136 mmol) and PdCl₂(dppf) (2.96 mg, 0.00454 mmol) were added thereto, and the mixture was stirred at 80°C for 2 hours. After cooling, the reaction mixture was added to a saturated aqueous solution of ammonium chloride, and the mixture was extracted with ethyl acetate. The extract was dried with sodium sulfate, filtered and concentrated under reduced pressure. The obtained residue was purified by silica gel column chromatography to obtain Compound 76. To Compound 76 was added TBAF (1 mol/L in THF, 0.25 mL), and the mixture was heated under reflux for 24 hours. The reaction mixture was cooled to room temperature, and poured into separating funnel. Ethyl acetate was added thereto, and the mixture was washed with a saturated aqueous solution of citric acid and a saturated aqueous solution of sodium chloride. The obtained organic layer was concentrated under reduced pressure, and the obtained residue was purified by silica gel column chromatography to obtain Compound (I-2-03) (2.5 mg, 11%).

### Compound (1-2-03); Method B

LC/MS retention time =1.62 min.
MS (ESI) m/z =503.1 (M+H)+.

### [Example 40]

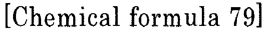

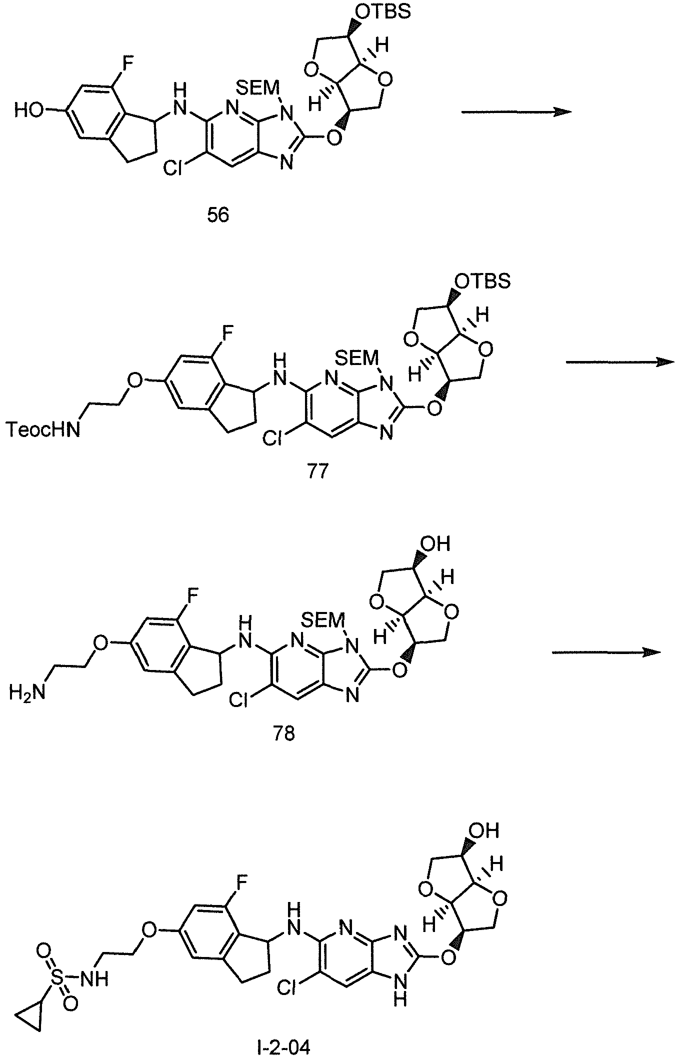

Compound 56 (160 mg, 0.226 mmol) was dissolved in toluene (1600 µl), and 2-[N-[2-trimethylsilyl)ethoxycarbonyl]amino] ethanol (93 mg, 0.452 mmol) and CMBP (109 mg, 0.452 mmol) were added thereto, and the mixture was stirred at 100°C. After completion of the reaction, the mixture was concentrated. The obtained residue was purified by silica gel column chromatography to obtain Compound 77 (162 mg, 80.1%) as a yellow oil.

Compound 77 (151mg, 0.169 mmol) was dissolved in THF (1.5 mL), and TBAF (1mol/L in THF, 0.422mL) was added thereto, and the mixture was stirred at room temperature for 90 hours. A saturated aqueous solution of sodium hydrogen carbonate was added thereto, and the reaction mixture was poured into separating funnel. The mixture was extracted with ethyl acetate. The extract was washed with a saturated aqueous solution of sodium chloride. The obtained organic layer was concentrated under reduced pressure, and the obtained residue was purified by amino silica gel column chromatography to obtain Compound 78 (99.5 mg, 93%).

Compound 78 (20 mg, 0.031 mmol) was dissolved in dichloromethane (0.4 mL), and triethylamine (0.00654 mL, 0.047 mmol) and cyclopropanesulfonyl chloride (4.92 mg, 0.035 mmol) were added thereto, and the mixture was stirred at room temperature. After completion of the reaction, a saturated aqueous solution of ammonium chloride was added thereto, and the mixture was extracted with ethyl acetate. The extract was washed with a saturated aqueous solution of sodium chloride and the mixture was concentrated. To the obtained residue was added TBAF (1mol/L in THF, 0.566mL), the mixture was heated under reflux. The reaction mixture was cooled to room temperature, and poured into separating funnel. Ethyl acetate was added thereto and washed with a saturated aqueous solution of citric acid and a saturated aqueous solution of sodium chloride. The obtained organic layer was concentrated under reduced pressure, and the obtained residue was purified by silica gel column chromatography to obtain Compound (I-2-04) (15 mg).

### Compound (1-2-04); Method B

LC/MS retention time =1.63 min.
MS (ESI) m/z =610.3 (M+H)+.

### [Example 41]

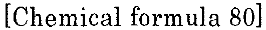

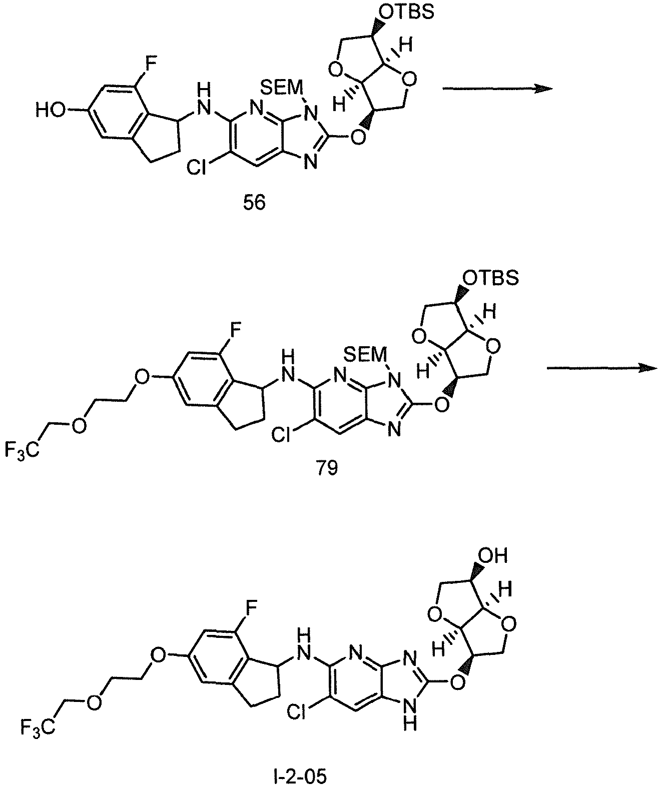

Compound 56 (28.7 mg, 0.041 mmol) was dissolved in DMF (287 µl), and 2-(2-bromoethoxy)-1,1,1-trifluoroethane (10.6 µl, 0.081 mmol) and cesium carbonate (26.4 mg, 0.081 mmol) were added thereto, and the mixture was stirred at 60°C for 1 hour. A saturated aqueous solution of ammonium chloride was added thereto, and the mixture was extracted with ethyl acetate. The extract was washed with a saturated aqueous solution of sodium chloride. The mixture was concentrated under reduced pressure, and the obtained residue was purified by silica gel column chromatography to obtain Compound 79 (22.4 mg).

To Compound 79 (22.4 mg) was added TBAF (1mol/L in THF, 0.484mL), and the mixture was heated under reflux. The reaction mixture was cooled to room temperature, and poured into separating funnel. Ethyl acetate was added thereto and washed with a saturated aqueous solution of citric acid and a saturated aqueous solution of sodium chloride. The obtained organic layer was concentrated under reduced pressure, and the obtained residue was purified by silica gel column chromatography to obtain Compound (1-2-05).

### Compound (1-2-05); Method B

LC/MS retention time =2.00 min.
MS (ESI) m/z =589.3 (M+H)+.

### [Example 42]

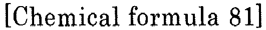

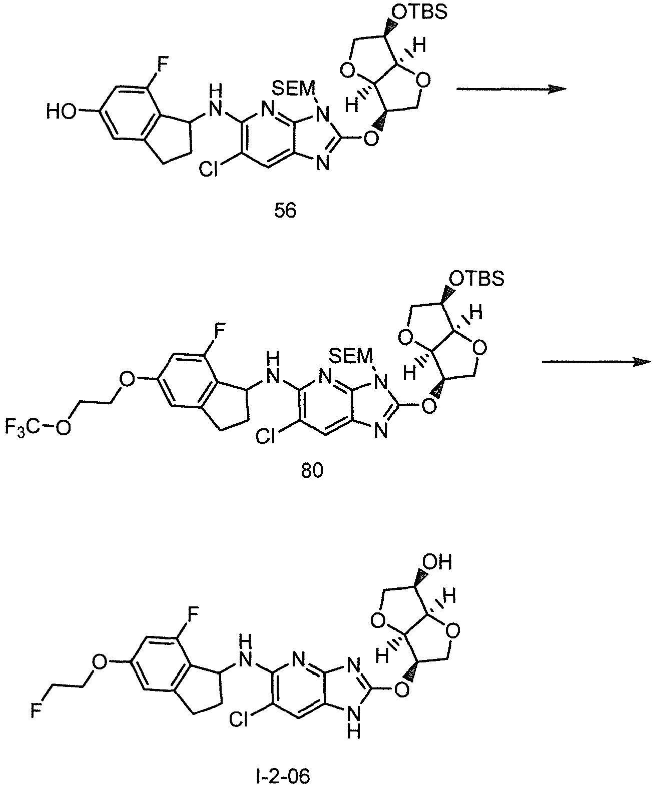

Compound 80 was synthesized from Compound 56 and 1-bromo-2-(trifluoromethoxy)ethane, in a similar way that Compound 79 was synthesized from Compound 56.

To Compound 80 (25.4 mg) was added TBAF (1mol/L in THF, 0.558mL), and the mixture was heated under reflux. The reaction mixture was cooled to room temperature, and poured into separating funnel. Ethyl acetate was added thereto, and the mixture was washed with a saturated aqueous solution of citric acid and a saturated aqueous solution of sodium chloride. The obtained organic layer was concentrated under reduced pressure, and the obtained residue was purified by silica gel column chromatography to obtain Compound (1-2-06).

### Compound (1-2-06); Method B

LC/MS retention time =1.76 min.
MS (ESI) m/z =509.3 (M+H)+.

### [Example 43]

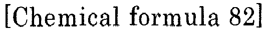

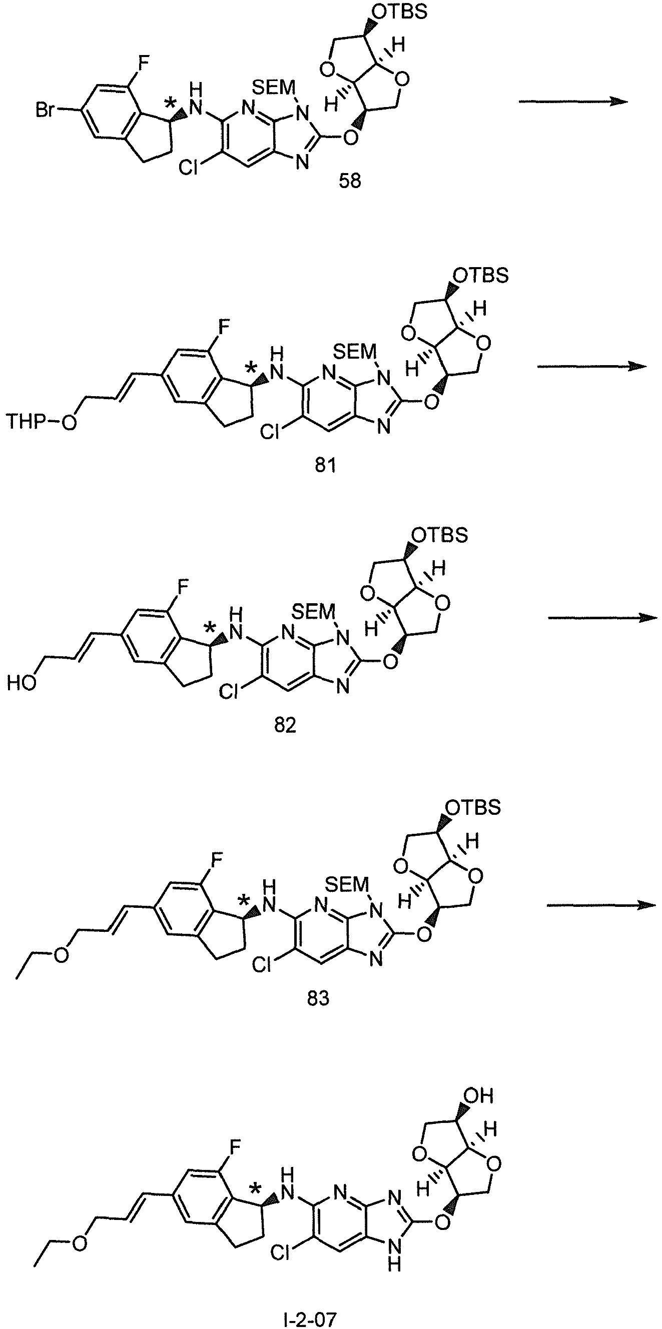

Compound 58 (30 mg, 0.039 mmol) was dissolved in 1,4-dioxane (0.9 mL) under nitrogen atmosphere, and (E)-4,4,5,5-tetramethyl-2-(3-((tetrahydro-2H-pyran-2-yl)oxy) propylene-1-yl)-1,3,2-dioxaborolane (16 mg, 0.058 mmol), a 2 mol/L aqueous solution of potassium carbonate (58.4ul, 0.117mmol) and PdCl₂(dppf) (2.54mg, 0.00389mmol) were added thereto, and the mixture was stirred at 80°C for 2 hours. After cooling, the reaction mixture was added to a saturated aqueous solution of ammonium chloride, and the mixture was extracted with ethyl acetate. The extract was dried with sodium sulfate, filtered and concentrated under reduced pressure. The obtained residue was purified by silica gel column chromatography to obtain Compound 81 (28.4mg, 87%).

To Compound 81 (83mg, 0.100mmol) dissolved in methanol (2.5 ml) was added PPTS (7.52mg, 0.030mmol), and the mixture was stirred at 60°C for 1 hour. After cooling, water was added thereto, and the mixture was extracted with ethyl acetate. The extract was dried with sodium sulfate, filtered and concentrated under reduced pressure. The obtained residue was purified by silica gel column chromatography to obtain Compound 82 (52.9 mg, 70.9%).

Compound 82 (33 mg) was dissolved in DMF (0.4 ml), and sodium hydride (60%, 3.53 mg, 0.088 mmol) and ethyl iodide (0.0107 mL, 0.132 mmol) were added thereto, and the mixture was stirred at room temperature for 1 hour. After cooling, a saturated aqueous solution of ammonium chloride was added thereto, and the mixture was extracted with ethyl acetate. The extract was washed with a saturated aqueous solution of sodium chloride, dried with sodium sulfate, filtered and concentrated under reduced pressure. The obtained residue was purified by silica gel column chromatography to obtain Compound 83 (18.9 mg, 55.2%).

Compound (I-2-07) was synthesized from Compound 83, in a similar way that Compound (I-2-03) was synthesized from Compound 76.

### Compound (I-2-07); Method B

LC/MS retention time =2.03 min.
MS (ESI) m/z =531.15 (M+H)+.

### [Example 44]

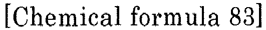

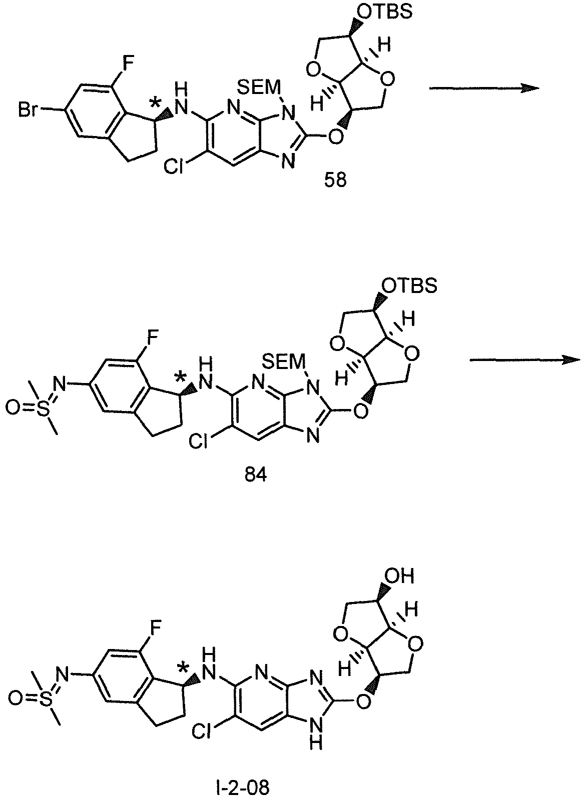

Compound 58 (30 mg, 0.039 mmol) was dissolved in 1,4-dioxane (0.3 mL) under nitrogen atmosphere, and dimethylsulfoximine (5.08 mg, 0.055 mmol), cesium carbonate (17.8 mg, 0.055 mmol), Xantpohs (3.38 mg, 0.00584 mmol) and Pd₂(dba)₃ (1.78 mg, 0.00195 mmol) were added thereto, and the mixture was stirred at 100°C for 3 hours. The reaction mixture was purified by silica gel column chromatography to obtain Compound 84 (30.3mg, 99%).

Compound (I-2-08) was synthesized from Compound 84, in a similar way that Compound (I-2-03) was synthesized from Compound 76.

### Compound (I-2-08); Method B

LC/MS retention time =1.30 min.
MS (ESI) m/z =538.10 (M+H)+.

### [Example 45]

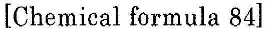

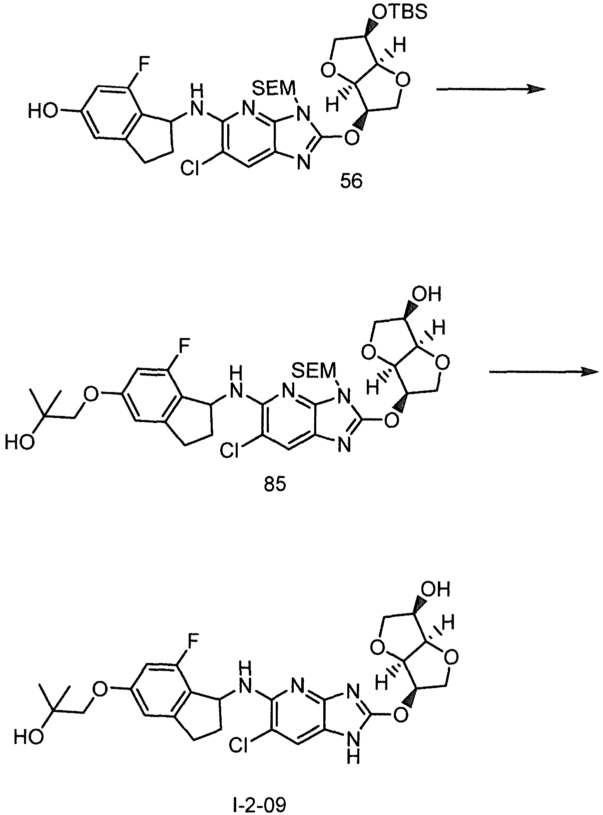

Compound 56 (40 mg, 0.057 mmol) was dissolved in DMF (400 µl), and 2,2-dimethyloxirane (100 µl, 1.13 mmol) and cesium carbonate (36.8 mg, 0.113 mmol) were added thereto, and the mixture was stirred at 100°C for 3 hours. A saturated aqueous solution of ammonium chloride was added thereto, and the mixture was extracted with ethyl acetate. The extract was washed with a saturated aqueous soluttion of sodium chloride and then concentrated. The obtained residue was purified by silica gel column chromatography to obtain Compound 85 (17.4 mg).

Compound (1-2-09) was synthesized from Compound 85, in a similar way that Compound (I-2-03) was synthesized from Compound 76.

### Compound (I-2-09); Method B

LC/MS retention time =1.66 min.
MS (ESI) m/z =535.35 (M+H)+.

### [Example 46]

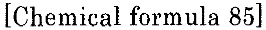

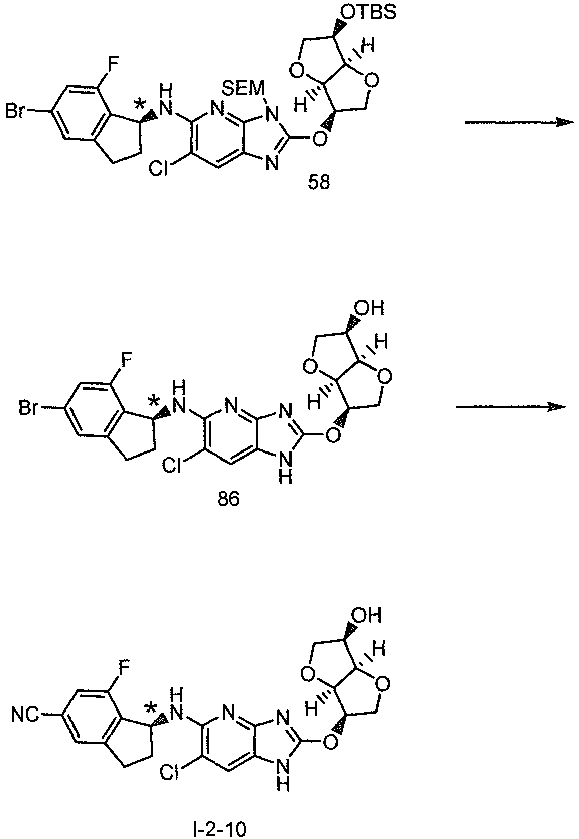

Compound 86 was synthesized from Compound 58, in a similar way that Compound (1-2-03) was synthesized from Compound 76.

Compound 86 (30 mg, 0.057 mmol) was dissolved in DMA (0.3 mL), and zinc cyanide (10.5 mg, 0.086 mmol), zinc (5.6 mg, 0.086 mmol) and bis(tri-tert-butylphosphine) palladium (8.75 mg, 0.017 mmol) were added thereto, and the mixture was stirred at 160°C for 1 hour in a microwave. A 1 mol/L aqueous solution of sodium hydroxide was added thereto, and the mixture was extracted with ethyl acetate. The extract was washed with water and a saturated aqueous solution of sodium chloride and then concentrated. The obtained residue was purified by silica gel column chromatography to obtain Compound (I-2-10) (9.4 mg 34.9%).

### Compound (I-2-10); Method B

LC/MS retention time =1.65 min.
MS (ESI) m/z =471.95 (M+H)+.

### [Example 47]

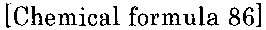

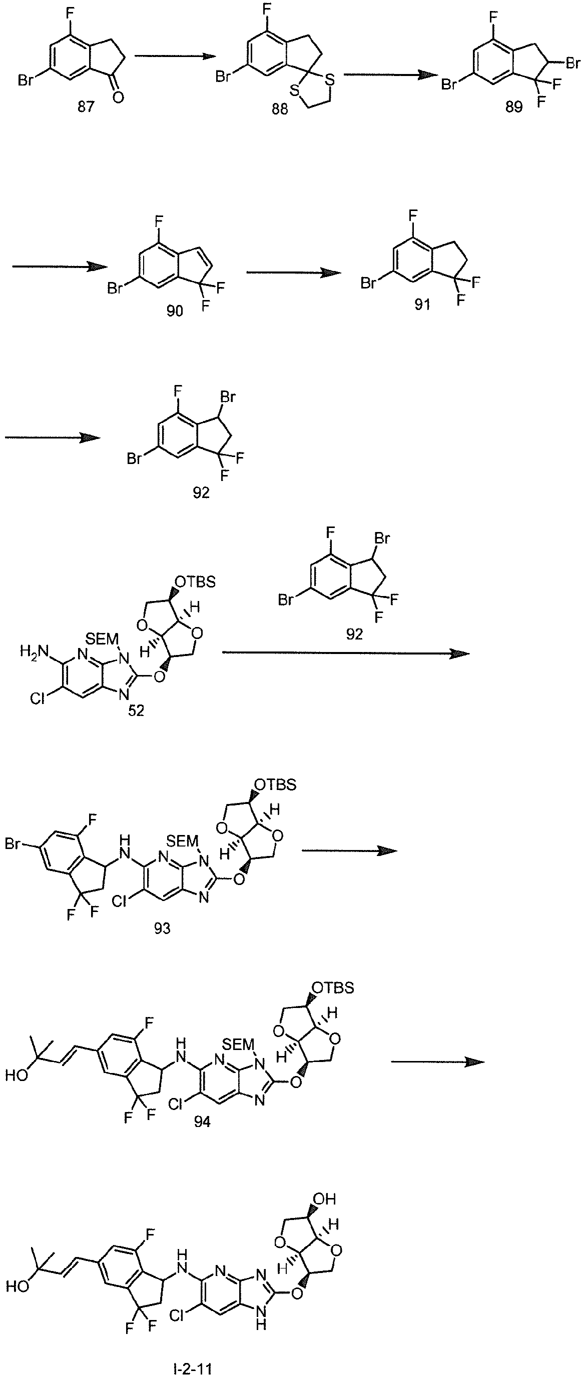

Compound 87 was dissolved in toluene (6 mL), and toluenesulfonic acid monohydrate (33.2 mg, 0.175 mmol) and ethanedithiol (88 µl, 01.048 mmol) were added thereto, and the mixture was refluxed for 8 hours. The reaction mixture was cooled to room temperature, an aqueous solution of sodium hypochlorite was added thereto, and the mixture was extracted with ethyl acetate. The extract was washed with a saturated aqueous solution of sodium chloride. The mixture was dried with anhydrous sodium sulfate and then concentrated. The obtained residue was purified by silica gel column chromatography to obtain Compound 88 (218 mg 81.6%).

1,3-bromo-5,5-dimethylhydantoin(817mg, 2.86mmol) was dissolved in dichloromethane (0.5 mL), and hydrogen fluoride-pyridine (0.827 ml, 6.43 mmol) and Compound 88 were added thereto at -78°C. The mixture was stirred for 2 hours. The reaction mixture was heated to 0°C, a 1 mol/L aqueous solution of sodium hydroxide and a saturated aqueous solution of sodium thiosulfate were added thereto. The mixture was extracted with dichloromethane. The extract was concentrated and the obtained residue was purified by silica gel column chromatography to obtain Compound 89 (177 mg, 75.1%) as a yellow oil.

Compound 89 (177mg, 0.536mmol) was dissolved in dichloromethane (0.7 mL), and DBU (0.121 mL, 0.805 mmol) was added thereto. The mixture was stirred at room temperature. After completion of the reaction, a 0.1 mol/L aqueous solution of hydrochloric acid was added thereto. The mixture was extracted with ethyl acetate. The extract was washed with a saturated aqueous solution of sodium chloride and concentrated. The obtained residue was purified by silica gel column chromatography to obtain Compound 90. Obtainted Compound 90 was dissolved in acetonitrile (4.4 mL), and then 2-nitrobenzenesulfonyl chloride (238mg, 1.07 mmol) and hydrazine monohydrate (104 µl, 2.15 mmol) were added thereto in an ice bath. The mixture was stirred at room temperature for 20 hours. The precipitated solid was filtered and the filtrate was concentrated. The obtained residue was purified by silica gel column chromatography to obtain Compound 91 as a yellow oil.

Compound 91 (50 mg, 0.199 mmol) was dissolved in water (0.5 mL), and a 0.5 mL aqueous solution of sodium bromite (60.1 mg, 0.398 mmol) and a 0.5 mL aqueous solution of sodium hydrogen sulfite (41.4 mg, 0.398 mmol) were added thereto. The mixture was stirred for 3 hours. An aqueous solution of sodium bromite (90.0 mg, 0.597 mmol) and an aqueous solution of sodium hydrogen sulfite (62.2 mg, 0.597 mmol) were added thereto. The mixture was stirred for 2 hours. An aqueous solution of sodium bromite (60.1 mg, 0.398mmol) and an aqueous solution of sodium hydrogen sulfite (41.4 mg, 0.398 mmol) were further added thereto. The mixture was stirred for 0.5 hours. To the reaction mixture was added a saturated aqueous solution of ammonium chloride, and the mixture was extracted with ethyl acetate. The extract was concentrated and the obtained residue was purified by silica gel column chromatography to obtain Compound 92 (53 mg, 80.7%) as a yellow oil.
Compound 92; ¹H-NMR (CDCl₃) δ: 7.55-7.52 (1H, m), 7.41-7.37 (1H, m), 5.45-5.39 (1H, m), 3.31-3.15 (1H, m), 3.11-3.00 (1H, m).

Compound 93 was obtained from Compound 92, in a similar way that Compound 54 was obtained from Compound 52.

Compound (I-2-11) was obtained from Compound 93, in a similar way that Compound (I-2-03) was obtained from Compound 58.

### Compound (I-2-11); Method B

LC/MS retention time =1.75 min.
MS (ESI) m/z =567.10 (M+H)+.

### [Example 48]

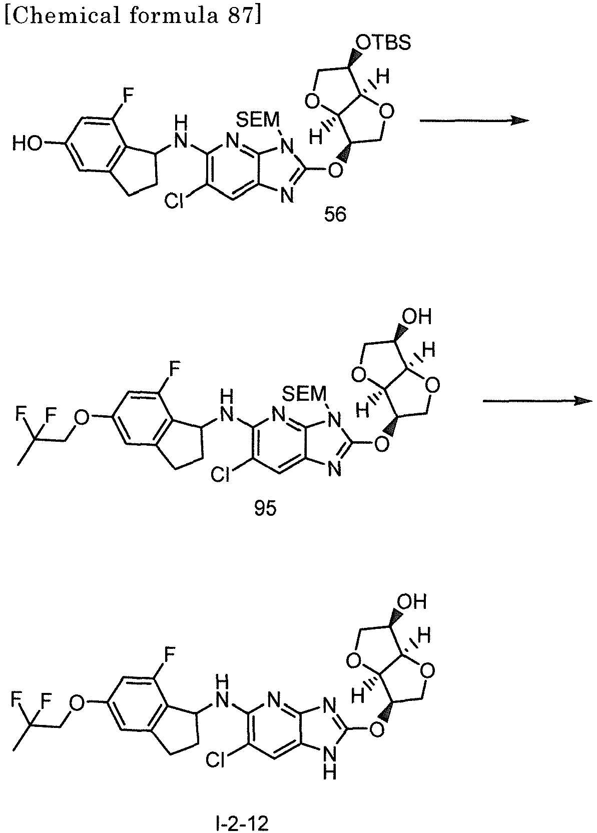

3,3-difluorobutan-1-ol (21.9 µl, 0.121 mmol) was dissolved in THF (500 µl), and MsCl (24.8 µl, 0.318 mmol) and trimethylamine (58.8 µl, 0.424 mmol) were added thereto. The mixture was stirred at room temperature for 1 hour. To the reaction mixture was added water and the mixture was extracted with ethyl acetate. The extract was washed with a saturated aqueous solution of sodium chloride and then concentrated. The obtained residue and Compound 56 (50 mg, 0.071 mmol) were dissolved in DMF (500 µl) and potassium carbonate (39.1 mg, 0.283 mmol) was added thereto. The mixture was stirred at 80°C for 3 hours. To the reaction mixture was added a saturated aqueous solution of ammonium chloride, and the mixture was extracted with ethyl acetate. The extract was washed with a saturated aqueous solution of sodium chloride and then concentrated, and the obtained residue was purified by silica gel column chromatography to obtain Compound 95 (38.6 mg, 68.3%).

Compound (I-2-12) was obtained from Compound 95, in a similar way that Compound (1-2-03) was obtained from Compound 76.

### Compound (1-2-12); Method B

LC/MS retention time =2.05 min.
MS (ESI) m/z =555.10 (M+H)+.

### [Example 49]

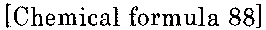

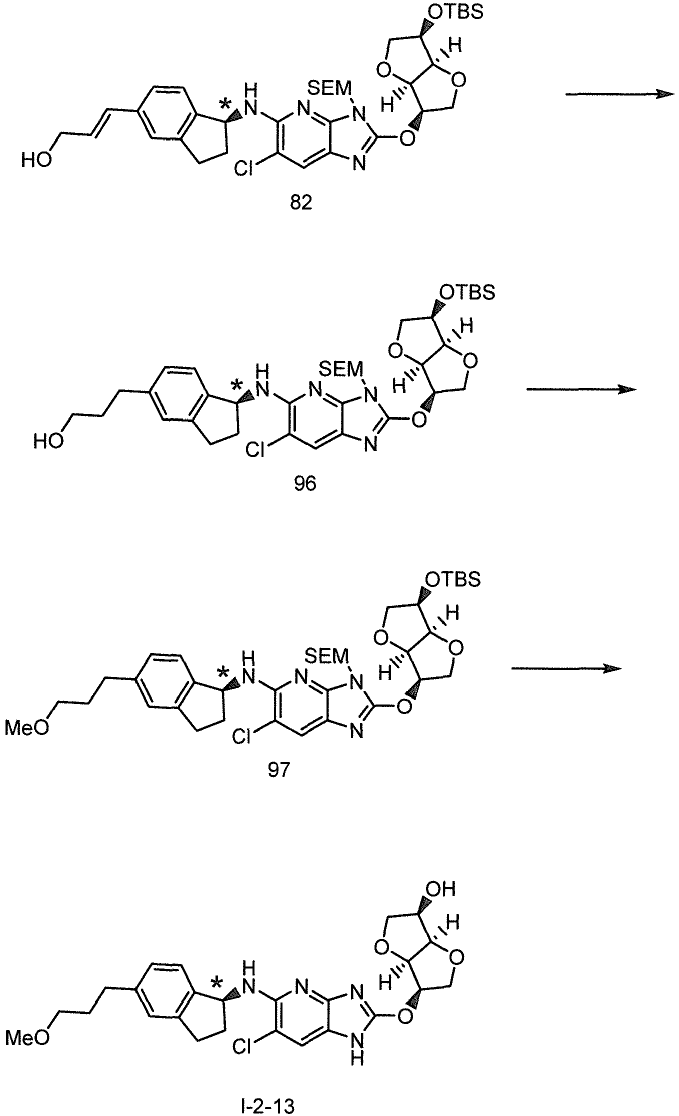

Compound 82 (130mg, 0.040mmol) was dissolved in ethanol (5 mL), and palladium 5% on carbon (52 mg) was added thereto. The mixture was stirred for 40 minutes under hydrogen atmosphere. The reaction mixture was filtered and concentrated. The obtained residue was purified by silica gel column chromatography to obtain Compound 96 (13.9 mg, 46.2%).

Compound 96 (13.9mg, 0.019mmol) was dissolved in DMF (0.14 mL), and sodium borohydride (60%, 1.85 mg, 0.046 mmol) and iodomethane (3.13µl, 0.050 mmol) were added thereto. The mixture was stirred at room temperature for 3 hours. The reaction mixture was purified by silica gel column chromatography to obtain Compound 97 (5.6 mg, 39.5%).

Compound (1-2-13) was synthesized from Compound 97, in a similar way that Compound (I-2-3) was synthesized from Compound 76.

### Compound (1-2-13); Method B

LC/MS retention time =1.94 min.
MS (ESI) m/z =519.15 (M+H)+.

### [Example 50]

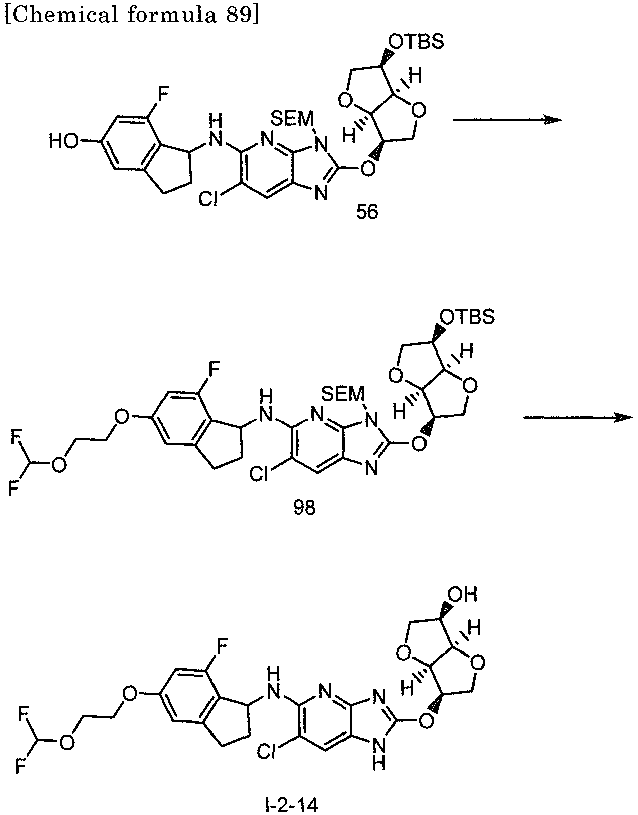

Compound 98 was synthesized from Compound 56 and 2-(difluoromethoxy) ethyl 4-methylbenzenesulfonate, in a similar way that Compound (I-2-05) was synthesized from Compound 56.

Compound 1-2-14 was synthesized from Compound 98, in a similar way that Compound (1-2-05) was synthesized from Compound 79.

### Compound (1-2-14); Method B

LC/MS retention time =1.90 min.
MS (ESI) m/z =557.15 (M+H)+.

### [Example 51]

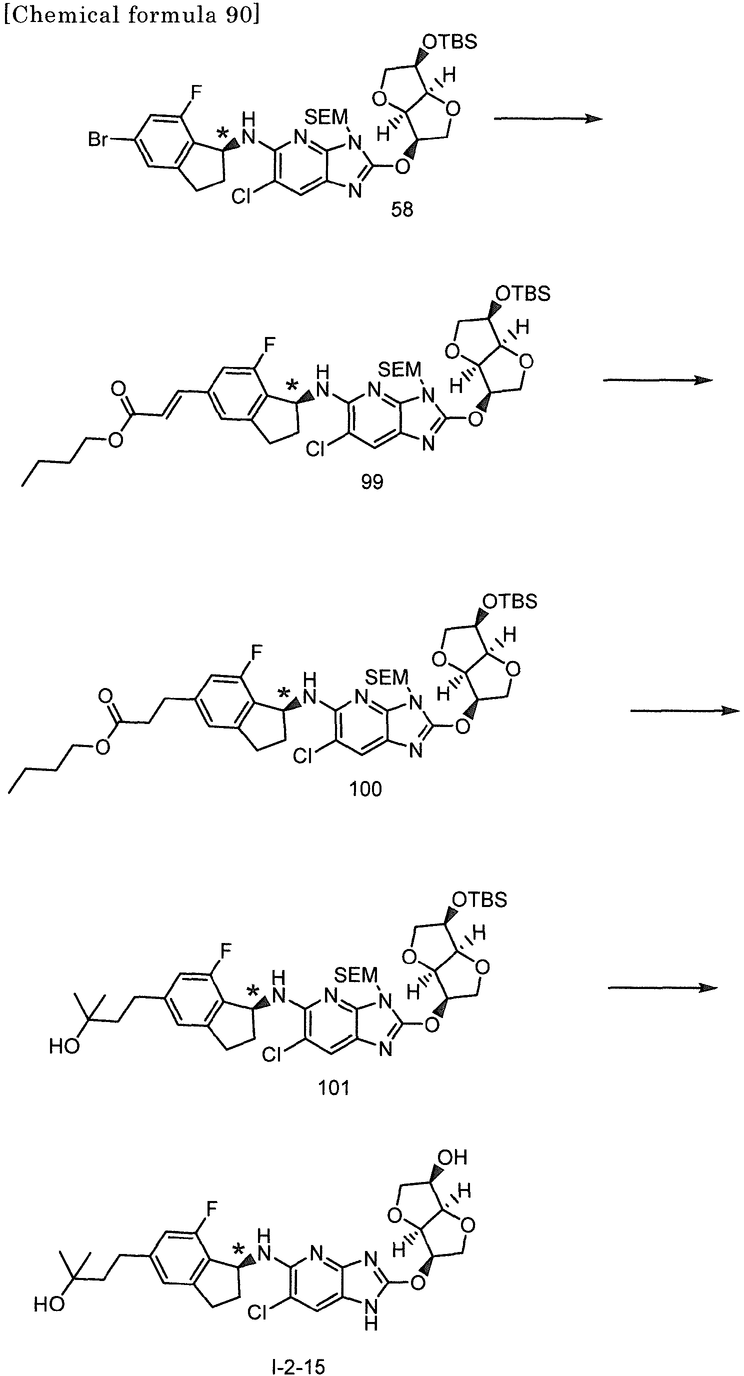

Compound 58 (250 mg, 0.325mmol) was dissolved in DMF (2.5 mL) under nitrogen atmosphere, and butyl acrylate (79 ul, 0.552 mmol), potassium carbonate (53.8 mg, 0.389mmol), acetic acid palladium (7.29 mg, 0.032 mmol) and DABCO (3.64 mg, 0.032 mmol) were added thereto and the reaction mixture was stirred at 120°C for 1.5 hours. The reaction mixture was cooled and purified by silica gel column chromatography to obtain Compound 99 (196.1 mg, 73.9%).

Compound 100 was synthesized from Compound 99, in a similar way that Compound 96 was synthesized from Compound 82.

Compound 100 (30 mg, 0.037mmol) was dissolved in THF (0.2 mL) under nitrogen atmosphere, and methylmagnesium bromide (in 3 mol/L diethyl ether, 48.8 ul, 0.146mmol) was added thereto. The mixture was stirred at room temperature for 6 hours. To the reaction mixture was added a saturated aqueous solution of ammonium chloride and the mixture was extracted with ethyl acetate. The extract was washed with a saturated aqueous solution of sodium chloride and then concentrated, and the obtained residue was purified by silica gel column chromatography to obtain Compound 101 (11.9 mg, 41.8%).

Compound (I-2-15) was obtained from Compound 101, in a similar way that Compound (1-2-05) was obtained from Compound 79.

### Compound (1-2-15); Method B

LC/MS retention time =1.74 min.
MS (ESI) m/z =533.20 (M+H)+.

### [Example 52]

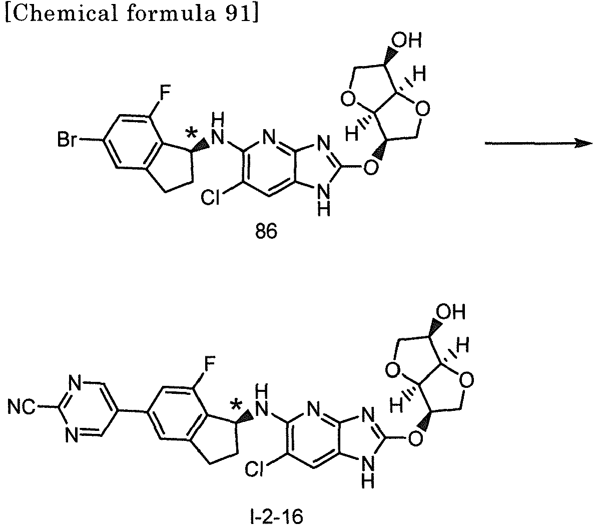

Compound 86 (20 mg, 0.038 mmol) was dissolved in 1,2-dioxane (0.2 mL), and then 5-(4,4,5,5-tetramethyl-1,3,2-dioxaborolan-2-yl)pyrimidine-2-carbonitrile (11.4 mg, 0.049 mmol), PdCl₂(dppf) (2.48 mg, 0.0038 mmol) and a 2 mol/L aqueous solution of potassium carbonate (38.0 ul, 0.076mmol) were added thereto, and the mixture was stirred at 100°C for 2 hours. After cooling, the reaction mixture was added to a saturated aqueous solution of ammonium chloride, and the mixture was extracted with ethyl acetate. The extract was dried with sodium sulfate, filtered and concentrated under reduced pressure. The obtained residue was purified by silica gel column chromatography to obtain Compound (1-2-16) (5.9 mg, 0.0107mmol).

### Compound (1-2-16); Method B

LC/MS retention time =1.82 min.
MS (ESI) m/z =550.35 (M+H)+.

### [Example 53]

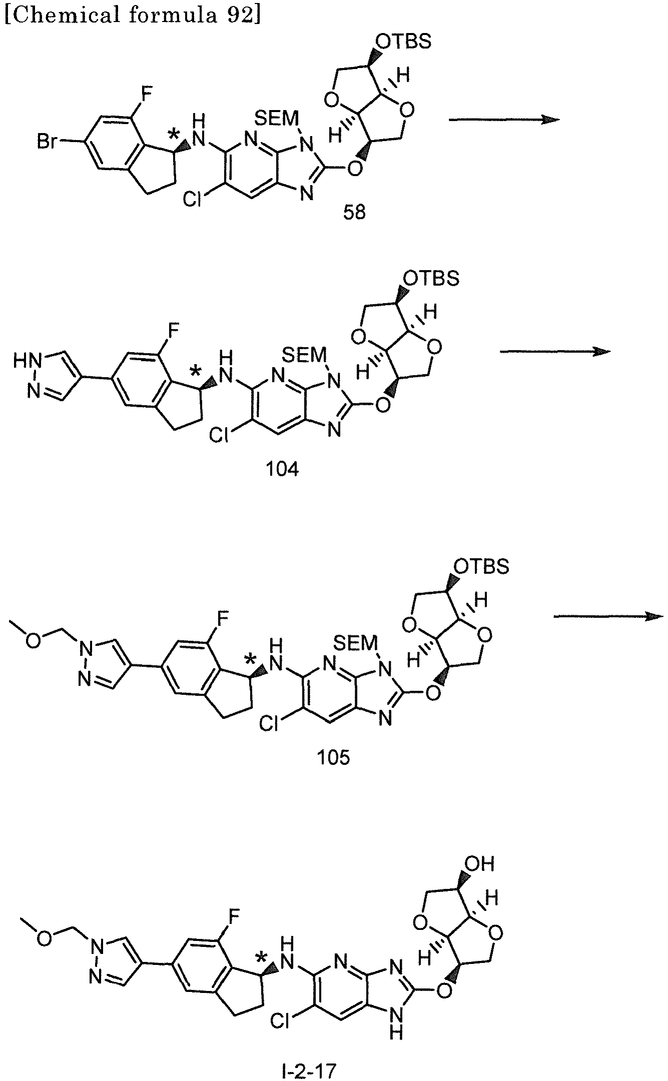

Compound 104 was obtained from Compound 58 and 4-(4,4,5,5-tetramethyl-1,3,2-dioxaborolane-2-yl)-1H-pyrazole, in a similar way that Compound 76 was obtained from Compound 58.

Compound 104 (16 mg, 0.021mmol) was dissolved in DMF (0.16 mL) under nitrogen atmosphere, and sodium hydride (60%, 2.54 mg, 0.064mmol) and MOMCl (3.85 ul, 0.050mmol) were added thereto. The mixture was stirred at room temperature for 4 hours. To the reaction mixture was added a saturated aqueous solution of ammonium chloride and the mixture was extracted with ethyl acetate. The extract was washed with a saturated aqueous solution of sodium chloride and then concentrated. The obtained residue was purified by silica gel column chromatography to obtain Compound 105 (13.1 mg).

Compound (1-2-17) was obtained from Compound 105, in a similar way that Compound (I-2-05) was obtained from Compound 79.

### Compound (1-2-17); Method B

LC/MS retention time =1.66 min.
MS (ESI) m/z =557.15 (M+H)+.

### [Example 54]

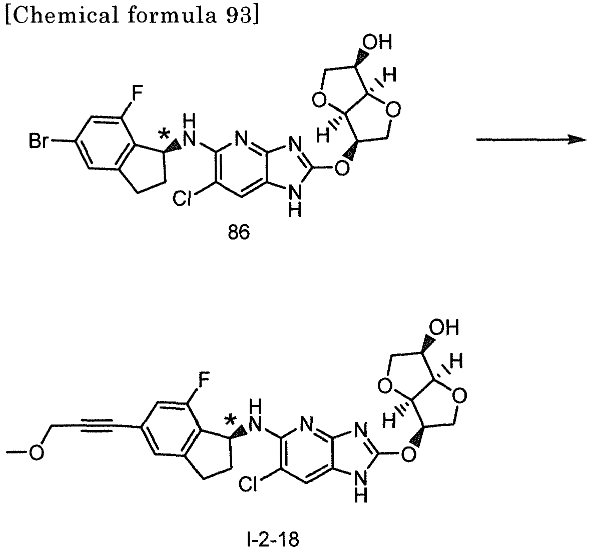

Compound 86 (20 mg, 0.038 mmol) was dissolved in DMF (0.3 mL) and copper chloride (1.44 mg, 0.00761mmol), PdCl₂(PPh₃)₂ (5.34mg, 0.00761mmol) and 3-methoxy-1-propyne (6.42 ul, 0.076mmol) were added thereto, and the mixture was stirred at 80°C for 1 hour in a microwave. Copper chloride (1.44 mg, 0.00761mmol), PdCl₂(PPh₃)₂ (5.34mg, 0.00761mmol) and 3-methoxy-1-propyne (6.42 ul, 0.076mmol) were further added thereto, and the mixture was stirred at 100°C for 1 hour. After cooling, to the reaction mixture was added a saturated aqueous solution of ammonium chloride, and the mixture was extracted with ethyl acetate. The extract was dried with sodium sulfate, filtered and concentrated under reduced pressure. The obtained residue was purified by silica gel column chromatography to obtain Compound (1-2-18) (8.4 mg, 42.9%).

### Compound (1-2-18); Method B

LC/MS retention time =1.90 min.
MS (ESI) m/z =515.10 (M+H)+.

### [Example 55]

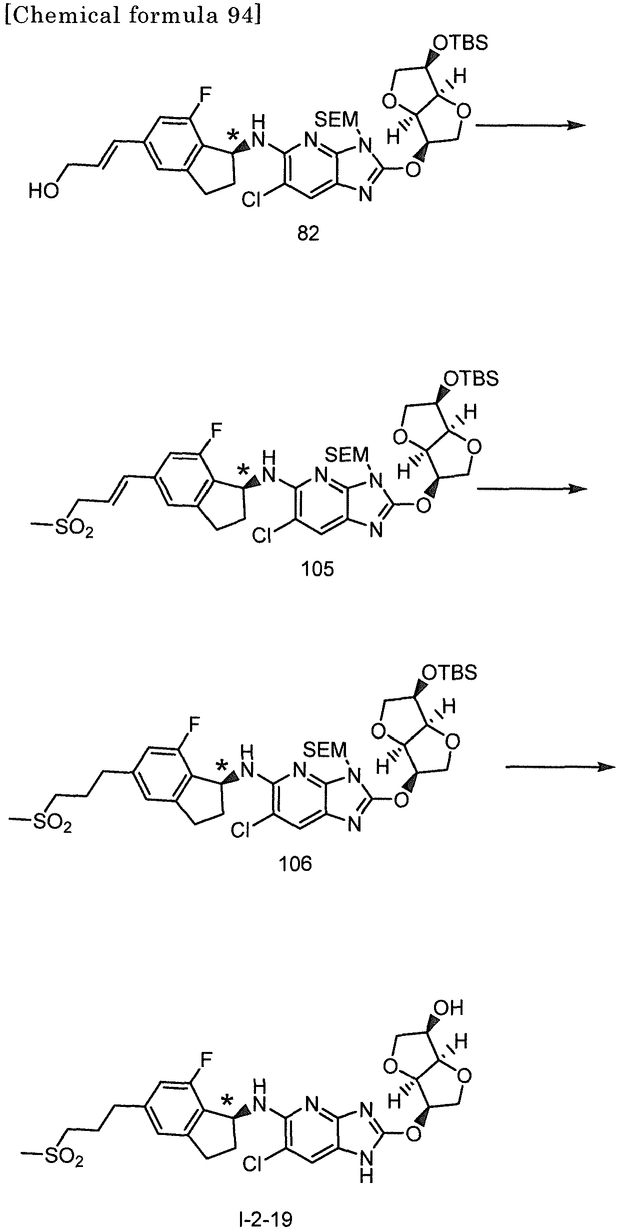

Compound 82 (68 mg, 0.091 mmol) was dissolved in dichloromethane (0.68 mL), and phosphorus tribromide (25.7µl, 0.273 mmol) was added thereto, and the mixture was stirred for 30 minutes in an ice bath. A saturated aqueous solution of ammonium chloride was added thereto, and the mixture was extracted with ethyl acetate. The extract was dried with sodium sulfate, filtered and then concentrated under reduced pressure. The obtained residue was dissolved in DMF (1.7 mL) and sodium methanesulfinate (13.9 mg, 0.136 mmol) was added thereto, and the mixture was stirred at 90°C for 30 minutes. The reaction mixture was purified by silica gel column chromatography to obtain Compound 105 (34 mg, 46.2%).

Compound 106 was synthesized from Compound 105, in a similar way that Compound 96 was synthesized from Compound 82.

Compound (I-2-19) was synthesized from Compound 106, in a similar way that Compound (I-2-05) was synthesized from Compound 79.

### Compound (I-2-19); Method B

LC/MS retention time =1.55 min.
MS (ESI) m/z =567.2 (M+H)+.

The compounds shown below were synthesized in a similar way. The measurement results of NMR or LC/MS of the respective compounds are shown.

**[Table 1]**

| No. | M: Mixture of diastereomers | Structure | retention time | Mass (M+H) | method |
|---|---|---|---|---|---|
| | C: Chiral (Optically active) | | | | |
| I-1-46 | C | 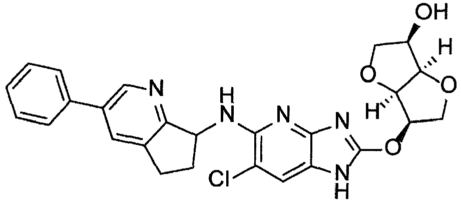 | 1.44 | 506.5 | B |
| I-1-47 | C | 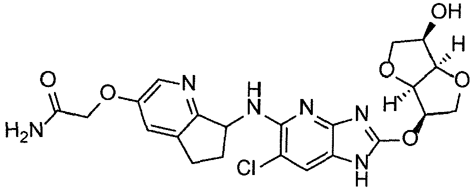 | 0.88 | 503.5 | B |
| I-1-48 | C | 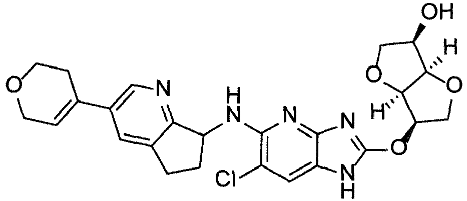 | 1.09 | 512.4 | B |
| I-1-49 | C | 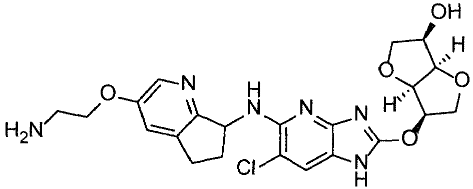 | 0.72 | 489.3 | B |
| I-1-50 | C | 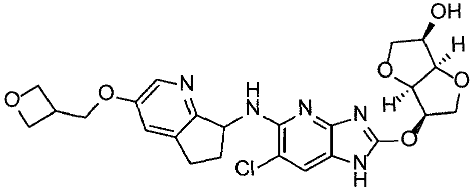 | 1.03 | 516.4 | B |
| I-1-51 | M | 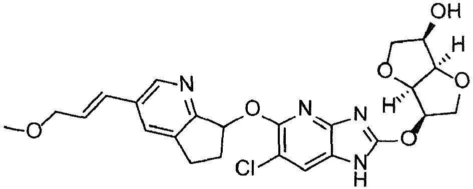 | 1.38 | 501.4 | B |

**[Table 2]**

| | | | | | |
|---|---|---|---|---|---|
| I-1-52 | C | 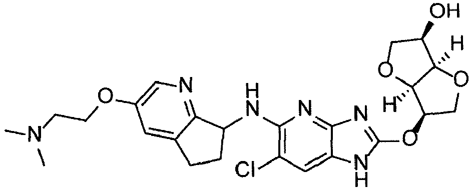 | 0.78 | 517.5 | B |
| I-1-53 | C | 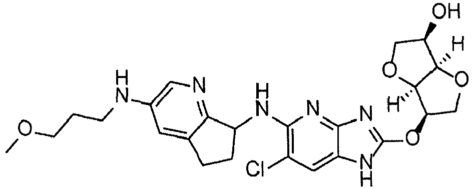 | 0.97 | 517.4 | B |
| I-1-54 | M | 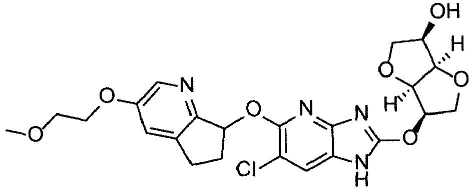 | 1.27 | 505.3 | B |
| I-1-55 | M | 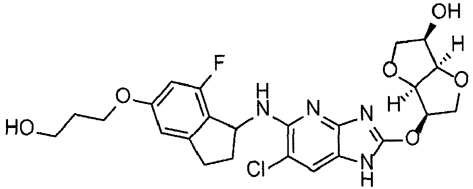 | 1.51 | 521.5 | B |
| I-1-56 | M | 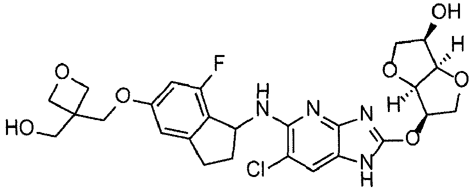 | 1.42 | 563.3 | B |
| I-1-57 | M | 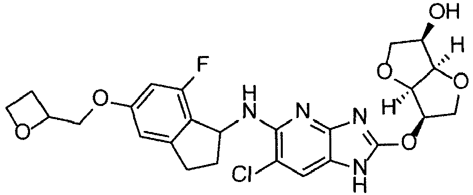 | 1.68 | 533.3 | B |

**[Table 3]**

| | | | | | |
|---|---|---|---|---|---|
| I-1-58 | M | 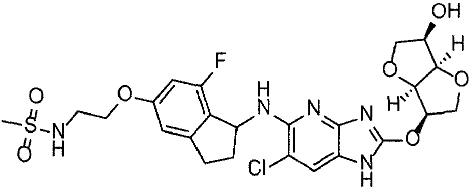 | 1.48 | 584.3 | B |
| I-1-59 | M | 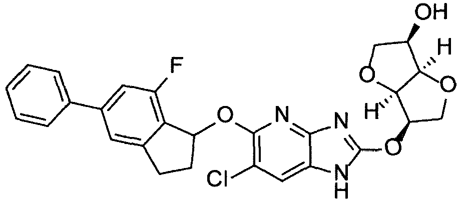 | 2.17 | 524.1 | B |
| I-1-60 | M | 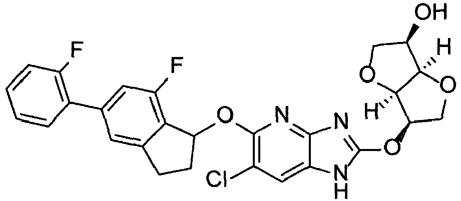 | 2.43 | 542.1 | B |
| I-1-61 | M | 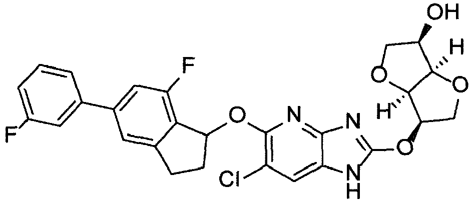 | 2.47 | 542.1 | B |
| I-1-62 | M | 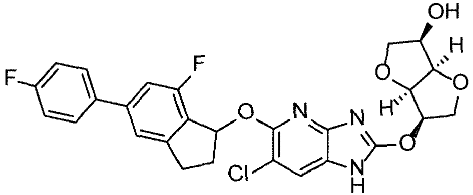 | 2.47 | 542.1 | B |
| I-1-63 | M | 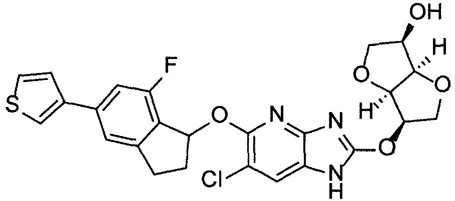 | 2.36 | 530.1 | B |

**[Table 4]**

| | | | | | |
|---|---|---|---|---|---|
| I-1-64 | M | 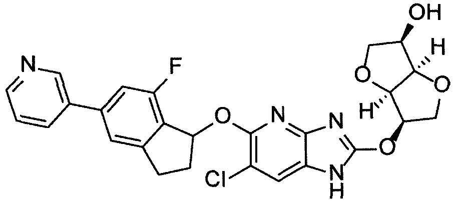 | 1.49 | 525.1 | B |
| I-1-65 | M | 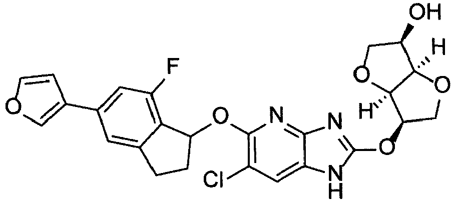 | 2.21 | 514.1 | B |
| I-1-66 | M | 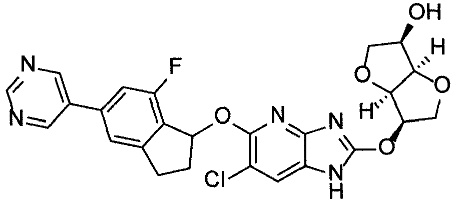 | 1.70 | 526.1 | B |
| I-1-67 | C | 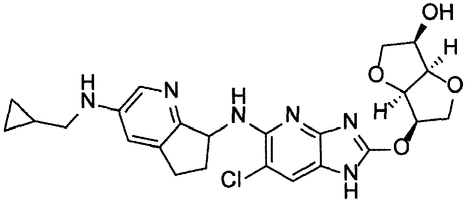 | 1.08 | 499.3 | B |
| I-1-68 | M | 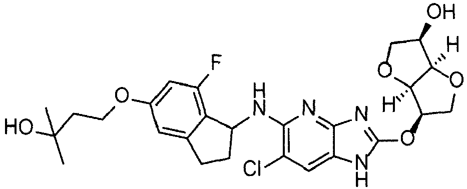 | 1.74 | 549.4 | B |
| I-1-69 | M | 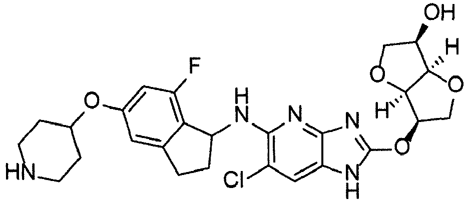 | 1.03 | 546.5 | B |

**[Table 5]**

| | | | | | |
|---|---|---|---|---|---|
| I-1-70 | C | 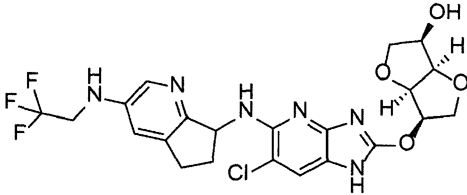 | 1.04 | 527.5 | B |
| I-1-71 | M | 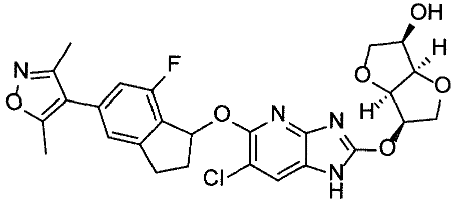 | 2.04 | 543.1 | B |
| I-1-72 | M | 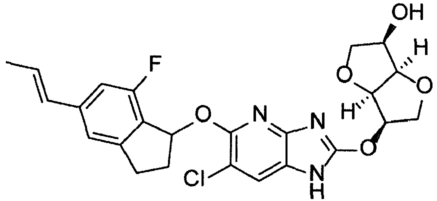 | 2.20 | 488.1 | B |
| I-1-73 | M | 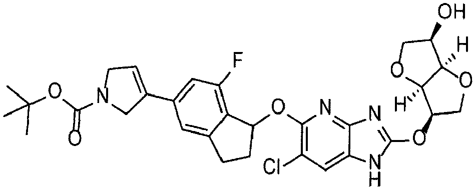 | 2.45 | 615.2 | B |
| I-1-74 | M | 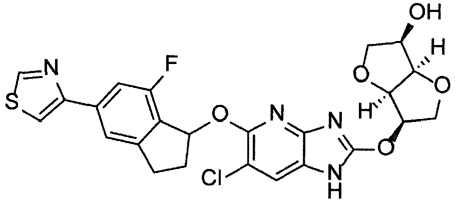 | 2.00 | 531.1 | B |
| I-1-75 | M | 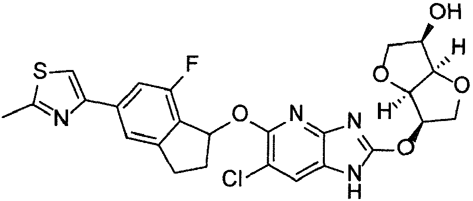 | 2.15 | 545.1 | B |

**[Table 6]**

| | | | | | |
|---|---|---|---|---|---|
| I-1-76 | M | 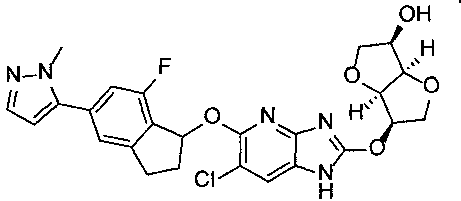 | 1.83 | 528.1 | B |
| I-1-77 | M | 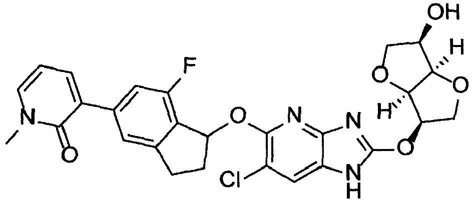 | 1.72 | 555.1 | B |
| I-1-78 | M | 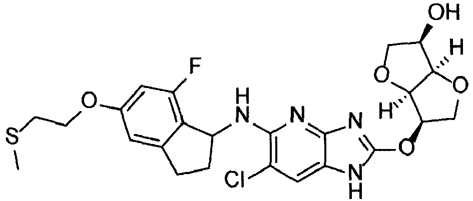 | 2.10 | 537.0 | B |
| I-1-79 | M | 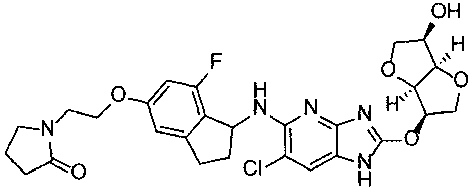 | 1.60 | 574.1 | B |
| I-1-80 | C | 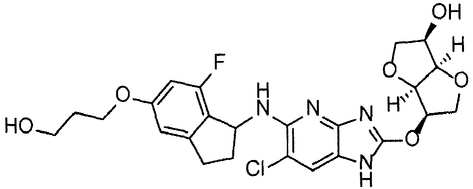 | 1.58 | 521.1 | B |
| I-1-81 | C | 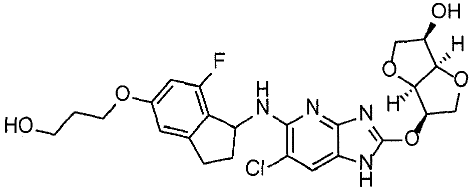 | 1.58 | 521.1 | B |

**[Table 7]**

| | | | | | |
|---|---|---|---|---|---|
| I-1-82 | C | 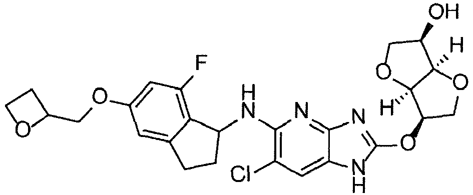 | 1.74 | 533.1 | B |
| I-1-83 | C | 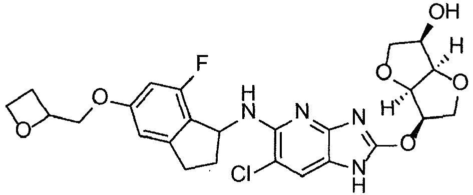 | 1.73 | 533.1 | B |
| I-1-84 | C | 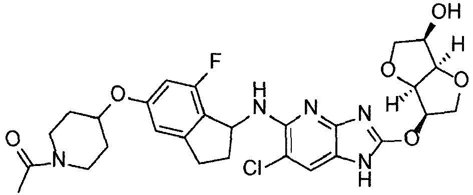 | 1.64 | 588.1 | B |
| I-1-85 | C | 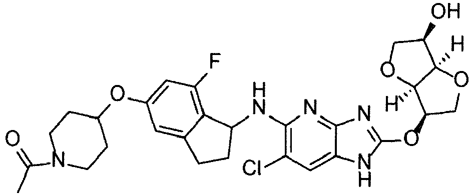 | 1.64 | 588.1 | B |
| I-1-86 | M | 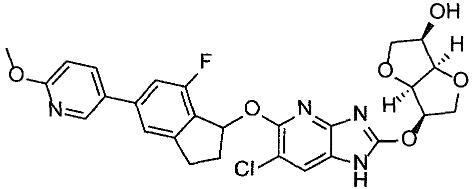 | 2.03 | 555.5 | B |
| I-1-87 | C | 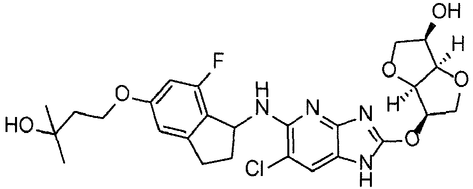 | 1.74 | 549.3 | B |

**[Table 8]**

| | | | | | |
|---|---|---|---|---|---|
| I-1-88 | C | 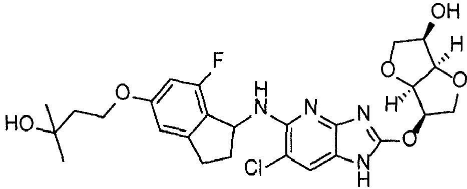 | 1.73 | 549.3 | B |
| I-1-89 | M | 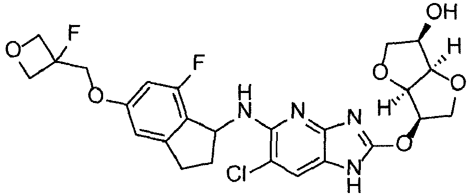 | 1.73 | 551.5 | B |
| I-1-90 | M | 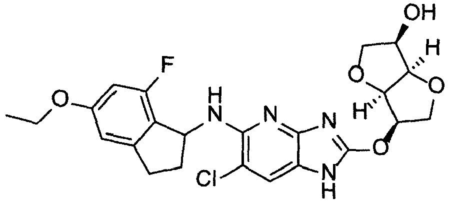 | 1.96 | 491.5 | B |
| I-1-91 | M | 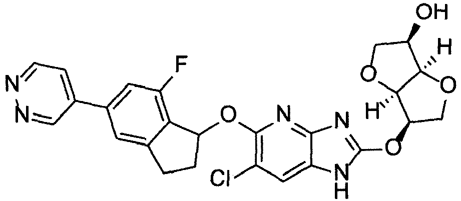 | 1.44 | 526.3 | B |
| I-1-92 | C | 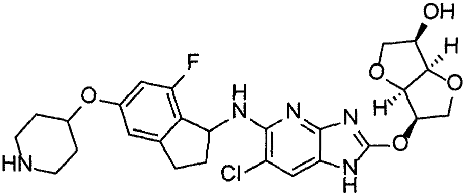 | 1.03 | 546.3 | B |
| I-1-93 | C | 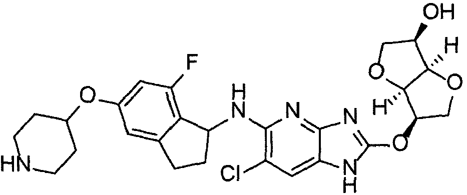 | 1.03 | 546.5 | B |

**[Table 9]**

| | | | | | |
|---|---|---|---|---|---|
| I-1-94 | C | 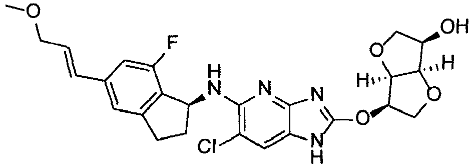 | 1.88 | 517.3 | B |
| I-1-95 | C | 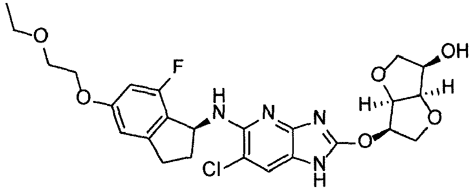 | 1.88 | 535.3 | B |
| I-1-96 | C | 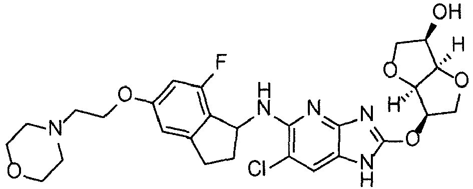 | 0.99 | 576.3 | B |
| I-1-97 | C | 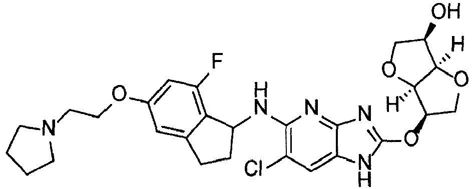 | 1.03 | 560.4 | B |
| I-1-98 | C | 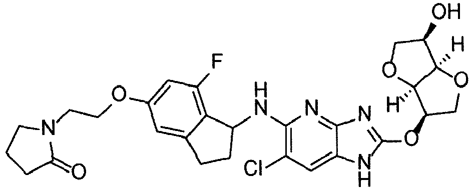 | 1.54 | 574.3 | B |
| I-1-99 | C | 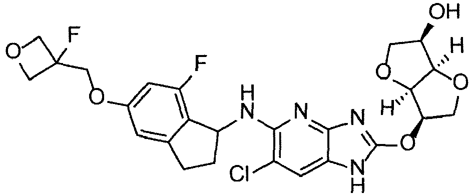 | 1.73 | 551.3 | B |
| I-1-100 | C | 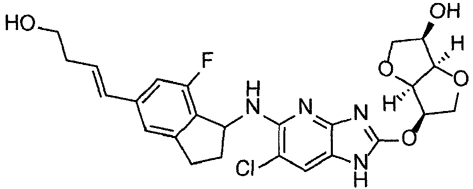 | 1.60 | 517.3 | B |

**[Table 10]**

| | | | | | |
|---|---|---|---|---|---|
| I-1-101 | M | 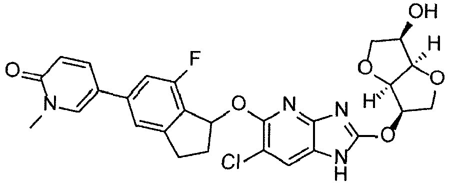 | 1.49 | 555.6 | B |
| I-1-102 | M | 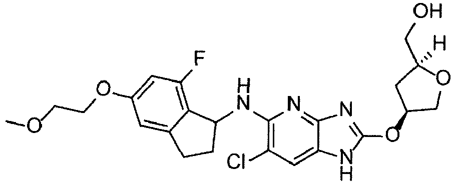 | 1.77 | 493.6 | B |
| I-1-103 | C | 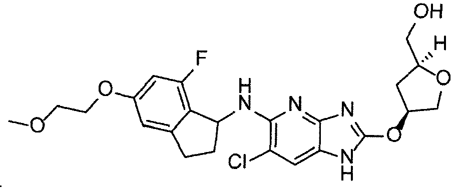 | 1.76 | 493.6 | B |
| I-1-104 | C | 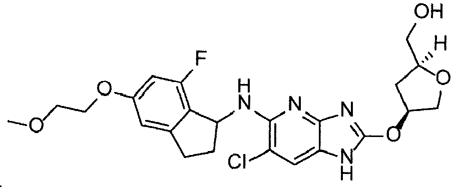 | 1.76 | 493.3 | B |
| I-1-105 | C | 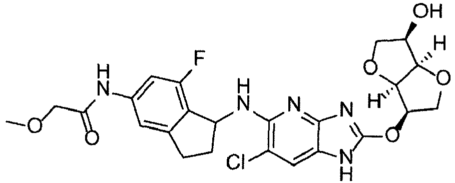 | 1.47 | 534.3 | B |
| I-1-106 | C | 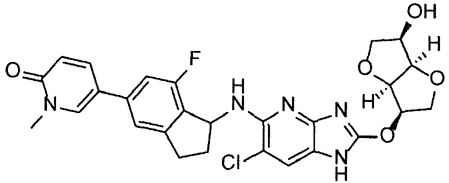 | 1.45 | 554.5 | B |
| I-1-107 | C | 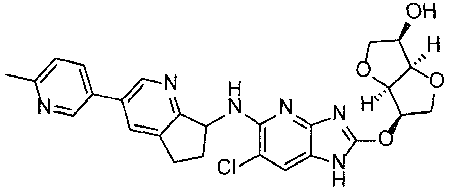 | 0.94 | 521.3 | B |

**[Table 11]**

| | | | | | |
|---|---|---|---|---|---|
| I-1-108 | M | 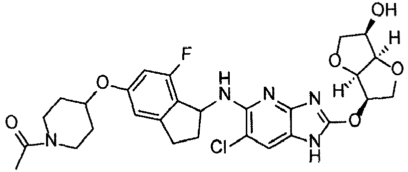 | 1.59 | 586.3 | B |
| I-1-109 | M | 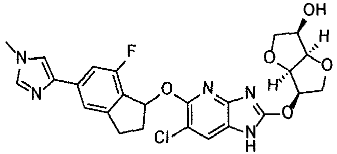 | 1.01 | 526.3 | B |
| I-1-110 | M | 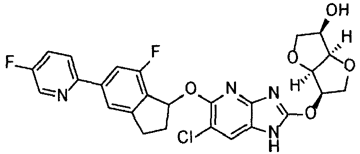 | 2.01 | 541.3 | B |
| I-1-111 | M | 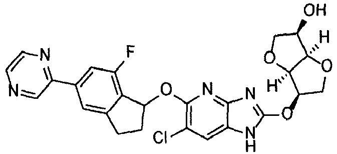 | 1.72 | 524.2 | B |

**[Table 12]**

| No. | M: Mixture of diastereomers | Structure | retention time | Mass (M+H) | method |
|---|---|---|---|---|---|
| | C: Chiral (Optically active) | | | | |
| I-2-20 | C | 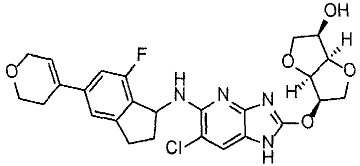 | 1.96 | 529.1 | B |
| I-2-21 | C | 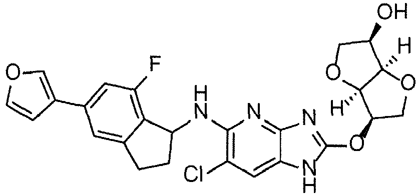 | 2.14 | 513.1 | B |
| I-2-22 | C | 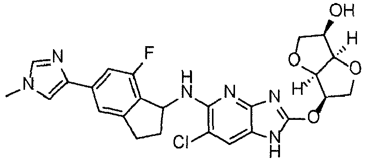 | 2.18 | 527.0 | B |
| I-2-23 | C | 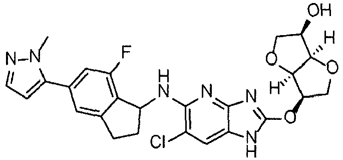 | 1.77 | 527.1 | B |
| I-2-24 | C | 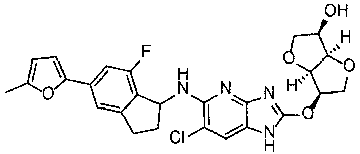 | 2.38 | 527.1 | B |
| I-2-25 | C | 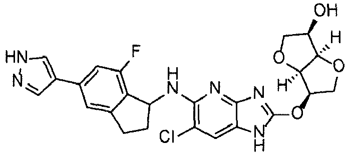 | 1.58 | 513.1 | B |

**[Table 13]**

| | | | | | |
|---|---|---|---|---|---|
| I-2-26 | C | 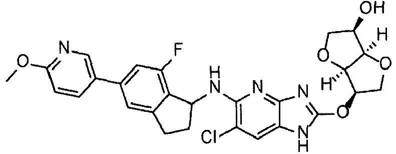 | 2.13 | 554.1 | B |
| I-2-27 | C | 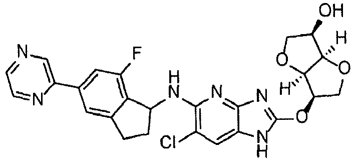 | 2.18 | 525.0 | B |
| I-2-28 | C | 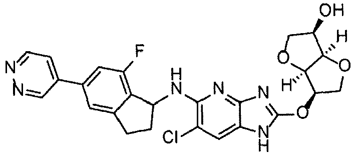 | 1.51 | 525.1 | B |
| I-2-29 | C | 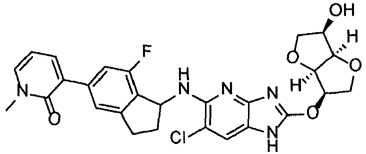 | 1.65 | 554.1 | B |
| I-2-30 | C | 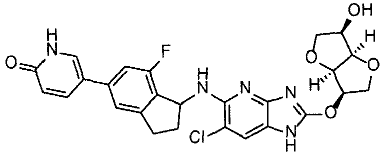 | 1.47 | 540.1 | B |
| I-2-31 | C | 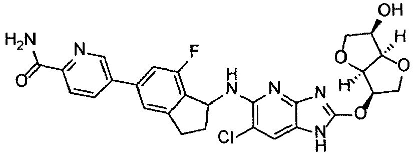 | 1.64 | 567.1 | B |
| I-2-32 | M | 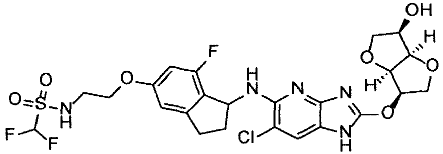 | 1.74 | 620.2 | B |

**[Table 14]**

| | | | | | |
|---|---|---|---|---|---|
| I-2-33 | M | 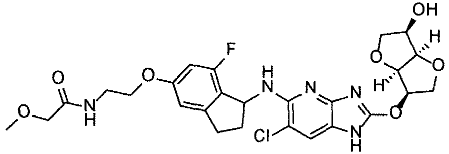 | 1.48 | 578.3 | B |
| I-2-34 | M | 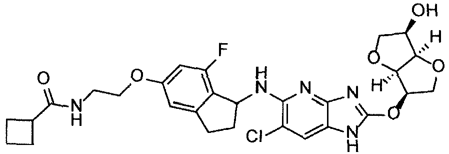 | 1.66 | 588.3 | B |
| I-2-35 | M | 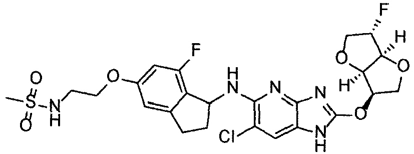 | 1.75 | 586.3 | B |
| I-2-36 | M | 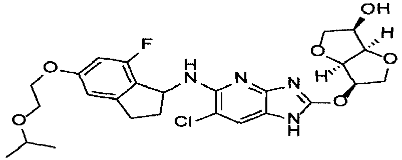 | 2.02 | 549.4 | B |
| I-2-37 | C | 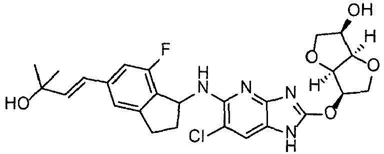 | 1.72 | 531.6 | B |
| I-2-38 | M | 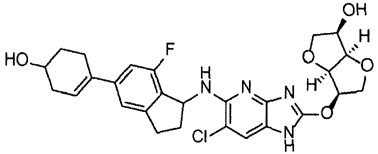 | 1.66 | 543.4 | B |
| I-2-39 | M | 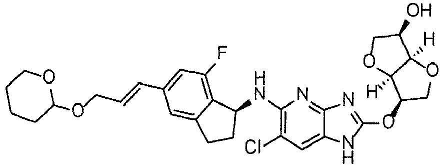 | 2.31 | 587.3 | B |

**[Table 15]**

| | | | | | |
|---|---|---|---|---|---|
| I-2-40 | M | 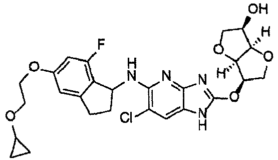 | 1.96 | 547.1 | B |
| I-2-41 | M | 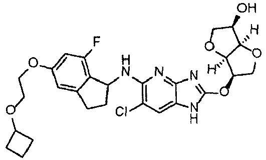 | 2.10 | 561.2 | B |
| I-2-42 | M | 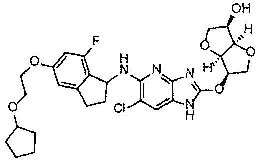 | 2.25 | 575.2 | B |
| I-2-43 | M | 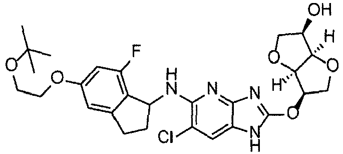 | 2.15 | 563.2 | B |
| I-2-44 | M | 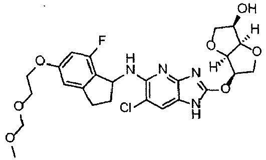 | 1.77 | 551.3 | B |
| I-2-45 | C | 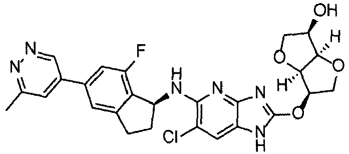 | 1.43 | 539.1 | B |
| I-2-46 | C | 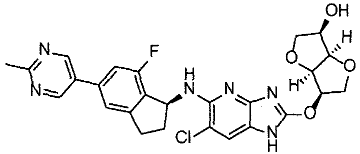 | 1.59 | 539.1 | B |

**[Table 16]**

| | | | | | |
|---|---|---|---|---|---|
| I-2-47 | C | 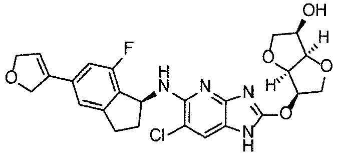 | 1.77 | 515.1 | B |
| I-2-48 | C | 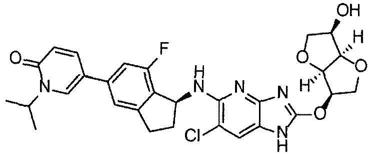 | 1.68 | 582.2 | B |
| I-2-49 | M | 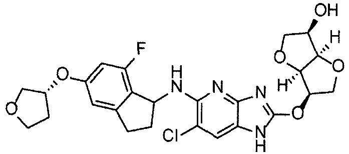 | 1.72 | 533.3 | B |
| I-2-50 | M | 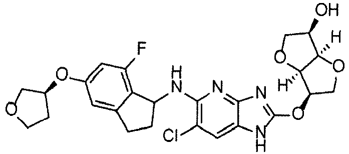 | 1.72 | 533.1 | B |
| I-2-51 | M | 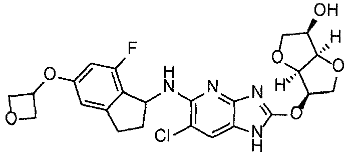 | 1.64 | 519.1 | B |
| I-2-52 | M | 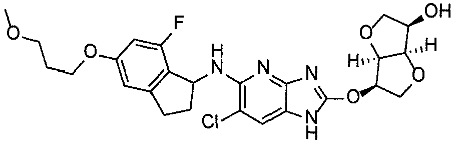 | 1.89 | 535.2 | B |
| I-2-53 | M | 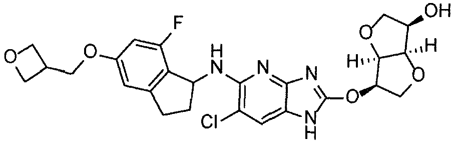 | 1.65 | 533.1 | B |

**[Table 17]**

| | | | | | |
|---|---|---|---|---|---|
| I-2-54 | M | 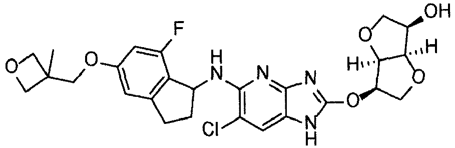 | 1.80 | 547.3 | B |
| I-2-55 | M | 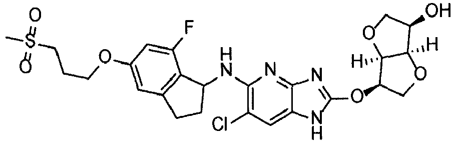 | 1.52 | 583.3 | B |
| I-2-56 | C | 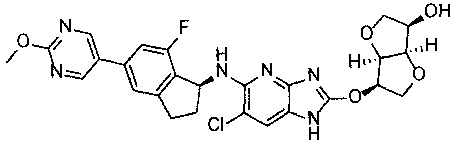 | 1.73 | 555.2 | B |
| I-2-57 | C | 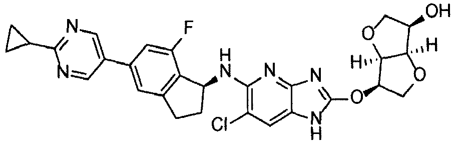 | 1.91 | 565.2 | B |
| I-2-58 | C | 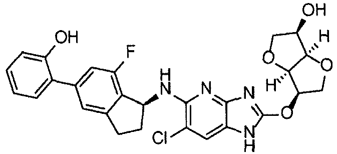 | 1.88 | 539.2 | B |
| I-2-59 | M | 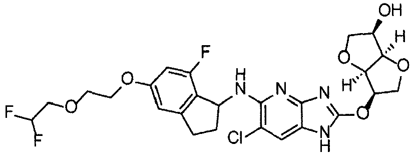 | 1.86 | 571.1 | B |
| I-2-60 | M | 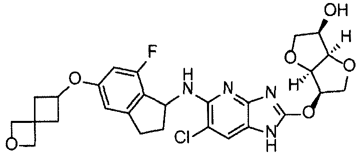 | 1.79 | 559.2 | B |

**[Table 18]**

| | | | | | |
|---|---|---|---|---|---|
| I-2-61 | C | 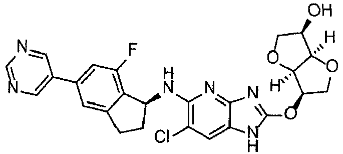 | 1.53 | 525.1 | B |
| I-2-62 | C | 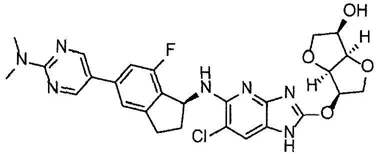 | 1.92 | 568.2 | B |
| I-2-63 | C | 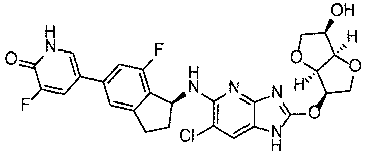 | 1.42 | 558.1 | B |
| I-2-64 | C | 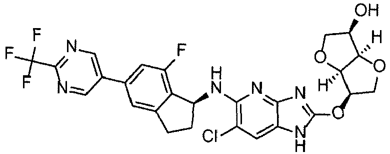 | 2.02 | 593.2 | B |
| I-2-65 | C | 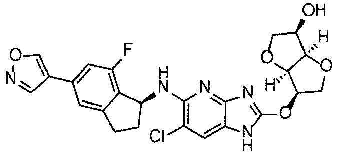 | 1.54 | 514.3 | B |
| I-2-66 | C | 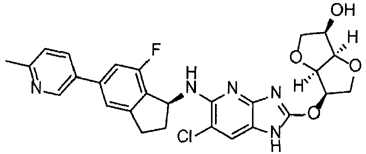 | 1.22 | 538.3 | B |
| I-2-67 | C | 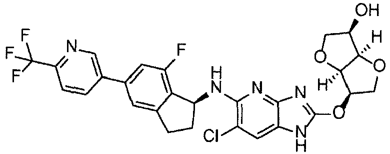 | 2.11 | 592.1 | B |

**[Table 19]**

| | | | | | |
|---|---|---|---|---|---|
| I-2-68 | C | 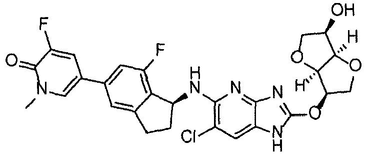 | 1.55 | 572.2 | B |
| I-2-69 | C | 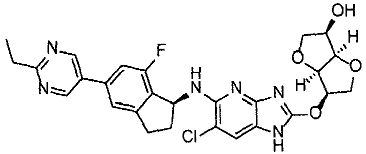 | 1.77 | 553.2 | B |
| I-2-70 | C | 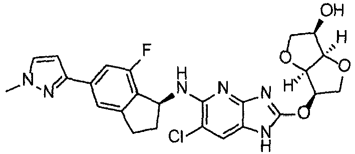 | 1.69 | 527.2 | B |
| I-2-71 | C | 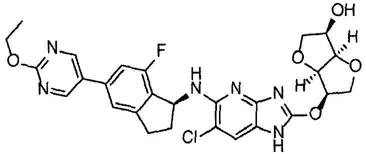 | 1.87 | 569.2 | B |
| I-2-72 | M | 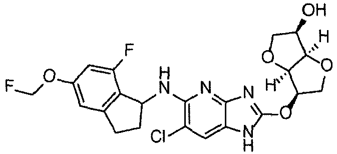 | 1.78 | 495.1 | B |
| I-2-73 | C | 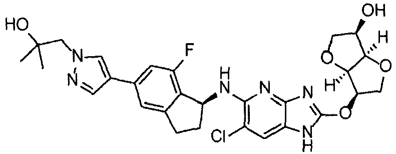 | 1.60 | 585.2 | B |
| I-2-74 | C | 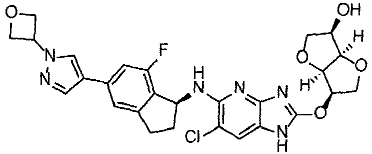 | 1.59 | 569.1 | B |

**[Table 20]**

| | | | | | |
|---|---|---|---|---|---|
| I-2-75 | M | 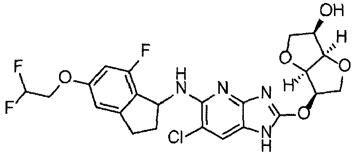 | 1.86 | 527.3 | B |
| I-2-76 | C | 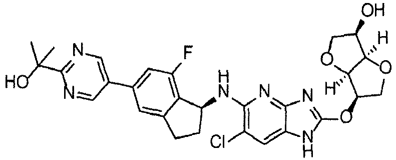 | 1.68 | 583.3 | B |
| I-2-77 | C | 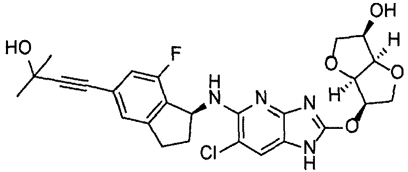 | 1.74 | 529.2 | B |
| I-2-78 | C | 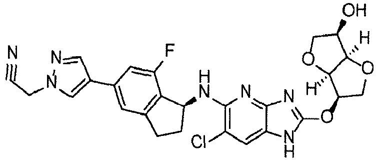 | 1.63 | 552.1 | B |

### Evaluation method of an activator for AMP-activated protein kinase (AMPK) (Test Example 1)

To a buffer solution consisting of a 50 mM HEPES-NaOH buffer solution (pH 7.0), 100 mM NaCl, 10 mM magnesium chloride, 0.1% bovine serum albumin, 0.2 mM sodium orthovanadate(V), 1 mM ethylene glycol-bis(2-aminoethyl ether)-N,N,N',N'-tetraacetic acid (EGTA), 5 mM disodium β-glycerophosphate and 2 mM dithiothreitol, a human AMPK α1β1γ1 enzyme (manufactured by Carna Biosciences, Inc.) was added in an amount to give a conversion rate of approximately 10% by reaction for 2 hours, and a compound dissolved in DMSO was added thereto so as to have a 1% DMSO concentration. The resulting liquid was left to stand for 10 minutes.

To the liquid, a substrate solution consisting of a 50 mM HEPES-NaOH buffer solution (pH 7.0), 100 mM NaCl, 10 mM magnesium chloride, 0.1% bovine serum albumin, 0.2 mM sodium orthovanadate(V), 1 mM ethylene glycol-bis(2-aminoethyl ether)-N,N,N',N'-tetraacetic acid (EGTA), 5 mM disodium β-glycerophosphate, 2 mM dithiothreitol, 0.4 mM ATP and 3 µM FL-Peptide 7 (manufactured by Caliper Life Sciences, Inc.) was added in equal amount (10 µl in total). The resulting liquid was allowed to react at 25°C for 2 hours, then 10 µl of 20 mM EDTA was added thereto to stop the reaction.

To detect phosphorylated fluorescent substrates, the reaction mixture was applied to a measuring device, LabChip EZ Reader II manufactured by Caliper Life Science, Inc., for detecting fluorescence by using differences in mobility due to differences in charge. The setting conditions for the device were pressure, -1.5 PSI; upstream voltage, -2250 V; downstream voltage, -400 V; post sample buffer sip time, 40 seconds; final delay, 120 seconds; and peak order, Product First.

A conversion rate was calculated from the peak heights of the obtained substrate and product. The conversion rate when not containing a compound was used as a control, and a concentration dependent curve was made by plotting the rate of increase in activity to the control at each concentration of a compound. The compound concentration showing 150% relative to the control (100%) was used as the EC 150 value, and the maximum rate of increase in activity within the measurement range was used as Emax.

### Preparation method of human AMPK α2β2γ1

The full length cDNAs of human AMPK β2 (NM_005399.3) and human AMPK α2 (NM_006252.3) were inserted into the MCS1 and MCS2 of the pETDuet-1 vector to prepare a human AMPK β2 and human AMPK α2 (6x His tag at the 5' terminus) expressing plasmid. The plasmid was cotransfected with an expression plasmid, in which the full length cDNA of human AMPK γ1 (NM_002733.3) had been inserted into pET28b(+), into BL21 CodonPlus (DE3)-RIL to obtain an expression strain. The expression strain was cultured in TB medium, followed by induction with 0.5 mM IPTG, and cultured at 25°C for 3 hours and then harvested. After ultrasonication, supernatant was collected and applied to Histrap FF column (GE) and RESOUECE Q column (GE) to prepare 12.5 mg of purified sample containing three types of subunit from 1.8 L of broth.

### Preparation method of human CaMKK2 used to impart activity to AMPK

An expression vector, in which the full length cDNA of human CAMKK β (NM_172226.1) had been inserted into pGEX-6P-3, was transfected into BL21 Star (DE3). The expression strain was cultured in TB medium, followed by induction with 0.5 mM IPTG, and cultured at 25°C for 3 hours and then harvested. After ultrasonication, supernatant was collected and applied to GSTrap FF column (GE) to prepare 14 mg of GST-fused CAMKK β from 720 ml of broth.

### Evaluation method of an activator for AMP-activated protein kinase (AMPK) (Test Example 2)

Human AMPK α2β2γ1 prepared in Escherichia coli was not phosphorylated and did not exhibit activity. Thus, phosphorylation treatment was carried out as pretreatment.

Human AMPK α2β2γ1 in an amount to give a conversion rate of approximately 10% by reaction for 2 hours, and CaMKK2 in an amount capable of sufficiently imparting activity to AMPK for one hour were mixed in a buffer solution consisting of a 50 mM HEPES-NaOH buffer solution (pH 7.0), 100 mM NaCl, 5 mM magnesium chloride, 0.1% bovine serum albumin, 0.2 mM sodium orthovanadate(V), 1 mM ethylene glycol-bis(2-aminoethyl ether)-N,N,N',N'-tetraacetic acid (EGTA), 5 mM disodium β-glycerophosphate, 1 mM dithiothreitol and 0.2 mM ATP, and the resulting liquid was left to stand at 25°C for 1 to 1.5 hours to sufficiently phosphorylate AMPK.

After that, to the enzyme liquid, which had been subjected to phosphorylation treatment, a compound dissolved in DMSO was added so as to have a 1% DMSO concentration. The resulting liquid was left to stand for 10 minutes.

To the liquid, a substrate solution consisting of a 50 mM HEPES-NaOH buffer solution (pH 7.0), 100 mM NaCl, 10 mM magnesium chloride, 0.1% bovine serum albumin, 0.2 mM sodium orthovanadate(V), 1 mM ethylene glycol-bis(2-aminoethyl ether)-N,N,N',N'-tetraacetic acid (EGTA), 5 mM disodium β-glycerophosphate, 2 mM dithiothreitol, 0.4 mM ATP and 3 µM FL-Peptide 7 (manufactured by Caliper Life Sciences, Inc.) was added in equal amount (10 µl in total). The resulting liquid was allowed to react at 25°C for 2 hours, and 10 µl of 20 mM EDTA was added thereto to stop the reaction.

To detect phosphorylated fluorescent substrates, the reaction mixture was applied to a measuring device, LabChip EZ Reader II manufactured by Caliper Life Science, Inc., for detecting fluorescence by using differences in mobility due to differences in charge. The setting conditions for the device were pressure, -1.5 PSI; upstream voltage, -2250 V; downstream voltage, -400 V; post sample buffer sip time, 40 seconds; final delay, 120 seconds; and peak order, Product First.

A conversion rate was calculated from the peak heights of the obtained substrate and product. The conversion rate when not containing a compound was used as a control, and a concentration dependent curve was made by plotting the rate of increase in activity to the control at each concentration of a compound. The compound concentration showing 150% relative to the control (100%) was used as the EC 150 value, and the maximum rate of increase in activity within the measurement range was used as Emax.

The results of Test Example 2 are shown below.
Compound (I-1-4): EC150 = 11 nM, Emax = 547%
Compound (I-1-6): EC150 = 6.8 nM, Emax = 516%
Compound (I-1-7): EC150 = 1.6 nM, Emax = 614%
Compound (I-1-10): EC150 = 8.4 nM, Emax = 608%
Compound (I-1-11): EC150 = 9.8 nM, Emax = 606%
Compound (I-1-13): EC150 = 2 nM, Emax = 604%
Compound (I-1-14): EC150 = 33 nM, Emax = 371%
Compound (I-1-15): EC150 = 10 nM, Emax = 388%
Compound (I-1-19): EC150 = 3.5 nM, Emax = 404%
Compound (I-1-20): EC150 = 4.3 nM, Emax = 403%
Compound (I-1-23): EC150 = 1.3 nM, Emax = 454%
Compound (I-1-24): EC150 = 25 nM, Emax = 423%
Compound (I-1-29): EC150 = 1.9 nM, Emax = 426%
Compound (I-1-32): EC150 = 2.3 nM, Emax = 389%
Compound (I-1-35): EC150 = 4.3 nM, Emax = 402%
Compound (I-1-38): EC150 = 8 nM, Emax = 392%
Compound (I-1-39): EC150 = 2.9 nM, Emax = 384%
Compound (I-1-42): EC150 = 2.1 nM, Emax = 391%
Compound (I-1-43): EC150 = 6.5 nM, Emax = 392%
Compound (I-1-44): EC150 = 5.6 nM, Emax = 422%
Compound (I-1-45): EC150 = 4.7 nM, Emax = 435%
Compound (I-1-46): EC150 = 54 nM, Emax = 381%
Compound (I-1-47): EC150 = 14 nM, Emax = 390%
Compound (I-1-48): EC150 = 15 nM, Emax = 393%
Compound (I-1-70): EC150 = 21 nM, Emax = 384%
Compound (I-1-72): EC150 = 13 nM, Emax = 375%
Compound (I-1-74): EC150 = 9.9 nM, Emax = 373%
Compound (I-1-76): EC150 = 15 nM, Emax = 378%
Compound (I-1-77): EC150 = 3.3 nM, Emax = 386%
Compound (I-1-79): EC150 = 4.2 nM, Emax = 385%
Compound (I-1-81): EC150 = 1.6 nM, Emax = 387%
Compound (I-1-83): EC150 = 2.2 nM, Emax = 414%
Compound (I-1-86): EC150 = 28 nM, Emax = 379%
Compound (I-1-88): EC150 = 5.9 nM, Emax = 376%
Compound (I-1-90): EC150 = 11 nM, Emax = 382%
Compound (I-1-91): EC150 = 1.2 nM, Emax = 466%
Compound (I-1-93): EC150 = 4 nM, Emax = 444%
Compound (I-1-94): EC150 = 5.6 nM, Emax = 416%
Compound (I-1-95): EC150 = 1.3 nM, Emax = 424%
Compound (I-1-96): EC150 = 3.6 nM, Emax = 430%
Compound (I-1-97): EC150 = 2.1 nM, Emax = 433%
Compound (I-1-98): EC150 = 3.6 nM, Emax = 434%
Compound (I-1-99): EC150 = 4.6 nM, Emax = 422%
Compound (I-1-100): EC150 = 3.5 nM, Emax = 441%
Compound (I-1-104): EC150 = 2.3 nM, Emax = 423%
Compound (I-1-105): EC150 = 4.1 nM, Emax = 434%
Compound (I-1-106): EC150 = 1.4 nM, Emax = 462%
Compound (I-1-111): EC150 = 4.5 nM, Emax = 424%
Compound (I-2-01): EC150 = 1.8nM, Emax = 646%
Compound (I-2-02): EC150 = 2.4nM, Emax = 557%
Compound (I-2-03): EC150 = 1.6nM, Emax = 678%
Compound (I-2-04): EC150 = 2.4nM, Emax = 736%
Compound (I-2-05): EC150 = 6nM, Emax = 680%
Compound (I-2-06): EC150 = 3.6nM, Emax = 707%
Compound (I-2-07): EC150 = 2nM, Emax = 727%
Compound (I-2-08): EC150 = 3.5nM, Emax = 688%
Compound (I-2-09): EC150 = 3.9nM, Emax = 633%
Compound (I-2-10): EC150 = 3.4nM, Emax = 642%
Compound (I-2-12): EC150 = 25nM, Emax = 690%
Compound (I-2-13): EC150 = 1.6nM, Emax = 711%
Compound (I-2-14): EC150 = 5.6nM, Emax = 594%
Compound (I-2-17): EC150 = 1.4nM, Emax = 720%
Compound (I-2-18): EC150 = 5nM, Emax = 650%
Compound (I-2-21): EC150 = 4.9nM, Emax = 650%
Compound (1-2-23): EC150 = 12nM, Emax = 709%
Compound (I-2-26): EC150 = 7.1nM, Emax = 669%
Compound (I-2-32): EC150 = 9nM, Emax = 714%
Compound (I-2-33): EC150 = 3nM, Emax = 704%
Compound (I-2-34): EC150 = 6.6nM, Emax = 719%
Compound (I-2-35): EC150 = 10nM, Emax = 679%
Compound (I-2-36): EC150 = 3.9nM, Emax = 698%
Compound (I-2-37): EC150 = 3.2nM, Emax = 645%
Compound (I-2-38): EC150 = 3.1nM, Emax = 725%
Compound (I-2-39): EC150 = 11nM, Emax = 583%
Compound (I-2-40): EC150 = 4.1nM, Emax = 604%
Compound (I-2-41): EC150 = 10nM, Emax = 604%
Compound (I-2-42): EC150 = 9.1nM, Emax = 600%
Compound (I-2-43): EC150 = 31nM, Emax = 612%
Compound (I-2-44): EC150 = 3.8nM, Emax = 595%
Compound (I-2-45): EC150 = 2.1nM, Emax = 711%
Compound (I-2-46): EC150 = 3.3nM, Emax = 708%
Compound (I-2-47): EC150 = 2.3nM, Emax = 724%
Compound (I-2-48): EC150 = 6.2nM, Emax = 737%
Compound (I-2-49): EC150 = 3.4nM, Emax = 667%
Compound (I-2-50): EC150 = 1.9nM, Emax = 678%
Compound (I-2-51): EC150 = 4.7nM, Emax = 688%
Compound (I-2-52): EC150 = 3.7nM, Emax = 629%
Compound (I-2-53): EC150 = 4nM, Emax = 637%
Compound (I-2-54): EC150 = 1.5nM, Emax = 643%
Compound (I-2-55): EC150 = 3.6nM, Emax = 637%
Compound (I-2-56): EC150 = 0.81nM, Emax = 681%
Compound (I-2-57): EC150 = 11nM, Emax = 636%
Compound (I-2-59): EC150 = 4.5nM, Emax = 634%
Compound (I-2-60): EC150 = 6.7nM, Emax = 634%
Compound (I-2-61): EC150 = 3.1nM, Emax = 649%
Compound (I-2-62): EC150 = 9.4nM, Emax = 638%
Compound (I-2-63): EC150 = 3.9nM, Emax = 653%
Compound (I-2-64): EC150 = 9.3nM, Emax = 676%
Compound (I-2-66): EC150 = 8.6nM, Emax = 711%
Compound (I-2-67): EC150 = 29nM, Emax = 683%
Compound (I-2-68): EC150 = 1.8nM, Emax = 600%
Compound (I-2-69): EC150 = 3nM, Emax = 598%
Compound (I-2-70): EC150 = 5.2nM, Emax = 583%
Compound (I-2-71): EC150 = 8.6nM, Emax = 590%
Compound (I-2-72): EC150 = 1.8nM, Emax = 592%
Compound (I-2-73): EC150 = 1.4nM, Emax = 608%
Compound (I-2-74): EC150 = 1.5nM, Emax = 609%
Compound (1-2-75): EC150 = 2.7nM, Emax = 581%
Compound (I-2-77): EC150 = 3.8nM, Emax = 650%
Compound (I-2-78): EC150 = 2.5nM, Emax = 692%

The compounds of the present invention have an excellent activating effect on an AMPK α1 trimer and/or an AMPK α2 trimer.

Usefulness as a medicament can be examined by the following tests, etc.

### CYP3A4 fluorescent MBI test

The CYP3A4 fluorescent MBI test is a test of evaluating Mechanism based inhibition (MBI) ability from enhancement by a metabolism reaction for CYP3A4 inhibition of the compound of the present invention. CYP3A4 inhibition was evaluated as an index 1-hydroxylation reaction of midazolam (MDZ) using pooled human hepatic microsomes.

The reaction conditions were as follows: substrate, 10 µmol/L MDZ; pre-reaction time, 0 or 30 minutes; reaction time, 2 minutes; reaction temperature, 37°C; pooled human hepatic microsomes, at pre-reaction 0.5 mg/mL, at reaction 0.05 mg/mL (at 10-fold dilution); concentration of the compound of the present invention at pre-reaction, 1, 5, 10, 20 µmol/L (four points).

Pooled human hepatic microsomes in a K-Pi buffer (pH 7.4) and a solution of the compound of the present invention as a pre-reaction mixture were added to a 96-well plate at the composition of the pre-reaction, a part of it was transferred to another a 96-well plate at the composition of the pre-reaction, a part of it was transferred to another 96-well plate so that it was 1/10 diluted with a substrate and a K-Pi buffer, NADPH as a coenzyme was added to initiate a reaction as an index (without pre-reaction) and, after a predetermined time of a reaction, a methanol/acetonitrile = 1/1 (V/V) solution was added to stop the reaction. In addition, NADPH was added to a remaining pre-reaction mixture to initiate a pre-reaction (with pre-reaction) and, after a predetermined time of a pre-reaction, a part was transferred to another plate so that it was 1/10 diluted with a substrate and a K-Pi buffer to initiate a reaction as an index. After a predetermined time of a reaction, a methanol/acetonitrile = 1/1 (V/V) solution was added to stop the reaction. The plate on which each index reaction had been performed was centrifuged at 3000 rpm for 15 minutes, and then midazolam 1-hydroxylation in the centrifuge supernatant was quantified by LC/MS/MS.

Addition of only DMSO being a solvent dissolving the compound of the present invention to a reaction system was adopted as a control (100%), remaining activity (%) was calculated at each concentration of the compound of the present invention added, and IC was calculated by reverse presumption by a logistic model using a concentration and an inhibition rate. "IC value at start of pre-reaction /IC value at 30 minutes after start of pre-reaction" was defined as Shifted IC value. When Shifted IC was 1.5 or more, this was defined as (+), and when Shifted IC was 1.0 or less, this was defined as (-).

### CYP inhibition test

Using commercially available pooled human hepatic microsome, and employing, as markers, 7-ethoxyresorufin O-deethylation (CYP1A2), tolbutamide methyl-hydroxylation (CYP2C9), mephenytoin 4'-hydroxylation (CYP2C19), dextromethorphan O-demethylation (CYP2D6), and terfenadine hydroxylation (CYP3A4) as typical substrate metabolism reactions of human main five CYP enzyme forms (CYP1A2, 2C9, 2C19, 2D6, 3A4), an inhibitory degree of each metabolite production amount by a test compound was assessed.

The reaction conditions were as follows: substrate, 0.5 µmol/L ethoxyresorufin (CYP1A2), 100 µmol/L tolbutamide (CYP2C9), 50 µmol/L S-mephenytoin (CYP2C19), 5 µmol/L dextromethorphan (CYP2D6), 1 µmol/L terfenadine (CYP3A4); reaction time, 15 minutes; reaction temperature, 37°C; enzyme, pooled human hepatic microsome 0.2 mg protein/mL; test drug concentration, 1, 5, 10, 20 µmol/L (four points).

Each five kinds of substrates, human hepatic microsome, or a test drug in 50 mM Hepes buffer as a reaction solution was added to a 96-well plate at the composition as described above, NADPH, as a coenzyme was added to initiate metabolism reactions as markers and, after the incubation at 37°C for 15 minutes, a methanol/acetonitrile = 1/1 (v/v) solution was added to stop the reaction. After the centrifugation at 3000 rpm for 15 minutes, resorufin (CYP1A2 metabolite) in the centrifuge supernatant was quantified by a fluorescent multilabel counter, and tolbutamide hydroxide (CYP2C9 metabolite), mephenytoin 4' hydroxide (CYP2C19 metabolite), dextromethorphan (CYP2D6 metabolite), and terfenadine alcohol (CYP3A4 metabolite) were quantified by LC/MS/MS.

Addition of only DMSO being a solvent dissolving a drug to a reaction system was adopted as a control (100%), remaining activity (%) was calculated at each concentration of a test drug added as the solution, and IC₅₀ was calculated by reverse presumption by a logistic model using a concentration and an inhibition rate.

### FAT test

Each 20 µL of freeze-stored Salmonella typhimurium (strains TA98 and TA100) was inoculated in 10 mL of liquid nutrient medium (2.5% Oxoid nutrient broth No.2), and the cultures were preincubated at 37°C under shaking for 10 hours. 9 mL of TA98 culture was centrifuged (2000 × g, 10 minutes) to remove medium, and the bacteria was suspended in 9 mL of Micro F buffer (K₂HPO₄: 3.5 g/L, KH₂PO₄: 1 g/L, (NH₄)₂SO₄: 1 g/L, trisodium citrate dihydrate: 0.25 g/L, MgSO₄• 7H₂O: 0.1 g/L), and the suspension was added to 110 mL of Exposure medium (Micro F buffer containing Biotin: 8 µg/mL, histidine: 0.2 µg/mL, glucose: 8 mg/mL). 3.16 mL of TA100 culture was added to 120 mL of Exposure medium to prepare the test bacterial solution. 588 µL of the test bacterial solution (a mixed solution of 498 µL of the test bacterial solution and 90 µL of the S9 mix in the case with metabolic activation conditions) was mixed with each 12 µL of the following solution: DMSO solution of the test substance (eight dose levels from maximum dose 50 mg/mL at 2-fold ratio); DMSO as negative control; 50 µg/mL of 4-nitroquinoline-1-oxide DMSO solution as positive control for strain TA98 without metabolic activation conditions; 0.25 µg/mL of 2-(2-furyl)-3-(5-nitro-2-furyl)acrylamide DMSO solution as positive control for strain TA100 without metabolic activation conditions; 40 µg/mL of 2-aminoanthracene DMSO solution as positive control for strain TA98 with metabolic activation conditions; or 20 µg/mL of 2-aminoanthracene DMSO solution as positive control for strain TA100 with metabolic activation conditions, and the mixture is incubated at 37°C under shaking for 90 minutes. 460 µL of the culture exposed to the test substance was mixed with 2300 µL of Indicator medium (Micro F buffer containing biotin: 8 µg/mL, histidine: 0.2 µg/mL, glucose: 8 mg/mL, Bromo Cresol Purple: 37.5 µg/mL), each 50 µL is dispensed into 48 wells per dose in the microplates, and is subjected to stationary cultivation at 37°C for 3 days. A well containing the bacteria, which has obtained the ability of proliferation by mutation in the gene coding amino acid (histidine) synthetase, turns the color from purple to yellow due to pH change. The number of the yellow wells among the 48 total wells per dose was counted to evaluate the mutagenicity by comparing with the negative control group.

### Solubility test

The solubility of a compound was determined under a condition in which 1% DMSO was added. A 10 mM compound solution was prepared using DMSO, and then 6 µL of the compound solution was added to 594 µL of artificial intestinal juice in pH 6.8 (to 250 mL of a 0.2 mol/L potassium dihydrogen phosphate reagent solution were added 118 mL of a 0.2 mol/L NaOH reagent solution and water to provide a final volume of 1000 mL). After standing at 25°C for 16 hours, the mixed solution was filtrated with suction. The filtrate was diluted twice with methanol/water (1/1), and then a concentration in the filtration was measured with HPLC or LC/MS/MS by the absolute calibration method.

### Metabolic stability test

Using commercially available pooled human hepatic microsomes, a test compound was reacted for a constant time, a remaining rate was calculated by comparing a reacted sample and an unreacted sample, thereby, a degree of metabolism in hepatic was assessed.

A reaction was performed (oxidative reaction) at 37°C for 0 minute or 30 minutes in the presence of 1 mmol/L NADPH in 0.2 mL of a buffer (50 mmol/L Tris-HCl pH 7.4, 150 mmol/L potassium chloride, 10 mmol/L magnesium chloride) containing 0.5 mg protein/mL of human hepatic microsomes. After the reaction, 50 µL of the reaction mixture was added to 100 µL of a methanol/acetonitrile = 1/1 (v/v) solution, and the mixture was mixed and centrifuged at 3000 rpm for 15 minutes. The test compound in the centrifuge supernatant was quantified by LC/MS/MS, and a remaining amount of the test compound after the reaction was calculated, letting a compound amount at 0 minute reaction time to be 100%. Hydrolysis reaction was performed in the absence of NADPH and glucuronidation reaction was performed in the presence of 5 mM UDP-glucuronic acid in place of NADPH, followed by similar operations.

### hERG test

For the purpose of assessing risk of an electrocardiogram QT interval prolongation, effects on delayed rectifier K⁺current (I_{Kr}), which plays an important role in the ventricular repolarization process, was studied using HEK293 cells expressing human ether-a-go-go related gene (hERG) channel.

After a cell was retained at a membrane potential of -80 mV by whole cell patch clamp method using an automated patch clamp system (PatchXpress 7000A, Axon Instruments Inc.), I_{Kr} induced by depolarization pulse stimulation at +40 mV for 2 seconds and, further, repolarization pulse stimulation at -50 mV for 2 seconds was recorded. After the generated current was stabilized, extracellular solution (NaCl: 135 mmol/L, KCl: 5.4 mmol/L, NaH₂PO₄: 0.3 mmol/L, CaCl₂• 2H₂O: 1.8 mmol/L, MgCl₂• 6H₂O: 1 mmol/L, glucose: 10 mmol/L, HEPES (4-(2-hydroxyethyl)-1-piperazine ethanesulfonic acid): 10 mmol/L, pH=7.4) in which the test compound had been dissolved at an objective concentration was applied to the cell under the room temperature condition for 10 minutes. From the recording I_{Kr}, an absolute value of the tail peak current was measured based on the current value at the resting membrane potential using an analysis software (DataXpress ver. 1, Molecular Devices Corporation). Further, the % inhibition relative to the tail peak current before application of the test substance was calculated, and compared with the vehicle-applied group (0.1% dimethyl sulfoxide solution) to assess influence of the test substance on I_{Kr}.

### Powder solubility test

Appropriate amounts of the test substances were put into appropriate containers. To the respective containers were added 200 µL of JP-1 fluid (sodium chloride 2.0 g, hydrochloric acid 7.0 mL and water to reach 1000 mL), 200 µL of JP-2 fluid (phosphate buffer (pH 6.8) 500 mL and water 500 mL), and 200 µL of 20 mmol/L TCA (sodium taurocholate)/JP-2 fluid (TCA 1.08 g and water to reach 100 mL). In the case that the test compound was dissolved after the addition of the test fluid, the bulk powder was added as appropriate. The containers were sealed, and shaken for 1 hour at 37°C. The mixtures were filtered, and 100 µL of methanol was added to each of the filtrate (100 µL) so that the filtrates were twofold diluted. The dilution ratio was changed if necessary. After confirmation of no bubbles and precipitates, the containers were sealed and shaken. Quantification was performed by HPLC with an absolute calibration method.

### BA test

### Materials and methods for studies on oral absorption

(1) Animals: mice or rats
(2) Animal husbandry: Mice and rats had free access to solid food and sterilized bottled tap water.
(3) Setting of dose and group compositions: orally or intravenously administered at a predetermined dose; Group compositions were as shown below. (Dose depends on the compound)
   Oral: 1 to 30 mg/kg (n=2 to 3)
   Intravenous: 0.5 to 10 mg/kg (n=2 to 3)
(4) Preparation of administration formulation: for oral administration, in a solution or a suspension state; for intravenous administration, in a solubilized state.
(5) Dosing procedure: In oral administration study, the test substance was forcibly administered to the stomach by using a gavage tube. In intravenous administration study, the test substance was administered via tail vein using a syringe with a needle.
(6) Evaluation items: Blood was collected at each time point, and plasma concentration of the test substance was determined by LC/MS/MS.
(7) Data analysis: Regarding the transition of the plasma concentration, area under the plasma concentration-time curve (AUC) was calculated by means of WinNonlin® program, respectively. Bioavailability (BA) was calculated from AUCs of the oral administration group and intravenous administration group.

Formulation Examples are shown below.

### Formulation Example 1: Tablets

The compound of the present invention, lactose and calcium stearate are mixed. The mixture is crushed, granulated and dried to give a suitable size of granules. Next, calcium stearate is added to the granules, and the mixture is compressed and molded to give tablets.

### Formulation Example 2: Capsules

The compound of the present invention, lactose and calcium stearate are mixed uniformly to obtain powder medicines in the form of powder or fine granules. The powder medicines are filled into capsule containers to give capsules.

### Formulation Example 3: Granules

The compound of the present invention, lactose and calcium stearate are mixed uniformly, and the mixture is compressed and molded. Then, it is crushed, granulated and sieved to give a suitable size of granules.

### Formulation Example 4: Orally disintegrating tablets

The compound of the present invention and crystalline cellulose are mixed and granulated, then tableted to give orally disintegrating tablets.

### Formulation Example 5: Dry syrups

The compound of the present invention and lactose are mixed, crushed, granulated and sieved to give a suitable size of dry syrups.

### Formulation Example 6: Injections

The compound of the present invention and phosphate buffer are mixed to give injections.

### Formulation Example 7: Infusions

The compound of the present invention and phosphate buffer are mixed to give injections.

### Formulation Example 8: Inhalations

The compound of the present invention and lactose are mixed and crushed finely to give inhalations.

### Formulation Example 9: Ointments

The compound of the present invention and petrolatum are mixed to give ointments.

### Formulation Example 10: Patches

The compound of the present invention and base such as adhesive plaster or the like are mixed to give patches.

### [Industrial Applicability]

As is apparent from the above test examples, the compounds of the present invention show an AMPK activating effect. Therefore, the compounds of the present invention are very useful as a therapeutic agent for type I diabetes, type II diabetes, hyperglycemia, metabolic syndrome, obesity, hypercholesterolemia and hypertension.

## Claims

1. A compound represented by the formula (I): 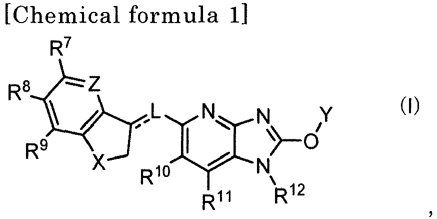 or its pharmaceutically acceptable salt,
wherein
L is -NR¹-, =N-, -O-, -S-, -SO₂-, -CR²R³-, or =CR²-;
dashed line indicates the presence or absence of a bond;
X is -CR⁴R⁵-, or -O-;
Y is substituted or unsubstituted heteroaryl, or substituted or unsubstituted heterocyclyl;
Z is =CR⁶-, or =N-;
R¹ is hydrogen, halogen, hydroxy, cyano, nitro, carboxy, substituted or unsubstituted alkyl, substituted or unsubstituted alkenyl, substituted or unsubstituted alkynyl, substituted or unsubstituted aryl, substituted or unsubstituted heteroaryl, substituted or unsubstituted cycloalkyl, substituted or unsubstituted cycloalkenyl, substituted or unsubstituted heterocyclyl, substituted or unsubstituted acyl, substituted or unsubstituted carbamoyl, substituted or unsubstituted alkylsulfonyl, substituted or unsubstituted alkenylsulfonyl, substituted or unsubstituted alkynylsulfonyl, substituted or unsubstituted arylsulfonyl, substituted or unsubstituted heteroarylsulfonyl, substituted or unsubstituted cycloalkylsulfonyl, substituted or unsubstituted cycloalkenylsulfonyl, substituted or unsubstituted heterocyclylsulfonyl, or substituted or unsubstituted sulfamoyl;
R² and R³ are each independently hydrogen, halogen, hydroxy, cyano, nitro, carboxy, substituted or unsubstituted alkyl, substituted or unsubstituted alkenyl, substituted or unsubstituted alkynyl, substituted or unsubstituted aryl, substituted or unsubstituted heteroaryl, substituted or unsubstituted cycloalkyl, substituted or unsubstituted cycloalkenyl, substituted or unsubstituted heterocyclyl, substituted or unsubstituted alkyloxy, substituted or unsubstituted aryloxy, substituted or unsubstituted heteroaryloxy, substituted or unsubstituted cycloalkyloxy, substituted or unsubstituted cycloalkenyloxy, substituted or unsubstituted heterocyclyloxy, substituted or unsubstituted alkylthio, substituted or unsubstituted arylthio, substituted or unsubstituted heteroarylthio, substituted or unsubstituted cycloalkylthio, substituted or unsubstituted cycloalkenylthio, substituted or unsubstituted heterocyclylthio, substituted or unsubstituted alkylsulfonyl, substituted or unsubstituted arylsulfonyl, substituted or unsubstituted heteroarylsulfonyl, substituted or unsubstituted cycloalkylsulfonyl, substituted or unsubstituted cycloalkenylsulfonyl, substituted or unsubstituted heterocyclylsulfonyl, substituted or unsubstituted acyl, substituted or unsubstituted carbamoyl, substituted or unsubstituted sulfamoyl, or substituted or unsubstituted amino;
R⁴ and R⁵ are each independently hydrogen, halogen, hydroxy, cyano, nitro, carboxy, substituted or unsubstituted alkyl, substituted or unsubstituted alkenyl, substituted or unsubstituted alkynyl, substituted or unsubstituted aryl, substituted or unsubstituted heteroaryl, substituted or unsubstituted cycloalkyl, substituted or unsubstituted cycloalkenyl, substituted or unsubstituted heterocyclyl, substituted or unsubstituted alkyloxy, substituted or unsubstituted aryloxy, substituted or unsubstituted heteroaryloxy, substituted or unsubstituted cycloalkyloxy, substituted or unsubstituted cycloalkenyloxy, substituted or unsubstituted heterocyclyloxy, substituted or unsubstituted alkylthio, substituted or unsubstituted arylthio, substituted or unsubstituted heteroarylthio, substituted or unsubstituted cycloalkylthio, substituted or unsubstituted cycloalkenylthio, substituted or unsubstituted heterocyclylthio, substituted or unsubstituted alkylsulfonyl, substituted or unsubstituted arylsulfonyl, substituted or unsubstituted heteroarylsulfonyl, substituted or unsubstituted cycloalkylsulfonyl, substituted or unsubstituted cycloalkenylsulfonyl, substituted or unsubstituted heterocyclylsulfonyl, substituted or unsubstituted acyl, substituted or unsubstituted carbamoyl, substituted or unsubstituted sulfamoyl, or substituted or unsubstituted amino;
R⁶ is hydrogen, halogen, hydroxy, cyano, nitro, carboxy, substituted or unsubstituted alkyl, substituted or unsubstituted alkenyl, substituted or unsubstituted alkynyl, substituted or unsubstituted aryl, substituted or unsubstituted heteroaryl, substituted or unsubstituted cycloalkyl, substituted or unsubstituted cycloalkenyl, substituted or unsubstituted heterocyclyl, substituted or unsubstituted alkyloxy, substituted or unsubstituted aryloxy, substituted or unsubstituted heteroaryloxy, substituted or unsubstituted cycloalkyloxy, substituted or unsubstituted cycloalkenyloxy, substituted or unsubstituted heterocyclyloxy, substituted or unsubstituted alkylthio, substituted or unsubstituted arylthio, substituted or unsubstituted heteroarylthio, substituted or unsubstituted cycloalkylthio, substituted or unsubstituted cycloalkenylthio, substituted or unsubstituted heterocyclylthio, substituted or unsubstituted alkylsulfonyl, substituted or unsubstituted arylsulfonyl, substituted or unsubstituted heteroarylsulfonyl, substituted or unsubstituted cycloalkylsulfonyl, substituted or unsubstituted cycloalkenylsulfonyl, substituted or unsubstituted heterocyclylsulfonyl, substituted or unsubstituted acyl, substituted or unsubstituted carbamoyl, substituted or unsubstituted sulfamoyl, or substituted or unsubstituted amino;
R⁷ , R⁸ and R⁹ are each independently hydrogen, halogen, hydroxy, cyano, nitro, carboxy, substituted or unsubstituted alkyl, substituted or unsubstituted alkenyl, substituted or unsubstituted alkynyl, substituted or unsubstituted aryl, substituted or unsubstituted heteroaryl, substituted or unsubstituted cycloalkyl, substituted or unsubstituted cycloalkenyl, substituted or unsubstituted heterocyclyl, substituted or unsubstituted alkyloxy, substituted or unsubstituted aryloxy, substituted or unsubstituted heteroaryloxy, substituted or unsubstituted cycloalkyloxy, substituted or unsubstituted cycloalkenyloxy, substituted or unsubstituted heterocyclyloxy, substituted or unsubstituted alkylthio, substituted or unsubstituted arylthio, substituted or unsubstituted heteroarylthio, substituted or unsubstituted cycloalkylthio, substituted or unsubstituted cycloalkenylthio, substituted or unsubstituted heterocyclylthio, substituted or unsubstituted alkylsulfonyl, substituted or unsubstituted arylsulfonyl , substituted or unsubstituted heteroarylsulfonyl, substituted or unsubstituted cycloalkylsulfonyl, substituted or unsubstituted cycloalkenylsulfonyl, substituted or unsubstituted heterocyclylsulfonyl, substituted or unsubstituted carbamoyl, substituted or unsubstituted sulfamoyl, substituted or unsubstituted amino,
R^{S}R^{S'}(O=)S=N-, R^{S}R^{S'}(O=)S=N-R^{2f}-, R^{S}R^{S'}(O=)S=N-C(=O)-, (R^{N})N=S(=O)(R^{S})-, (R^{N})N=S(=O)(R^{S})-R^{2f}-, R^{S}R^{S'}(R^{N'}-N=)S=N-, ((R^{N})N=)₂S(R^{S})-, (R^{N}R^{N'})N-C(=O)-O-, R^{O}O-C(=O)-N(R^{N})-, R^{O}O-C(=O)-O-, R^{S}(R^{N}R^{N'}N)(O=)S=N-, R^{S}(R^{N}R^{N'}N)(O=)S=N-R^{2f}-, (R^{N"})N=S(=O)(NR^{N}R^{N'})-, (R^{N"})N=S(=O)(NR^{N}R^{N'})-R^{2f}-, 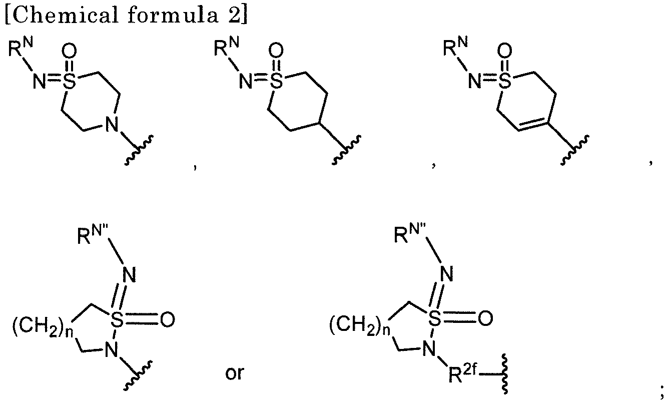
wherein n is an integer 1 or 2;
R^{S} and R^{S} are each independently substituted or unsubstituted alkyl, substituted or unsubstituted cycloalkyl, substituted or unsubstituted heterocyclyl, substituted or unsubstituted aryl, or substituted or unsubstituted heteroaryl;
R^{S} and R^{S'} bound to the same sulfur atom may form a substituted or unsubstituted ring together with the sulfur atom;
R^{2f} is substituted or unsubstituted alkylene;
R^{N} is each independently hydrogen, cyano, substituted or unsubstituted alkyl, substituted or unsubstituted alkylcarbonyl, substituted or unsubstituted alkyloxycarbonyl, substituted or unsubstituted cycloalkyl, substituted or unsubstituted cycloalkylcarbonyl, substituted or unsubstituted heterocyclyl, substituted or unsubstituted heterocyclylcarbonyl, substituted or unsubstituted aryl, substituted or unsubstituted arylcarbonyl, substituted or unsubstituted heteroaryl, substituted or unsubstituted heteroarylcarbonyl, or substituted or unsubstituted carbamoyl;
when R⁷, R⁸ or R⁹ is ((R^{N})N=)₂S(R^{S})-, two R^{N} together with the adjacent nitrogen atom may form a substituted or unsubstituted ring;
R^{N'} is hydrogen, cyano, substituted or unsubstituted alkyl, substituted or unsubstituted alkylcarbonyl, substituted or unsubstituted alkyloxycarbonyl, substituted or unsubstituted cycloalkyl, substituted or unsubstituted cycloalkylcarbonyl, substituted or unsubstituted heterocyclyl, substituted or unsubstituted heterocyclylcarbonyl, substituted or unsubstituted aryl, substituted or unsubstituted arylcarbonyl, substituted or unsubstituted heteroaryl, substituted or unsubstituted heteroarylcarbonyl, or substituted or unsubstituted carbamoyl;
R^{N} and R^{N'} bound to the same nitrogen atom may form a substituted or unsubstituted ring together with the nitrogen atom;
R^{N}" is hydrogen, cyano, substituted or unsubstituted alkyl, substituted or unsubstituted alkenyl, substituted or unsubstituted alkynyl, substituted or unsubstituted alkylcarbonyl, substituted or unsubstituted alkyloxycarbonyl, substituted or unsubstituted alkylcarbamoyl, substituted or unsubstituted cycloalkyl, substituted or unsubstituted cycloalkenyl, substituted or unsubstituted cycloalkynyl, substituted or unsubstituted aryl, substituted or unsubstituted heteroaryl, substituted or unsubstituted heterocyclyl, substituted or unsubstituted acyl, substituted or unsubstituted aryloxycarbonyl, substituted or unsubstituted arylsulfonyl, substituted or unsubstituted alkylsulfonyl, or substituted or unsubstituted carbamoyl;
R^{O} is hydrogen, substituted or unsubstituted alkyl, substituted or unsubstituted alkenyl, substituted or unsubstituted alkynyl, substituted or unsubstituted aryl, substituted or unsubstituted heteroaryl, substituted or unsubstituted cycloalkyl, substituted or unsubstituted cycloalkenyl, or substituted or unsubstituted heterocyclyl;
provided that R⁷, R⁸ and R⁹ are not hydrogen at the same time;
R¹⁰ and R¹¹ are each independently hydrogen, halogen, hydroxy, cyano, nitro, carboxy, substituted or unsubstituted alkyl, substituted or unsubstituted alkenyl, substituted or unsubstituted alkynyl, substituted or unsubstituted aryl, substituted or unsubstituted heteroaryl, substituted or unsubstituted cycloalkyl, substituted or unsubstituted cycloalkenyl, substituted or unsubstituted heterocyclyl, substituted or unsubstituted alkyloxy, substituted or unsubstituted aryloxy, substituted or unsubstituted heteroaryloxy, substituted or unsubstituted cycloalkyloxy, substituted or unsubstituted cycloalkenyloxy, substituted or unsubstituted heterocyclyloxy, substituted or unsubstituted alkylthio, substituted or unsubstituted arylthio, substituted or unsubstituted heteroarylthio, substituted or unsubstituted cycloalkylthio, substituted or unsubstituted cycloalkenylthio, substituted or unsubstituted heterocyclylthio, substituted or unsubstituted alkylsulfonyl, substituted or unsubstituted arylsulfonyl, substituted or unsubstituted heteroarylsulfonyl, substituted or unsubstituted cycloalkylsulfonyl, substituted or unsubstituted cycloalkenylsulfonyl, substituted or unsubstituted heterocyclylsulfonyl, substituted or unsubstituted acyl, substituted or unsubstituted carbamoyl, substituted or unsubstituted sulfamoyl, or substituted or unsubstituted amino;
R¹² is hydrogen, or substituted or unsubstituted alkyl;
with the proviso that
compounds wherein Z is =CR⁶-, and R⁶ and R⁸ are fuluoro at the same time; and compounds shown below; 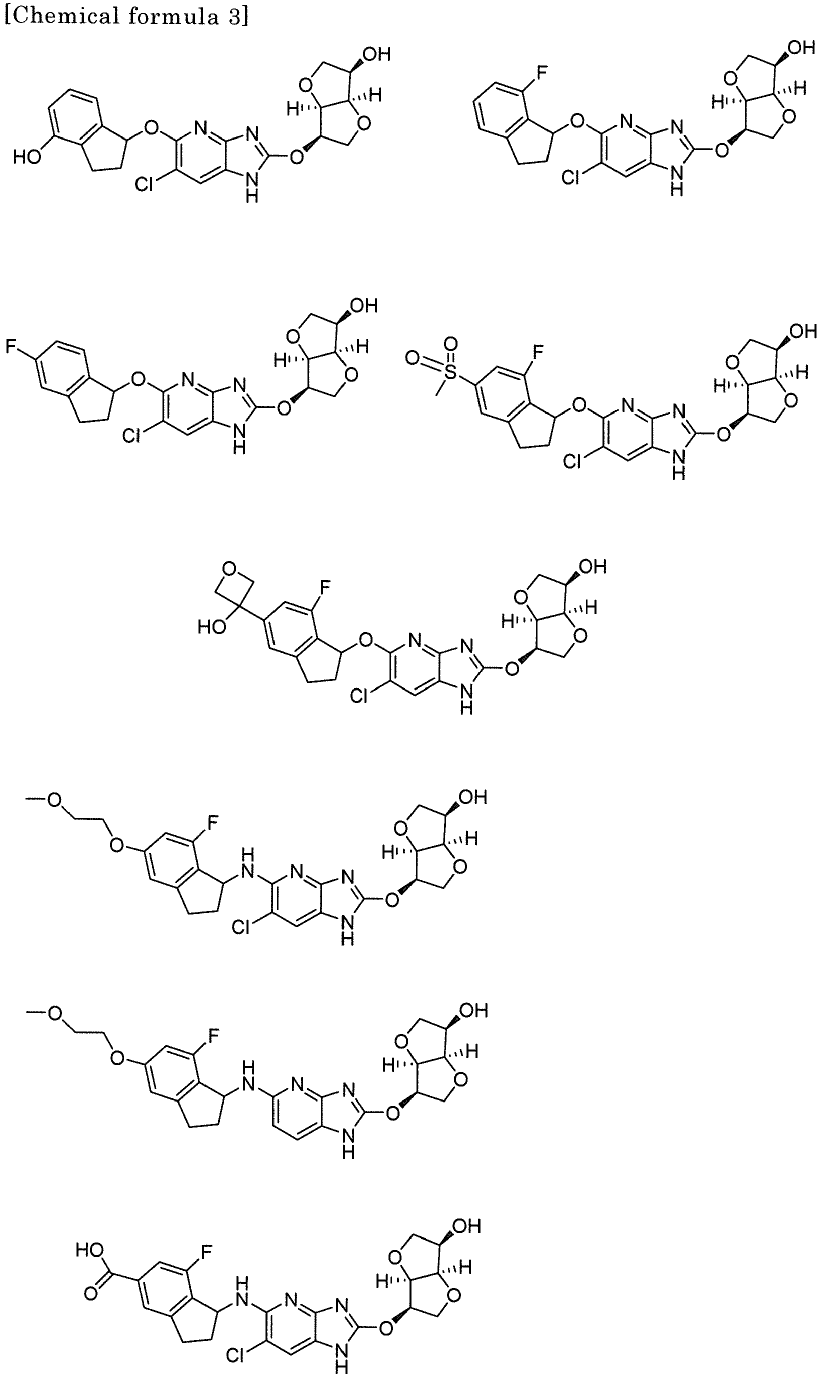 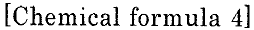 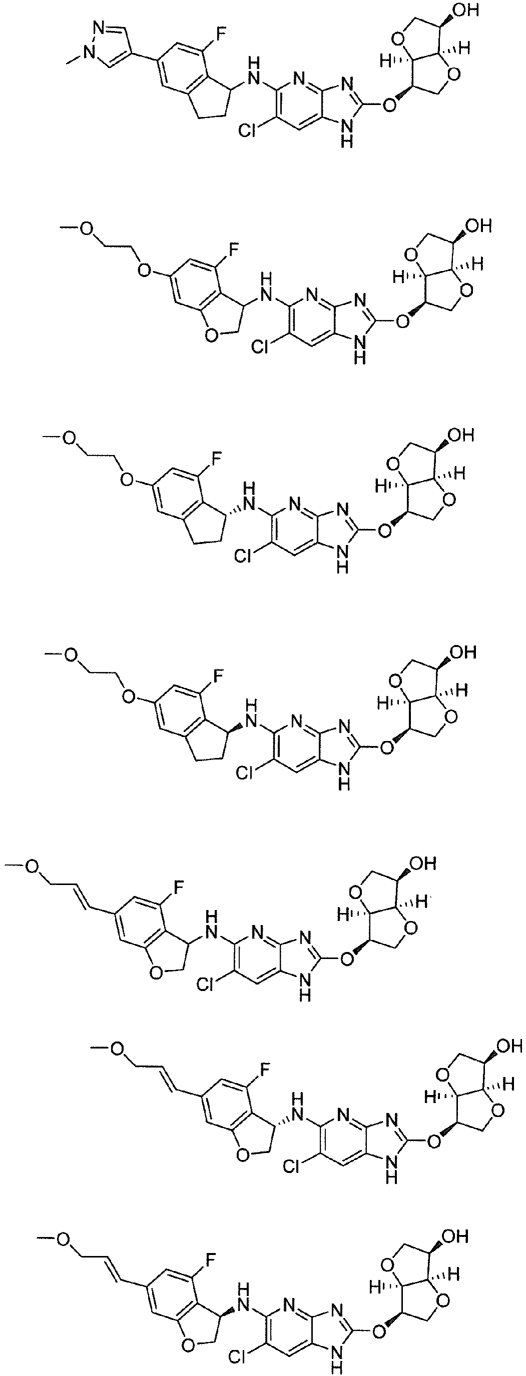 and mixture of diastereomers of compounds shown below are excluded

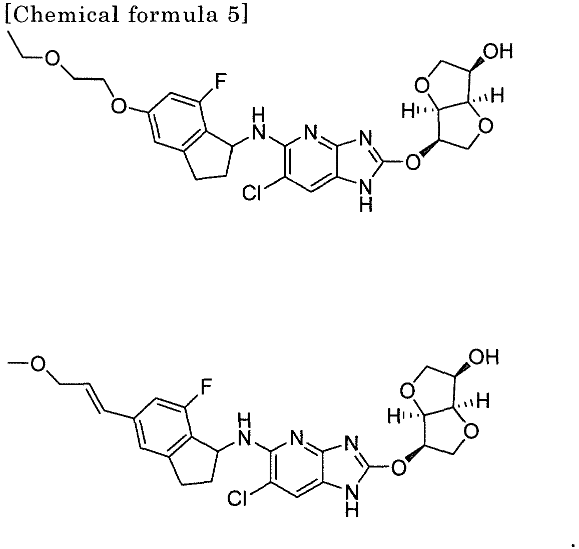

2. The compound according to claim 1 or its pharmaceutically acceptable salt, wherein L is -NR¹-, or -O-.

3. The compound according to claim 1 or its pharmaceutically acceptable salt, wherein L is -NR¹-.

4. The compound according to claim 1 or its pharmaceutically acceptable salt, wherein the compound represented by the formula (I) is a compound represented by the formula (II): 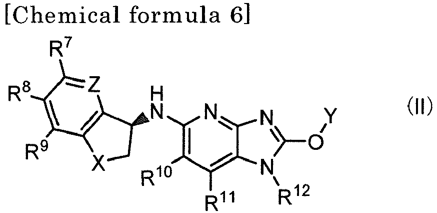 wherein each substituent in the formula (II) is defined in claim 1.

5. The compound according to any one of claims 1 to 3, or its pharmaceutically acceptable salt, wherein R¹ is hydrogen.

6. The compound according to any one of claims 1 to 5 or its pharmaceutically acceptable salt, wherein Y is substituted or unsubstituted heterocyclyl.

7. The compound according to claim 6 or its pharmaceutically acceptable salt, wherein Y is 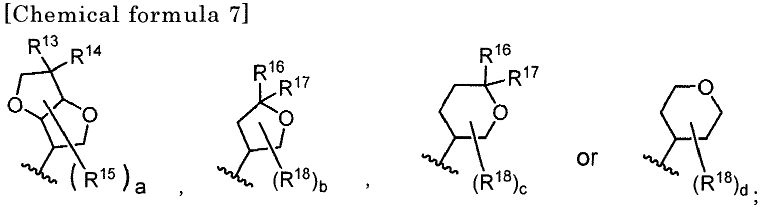
wherein R¹³ and R¹⁴ are each independently hydrogen, halogen, hydroxy, cyano, nitro, carboxy, substituted or unsubstituted alkyl, substituted or unsubstituted alkenyl, substituted or unsubstituted alkynyl, substituted or unsubstituted alkyloxy, substituted or unsubstituted alkylthio, substituted or unsubstituted alkylsulfonyl, substituted or unsubstituted acyl, substituted or unsubstituted carbamoyl, substituted or unsubstituted sulfamoyl, or substituted or unsubstituted amino;
R¹⁵ is each independently halogen, hydroxy, cyano, nitro, carboxy, substituted or unsubstituted alkyl, substituted or unsubstituted alkenyl, substituted or unsubstituted alkynyl, substituted or unsubstituted alkyloxy, substituted or unsubstituted alkylthio, substituted or unsubstituted alkylsulfonyl, substituted or unsubstituted acyl, substituted or unsubstituted carbamoyl, substituted or unsubstituted sulfamoyl, or substituted or unsubstituted amino;
a is an integer from 0 to 6;
R¹⁶ and R¹⁷ are each independently hydrogen, halogen, hydroxy, cyano, nitro, carboxy, substituted or unsubstituted alkyl, substituted or unsubstituted alkenyl, substituted or unsubstituted alkynyl, substituted or unsubstituted alkyloxy, substituted or unsubstituted alkylthio, substituted or unsubstituted alkylsulfonyl, substituted or unsubstituted acyl, substituted or unsubstituted carbamoyl, substituted or unsubstituted sulfamoyl, or substituted or unsubstituted amino; R¹⁸ is each independently halogen, hydroxy, cyano, nitro, carboxy, substituted or unsubstituted alkyl, substituted or unsubstituted alkenyl, substituted or unsubstituted alkynyl, substituted or unsubstituted alkyloxy, substituted or unsubstituted alkylthio, substituted or unsubstituted alkylsulfonyl, substituted or unsubstituted acyl, substituted or unsubstituted carbamoyl, substituted or unsubstituted sulfamoyl, or substituted or unsubstituted amino;
b is an integer from 0 to 5;
c is an integer from 0 to 7;
d is an integer from 0 to 9.

8. The compound according to any one of claims 1 to 7 or its pharmaceutically acceptable salt, wherein X is -CR⁴R⁵-.

9. The compound according to any one of claims 1 to 8 or its pharmaceutically acceptable salt, wherein R⁴ and R⁵ are hydrogen.

10. The compound according to any one of claims 1 to 9 or its pharmaceutically acceptable salt, wherein Z is =CR⁶-, and R⁶ is halogen.

11. The compound according to any one of claims 1 to 9 or its pharmaceutically acceptable salt, wherein Z is =N-.

12. The compound according to any one of claims 1 to 11 or its pharmaceutically acceptable salt, wherein R⁷ , R⁸ and R⁹ are each independently hydrogen, halogen, hydroxy, cyano, nitro, carboxy, substituted or unsubstituted alkyl, substituted or unsubstituted alkenyl, substituted or unsubstituted alkynyl, substituted or unsubstituted aryl, substituted or unsubstituted heteroaryl, substituted or unsubstituted cycloalkyl, substituted or unsubstituted cycloalkenyl, substituted or unsubstituted heterocyclyl, substituted or unsubstituted alkyloxy, substituted or unsubstituted aryloxy, substituted or unsubstituted heteroaryloxy, substituted or unsubstituted cycloalkyloxy, substituted or unsubstituted cycloalkenyloxy, substituted or unsubstituted heterocyclyloxy, substituted or unsubstituted alkylsulfonyl, or substituted or unsubstituted amino.

13. The compound according to any one of claims 1 to 12 or its pharmaceutically acceptable salt, wherein R¹⁰ is hydrogen, halogen, cyano, carboxy, or substituted or unsubstituted alkyl.

14. The compound according to claim 13 or its pharmaceutically acceptable salt, wherein R¹⁰ is hydrogen, fluoro, chloro, cyano, or substituted or unsubstituted alkyl, wherein the substituent of the substituted alkyl is halogen.

15. The compound according to claim 13 or its pharmaceutically acceptable salt, wherein R¹⁰ is fluoro, or chloro.

16. The compound according to any one of claims 1 to 15 or its pharmaceutically acceptable salt, wherein R¹¹ is hydrogen.

17. The compound according to any one of claims 1 to 16 or its pharmaceutically acceptable salt, wherein R¹² is hydrogen.

18. The compound according to claim 1 or its pharmaceutically acceptable salt, wherein the compound is selected from compound (I-1-4), (I-1-6), (I-1-7), (I-1-10), (I-1-11), (I-1-13), (I-1-14), (I-1-15), (I-1-19), (I-1-20), (I-1-23), (I-1-24), (I-1-29), (I-1-32), (I-1-35), (I-1-38), (I-1-39), (I-1-42), (I-1-43), (I-1-44), (I-1-45), (I-1-46), (I-1-47), (I-1-48), (I-1-70), (I-1-72), (I-1-74), (I-1-76), (I-1-77), (I-1-79), (I-1-81), (I-1-83), (I-1-86), (I-1-88), (I-1-90), (I-1-91), (I-1-93), (I-1-94), (I-1-95), (I-1-96), (I-1-97), (I-1-98), (I-1-99), (I-1-100), (I-1-104), (I-1-105), (I-1-106), or (I-1-111).

19. A compound represented by the formula (III) or (IV) or its pharmaceutically acceptable salt, wherein the compound represented by the formula (III) is 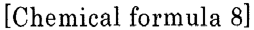 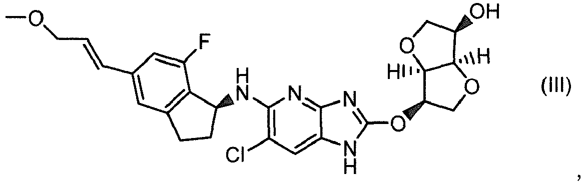 and the compound represented by the formula (IV) is

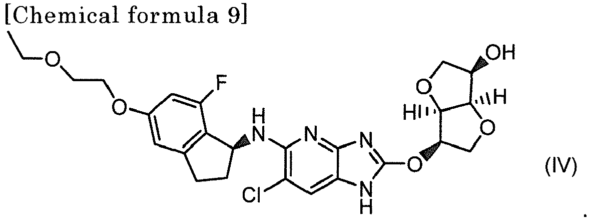

20. The compound according to claim 1 or its pharmaceutically acceptable salt, wherein the compound is selected from compound (I-2-01), (I-2-02), (I-2-03), (I-2-04), (I-2-05), (I-2-06), (I-2-07), (I-2-08), (I-2-09), (I-2-10), (I-2-12), (I-2-13), (I-2-14), (I-2-17), (I-2-18), (I-2-21), (I-2-23), (I-2-26), (I-2-32), (I-2-33), (I-2-34), (I-2-35), (I-2-36), (I-2-37), (I-2-38), (I-2-39), (I-2-40), (I-2-41), (I-2-42), (I-2-43), (I-2-44), (I-2-45), (I-2-46), (I-2-47), (I-2-48), (I-2-49), (I-2-50), (I-2-51), (I-2-52), (I-2-53), (I-2-54), (I-2-55), (I-2-56), (I-2-57), (I-2-59), (I-2-60), (I-2-61), (I-2-62), (I-2-63), (I-2-64), (I-2-66), (I-2-67), (I-2-68), (I-2-69), (I-2-70), (I-2-71), (I-2-72), (I-2-73), (I-2-74), (I-2-75), (I-2-77) or (I-2-78).

21. A pharmaceutical composition comprising the compound according to any one of claims 1 to 20 or its pharmaceutically acceptable salt.

22. The pharmaceutical composition according to claim 21, which has an activating effect on adenosine monophosphate-activated protein kinase.

23. The pharmaceutical composition according to claim 21 or 22, for the treatment and/or prevention of diabetes.

24. A method for preventing or treating diabetes, comprising administering the compound according to any one of claims 1 to 20, or its pharmaceutically acceptable salt.

25. The compound according to any one of claims 1 to 20, or its pharmaceutically acceptable salt, for the treatment and/or prevention of diabetes.
